# EUROPEAN PATENT SPECIFICATION

(11) **EP 3 976 587 B1**
(45) Date of publication and mention of the grant of the patent: **04.12.2024**
(21) Application number: 19836340.0
(22) Date of filing: 12.12.2019
(51) Int. Cl.: C07D 215/233, A61K 31/47, A61P 35/00

(54) **CRYSTALLINE SALT FORMS OF A KINASE INHIBITOR**
KRISTALLINE SALZFORMEN EINES KINASE-INHIBITORS
FORMES DE SEL CRISTALLIN D'UN INHIBITEUR DE KINASE

(30) Priority: 03.06.2019 US 201962856404 P
(43) Date of publication of application: 06.04.2022
(73) Proprietor: Exelixis, Inc., Alameda, CA 94502 (US)
(72) Inventor: DEMORIN, Frenel, Long Beach, CA 90815 (US); SHAH, Khalid, Half Moon Bay, CA 94019 (US); SHAKYA, Sagar, San Diego, CA 92129 (US); WONG, Peter, Brisbane, CA 94005 (US); JOHNSON, Courtney S., West Lafayette, IN 47906 (US); BEVILL, Melanie Janelle, West Lafayette, IN 47906 (US); PARENT, Stephan D., West Lafayette, IN 47906 (US)
(74) Representative: Arch, Peter Jonathan Sanders
(86) International application number: PCT/US2019/065980
(87) International publication number: WO 2020/247019

(56) References cited:
- WO-A1-2018/218233
- WO-A1-2019/148044
- WO-A1-2020/123800
- WO-A2-2005/030140
- CN-A- 109 761 899
- US-A1- 2018 009 758
- DATABASE WPI Week 201955, Derwent World Patents Index; AN 2019-46438P

## Description

### TECHNICAL FIELD OF THE INVENTION

The present invention relates to crystalline forms of salts of Compound 1. The invention also relates to pharmaceutical compositions comprising the solid crystalline salts of Compound 1. The invention further relates to the crystalline salt forms for use in a method of treating a disease, disorder, or syndrome mediated at least in part by modulating *in vivo* activity of a protein kinase, wherein the disease, disorder or syndrome is cancer.

### BACKGROUND OF THE INVENTION

Human Axl belongs to the Tyro3, Axl, and Mer (TAM) subfamily of receptor tyrosine kinases that includes Mer. TAM kinases are characterized by an extracellular ligand binding domain consisting of two immunoglobulin -like domains and two fibronectin type III domains. Axl is overexpressed in a number of tumor cell types and was initially cloned from patients with chronic myelogenous leukemia. When overexpressed, Axl exhibits transforming potential. Axl signaling is believed to cause tumor growth through activation of proliferative and anti-apoptotic signaling pathways. Axl has been associated with cancers such as lung cancer, myeloid leukemia, uterine cancer, ovarian cancer, gliomas, melanoma, thyroid cancer, renal cell carcinoma, osteosarcoma, gastric cancer, prostate cancer, and breast cancer. The over-expression of Axl results in a poor prognosis for patients with the indicated cancers.

Activation of Mer, like Axl, conveys downstream signaling pathways that cause tumor growth and activation. Mer binds ligands such as the soluble protein Gas-6. Gas-6 binding to Mer induces autophosphorylation of Mer on its intracellular domain, resulting in downstream signal activation. Over-expression of Mer in cancer cells leads to increased metastasis, most likely by generation of soluble Mer extracellular domain protein as a decoy receptor. Tumor cells secrete a soluble form of the extracellular Mer receptor which reduces the ability of soluble Gas-6 ligand to activate Mer on endothelial cells, leading to cancer progression.

A need therefore exists for compounds that inhibit TAM receptor tyrosine kinases such as Axl and Mer for the treatment of selected cancers.

CN109761899A discloses quinoline derivatives, and pharmaceutically acceptable salts or solvates, applications and medicines and pharmaceutical compositions thereof. The compounds are described as having inhibitory activity towards several protein tyrosine kinases (e.g. MET, VEGFR, BRAF, PDGFR and RET). It is stated that the compounds can be used in the treatment of cancers.

WO 2018/218233 discloses crystalline solid forms of N-(4-((6,7-dimethoxyquinolin-4-yl)oxy)phenyl)-N'-(4-fluorophenyl) cyclopropane-1,1-dicarboxamide salts, which are stated as being useful for the treatment of cancer. Also disclosed are pharmaceutical compositions and methods of using such compositions for the treatment of cancer.

WO 2005/030140 discloses quinazoline and quinoline compounds suitable for modulating the activity of protein kinases, in particular c-Met. Also disclosed are compositions containing these compounds, and methods of using such compositions for the treatment of kinase-dependent diseases which may include cancer.

US 2018/009758 discloses multi-tyrosine kinase inhibitor compounds, compositions comprising these compounds, and methods of treating eye conditions using the disclosed compounds and compositions.

### SUMMARY OF THE INVENTION

The present disclosure provides crystalline forms of salts of Compound 1, N-(4-fluorophenyl)-N-(4-((7-methoxy-6-(methylcarbamoyl)quinolin-4-yl)oxy)phenyl)cyclopropane-1,1-dicarboxamide, which has the structure:

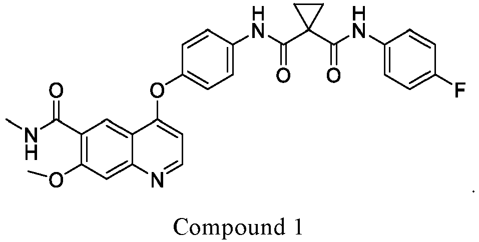

Compound 1 is disclosed in WO 2019/148044.

Specific crystalline salt forms of an active pharmaceutical ingredient (API), such as Compound 1, can have several advantages over other polymorphic or amorphous forms, such as increased stability during storage or processing, more favorable solubility, and increased bioavailability. Reported herein are several stable crystalline salt forms of Compound 1.

In one aspect, the invention includes a crystalline salt form of Compound 1 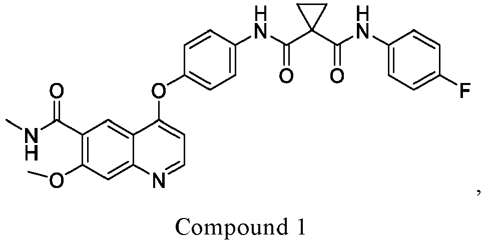 wherein the crystalline salt form is selected from Compound 1 Besylate Form A, Compound 1 Besylate Form B, Compound 1 Benzoate Form A, Compound 1 Camsylate Form A, Compound 1 Camsylate Form B, Compound 1 Citrate Form A, Compound 1 Citrate Form B, Compound 1 Esylate Form A, Compound 1 Esylate Form B, Compound 1 HBr Form A, Compound 1 HBr Form B, Compound 1 Lactate Form A, Compound 1 Malate Form A, Compound 1 Maleate Form A, Compound 1 Maleate Form B, Compound 1 Mesylate Form A, Compound 1 Mesylate Form B, Compound 1 Mesylate Form C, Compound 1 Oxalate Form A, Compound 1 Oxalate Form B, Compound 1 Oxalate Form C, Compound 1 Propionate Form A, Compound 1 Succinate Form A, Compound 1 Sulfate Form A, Compound 1 Sulfate Form B, Compound 1 Sulfate Form C, Compound 1 Tartrate Form A, Compound 1 Hemitartrate Form B, Compound 1 Tartrate Form C, Compound 1 Tosylate Form A, and Compound 1 Tosylate Form B, wherein the crystalline salt forms are characterized according to claim 1.

In one aspect, the invention includes a pharmaceutical composition comprising a crystalline salt form described herein and a pharmaceutically acceptable excipient.

In another aspect, the invention includes a crystalline salt form of the invention, or a pharmaceutical composition of the invention, for use in a method of treating a disease, disorder, or syndrome mediated at least in part by modulating in vivo activity of a protein kinase, wherein the disease, disorder or syndrome is cancer.

In another aspect, which is not claimed, the disclosure includes a method of treating a disease, disorder, or syndrome mediated at least in part by modulating *in vivo* activity of a protein kinase, comprising administering to a subject in need thereof a crystalline salt form or pharmaceutical composition described herein.

In one instance of this aspect, the disease, disorder, or syndrome mediated at least in part by modulating *in vivo* activity of a protein kinase is cancer.

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis). In another aspect, the disclosure includes a method for inhibiting a protein kinase, the method comprising contacting the protein kinase with a crystalline salt form or pharmaceutical composition described herein.

In one instance of this aspect, the protein kinase is Axl, Mer, c-Met, KDR, or a combination thereof.

A further aspect of the disclosure provides processes for making the solid forms described herein.

### BRIEF DESCRIPTION OF THE FIGURES

**FIG. 1** is an XRPD pattern of Compound 1 Besylate Form A.
**FIG. 2** is a DSC thermogram of Compound 1 Besylate Form A.
**FIG. 3** is a TGA thermogram of Compound 1 Besylate Form A.
**FIG. 4** is an XRPD pattern of Compound 1 Besylate Form B (mixture with Compound 1 Besylate Form A).
**FIG. 5** is an XRPD pattern of Compound 1 Benzoate Form A.
**FIG. 6** is an XRPD pattern of Compound 1 Camsylate Form A.
**FIG. 7** is a DSC thermogram of Compound 1 Camsylate Form A.
**FIG. 8** is a TGA thermogram of Compound 1 Camsylate Form A.
**FIG. 9** is an XRPD pattern of Compound 1 Camsylate Form B.
**FIG. 10** is an XRPD pattern of Compound 1 Citrate Form A (mixture with Compound 1 Form A).
**FIG. 11** is an XRPD pattern of Compound 1 Citrate Form B.
**FIG. 12** is a DSC thermogram of Compound 1 Citrate Form B.
**FIG. 13** is a TGA thermogram of Compound 1 Citrate Form B.
**FIG. 14** is a DVS isotherm plot of Compound 1 Citrate Form B
**FIG. 15** is an XRPD pattern of Compound 1 Esylate Form A.
**FIG. 16** is a DSC thermogram of Compound 1 Esylate Form A.
**FIG. 17** is a TGA thermogram of Compound 1 Esylate Form A.
**FIG. 18** is an XRPD pattern of Compound 1 Esylate Form B (mixture with Compound 1 Esylate Form A).
**FIG. 19** is an XRPD pattern of Compound 1 HBr Form A.
**FIG. 20** is an XRPD pattern of Compound 1 HBr Form B.
**FIG. 21** is an XRPD pattern of Compound 1 Lactate Form A (mixture with Compound 1 Form A).
**FIG. 22** is an XRPD pattern of Compound 1 Malate Form A (mixture with Compound 1 Form A).
**FIG. 23** is an XRPD pattern of Compound 1 Maleate Form A.
**FIG. 24** is a DSC thermogram of Compound 1 Maleate Form A.
**FIG. 25** is a TGA thermogram of Compound 1 Maleate Form A.
**FIG. 26A - 26E** are hot stage photomicrographs showing Compound 1 Maleate Form A at (A) 28.0 °C showing the crystalline form at ambient temperature, (B) 129.3 °C with no change in crystalline form, (C) 133.0 °C with some melting, (D) 140.0 °C with complete melting, and (E) cooled down to 28.3 showing no recrystallization at ambient temperature.
**FIG. 27** is a DVS isotherm plot of Compound 1 Maleate Form A.
**FIG. 28** is an XRPD pattern of Compound 1 Maleate Form B (mixture with Compound 1 Maleate Form A).
**FIG. 29** is an XRPD pattern of Compound 1 Mesylate Form A.
**FIG. 30** is an XRPD pattern of Compound 1 Mesylate Form B.
**FIG. 31** is an XRPD pattern of Compound 1 Mesylate Form C.
**FIG. 32** is a DSC thermogram of Compound 1 Mesylate Form C.
**FIG. 33** is a TGA thermogram of Compound 1 Mesylate Form C.
**FIG. 34** is a DVS isotherm plot of Compound 1 Mesylate Form C.
**FIG. 35** is an XRPD pattern of Compound 1 Oxalate Form A.
**FIG. 36** is an XRPD pattern of Compound 1 Oxalate Form B.
**FIG. 37** is an XRPD pattern of Compound 1 Oxalate Form C.
**FIG. 38** is an XRPD pattern of Compound 1 Propionate Form A (mixture with Compound 1 Form A).
**FIG. 39** is an XRPD pattern of Compound 1 Succinate Form A (mixture with Compound 1 Form A).
**FIG. 40** is an XRPD pattern of Compound 1 Sulfate Form A.
**FIG. 41** is an XRPD pattern of Compound 1 Sulfate Form B.
**FIG. 42** is an XRPD pattern of Compound 1 Sulfate Form C.
**FIG. 43** is an XRPD pattern of Compound 1 Tartrate Form A (mixture with Compound 1 Form A).
**FIG. 44** is an XRPD pattern of Compound 1 Hemitartrate Form B.
**FIG. 45** is a DSC thermogram of Compound 1 Hemitartrate Form B.
**FIG. 46** is a TGA thermogram of Compound 1 Hemitartrate Form B.
**FIG. 47** is an XRPD pattern of Compound 1 Tosylate Form A.
**FIG. 48** is a DSC thermogram of Compound 1 Tosylate Form A.
**FIG. 49** is a TGA thermogram of Compound 1 Tosylate Form A.
**FIG. 50** is a DVS isotherm plot of Compound 1 Tosylate Form A.
**FIG. 51A** - **51D** are hot stage photomicrographs showing Compound 1 Tosylate Form A at (A) 29.9 °C showing the crystalline form at ambient temperature, (B) 199.3 °C with no change in crystalline form, (C) 214.4 °C with some melting, and (D) 217.9 °C with complete melting.
**FIG. 52** is an XRPD pattern of Compound 1 Tosylate Form B.
**FIG. 53** is a DSC thermogram of Compound 1 Tosylate Form B.
**FIG. 54** is a TGA thermogram of Compound 1 Tosylate Form B.
**FIG. 55** is a DVS isotherm plot of Compound 1 Tosylate Form B.
**FIG. 56A** - **56E** are hot stage photomicrographs showing Compound 1 Tosylate Form B at (A) 25.5 °C showing the crystalline form at ambient temperature, (B) 172.0 °C with no change in crystalline form, (C) 188.0 °C with very minor melting, (D) 194.0 °C with nearly complete melting, (E) 200.1 °C with complete melting, (E) 28.4 °C with initial recrystallization.

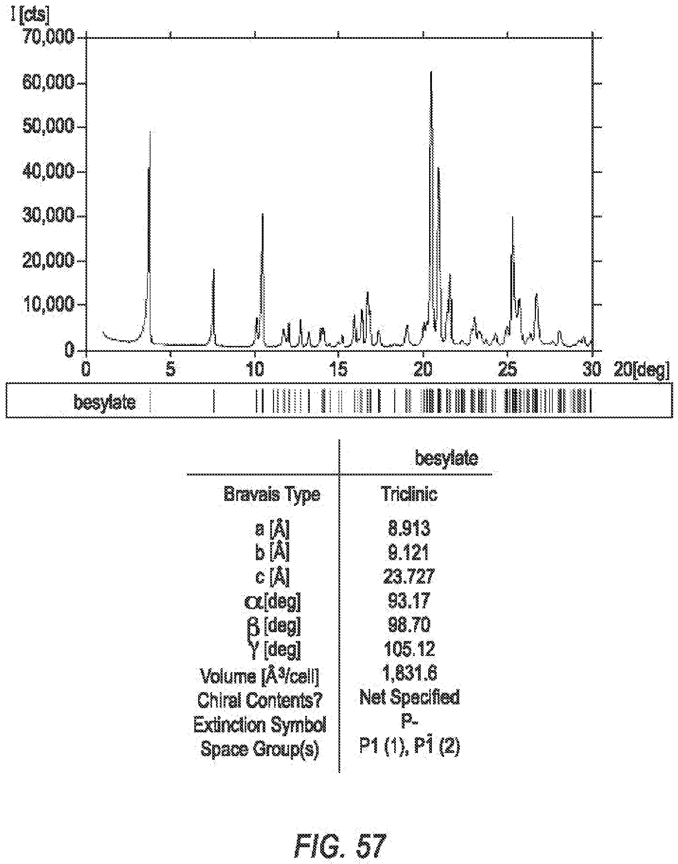

**FIG. 57** is the indexing results for Compound 1 Besylate Form A, including the tabulated space group consistent with the assigned extinction symbol, unit cell parameters, and derived quantities.

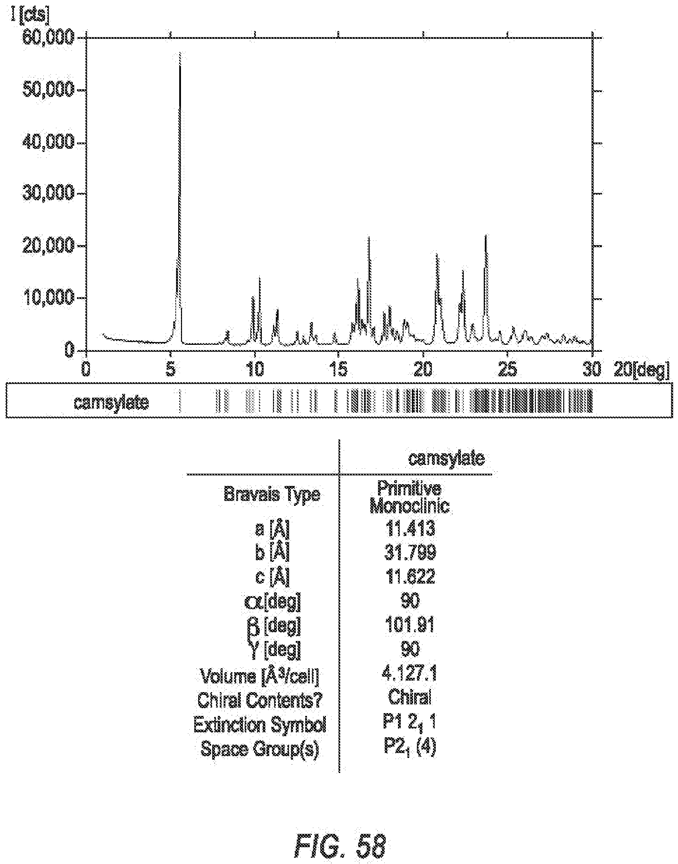

**FIG. 58** is the indexing results for Compound 1 Camsylate Form A, including the tabulated space group consistent with the assigned extinction symbol, unit cell parameters, and derived quantities.

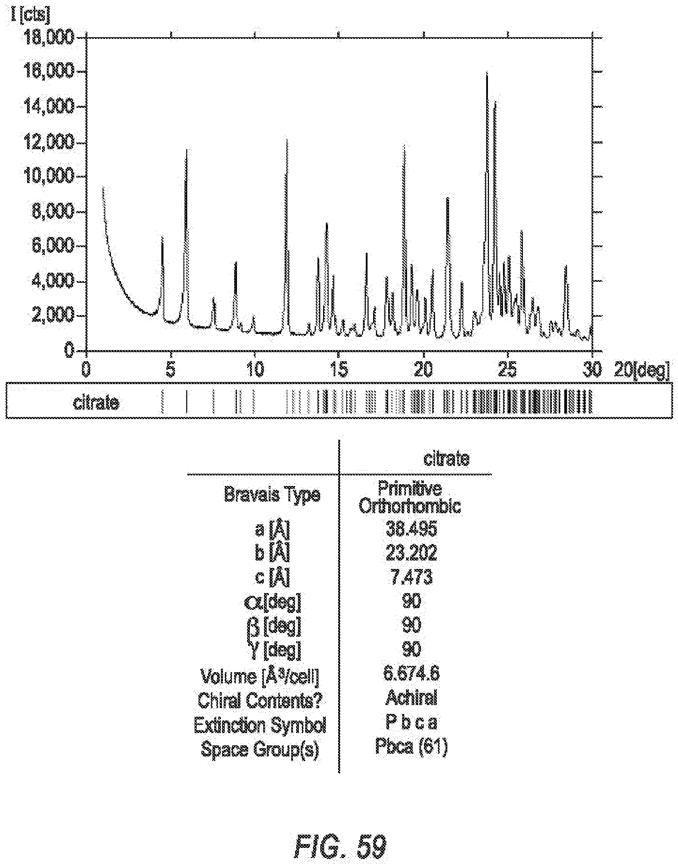

**FIG. 59** is the indexing results for Compound 1 Citrate Form B, including the tabulated space group consistent with the assigned extinction symbol, unit cell parameters, and derived quantities.

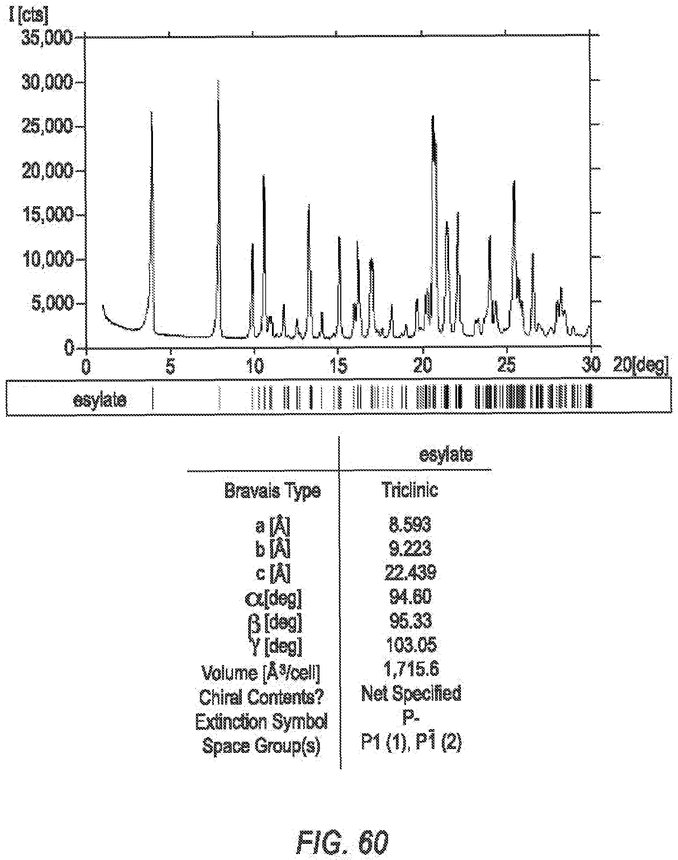

**FIG. 60** is the indexing results for Compound 1 Esylate Form A, including the tabulated space group consistent with the assigned extinction symbol, unit cell parameters, and derived quantities.

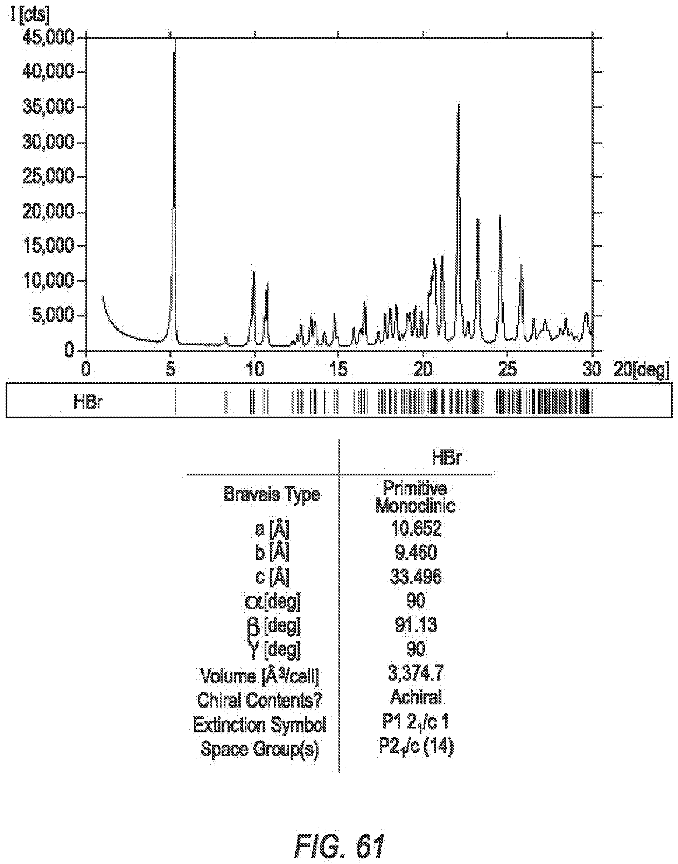

**FIG. 61** is the indexing results for Compound 1 HBr Form B, including the tabulated space group consistent with the assigned extinction symbol, unit cell parameters, and derived quantities.

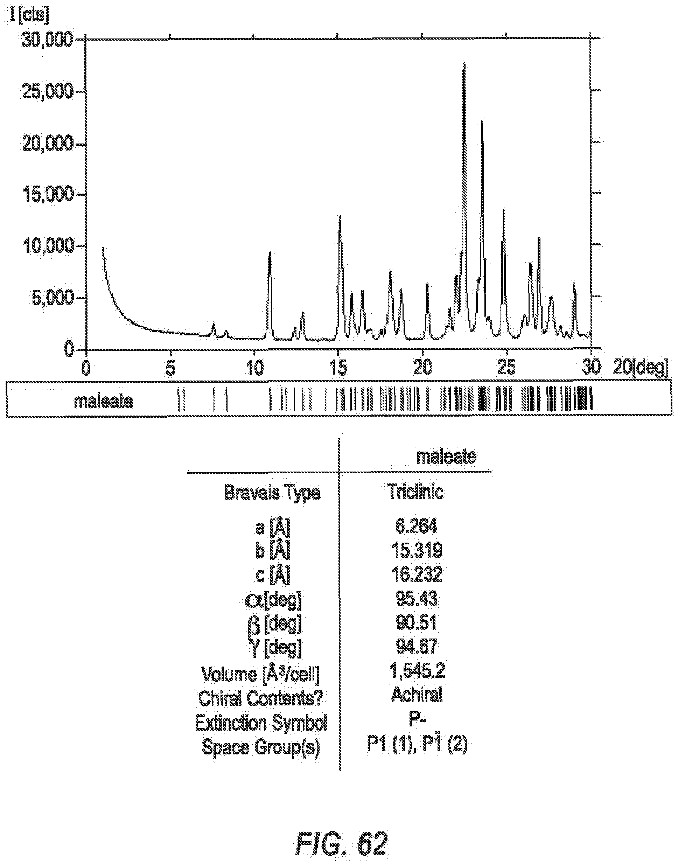

**FIG. 62** is the indexing results for Compound 1 Maleate Form A, including the tabulated space group consistent with the assigned extinction symbol, unit cell parameters, and derived quantities.

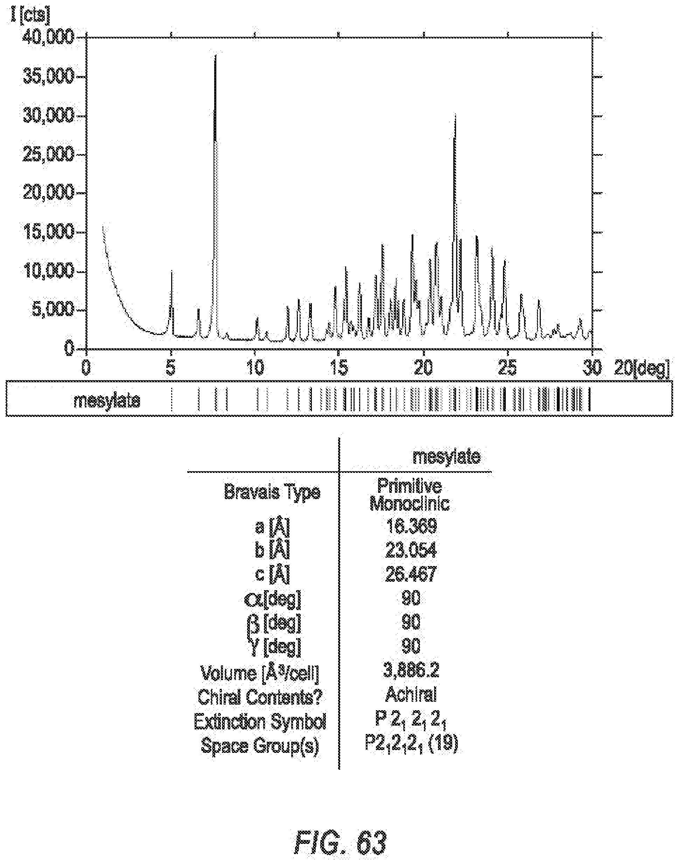

**FIG. 63** is the indexing results for Compound 1 Mesylate Form A, including the tabulated space group consistent with the assigned extinction symbol, unit cell parameters, and derived quantities.

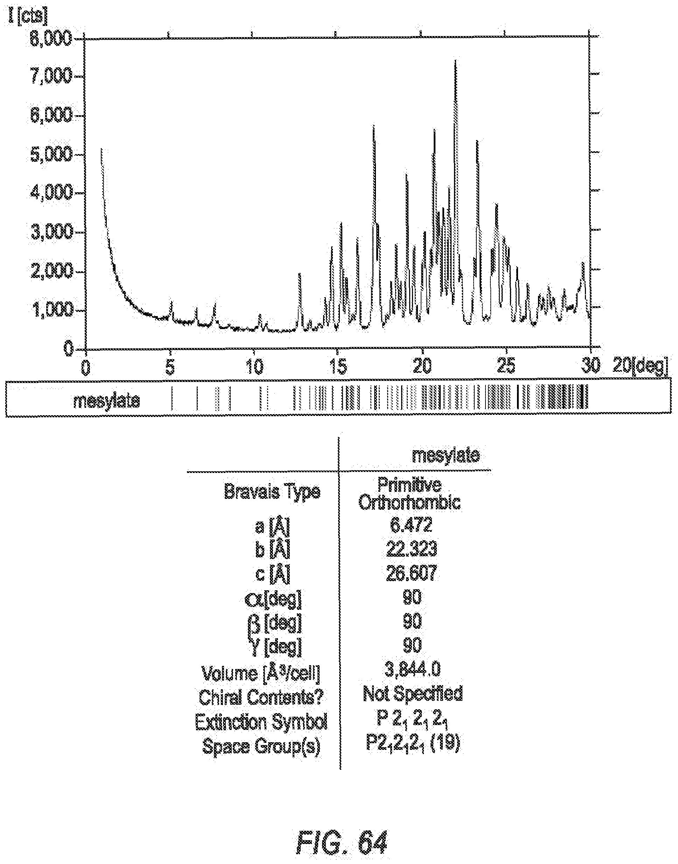

**FIG. 64** is the indexing results for Compound 1 Mesylate Form B, including the tabulated space group consistent with the assigned extinction symbol, unit cell parameters, and derived quantities.

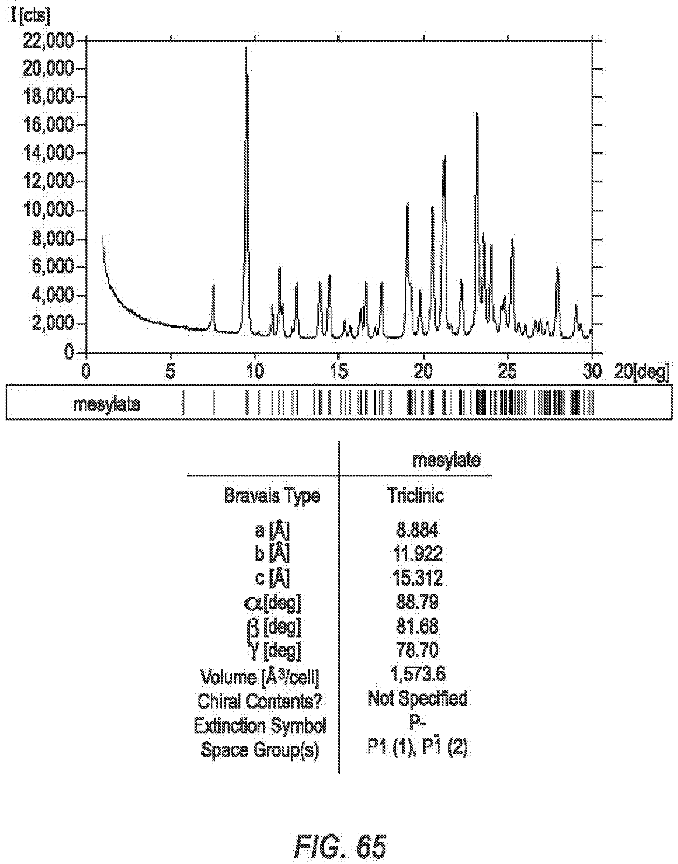

**FIG. 65** is the indexing results for Compound 1 Mesylate Form C, including the tabulated space group consistent with the assigned extinction symbol, unit cell parameters, and derived quantities.

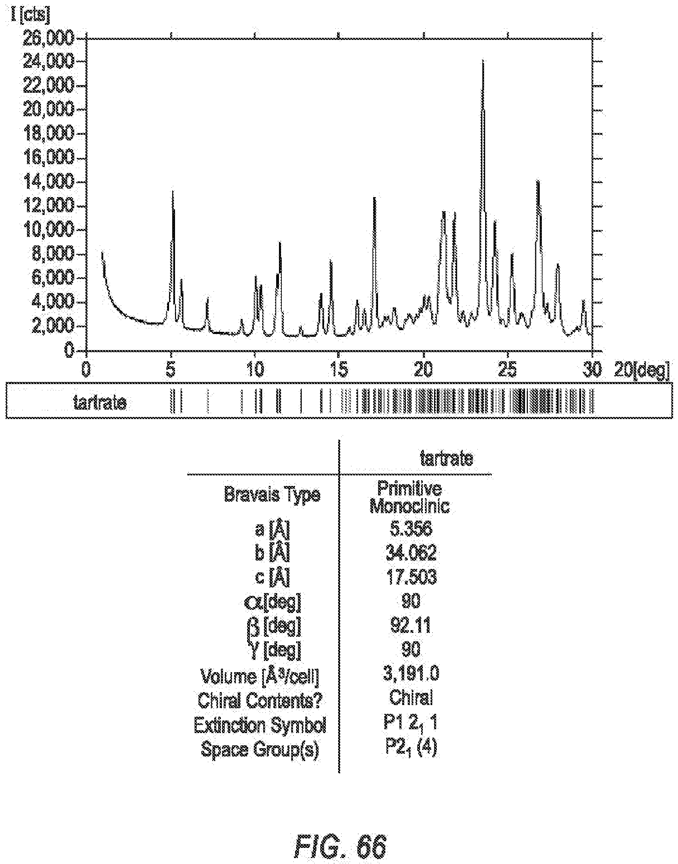

**FIG. 66** is the indexing results for Compound 1 Hemitartarate Form B, including the tabulated space group consistent with the assigned extinction symbol, unit cell parameters, and derived quantities.

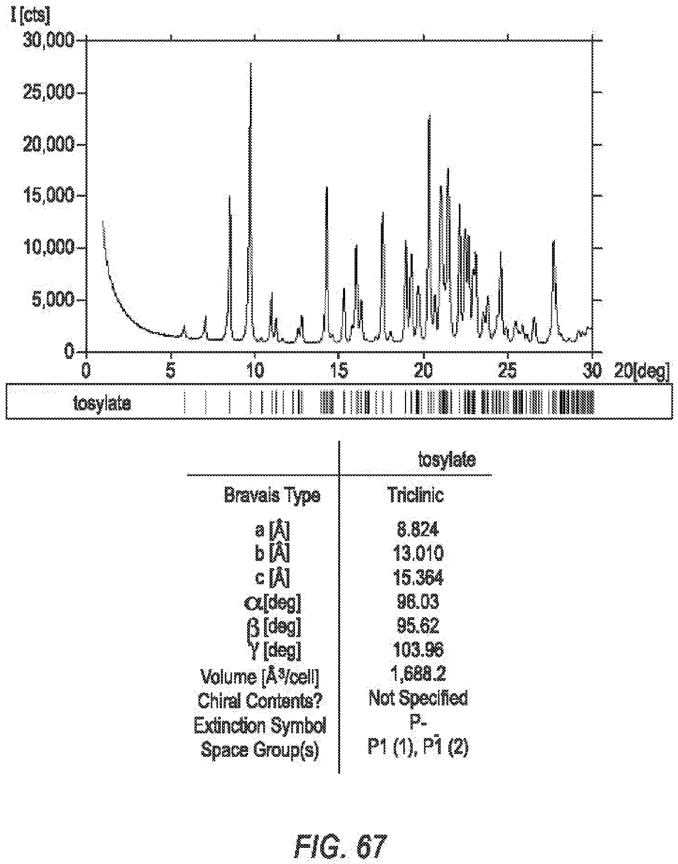

**FIG. 67** is the indexing results for Compound 1 Tosylate Form A, including the tabulated space group consistent with the assigned extinction symbol, unit cell parameters, and derived quantities.

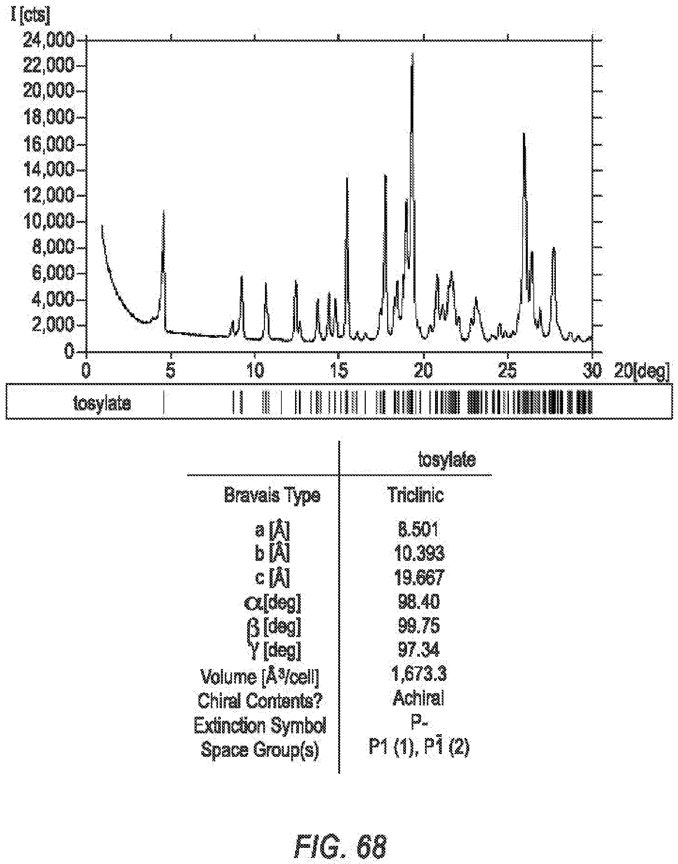

**FIG. 68** is the indexing results for Compound 1 Tosylate Form B, including the tabulated space group consistent with the assigned extinction symbol, unit cell parameters, and derived quantities.
**FIG. 69** is the XRPD pattern of Compound 1 Tartrate Form C.

### DETAILED DESCRIPTION OF THE DISCLOSURE

### DEFINITIONS, ABBREVIATIONS AND ACRONYMS

### Analytical Techniques

| **Abbreviations/Acronyms** | **Full Name/Description** |
|---|---|
| DSC | Differential scanning calorimetry |
| DVS | Dynamic (water) vapor sorption |
| HSM | Hot stage microscopy |
| NMR | Nuclear magnetic resonance spectroscopy |
| OM | Optical microscopy |
| PLM | Polarized light microscopy |
| TGA | Thermogravimetry or Thermogravimetric analysis |
| XRPD | X-ray powder diffraction |

### Experimental techniques

| **Abbreviations/Acronyms** | **Full Name/Description** |
|---|---|
| CC | Crash cooling |
| CP | Crash precipitation |
| FC | Fast cooling |
| FE | Fast evaporation |
| RC | Reaction crystallization |
| SC | Slow cooling |
| SE | Slow evaporation |
| VD | Vapor diffusion |
| VS | Vapor stress |

### Miscellaneous

| **Abbreviations/Acronyms** | **Full Name/Description** |
|---|---|
| | About or approximately |
| API | Active pharmaceutical ingredient |
| B/E | Birefringence and extinction |
| Endo/endo | Endotherm or endothermic |
| eq | Equivalent |
| Exo/exo | Exotherm or exothermic |
| FB | Free base |
| FF | Free form |
| frz | Freezer |
| LIMS | Laboratory Information Management System |
| Max/max | Maximum or maxima |
| Obs | Observation or observed |
| PO | Preferred orientation |
| ppt | Precipitate or precipitation |
| ref | Refrigerator |
| RH | Relative humidity |
| RT | Room temperature |
| Soln/soln | Solution |
| vac | Vacuum |
| wt% | Weight percent |

### Solvents

| **Abbreviations/Acronyms** | **Full Name/Description** |
|---|---|
| ACN | Acetonitrile |
| AcOH | Acetic acid |
| DCM | Dichloromethane |
| DMSO | Dimethylsulfoxide |
| EtOAc | Ethyl acetate |
| EtOH | Ethanol |
| HFIPA | Hexafluoroisopropanol |
| IPA | Isopropyl alcohol, 2-propanol |
| MEK | Methyl ethyl ketone |
| MeOH | Methanol |
| MTBE | Methyl-tertiary-butyl ether |
| TFE | 2,2,2-Trifluoroethanol |
| THF | Tetrahydrofuran |

As used herein, the following definitions shall apply unless otherwise indicated.

For purposes of this disclosure, the chemical elements are identified in accordance with the Periodic Table of the Elements, CAS version, Handbook of Chemistry and Physics, 95th Ed. Additionally, general principles of organic chemistry are described in "Organic Chemistry," 2nd Ed., Thomas Sorrell, University Science Books, Sausalito: 2006, and "March's Advanced Organic Chemistry," 7th Ed., Ed.: Smith, M.B. and March, J., John Wiley & Sons, New York: 2013.

As used herein, the term "Low/limited/significant hygroscopisity" refers to a material that exhibits < 0.5/< 2.0/≥ 2.0 wt% water uptake over a specified RH range.

As used herein, the term "stoichiometric hydrate" refers to crystalline material with a defined water content over an extended RH range. Typical stoichiometric hydrates are hemihydrates, monohydrates, sesquihydrates, dihydrates, etc.

As used herein, the term "variable hydrate" refers to crystalline material with variable water content over an extended RH range, yet with no phase change.

As used herein, a chemical term designated as a "Form" refers to a chemical compound, or salt thereof, that consists of a single phase.

As used herein, the term "low/limited/intermediate/good/high solubility" refers to a material having a solubility of < 1/1 - 20/20 - 100/100 - 200/> 200 mg/mL.

As used herein, the term "crystalline" refers to a material that produces an XRPD pattern with sharp peaks (similar to instrumental peak widths) and weak diffuse scattering relative to the peaks.

As used herein, the term "disordered crystalline" refers to a material that produces XRPD pattern with broad peaks (relative to instrumental peak widths) and/or strong diffuse scattering relative to the peaks. Disordered materials may be:
1) microcrystalline,
2) crystalline with large defect density,
3) mixtures of crystalline and X-ray amorphous phases, or
4) a combination of the above.

As used herein, the term "insufficient signal" means that spectrographic analysis of a sample produced a spectrum or pattern (output) having insufficient signal above the expected background noise.

As used herein, the term "single crystalline phase" refers to an XRPD pattern that is judged to contain evidence of a single crystalline form due to the Bragg peaks being indexed with a single unit cell. Indexing is the process of assigning Miller index labels to each peak in a diffraction pattern. Also, the size and shape of the crystal unit cell is determined during the indexing process.

As used herein, the term "slurry" refers to a suspension prepared by adding enough solids to a given solvent at ambient conditions so that undissolved solids are present. A typical slurry includes agitation (typically by stirring or oscillation), an act that is also referred to as "slurrying," in a sealed vial at a given temperature for an extended period of time. Typically, the solids are recovered after a given period of time using a method described herein.

As used herein, the term "X-ray amorphous" or "amorphous" refers to a material having diffuse scatter present, but no evidence for Bragg peaks in the XRPD pattern.

As used herein, the term "crystalline" refers to compounds in a solid state having a periodic and repeating three-dimensional internal arrangement of atoms, ions or molecules characteristic of crystals, for example, arranged in fixed geometric patterns or lattices that have rigid long range order. The term crystalline does not necessarily mean that the compound exists as crystals, but that it has this crystal-like internal structural arrangement.

As used herein, the term "substantially crystalline" refers to a solid material that is predominately arranged in fixed geometric patterns or lattices that have rigid long range order. For example, substantially crystalline materials have more than about 85% crystallinity (e.g., more than about 90% crystallinity, more than about 95% crystallinity, or more than about 99% crystallinity). It is also noted that the term 'substantially crystalline' includes the descriptor 'crystalline,' which is defined in the previous paragraph.

"Patient" for the purposes of the present disclosure includes humans and any other animals, particularly mammals, and other organisms. Thus the methods are applicable to both human therapy and veterinary applications. In a preferred instance the patient is a mammal, and in a most preferred instance the patient is human. Examples of the preferred mammals include mice, rats, other rodents, rabbits, dogs, cats, swine, cattle, sheep, horses, and primates.

"Kinase-dependent diseases or conditions" refer to pathologic conditions that depend on the activity of one or more kinases. Kinases either directly or indirectly participate in the signal transduction pathways of a variety of cellular activities including proliferation, adhesion, migration, differentiation, and invasion. Diseases associated with kinase activities include tumor growth, the pathologic neovascularization that supports solid tumor growth, and associated with other diseases where excessive local vascularization is involved such as ocular diseases (diabetic retinopathy, age-related macular degeneration, and the like) and inflammation (psoriasis, rheumatoid arthritis, and the like).

"Therapeutically effective amount" is an amount of a crystalline salt form of Compound 1 that, when administered to a patient, ameliorates a symptom of the disease. The amount of a crystalline salt form of Compound 1 which constitutes a "therapeutically effective amount" will vary depending on the compound, the disease state and its severity, the age of the patient to be treated, and the like. The therapeutically effective amount can be determined routinely by one of ordinary skill in the art having regard to his own knowledge and to this disclosure.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, immunogenicity or other problem or complication, commensurate with a reasonable benefit risk ratio.

As used herein, the phrase "pharmaceutically acceptable excipient" refers to a pharmaceutically-acceptable material, composition, or vehicle, such as a liquid or solid filler, diluent, solvent, or encapsulating material. Excipients are generally safe, non-toxic and neither biologically nor otherwise undesirable and include excipients that are acceptable for veterinary use as well as human pharmaceutical use. In one instance, each component is "pharmaceutically acceptable" as defined herein. See, e.g., Remington: The Science and Practice of Pharmacy, 21st ed.; Lippincott Williams & Wilkins: Philadelphia, Pa., 2005; Handbook of Pharmaceutical Excipients, 6th ed.; Rowe et al, Eds.; The Pharmaceutical Press
and the American Pharmaceutical Association: 2009; Handbook of Pharmaceutical Additives, 3rd ed.; Ash and Ash Eds.; Gower Publishing Company: 2007; Pharmaceutical Pref or mulation and Formulation, 2nd ed.; Gibson Ed. ; CRC Press LLC: Boca Raton, Fla., 2009.

"Cancer" refers to cellular-proliferative disease states, including, but not limiting to: Cardiac: sarcoma (angiosarcoma, fibrosarcoma, rhabdomyosarcoma, liposarcoma), myxoma, rhabdomyoma, fibroma, lipoma and teratoma; Head and neck: squamous cell carcinomas of the head and neck, laryngeal and hypopharyngeal cancer, nasal cavity and paranasal sinus cancer, nasopharyngeal cancer, salivary gland cancer, oral and orppharyngeal cancer; Lung: bronchogenic carcinoma (squamous cell, undifferentiated small cell, undifferentiated large cell, adenocarcinoma, non-small cell lung cancer), alveolar (bronchiolar) carcinoma, alveolar sarcoma, alveolar soft part sarcoma, bronchial adenoma, sarcoma, lymphoma, chondromatous hamartoma, mesothelioma; Colon: colorectal cancer, adenocarcinoma, gastrointestinal stromal tumors, lymphoma, carcinoids, Turcot Syndrome; Gastrointestinal: gastric cancer, gastroesophageal junction adenocarcinoma, esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Breast: metastatic breast cancer, ductal carcinoma in situ, invasive ductal carcinoma, tubular carcinoma, medullary carcinoma, mucinous carcinoma, lobular carcinoma in situ, triple negative breast cancer; Genitourinary tract: kidney (adenocarcinoma, Wilm's tumor [nephroblastoma], lymphoma, leukemia, renal cell carcinoma, metastatic renal cell carcinoma), bladder and urethra (squamous cell carcinoma, transitional cell carcinoma, adenocarcinoma, urothelial carcinoma), prostate (adenocarcinoma, sarcoma, castrate resistant prostate cancer, bone metastases, bone metastases associated with castrate resistant prostate cancer,), testis (seminoma, teratoma, embryonal carcinoma, teratocarcinoma, choriocarcinoma, sarcoma, interstitial cell carcinoma, fibroma, fibroadenoma, adenomatoid tumors, lipoma), clear cell carcinoma, papillary carcinoma, penile cancer, penile squamous cell carrcinoma ; Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochrondroma (osteocartilaginous exostoses), benign chondroma, chondroblastoma, chondromyxofibroma, osteoid osteoma, and giant cell tumors; Thyroid: medullary thyroid cancer, differentiated thyroid cancer, papillary thyroid cancer, follicular thyroid cancer, hurthle cell cancer, and anaplastic thyroid cancer; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma), NF1, neurofibromatosis, plexiform neurofibromas; Gynecological: uterus (endometrial cancer), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), myelofibrosis, polycythemia vera, essential thrombocythemia, Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; and Adrenal glands: neuroblastoma. Thus, the term "cancerous cell" as provided herein, includes a cell afflicted by any one of the above-identified conditions. In some instances, a compound or combination as disclosed herein can be used for the treatment of diseases including HIV, sickle cell disease, graft-versus-host disease, acute graft-versus-host disease, chronic graft-versus-host disease, and sickle cell anemia.

In general, the nomenclature used in this application is based on naming conventions adopted by the International Union of Pure and Applied Chemistry (IUPAC). Chemical structures shown herein were prepared using CHEMDRAW^{®}. Any open valency appearing on a carbon, oxygen, or nitrogen atom in the structures herein indicates the presence of a hydrogen atom.

In one aspect, the invention includes a crystalline salt form of Compound 1 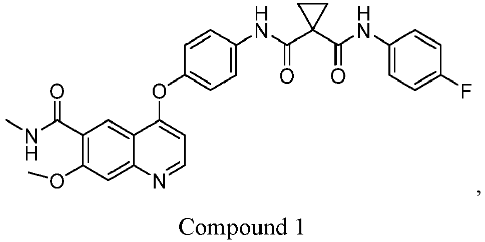 which is 1-N'-(4-Fluorophenyl)-1-N-[4-[7-methoxy-6-(methylcarbamoyl)quinolin-4-yl]oxyphenyl]cyclopropane-1,1-dicarboxamide, or N'-(4-Fluorophenyl)-N-[4-[7-methoxy-6-(methylcarbamoyl)quinolin-4-yl]oxyphenyl]cyclopropane-1,1-dicarboxamide, wherein the crystalline salt form is selected from Compound 1 Besylate Form A, Compound 1 Besylate Form B, Compound 1 Benzoate Form A, Compound 1 Camsylate Form A, Compound 1 Camsylate Form B, Compound 1 Citrate Form A, Compound 1 Citrate Form B, Compound 1 Esylate Form A, Compound 1 Esylate Form B, Compound 1 HBr Form A, Compound 1 HBr Form B, Compound 1 Lactate Form A, Compound 1 Malate Form A, Compound 1 Maleate Form A, Compound 1 Maleate Form B, Compound 1 Mesylate Form A, Compound 1 Mesylate Form B, Compound 1 Mesylate Form C, Compound 1 Oxalate Form A, Compound 1 Oxalate Form B, Compound 1 Oxalate Form C, Compound 1 Propionate Form A, Compound 1 Succinate Form A, Compound 1 Sulfate Form A, Compound 1 Sulfate Form B, Compound 1 Sulfate Form C, Compound 1 Tartrate Form A, Compound 1 Hemitartrate Form B, Compound 1 Tartrate Form C, Compound 1 Tosylate Form A, and Compound 1 Tosylate Form B, wherein these crystalline salt forms are characterized according to claim 1.

In one embodiment of this aspect, the crystalline form is characterized as Compound 1 Besylate Form A.

The Compound 1 Besylate Form A is characterized by one or more peaks in an XRPD pattern on a 2 Theta scale, wherein the one or more peaks is selected from 3.78, 7.56, 10.08, 10.42, 11.70, 11.78, 12.00, 12.71, 13.20, 13.92, 14.06, 14.44, 14.93, 15.18, 15.96, 16.21, 16.40, 16.73, 16.86, 17.38, 18.32, 19.07, 20.07, 20.26, 20.51, 20.94, 21.45, 21.60, 22.30, 22.90, 23.05, 23.28, 23.71, 24.28, 24.97, 25.30, 25.69, 26.33, 26.71, 27.29, 27.65, 28.07, 29.22, and 29.50.

In an embodiment, the Compound 1 Besylate Form A is characterized by one or more peaks in an XRPD pattern on a 2 Theta scale, wherein the one or more peaks is selected from 3.78, 7.56, 10.42, 20.51, 20.94, 21.60, and 25.30.

In a further embodiment, the Compound 1 Besylate Form A is characterized by all of the following peaks in an XRPD pattern on a 2 Theta scale, selected from 3.78, 7.56, 10.42, 20.51, 20.94, 21.60, and 25.30.

In still a further embodiment, the Compound 1 Besylate Form A is characterized by all of the following peaks in an XRPD pattern on a 2 Theta scale, selected from 3.78, 7.56, 10.08, 10.42, 11.70, 11.78, 12.00, 12.71, 13.20, 13.92, 14.06, 14.44, 14.93, 15.18, 15.96, 16.21, 16.40, 16.73, 16.86, 17.38, 18.32, 19.07, 20.07, 20.26, 20.51, 20.94, 21.45, 21.60, 22.30, 22.90, 23.05, 23.28, 23.71, 24.28, 24.97, 25.30, 25.69, 26.33, 26.71, 27.29, 27.65, 28.07, 29.22, and 29.50.

In another embodiment, the Compound 1 Besylate Form A is characterized by a first endotherm at about 144°C and a second endotherm at a temperature of about 170 °C in a DSC thermogram.

In another embodiment, the Compound 1 Besylate Form A is characterized by a weight loss of about 7.9% over a temperature range of 39-177 °C in a TGA thermogram.

In one embodiment of this aspect, the crystalline form is characterized as Compound 1 Besylate Form B.

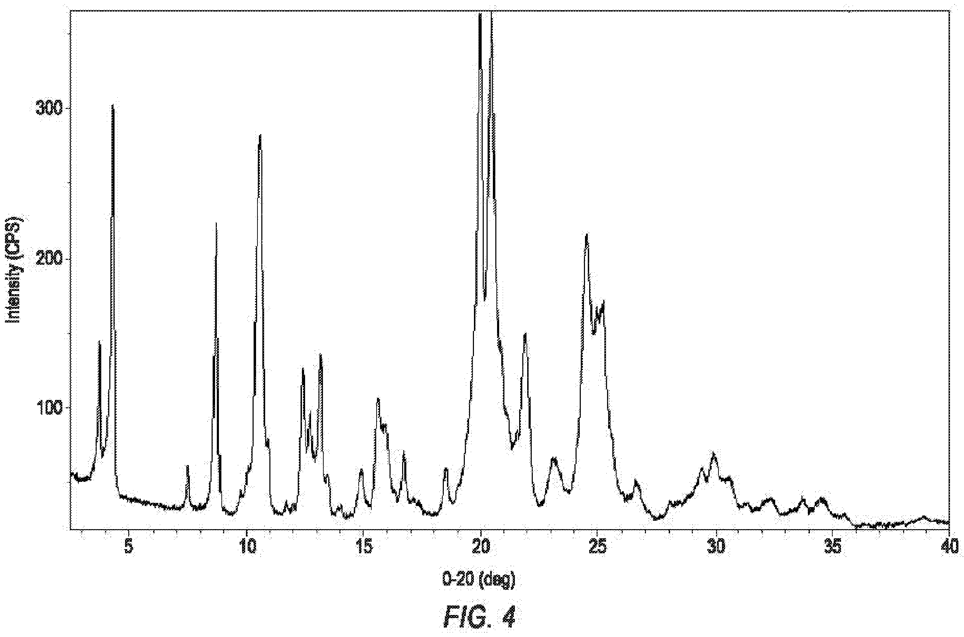

The Compound 1 Besylate Form B is characterized by the XRPD pattern provided in Figure 4.

In one embodiment of this aspect, the crystalline form is characterized as Compound 1 Benzoate Form A.

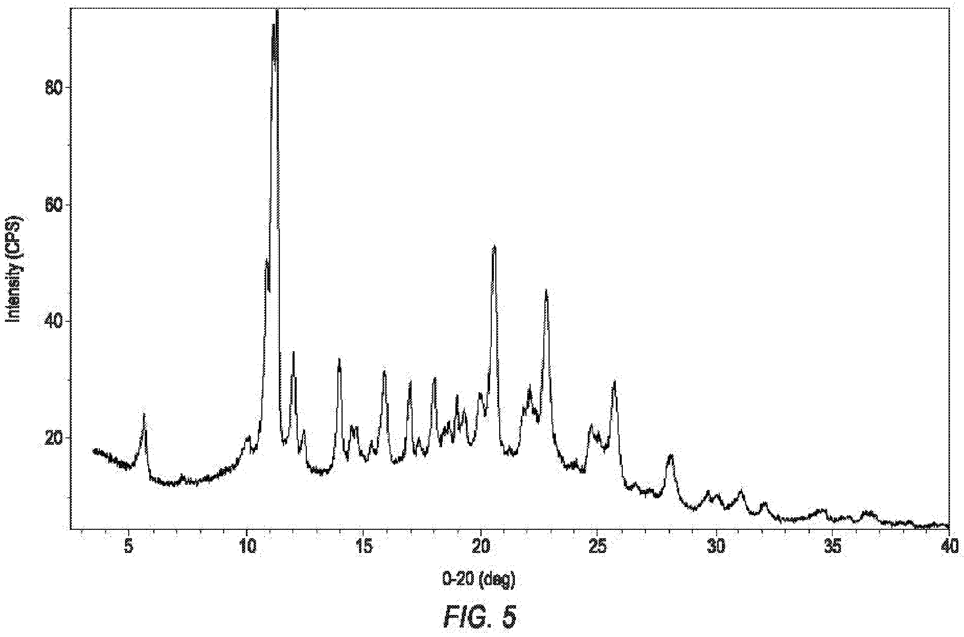

The Compound 1 Benzoate Form A is characterized by the XRPD pattern provided in Figure 5.

In one embodiment of this aspect, the crystalline form is characterized as Compound 1 Camsylate Form A.

The Compound 1 Camsylate Form A is characterized by one or more peaks in an XRPD pattern on a 2 Theta scale, wherein the one or more peaks is selected from 5.55, 7.90, 8.25, 8.38, 9.55, 9.87, 10.26, 11.12, 11.34, 12.50, 12.94, 13.40, 13.65, 14.79, 15.83, 16.16, 16.41, 16.54, 16.82, 17.10, 17.69, 18.02, 18.22, 18.47, 18.91, 19.10, 19.47, 20.04, 20.87, 21.05, 22.16, 22.36, 22.93, 23.71, 24.55, 25.33, 26.02, 26.40, 27.09, 27.33, 27.97, 28.32, 28.68, and 28.98.

In an embodiment, the Compound 1 Camsylate Form A is characterized by one or more peaks in an XRPD pattern on a 2 Theta scale, wherein the one or more peaks is selected from 5.55, 9.87, 10.26, 16.16, 16.82, 20.87, 22.36, and 23.71.

In a further embodiment, the Compound 1 Camsylate Form A is characterized by all of the following peaks in an XRPD pattern on a 2 Theta scale, selected from 5.55, 9.87, 10.26, 16.16, 16.82, 20.87, 22.36, and 23.71.

In still a further embodiment, the Compound 1 Camsylate Form A is characterized by all of the following peaks in an XRPD pattern on a 2 Theta scale, selected from 5.55, 7.90, 8.25, 8.38, 9.55, 9.87, 10.26, 11.12, 11.34, 12.50, 12.94, 13.40, 13.65, 14.79, 15.83, 16.16, 16.41, 16.54, 16.82, 17.10, 17.69, 18.02, 18.22, 18.47, 18.91, 19.10, 19.47, 20.04, 20.87, 21.05, 22.16, 22.36, 22.93, 23.71, 24.55, 25.33, 26.02, 26.40, 27.09, 27.33, 27.97, 28.32, 28.68, and 28.98.

In another embodiment, the Compound 1 Camsylate Form A is characterized by a first endotherm at a temperature of about 110 °C and a second endotherm at about 174 °C in a DSC thermogram.

In another embodiment, the Compound 1 Camsylate Form A is characterized by a weight loss of about 5.9% over a temperature range of 38-170 °C in a TGA thermogram.

In one embodiment of this aspect, the crystalline form is characterized as Compound 1 Camsylate Form B.

The Compound 1 Camsylate Form B is characterized by one or more peaks in an XRPD pattern on a 2 Theta scale, wherein the one or more peaks is selected from 5.25, 8.32, 8.51, 8.76, 9.05, 9.58, 9.88, 10.00, 10.52, 10.80, 11.06, 11.96, 12.24, 12.58, 13.98, 15.62, 15.81, 16.15, 16.70, 17.12, 17.42, 17.71, 18.41, 19.37, 19.55, 20.63, 21.13, 21.56, 21.89, 22.50, 23.04, 24.43, 26.08, 27.07, 27.97, 28.22, and 28.73.

In an embodiment, the Compound 1 Camsylate Form B is characterized by one or more peaks in an XRPD pattern on a 2 Theta scale, wherein the one or more peaks is selected from 5.25, 10.52, 16.15, 21.13, 21.56, and 21.89.

In a further embodiment, the Compound 1 Camsylate Form B is characterized by all of the following peaks in an XRPD pattern on a 2 Theta scale, selected from 5.25, 10.52, 16.15, 21.13, 21.56, and 21.89.

In a further embodiment, the Compound 1 Camsylate Form B is characterized by all of the following peaks in an XRPD pattern on a 2 Theta scale, selected from 5.25, 8.32, 8.51, 8.76, 9.05, 9.58, 9.88, 10.00, 10.52, 10.80, 11.06, 11.96, 12.24, 12.58, 13.98, 15.62, 15.81, 16.15, 16.70, 17.12, 17.42, 17.71, 18.41, 19.37, 19.55, 20.63, 21.13, 21.56, 21.89, 22.50, 23.04, 24.43, 26.08, 27.07, 27.97, 28.22, and 28.73.

In one embodiment of this aspect, the crystalline form is characterized as Compound 1 Citrate Form A.

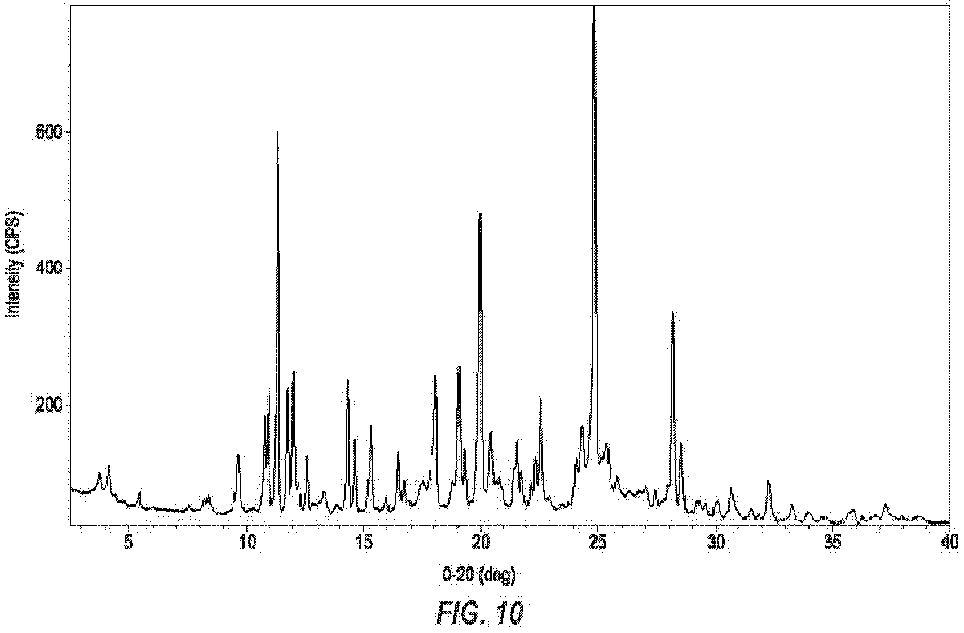

The Compound 1 Citrate Form A is characterized by the XRPD pattern provided in Figure 10.

In one embodiment of this aspect, the crystalline form is characterized as Compound 1 Citrate Form B.

The Compound 1 Citrate Form B is characterized by one or more peaks in an XRPD pattern on a 2 Theta scale, wherein the one or more peaks is selected from 4.58, 5.95, 7.61, 8.88, 9.19, 9.94, 11.93, 13.26, 13.79, 14.29, 14.68, 15.26, 15.95, 16.64, 17.11, 17.87, 18.21, 18.43, 18.90, 19.36, 19.67, 20.14,20.57, 21.47, 22.30, 22.64,23.06, 23.80, 24.25, 24.55, 24.81, 25.10, 25.45, 25.54, 25.85, 26.35, 26.51, 26.83, 27.16, 27.57, 27.85, 28.08, and 28.47.

In an embodiment, the Compound 1 Citrate Form B is characterized by one or more peaks in an XRPD pattern on a 2 Theta scale, wherein the one or more peaks is selected from 4.58, 5.95, 8.88, 11.93, 13.79, 14.29, 16.64, 18.90, 21.47, 23.80, 24.25, and 25.85.

In a further embodiment, the Compound 1 Citrate Form B is characterized by all of the following peaks in an XRPD pattern on a 2 Theta scale, selected from 4.58, 5.95, 8.88, 11.93, 13.79, 14.29, 16.64, 18.90, 21.47, 23.80, 24.25, and 25.85.

In still a further embodiment, the Compound 1 Citrate Form B is characterized by all of the following peaks in an XRPD pattern on a 2 Theta scale, selected from 4.58, 5.95, 7.61, 8.88, 9.19, 9.94, 11.93, 13.26, 13.79, 14.29, 14.68, 15.26, 15.95, 16.64, 17.11, 17.87, 18.21, 18.43, 18.90, 19.36, 19.67, 20.14, 20.57, 21.47, 22.30,22.64, 23.06, 23.80, 24.25, 24.55, 24.81, 25.10, 25.45, 25.54, 25.85, 26.35, 26.51, 26.83, 27.16, 27.57, 27.85, 28.08, and 28.47.

In another embodiment, the Compound 1 Citrate Form B is characterized by an endotherm at about 175 °C in a DSC thermogram. In another embodiment, the Compound 1 Citrate Form B is characterized by an endotherm with an onset temperature at about 175 °C in a DSC thermogram.

In another embodiment, the Compound 1 Citrate Form B is characterized by a weight loss above a temperature of 175 °C in a TGA thermogram.

In another embodiment, the Compound 1 Citrate Form B is characterized by a weight loss beginning at a temperature of about 159 °C and ending at about 220 °C in a TGA thermogram.

In one embodiment, the Compound 1 Citrate Form B is characterized by about a 4.1 wt% increase, as measured by DVS, when taken from an environment of 5% relative humidity to an environment of 95% relative humidity.

In one embodiment of this aspect, the crystalline form is characterized as Compound 1 Esylate Form A.

The Compound 1 Esylate Form A is characterized by one or more peaks in an XRPD pattern on a 2 Theta scale, wherein the one or more peaks is selected from 3.97, 7.95, 9.89, 10.62, 10.92, 11.03, 11.34, 11.77, 12.03, 12.53, 12.71, 13.27, 14.00, 14.70, 15.08, 15.94, 16.17, 16.92, 17.01, 17.19, 17.35, 17.61, 18.15, 18.76, 19.03, 19.66, 19.89, 20.26, 20.65, 20.78, 21.44, 22.08, 23.14, 23.27, 23.99, 24.32, 25.43, 25.66, 25.85,26.52, 26.87, 27.61, 27.96, 28.18, 28.40, and 28.91.

In an embodiment, the Compound 1 Esylate Form A is characterized by one or more peaks in an XRPD pattern on a 2 Theta scale, wherein the one or more peaks is selected from 3.97, 7.95, 9.89, 10.62, 13.27, 15.08, 16.17, 16.92, 17.01, 20.65, 20.78, 21.44, 22.08, 23.99, 25.43, and 26.52.

In a further embodiment, the Compound 1 Esylate Form A is characterized by all of the following peaks in an XRPD pattern on a 2 Theta scale, selected from 3.97, 7.95, 9.89, 10.62, 13.27, 15.08, 16.17, 16.92, 17.01, 20.65, 20.78, 21.44, 22.08, 23.99, 25.43, and 26.52.

In still a further embodiment, the Compound 1 Esylate Form A is characterized by all of the following peaks in an XRPD pattern on a 2 Theta scale, selected from 3.97, 7.95, 9.89, 10.62, 10.92, 11.03, 11.34, 11.77, 12.03, 12.53, 12.71, 13.27, 14.00, 14.70, 15.08, 15.94, 16.17, 16.92, 17.01, 17.19, 17.35, 17.61, 18.15, 18.76, 19.03, 19.66, 19.89, 20.26, 20.65, 20.78, 21.44, 22.08, 23.14, 23.27, 23.99, 24.32, 25.43, 25.66, 25.85,26.52, 26.87, 27.61, 27.96, 28.18, 28.40, and 28.91.

In another embodiment, the Compound 1 Esylate Form A is characterized by a first endotherm at a temperature of about 69 °C, a second endotherm at a temperature of about 156 °C, and a third endotherm at a temperature of about 190 °C, in a DSC thermogram.

In another embodiment, the Compound 1 Esylate Form A is characterized by a weight loss of about 8.5 wt% over a temperature range of 38-188 °C in a TGA thermogram.

In one embodiment of this aspect, the crystalline form is characterized as Compound 1 Esylate Form B.

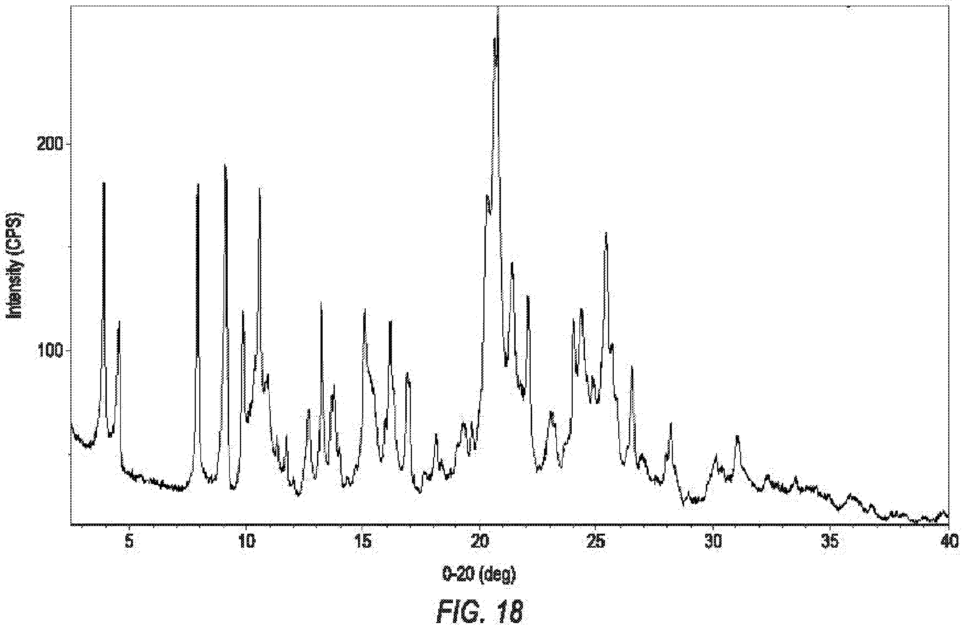

The Compound 1 Esylate Form B is characterized by the XRPD pattern provided in Figure 18.

In one embodiment of this aspect, the crystalline form is characterized as Compound 1 HBr Form A.

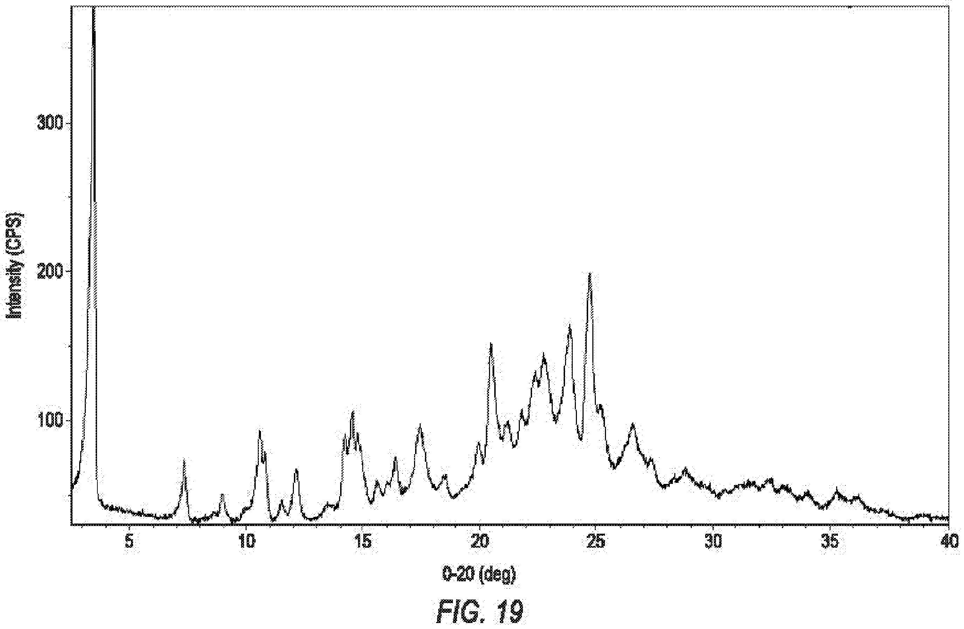

The Compound 1 HBr Form A is characterized by the XRPD pattern provided in Figure 19.

In one embodiment of this aspect, the crystalline form is characterized as Compound 1 HBr Form B.

The Compound 1 HBr Form B is characterized by one or more peaks in an XRPD pattern on a 2 Theta scale, wherein the one or more peaks is selected from 5.26, 8.29, 9.75, 9.91, 10.56, 10.72, 12.25, 12.50, 12.75, 13.31, 13.54, 14.11, 14.72, 14.89, 15.87, 16.29, 16.50, 17.36, 17.78, 18.09, 18.44, 18.75, 19.12, 19.24, 19.56, 19.92, 20.40, 20.55, 20.69, 21.18, 22.09, 22.64, 23.24, 24.56, 25.80, 26.52, 27.23, 27.43, 28.13, 28.46, 28.76, 29.08, 29.66, and 30.07.

In an embodiment, the Compound 1 HBr Form B is characterized by one or more peaks in an XRPD pattern on a 2 Theta scale, wherein the one or more peaks is selected from 5.26, 9.91, 10.72, 20.55, 20.69, 21.18, 22.09, 23.24, 24.56, and 25.80.

In a further embodiment, the Compound 1 HBr Form B is characterized by all of the following peaks in an XRPD pattern on a 2 Theta scale, selected from 5.26, 9.91, 10.72, 20.55, 20.69, 21.18, 22.09, 23.24, 24.56, and 25.80.

In still a further embodiment, the Compound 1 HBr Form B is characterized by all of the following peaks in an XRPD pattern on a 2 Theta scale, selected from 5.26, 8.29, 9.75, 9.91, 10.56, 10.72, 12.25, 12.50, 12.75, 13.31, 13.54, 14.11, 14.72, 14.89, 15.87, 16.29, 16.50, 17.36, 17.78, 18.09, 18.44, 18.75, 19.12, 19.24, 19.56, 19.92, 20.40, 20.55, 20.69, 21.18, 22.09, 22.64, 23.24, 24.56, 25.80, 26.52, 27.23, 27.43, 28.13, 28.46, 28.76, 29.08, 29.66, and 30.07.

In one embodiment of this aspect, the crystalline form is characterized as Compound 1 Lactate Form A.

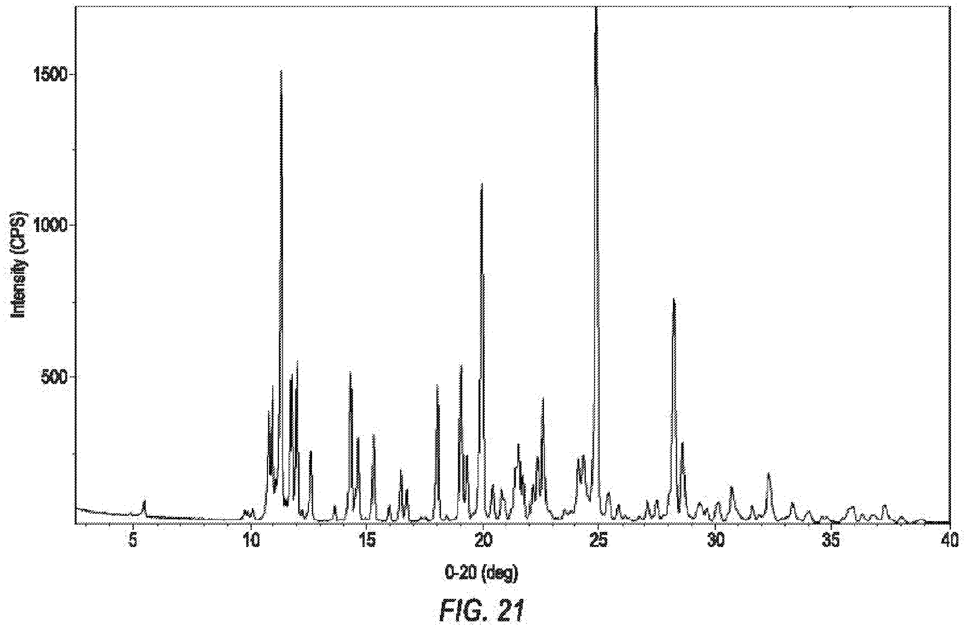

The Compound 1 Lactate Form A is characterized by the XRPD pattern provided in Figure 21.

In one embodiment of this aspect, the crystalline form is characterized as Compound 1 Malate Form A.

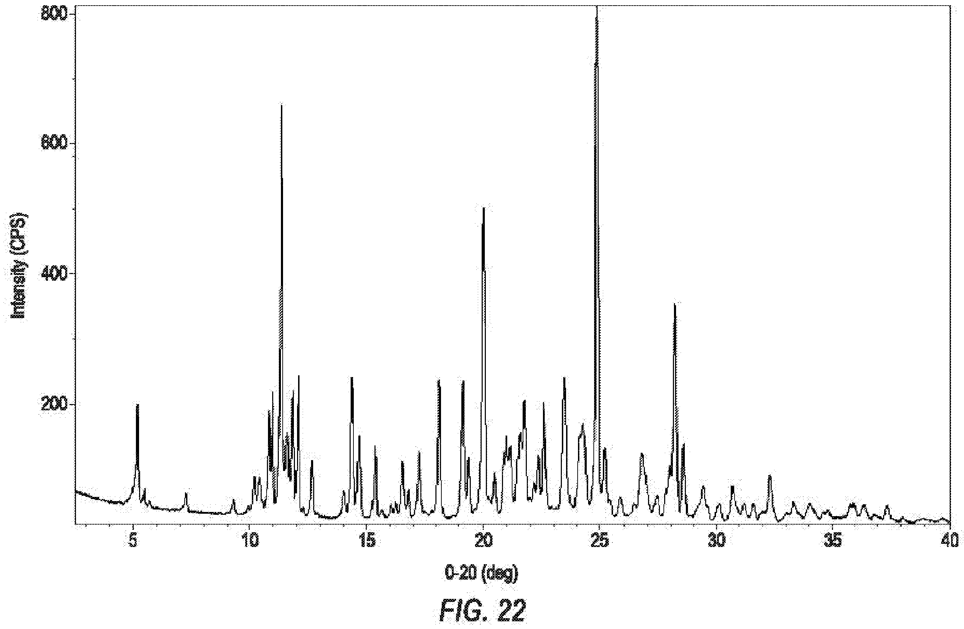

The Compound 1 Malate Form A is characterized by the XRPD pattern provided in Figure 22.

In one embodiment of this aspect, the crystalline form is Compound 1 Maleate Form A.

The Compound 1 Maleate Form A is characterized by one or more peaks in an XRPD pattern on a 2 Theta scale, wherein the one or more peaks is selected from 7.58, 8.34, 10.92, 12.38, 12.87, 14.17, 15.13, 15.78, 16.43, 16.91, 17.51, 18.09, 18.71, 20.30, 21.60, 21.99, 22.30, 22.49, 23.32, 23.56, 23.92, 24.79, 26.04, 26.41, 26.92, 27.62, 28.18, 28.54, and 29.01.

In an embodiment, the Compound 1 Maleate Form A is characterized by one or more peaks in an XRPD pattern on a 2 Theta scale, wherein the one or more peaks is selected from 10.92, 15.13, 15.78, 16.43, 18.09, 18.71, 20.30, 21.99,22.30, 22.49, 23.32, 23.56, 24.79, 26.41, 26.92, and 29.01.

In a further embodiment, the Compound 1 Maleate Form A is characterized by all of the following peaks in an XRPD pattern on a 2 Theta scale, selected from 10.92, 15.13, 15.78, 16.43, 18.09, 18.71, 20.30, 21.99, 22.30, 22.49, 23.32, 23.56, 24.79, 26.41, 26.92, and 29.01.

In still a further embodiment, the Compound 1 Maleate Form A is characterized by all of the following peaks in an XRPD pattern on a 2 Theta scale, selected from 7.58, 8.34, 10.92, 12.38, 12.87, 14.17, 15.13, 15.78, 16.43, 16.91, 17.51, 18.09, 18.71, 20.30, 21.60, 21.99, 22.30, 22.49, 23.32, 23.56, 23.92, 24.79, 26.04, 26.41, 26.92, 27.62, 28.18, 28.54, and 29.01.

In another embodiment, the Compound 1 Maleate Form A is characterized by an endotherm at a temperature of about 117 °C in a DSC thermogram.

In another embodiment, the Compound 1 Maleate Form A is characterized by a weight loss of about 3.1 wt% in the temperature range of 42-146 °C in a TGA thermogram.

In another embodiment, the Compound 1 Maleate Form A is characterized by about a 2.5 wt% increase, as measured by DVS, when taken from an environment of 5% relative humidity to an environment of 95% relative humidity.

In one embodiment of this aspect, the crystalline form is characterized as Compound 1 Maleate Form B.

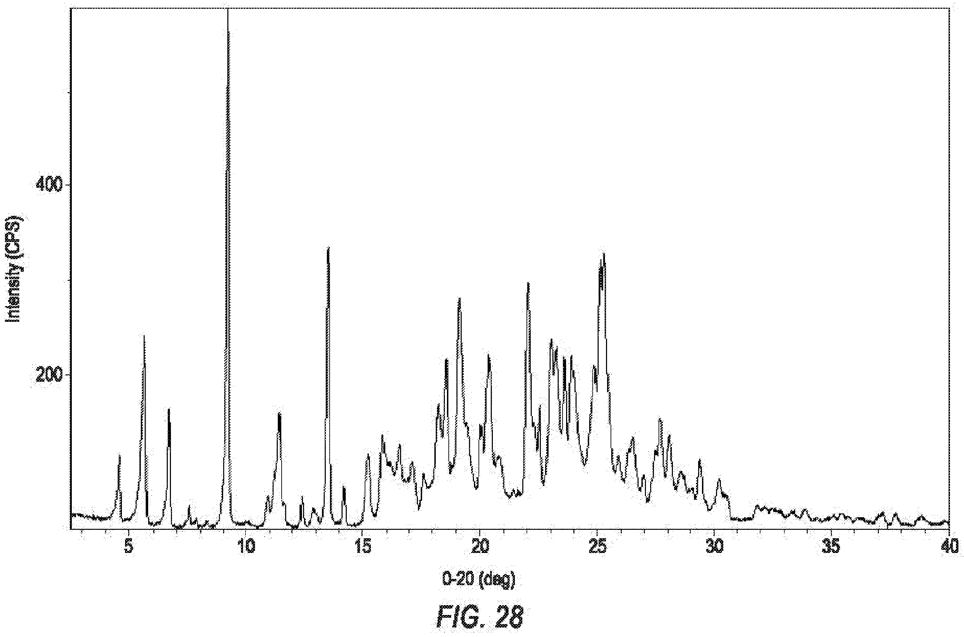

The Compound 1 Maleate Form B is characterized by the XRPD pattern provided in Figure 28.

In one embodiment of this aspect, the crystalline form is characterized as Compound 1 Mesylate Form A.

The Compound 1 Mesylate Form A is characterized by one or more peaks in an XRPD pattern on a 2 Theta scale, wherein the one or more peaks is selected from 5.06, 6.65, 7.66, 8.35, 10.16, 10.72, 11.98, 12.62, 13.32, 14.41, 14.79, 15.42, 15.72, 15.89, 16.23, 16.77, 17.20, 17.59, 18.08, 18.40, 18.81, 19.33, 19.53, 19.71, 20.14, 20.38, 20.73, 21.05, 21.56, 21.83, 22.16, 23.14, 24.06, 24.54, 24.77, 25.75, 26.82, 27.71, 27.94, 28.71, 29.27, and 29.85.

In an embodiment, the Compound 1 Mesylate Form A is characterized by one or more peaks in an XRPD pattern on a 2 Theta scale, wherein the one or more peaks is selected from 5.06, 7.66, 14.79, 15.42, 16.23, 17.20, 17.59, 18.40, 19.33, 19.53, 20.38, 20.73, 21.83, 22.16, 23.14, 24.06, and 24.77.

In a further embodiment, the Compound 1 Mesylate Form A is characterized by all of the following peaks in an XRPD pattern on a 2 Theta scale, selected from 5.06, 7.66, 14.79, 15.42, 16.23, 17.20, 17.59, 18.40, 19.33, 19.53, 20.38, 20.73, 21.83, 22.16, 23.14, 24.06, and 24.77.

In still a further embodiment, the Compound 1 Mesylate Form A is characterized by all of the following peaks in an XRPD pattern on a 2 Theta scale, selected from 5.06, 6.65, 7.66, 8.35, 10.16, 10.72, 11.98, 12.62, 13.32, 14.41, 14.79, 15.42, 15.72, 15.89, 16.23, 16.77, 17.20, 17.59, 18.08, 18.40, 18.81, 19.33, 19.53, 19.71, 20.14, 20.38, 20.73, 21.05, 21.56, 21.83, 22.16, 23.14, 24.06, 24.54, 24.77, 25.75, 26.82, 27.71, 27.94, 28.71, 29.27, and 29.85.

In one embodiment of this aspect, the crystalline form is characterized as Compound 1 Mesylate Form B.

The Compound 1 Mesylate Form B is characterized by one or more peaks in an XRPD pattern on a 2 Theta scale, wherein the one or more peaks is selected from 5.14, 6.62, 7.72, 7.89, 10.33, 10.73, 12.74, 13.31, 13.88, 14.23, 14.61, 15.21, 15.52, 15.87, 16.17, 17.18, 17.41, 17.88, 18.15, 18.46, 18.73, 19.13, 19.54, 20.15, 20.53, 20.75, 20.98, 21.27, 21.60, 22.03, 22.28, 23.07, 23.32, 24.16, 24.42, 24.87, 25.13, 25.64, 26.24, 26.95, 27.17, 27.49, 27.77, 28.42, and 29.54.

In an embodiment, the Compound 1 Mesylate Form B is characterized by one or more peaks in an XRPD pattern on a 2 Theta scale, wherein the one or more peaks is selected from 14.61, 15.21, 16.17, 17.18, 17.41, 18.46, 19.13, 19.54, 20.15, 20.53, 20.75, 20.98, 21.27, 21.60, 22.03, 23.32, 24.16, 24.42, 24.87, and 25.13.

In a further embodiment, the Compound 1 Mesylate Form B is characterized by all of the following peaks in an XRPD pattern on a 2 Theta scale, selected from 14.61, 15.21, 16.17, 17.18, 17.41, 18.46, 19.13, 19.54, 20.15, 20.53, 20.75, 20.98, 21.27, 21.60, 22.03, 23.32, 24.16, 24.42, 24.87, and 25.13.

In still a further embodiment, the Compound 1 Mesylate Form B is characterized by all of the following peaks in an XRPD pattern on a 2 Theta scale, selected from 5.14, 6.62, 7.72, 7.89, 10.33, 10.73, 12.74, 13.31, 13.88, 14.23, 14.61, 15.21, 15.52, 15.87, 16.17, 17.18, 17.41, 17.88, 18.15, 18.46, 18.73, 19.13, 19.54, 20.15, 20.53, 20.75, 20.98, 21.27, 21.60, 22.03, 22.28, 23.07, 23.32, 24.16, 24.42, 24.87, 25.13, 25.64, 26.24, 26.95, 27.17, 27.49, 27.77, 28.42, and 29.54.

In one embodiment of this aspect, the crystalline form is characterized as Compound 1 Mesylate Form C.

The Compound 1 Mesylate Form C is characterized by one or more peaks in an XRPD pattern on a 2 Theta scale, wherein the one or more peaks is selected from 7.55, 9.53, 10.25, 11.04, 11.49, 11.65, 12.24, 12.51, 13.88, 14.44, 15.36, 15.66, 16.29, 16.58, 17.16, 17.54, 19.04, 19.20, 19.79, 20.54, 21.12, 21.24, 21.62, 22.22, 23.15, 23.57, 23.99, 24.61, 24.77, 25.22, 25.61, 25.99, 26.60, 26.89, 27.29, 27.91, 29.03, and 29.29.

In an embodiment, the Compound 1 Mesylate Form C is characterized by one or more peaks in an XRPD pattern on a 2 Theta scale, wherein the one or more peaks is selected from 7.55, 9.53, 11.49, 12.51, 14.44, 19.04, 20.54, 21.12, 21.24, and 23.15.

In a further embodiment, the Compound 1 Mesylate Form C is characterized by all of the following peaks in an XRPD pattern on a 2 Theta scale, selected from 7.55, 9.53, 11.49, 12.51, 14.44, 19.04, 20.54, 21.12, 21.24, and 23.15.

In still a further embodiment, the Compound 1 Mesylate Form C is characterized by all of the following peaks in an XRPD pattern on a 2 Theta scale, selected from 7.55, 9.53, 10.25, 11.04, 11.49, 11.65, 12.24, 12.51, 13.88, 14.44, 15.36, 15.66, 16.29, 16.58, 17.16, 17.54, 19.04, 19.20, 19.79, 20.54, 21.12, 21.24, 21.62, 22.22, 23.15, 23.57, 23.99, 24.61, 24.77, 25.22, 25.61, 25.99, 26.60, 26.89, 27.29, 27.91, 29.03, and 29.29.

In another embodiment, the Compound 1 Mesylate Form C is characterized by a first endotherm from a temperature of about 96-98 °C, and a second endotherm from a temperature of about 198-203 °C in a DSC thermogram. In another embodiment, the Compound 1 Mesylate Form C is characterized by a first endotherm with a peak at a temperature of about 69 °C, wherein the first endotherm has a lower limit of a temperature of about 36 and an upper limit of a temperature of about 96 °C, and a second endotherm with a peak at a temperature of about 208 °C, wherein the second endotherm has a lower limit of a temperature of about 169 and an upper limit of a temperature of about 219 °C.

In another embodiment, the Compound 1 Mesylate Form C is characterized by a weight loss of from 0.5 to 3.0 wt% in the temperature range of 36-130 °C in a TGA thermogram.

In another embodiment, the Compound 1 Mesylate Form C is characterized by about a 5.1 wt% increase, as measured by DVS, when taken from an environment of 5% relative humidity to an environment of 95% relative humidity.

In one embodiment of this aspect, the crystalline form is characterized as Compound 1 Oxalate Form A.

The Compound 1 Oxalate Form A is characterized by one or more peaks in an XRPD pattern on a 2 Theta scale, wherein the one or more peaks is selected from 4.90, 5.25, 6.20, 8.05, 9.87, 10.54, 12.29, 14.79, 15.35, 15.84, 16.49, 16.89, 17.55, 18.27, 18.67, 19.12, 19.40, 19.81, 20.26, 20.50, 21.06, 21.74, 22.17, 22.93, 23.26, 23.50, 23.81, 24.66, 25.06, 25.95, 26.90, 27.82, and 28.24.

In an embodiment, the Compound 1 Oxalate Form A is characterized by one or more peaks in an XRPD pattern on a 2 Theta scale, wherein the one or more peaks is selected from 4.90, 5.25, 9.87, 10.54, 12.29, 15.35, 18.67, 19.40, 19.81, 20.26, 22.93, and 24.66.

In another embodiment, the Compound 1 Oxalate Form A is characterized by all of the following peaks in an XRPD pattern on a 2 Theta scale, selected from 4.90, 5.25, 9.87, 10.54, 12.29, 15.35, 18.67, 19.40, 19.81, 20.26, 22.93, and 24.66.

In a further embodiment, the Compound 1 Oxalate Form A is characterized by all of the following peaks in an XRPD pattern on a 2 Theta scale, selected from 4.90, 5.25, 6.20, 8.05, 9.87, 10.54, 12.29, 14.79, 15.35, 15.84, 16.49, 16.89, 17.55, 18.27, 18.67, 19.12, 19.40, 19.81, 20.26, 20.50, 21.06, 21.74, 22.17, 22.93, 23.26, 23.50, 23.81, 24.66, 25.06, 25.95, 26.90, 27.82, and 28.24.

In one embodiment of this aspect, the crystalline form is characterized as Compound 1 Oxalate Form B.

The Compound 1 Oxalate Form B is characterized by one or more peaks in an XRPD pattern on a 2 Theta scale, wherein the one or more peaks is selected from 4.20, 7.59, 8.02, 8.42, 10.53, 12.36, 12.66, 13.09, 13.35, 13.88, 14.15, 14.46, 15.06, 15.23, 17.42, 17.75, 18.28, 19.07, 19.52, 20.33, 21.18, 21.43, 21.74, 22.08, 22.59, 23.53, 24.21, 24.52, 24.71, 25.24, 25.85, 26.24, 26.55, 26.90, 27.43, 28.56, 29.30, and 29.51.

In an embodiment, the Compound 1 Oxalate Form B is characterized by one or more peaks in an XRPD pattern on a 2 Theta scale, wherein the one or more peaks is selected from 4.20, 7.59, 10.53, 15.06, 15.23, 19.52, 21.18, 21.43, 22.59, 24.21, and 25.85.

In a further embodiment, the Compound 1 Oxalate Form B is characterized by all of the following peaks in an XRPD pattern on a 2 Theta scale, selected from 4.20, 7.59, 10.53, 15.06, 15.23, 19.52, 21.18, 21.43, 22.59, 24.21, and 25.85.

In still a further embodiment, the Compound 1 Oxalate Form B is characterized by all of the following peaks in an XRPD pattern on a 2 Theta scale, selected from 4.20, 7.59, 8.02, 8.42, 10.53, 12.36, 12.66, 13.09, 13.35, 13.88, 14.15, 14.46, 15.06, 15.23, 17.42, 17.75, 18.28, 19.07, 19.52, 20.33, 21.18, 21.43, 21.74, 22.08, 22.59,23.53, 24.21, 24.52, 24.71, 25.24, 25.85, 26.24, 26.55, 26.90, 27.43, 28.56, 29.30, and 29.51.

In one embodiment of this aspect, the crystalline form is characterized as Compound 1 Oxalate Form C.

The Compound 1 Oxalate Form C is characterized by one or more peaks in an XRPD pattern on a 2 Theta scale, wherein the one or more peaks is selected from 4.13, 7.70, 10.29, 10.59, 13.09, 13.70, 14.13,15.33, 15.87, 16.57, 17.19, 17.50, 17.83, 18.15, 18.61, 19.24, 19.42, 20.11, 20.34, 20.64, 21.07,21.47, 21.59, 21.91, 22.56,22.92, 23.37, 23.87, 24.35, 25.35, 25.91, 26.47, 26.68, 27.74, 28.47, and 29.14.

In an embodiment, the Compound 1 Oxalate Form C is characterized by one or more peaks in an XRPD pattern on a 2 Theta scale, wherein the one or more peaks is selected from 7.70, 10.29, 10.59, 13.09, 13.70, 15.33, 17.83, 18.15, 20.11, 21.07, 21.91, and 22.56.

In a further embodiment, the Compound 1 Oxalate Form C is characterized by all of the following peaks in an XRPD pattern on a 2 Theta scale, selected from 7.70, 10.29, 10.59, 13.09, 13.70, 15.33, 17.83, 18.15, 20.11, 21.07, 21.91, and 22.56.

In still a further embodiment, the Compound 1 Oxalate Form C is characterized by all of the following peaks in an XRPD pattern on a 2 Theta scale, selected from 4.13, 7.70, 10.29, 10.59, 13.09, 13.70, 14.13,15.33, 15.87, 16.57, 17.19, 17.50, 17.83, 18.15, 18.61, 19.24, 19.42, 20.11, 20.34, 20.64, 21.07,21.47, 21.59, 21.91, 22.56,22.92, 23.37, 23.87, 24.35, 25.35, 25.91, 26.47, 26.68, 27.74, 28.47, and 29.14.

In one embodiment of this aspect, the crystalline form is characterized as Compound 1 Propionate Form A.

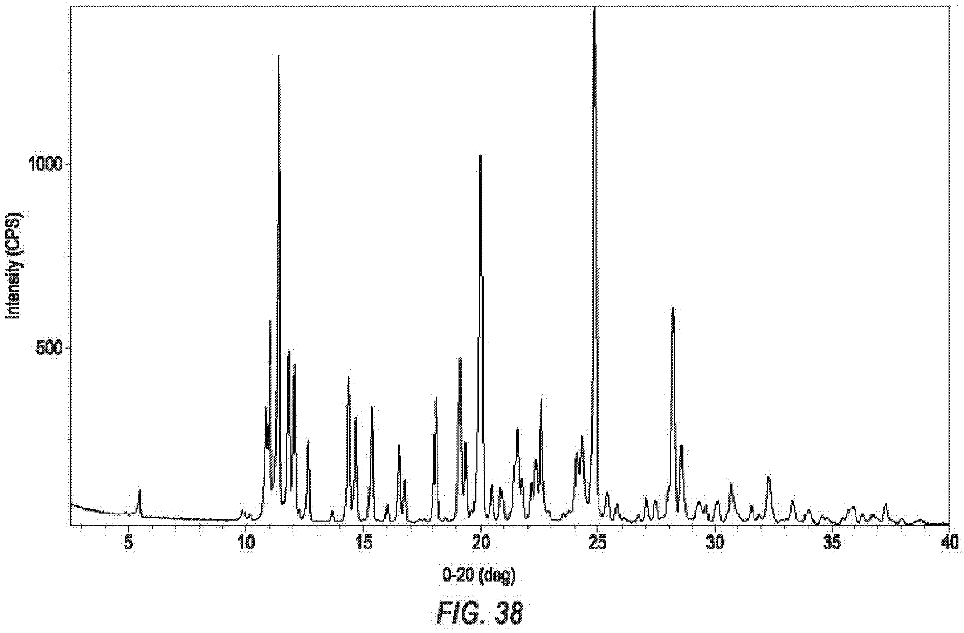

The Compound 1 Propionate Form A is characterized by the XRPD pattern provided in Figure 38.

In one embodiment of this aspect, the crystalline form is characterized as Compound 1 Succinate Form A.

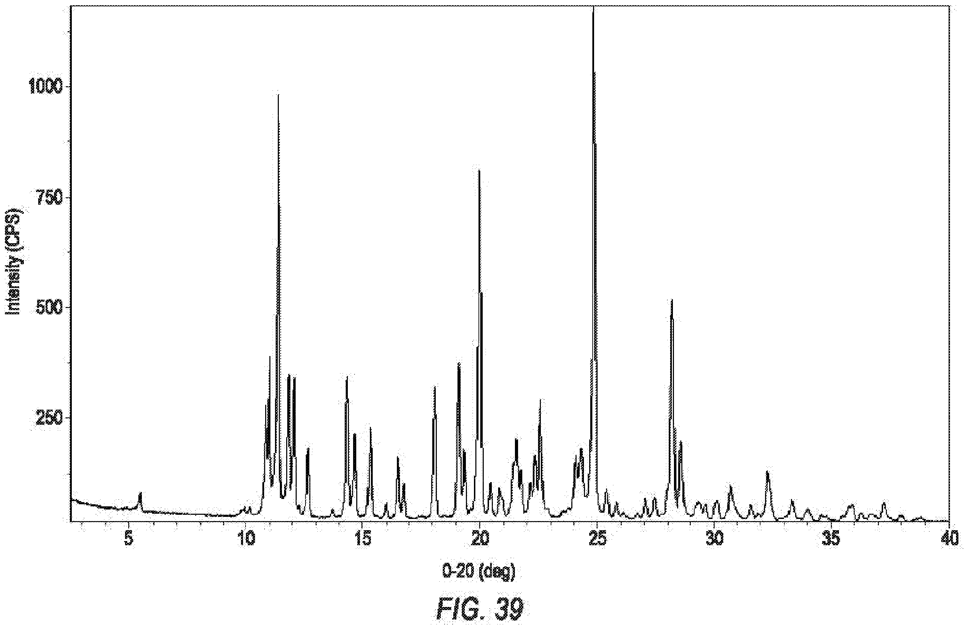

The Compound 1 Succinate Form A is characterized by the XRPD pattern provided in Figure 39.

In one embodiment of this aspect, the crystalline form is characterized as Compound 1 Sulfate Form A.

The Compound 1 Sulfate Form A is characterized by one or more peaks in an XRPD pattern on a 2 Theta scale, wherein the one or more peaks is selected from 4.74, 5.85, 9.47, 10.04, 11.74, 11.95, 13.77, 14.99, 15.42, 15.71, 16.48, 17.36, 17.66, 17.92, 18.59, 18.95, 19.36, 19.74, 20.22, 20.77, 21.38, 21.86, 22.74, 23.30, 23.70, 24.33, 25.07, 25.32, 25.67, 26.23, and 27.00.

In an embodiment, the Compound 1 Sulfate Form A is characterized by one or more peaks in an XRPD pattern on a 2 Theta scale, wherein the one or more peaks is selected from 4.74, 5.85, 9.47, 10.04, 11.74, 11.95, 16.48, 20.22, and 22.74.

In a further embodiment, the Compound 1 Sulfate Form A is characterized by all of the following peaks in an XRPD pattern on a 2 Theta scale, selected from 4.74, 5.85, 9.47, 10.04, 11.74, 11.95, 16.48, 20.22, and 22.74.

In still a further embodiment, the Compound 1 Sulfate Form A is characterized by all of the following peaks in an XRPD pattern on a 2 Theta scale, selected from 4.74, 5.85, 9.47, 10.04, 11.74, 11.95, 13.77, 14.99, 15.42, 15.71, 16.48, 17.36, 17.66, 17.92, 18.59, 18.95, 19.36, 19.74, 20.22, 20.77, 21.38, 21.86, 22.74, 23.30, 23.70, 24.33, 25.07, 25.32, 25.67, 26.23, and 27.00.

In one embodiment of this aspect, the crystalline form is characterized as Compound 1 Sulfate Form B.

The Compound 1 Sulfate Form B is characterized by one or more peaks in an XRPD pattern on a 2 Theta scale, wherein the one or more peaks is selected from 4.27, 8.57, 9.32, 10.95, 11.29, 12.07, 12.87, 13.25, 13.82, 14.13, 14.67, 16.94, 17.21, 17.64, 19.99, 20.26, 21.03, 21.44, 22.00, 22.71, 23.85, 25.20, and 26.02.

In an embodiment, the Compound 1 Sulfate Form B is characterized by one or more peaks in an XRPD pattern on a 2 Theta scale, wherein the one or more peaks is selected from 4.27, 8.57, 9.32, 16.94, 19.99, 21.03, 21.44, 22.00, and 26.02.

In a further embodiment, the Compound 1 Sulfate Form B is characterized by all of the following peaks in an XRPD pattern on a 2 Theta scale, selected from 4.27, 8.57, 9.32, 16.94, 19.99, 21.03, 21.44, 22.00, and 26.02.

In still a further embodiment, the Compound 1 Sulfate Form B is characterized by all of the following peaks in an XRPD pattern on a 2 Theta scale, selected from 4.27, 8.57, 9.32, 10.95, 11.29, 12.07, 12.87, 13.25, 13.82, 14.13, 14.67, 16.94, 17.21, 17.64, 19.99, 20.26, 21.03, 21.44, 22.00, 22.71, 23.85, 25.20, and 26.02.

In one embodiment of this aspect, the crystalline form is characterized as Compound 1 Sulfate Form C.

The Compound 1 Sulfate Form C is characterized by one or more peaks in an XRPD pattern on a 2 Theta scale, wherein the one or more peaks is selected from 3.19, 5.35, 9.04, 9.62, 10.73, 11.31, 11.60, 12.27, 12.67, 13.76, 14.31, 14.65, 15.21, 15.63, 16.11, 16.33, 16.63, 17.11, 18.32, 18.75, 18.99, 19.36, 19.47, 20.16, 20.55, 21.01, 21.52, 21.62, 21.89, 22.19, 22.53, 23.19, 23.79, 24.23, 24.81, 25.07,25.41, 26.06, 26.73, 27.58, 28.16, 28.63, 28.90, 29.22, 29.47, and 29.73.

In an embodiment, the Compound 1 Sulfate Form C is characterized by one or more peaks in an XRPD pattern on a 2 Theta scale, wherein the one or more peaks is selected from 3.19, 5.35, 9.62, 12.27, 16.33, 17.11, 18.75, 18.99, 19.36, 19.47, 20.55, 21.01, 21.52, 21.62, 21.89, 22.19, 23.79, 25.07, and 26.06.

In a further embodiment, the Compound 1 Sulfate Form C is characterized by all of the following peaks in an XRPD pattern on a 2 Theta scale, selected from 3.19, 5.35, 9.62, 12.27, 16.33, 17.11, 18.75, 18.99, 19.36, 19.47, 20.55, 21.01, 21.52, 21.62, 21.89, 22.19, 23.79, 25.07, and 26.06.

In still a further embodiment, the Compound 1 Sulfate Form C is characterized by all of the following peaks in an XRPD pattern on a 2 Theta scale, selected from 3.19, 5.35, 9.04, 9.62, 10.73, 11.31, 11.60, 12.27, 12.67, 13.76, 14.31, 14.65, 15.21, 15.63, 16.11, 16.33, 16.63, 17.11, 18.32, 18.75, 18.99, 19.36, 19.47, 20.16, 20.55,21.01, 21.52, 21.62, 21.89, 22.19, 22.53, 23.19, 23.79, 24.23, 24.81,25.07, 25.41, 26.06, 26.73,27.58, 28.16, 28.63, 28.90, 29.22, 29.47, and 29.73.

In one embodiment of this aspect, the crystalline form is characterized as Compound 1 Tartrate Form A.

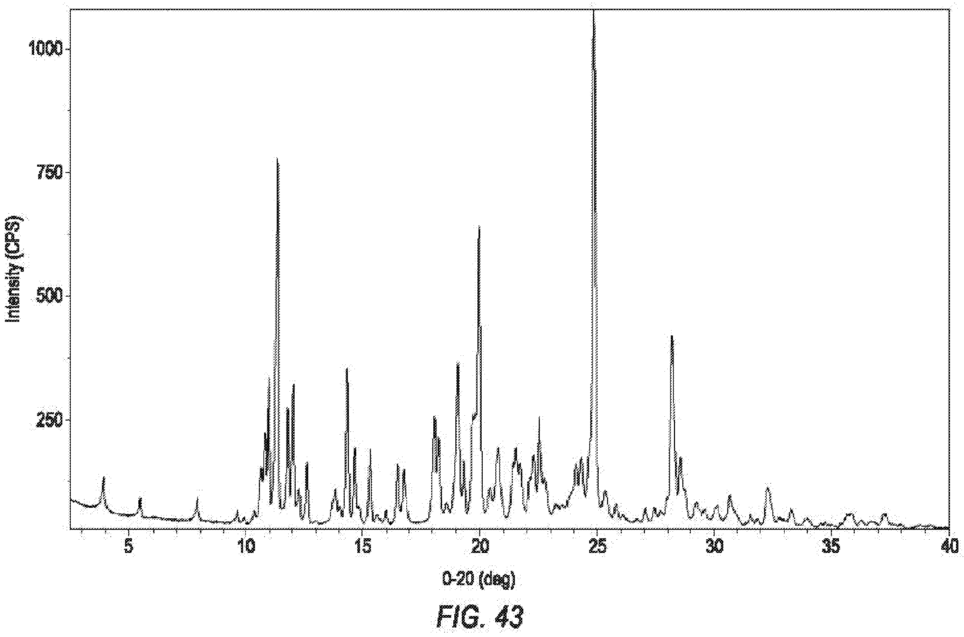

The Compound 1 Tartrate Form A is characterized by the XRPD pattern provided in Figure 43.

In one embodiment of this aspect, the crystalline form is characterized as Compound 1 Hemitartrate Form B.

The Compound 1 Hemitartrate Form B is characterized by one or more peaks in an XRPD pattern on a 2 Theta scale, wherein the one or more peaks is selected from 5.17, 5.66, 7.22, 9.27, 10.10, 10.40, 11.37, 11.55, 12.77, 13.94, 14.51, 15.60, 16.06, 16.48, 17.09, 17.69, 17.90, 18.27, 18.94, 19.15, 19.59, 19.81, 20.06, 20.32, 21.23, 21.83, 22.34, 22.84, 23.53, 24.26, 24.70, 25.26, 25.80, 26.78, 27.29, 27.90, 29.04, and 29.45.

In an embodiment, the Compound 1 Hemitartrate Form B is characterized by one or more peaks in an XRPD pattern on a 2 Theta scale, wherein the one or more peaks is selected from 5.17, 5.66, 10.10, 10.40, 11.37, 11.55, 14.51, 17.09, 21.23, 21.83, 23.53, 24.26, 25.26, and 26.78.

In a further embodiment, the Compound 1 Hemitartrate Form B is characterized by all of the following peaks in an XRPD pattern on a 2 Theta scale, selected from 5.17, 5.66, 10.10, 10.40, 11.37, 11.55, 14.51, 17.09, 21.23, 21.83, 23.53, 24.26, 25.26, and 26.78.

In still a further embodiment, the Compound 1 Hemitartrate Form B is characterized by all of the following peaks in an XRPD pattern on a 2 Theta scale, selected from 5.17, 5.66, 7.22, 9.27, 10.10, 10.40, 11.37, 11.55, 12.77, 13.94, 14.51, 15.60, 16.06, 16.48, 17.09, 17.69, 17.90, 18.27, 18.94, 19.15, 19.59, 19.81, 20.06, 20.32, 21.23, 21.83, 22.34, 22.84, 23.53, 24.26, 24.70, 25.26, 25.80, 26.78, 27.29, 27.90, 29.04, and 29.45.

In another embodiment, the Compound 1 Hemitartrate Form B is characterized by a first endotherm at a temperature of about 111 °C, and a second endotherm at a temperature of about 148 °C in a DSC thermogram.

In another embodiment, the Compound 1 Hemitartrate Form B is characterized by a weight loss of about 7.4 wt% in the temperature range of 39-157 °C in a TGA thermogram.

In one embodiment of this aspect, the crystalline form is characterized as Compound 1 Tartrate Form C.

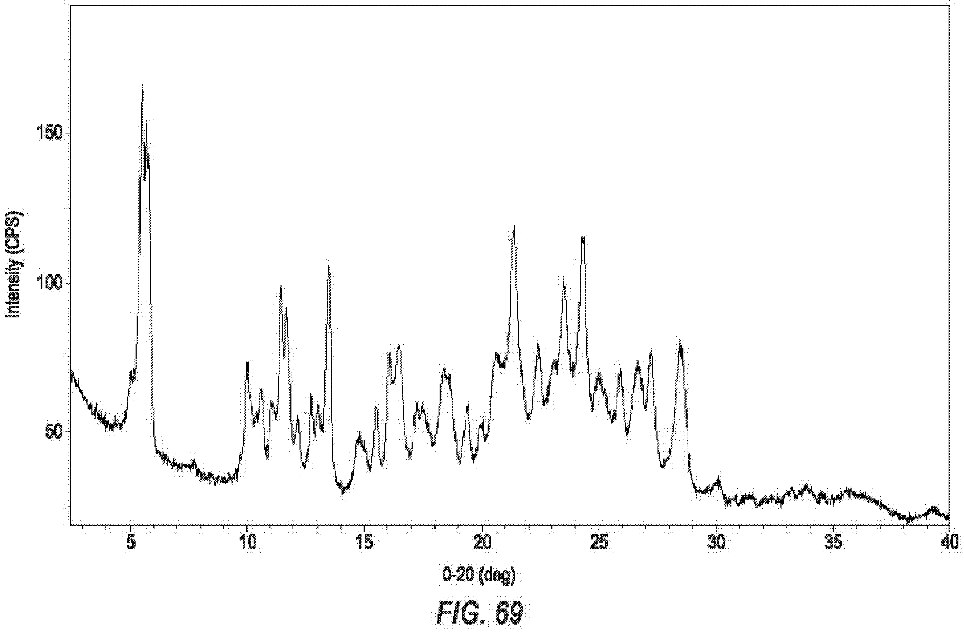

The Compound 1 Tartrate Form C is characterized by the XRPD pattern provided in Figure 69.

In one embodiment of this aspect, the crystalline form is characterized as Compound 1 Tosylate Form A.

The Compound 1 Tosylate Form A is characterized by one or more peaks in an

XRPD pattern on a 2 Theta scale, wherein the one or more peaks is selected from 5.81, 7.05, 8.52, 9.74, 10.40, 10.99, 11.26, 11.66, 12.58, 12.81, 14.28, 14.62, 15.30, 15.80, 16.03, 16.34, 17.12, 17.55, 18.03, 18.94, 19.26, 19.67, 20.31, 20.65,21.02, 21.43, 22.11, 22.44, 22.66, 22.94, 23.07, 23.54, 23.79, 24.55,24.88, 25.42, 25.83, 26.10,26.53, 27.70, 28.11, 28.62, 29.18, 29.40, 29.66, and 29.83.

In an embodiment, the Compound 1 Tosylate Form A is characterized by one or more peaks in an XRPD pattern on a 2 Theta scale, wherein the one or more peaks is selected from 8.52, 9.74, 14.28, 16.03, 17.55, 18.94, 19.26, 20.31, 21.02, 21.43, 22.11, 22.44, 22.66, 23.07, 24.55, and 27.70.

In a further embodiment, the Compound 1 Tosylate Form A is characterized by all of the following peaks in an XRPD pattern on a 2 Theta scale, selected from 8.52, 9.74, 14.28, 16.03, 17.55, 18.94, 19.26,20.31, 21.02, 21.43, 22.11, 22.44, 22.66, 23.07, 24.55, and 27.70.

In still a further embodiment, the Compound 1 Tosylate Form A is characterized by all of the following peaks in an XRPD pattern on a 2 Theta scale, selected from 5.81, 7.05, 8.52, 9.74, 10.40, 10.99, 11.26, 11.66, 12.58, 12.81, 14.28, 14.62, 15.30, 15.80, 16.03, 16.34, 17.12, 17.55, 18.03, 18.94, 19.26, 19.67, 20.31, 20.65, 21.02, 21.43, 22.11, 22.44, 22.66, 22.94, 23.07, 23.54, 23.79, 24.55, 24.88, 25.42, 25.83, 26.10, 26.53, 27.70, 28.11, 28.62, 29.18, 29.40, 29.66, and 29.83.

In another embodiment, the Compound 1 Tosylate Form A is characterized by an endotherm at a temperature of about 214 °C in a DSC thermogram. In another embodiment, the Compound 1 Tosylate Form A is characterized by an endotherm with an onset temperature at about 214 °C in a DSC thermogram.

In another embodiment, the Compound 1 Tosylate Form A is characterized by a weight loss of about 0.9 wt% in the temperature range of 72-231 °C in a TGA thermogram.

In another embodiment, the Compound 1 Tosylate Form A is characterized by about a 0.9 wt% increase, as measured by DVS, when taken from an environment of 5% relative humidity to an environment of 95% relative humidity.

In one embodiment of this aspect, the crystalline form is characterized as Compound 1 Tosylate Form B.

The Compound 1 Tosylate Form B is characterized by one or more peaks in an XRPD pattern on a 2 Theta scale, wherein the one or more peaks is selected from 4.61, 8.69, 9.25, 10.68, 12.44, 12.69, 13.76, 14.42, 14.81, 15.47, 16.08, 16.57, 17.45, 17.76, 18.44, 18.83, 19.01, 19.35, 20.42, 20.84, 21.15, 21.69, 22.09, 22.86, 23.12, 24.08, 24.53, 24.86, 25.22, 25.91, 26.33, 26.82, 27.63, 28.61, 29.11, and 29.77.

In an embodiment, the Compound 1 Tosylate Form B is characterized by one or more peaks in an XRPD pattern on a 2 Theta scale, wherein the one or more peaks is selected from 4.61, 9.25, 12.44, 15.47, 17.76, 19.01, 19.35, 25.91, 26.33, and 27.63.

In a further embodiment, the Compound 1 Tosylate Form B is characterized by all of the following peaks in an XRPD pattern on a 2 Theta scale, selected from 4.61, 9.25, 12.44, 15.47, 17.76, 19.01, 19.35, 25.91, 26.33, and 27.63.

In still a further embodiment, the Compound 1 Tosylate Form B is characterized by all of the following peaks in an XRPD pattern on a 2 Theta scale, selected from 4.61, 8.69, 9.25, 10.68, 12.44, 12.69, 13.76, 14.42, 14.81, 15.47, 16.08, 16.57, 17.45, 17.76, 18.44, 18.83, 19.01, 19.35, 20.42, 20.84, 21.15, 21.69, 22.09, 22.86, 23.12, 24.08, 24.53, 24.86, 25.22, 25.91, 26.33, 26.82, 27.63, 28.61, 29.11, and 29.77.

In another embodiment, the Compound 1 Tosylate Form B is characterized by a sharp endotherm at a temperature of ~183 °C in a DSC thermogram. In another embodiment, the Compound 1 Tosylate Form B is characterized by a sharp endotherm with an onset temperature at -183 °C in a DSC thermogram. In another embodiment, the Compound 1 Tosylate Form B is characterized by a sharp endotherm with a peak at a temperature of -191 °C in a DSC thermogram.

In another embodiment, the Compound 1 Tosylate Form B is characterized by a weight loss of about 0.7 wt% in the temperature range of 39-214 °C in a TGA thermogram.

In another embodiment, the Compound 1 Tosylate Form B is characterized by about a 0.9 wt% increase, as measured by DVS, when taken from an environment of 5% relative humidity to an environment of 95% relative humidity.

In one aspect, the invention includes a pharmaceutical composition comprising a crystalline salt form described herein and a pharmaceutically acceptable excipient.

In another aspect, which is not claimed, the disclosure includes a method of treating a disease, disorder, or syndrome mediated at least in part by modulating *in vivo* activity of a protein kinase, comprising administering to a subject in need thereof a crystalline salt form or pharmaceutical composition described herein.

In one instance of this aspect, the disease, disorder, or syndrome mediated at least in part by modulating *in vivo* activity of a protein kinase is cancer.

In another aspect, which is not claimed, the disclosure includes a method for inhibiting a protein kinase, the method comprising contacting the protein kinase with a crystalline salt form or pharmaceutical composition described herein.

In one instance of this aspect, the protein kinase is Axl, Mer, c-Met, KDR, or a combination thereof.

### Crystalline forms of the Invention

### Compound 1 Besylate Form A

Compound 1 Besylate Form A is a 1:1 salt of Compound 1 and benzenesulfonic acid, and further includes one molar equivalent of acetone in the crystal lattice.

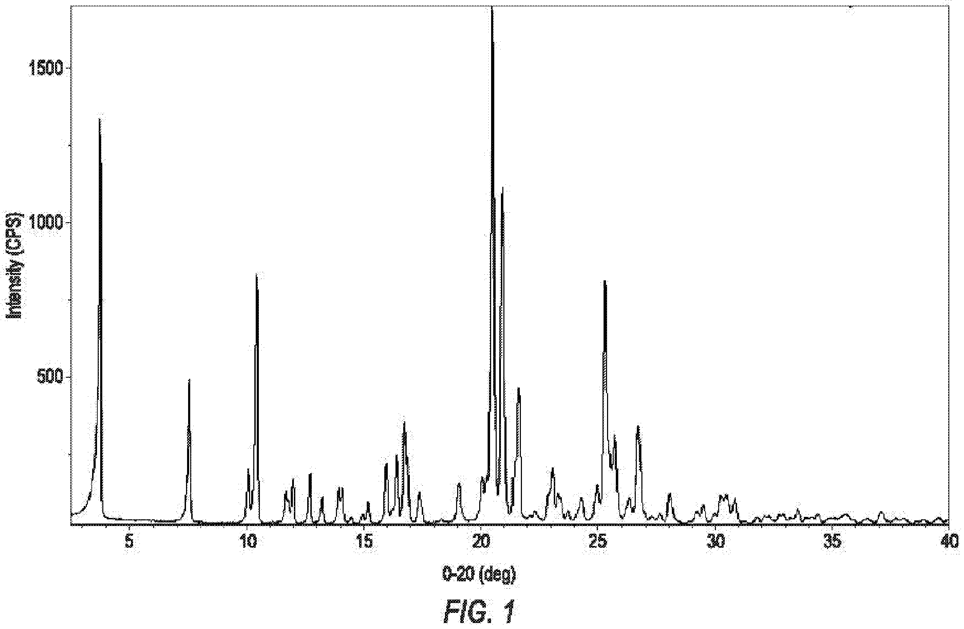

The XRPD pattern for Compound 1 Besylate Form A is provided in Figure 1, and a list of peaks from the pattern is provided in Table 1 below.

**Table 1: XRPD peaks of Compound 1 Besylate Form A**

| **2*θ* (°)** | ***d*-spacing (Å)** | **Intensity (%)** |
|---|---|---|
| 3.78 ± 0.20 | 23.386 ± 1.238 | 78 |
| 7.56 ± 0.20 | 11.677 ± 0.308 | 28 |
| 10.08 ± 0.20 | 8.766 ± 0.173 | 12 |
| 10.42 ± 0.20 | 8.480 ± 0.162 | 49 |
| 11.70 ± 0.20 | 7.561 ± 0.129 | 8 |
| 11.78 ± 0.20 | 7.506 ± 0.127 | 6 |
| 12.00 ± 0.20 | 7.369 ± 0.122 | 10 |
| 12.71 ± 0.20 | 6.958 ± 0.109 | 11 |
| 13.20 ± 0.20 | 6.702 ± 0.101 | 7 |
| 13.92 ± 0.20 | 6.358 ± 0.091 | 8 |
| 14.06 ± 0.20 | 6.294 ± 0.089 | 8 |
| 14.44 ± 0.20 | 6.128 ± 0.084 | 3 |
| 14.93 ± 0.20 | 5.928 ± 0.079 | 3 |
| 15.18 ± 0.20 | 5.833 ± 0.076 | 6 |
| 15.96 ± 0.20 | 5.547 ± 0.069 | 13 |
| 16.21 ± 0.20 | 5.465 ± 0.067 | 4 |
| 16.40 ± 0.20 | 5.401 ± 0.065 | 15 |
| 16.73 ± 0.20 | 5.295 ± 0.063 | 21 |
| 16.86 ± 0.20 | 5.254 ± 0.062 | 14 |
| 17.38 ± 0.20 | 5.099 ± 0.058 | 7 |
| 18.32 ± 0.20 | 4.840 ± 0.052 | 2 |
| 19.07 ± 0.20 | 4.650 ± 0.048 | 9 |
| 20.07 ± 0.20 | 4.420 ± 0.044 | 10 |
| 20.26 ± 0.20 | 4.380 ± 0.043 | 10 |
| 20.51 ± 0.20 | 4.327 ± 0.042 | 100 |
| 20.94 ± 0.20 | 4.238 ± 0.040 | 66 |
| 21.45 ± 0.20 | 4.139 ± 0.038 | 14 |
| 21.60 ± 0.20 | 4.112 ± 0.038 | 27 |
| 22.30 ± 0.20 | 3.984 ± 0.035 | 4 |
| 22.90 ± 0.20 | 3.880 ± 0.033 | 8 |
| 23.05 ± 0.20 | 3.856 ± 0.033 | 12 |
| 23.28 ± 0.20 | 3.818 ± 0.032 | 7 |
| 23.71 ± 0.20 | 3.750 ± 0.031 | 4 |
| 24.28 ± 0.20 | 3.662 ± 0.030 | 6 |
| 24.97 ± 0.20 | 3.564 ± 0.028 | 9 |
| 25.30 ± 0.20 | 3.518 ± 0.027 | 48 |
| 25.69 ± 0.20 | 3.464 ± 0.027 | 18 |
| 26.33 ± 0.20 | 3.382 ± 0.025 | 6 |
| 26.71 ± 0.20 | 3.335 ± 0.025 | 20 |
| 27.29 ± 0.20 | 3.265 ± 0.023 | 3 |
| 27.65 ± 0.20 | 3.224 ± 0.023 | 3 |
| 28.07 ± 0.20 | 3.176 ± 0.022 | 7 |
| 29.22 ± 0.20 | 3.054 ± 0.020 | 4 |
| 29.50 ± 0.20 | 3.025 ± 0.020 | 5 |

The XRPD pattern for Compound 1 Beslylate Form A was successfully indexed, suggesting the material consists primarily or exclusively of a single crystalline phase.

### Unit cell data for Compound 1 Besylate Form A:

| Bravais Type | Triclinic |
|---|---|
| a [Å] | 8.913 |
| b [Å] | 9.121 |
| c [Å] | 23.727 |
| α [deg] | 93.17 |
| β [deg] | 98.70 |
| γ [deg] | 105.12 |
| Volume [Å³/cell] | 1,831.6 |
| Chiral Contents? | Not Specified |
| Extinction Symbol | P - |
| Space Group(s) | P1 (1), PT (2) |

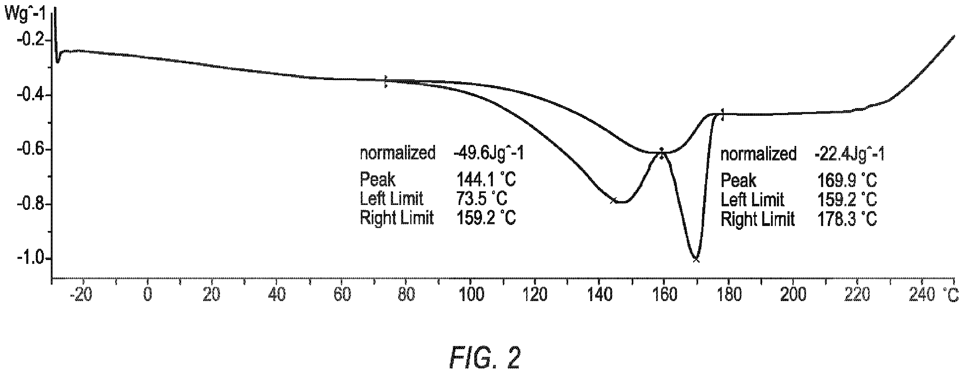

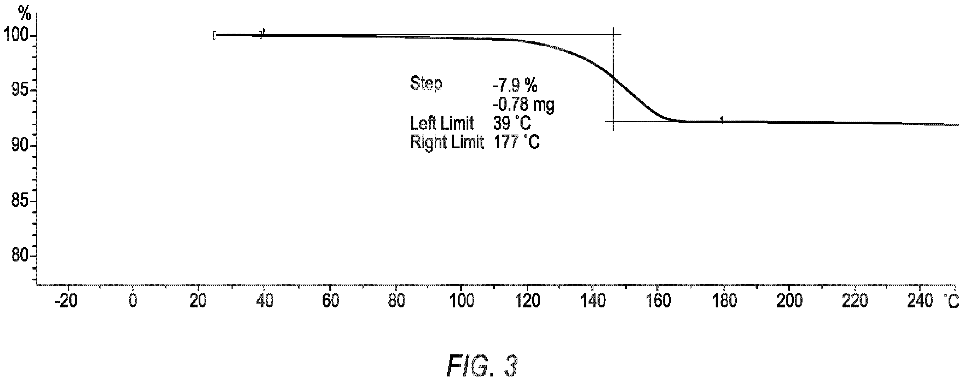

DSC and TGA thermograms for Compound 1 Besylate Form A are provided in Figures 2 and 3, respectively. A weight loss of ∼7.9% over the temperatures of 39-177 °C by TGA corresponds to 1 molar equivalent of acetone. DSC shows two broad endotherms at ∼144 °C and -170 °C.

### Compound 1 Besylate Form B

Compound 1 Besylate Form B is the resulting material after desolvation of Compound 1 Besylate Form A. It was detected as a mixture with Besylate Form A.

The XRPD pattern for Compound 1 Besylate Form B is provided in Figure 4.

### Compound 1 Benzoate Form A

Compound 1 Benzoate Form A is a 1:1 salt, formed by adding benzoic acid to Compound 1 in hot methanol.

The XRPD pattern for Compound 1 Benzoate Form A is provided in Figure 5.

### Compound 1 Camsylate Form A

Compound 1 Camsylate Form A is a 1:1 salt of Compound 1 and camphorsulfonic acid, and further includes one molar equivalent of acetone in the crystal lattice.

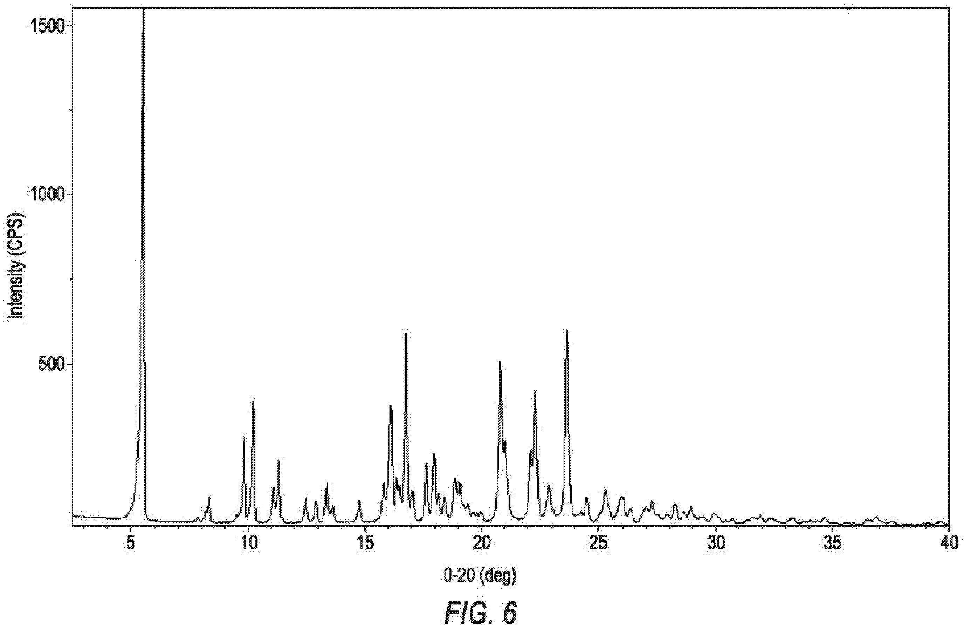

The XRPD pattern for Compound 1 Camsylate Form A is provided in Figure 6, and a list of peaks from the pattern is provided in Table 2 below.

**Table 2: XRPD peaks of Compound 1 Camsylate Form A**

| **2*θ* (°)** | ***d*-spacing (Å)** | **Intensity (%)** |
|---|---|---|
| 5.55 ± 0.20 | 15.916 ± 0.573 | 100 |
| 7.90 ± 0.20 | 11.179 ± 0.282 | 3 |
| 8.25 ± 0.20 | 10.704 ± 0.259 | 4 |
| 8.38 ± 0.20 | 10.545 ± 0.251 | 7 |
| 9.55 ± 0.20 | 9.250 ± 0.193 | 4 |
| 9.87 ± 0.20 | 8.951 ± 0.181 | 18 |
| 10.26 ± 0.20 | 8.617 ± 0.168 | 25 |
| 11.12 ± 0.20 | 7.951 ± 0.143 | 9 |
| 11.34 ± 0.20 | 7.800 ± 0.137 | 14 |
| 12.50 ± 0.20 | 7.075 ± 0.113 | 7 |
| 12.94 ± 0.20 | 6.837 ± 0.105 | 6 |
| 13.40 ± 0.20 | 6.601 ± 0.098 | 10 |
| 13.65 ± 0.20 | 6.480 ± 0.094 | 5 |
| 14.79 ± 0.20 | 5.986 ± 0.081 | 6 |
| 15.83 ± 0.20 | 5.593 ± 0.070 | 10 |
| 16.16 ± 0.20 | 5.480 ± 0.067 | 25 |
| 16.41 ± 0.20 | 5.397 ± 0.065 | 11 |
| 16.54 ± 0.20 | 5.354 ± 0.064 | 9 |
| 16.82 ± 0.20 | 5.266 ± 0.062 | 38 |
| 17.10 ± 0.20 | 5.180 ± 0.060 | 8 |
| 17.69 ± 0.20 | 5.010 ± 0.056 | 13 |
| 18.02 ± 0.20 | 4.918 ± 0.054 | 15 |
| 18.22 ± 0.20 | 4.866 ± 0.053 | 8 |
| 18.47 ± 0.20 | 4.799 ± 0.052 | 7 |
| 18.91 ± 0.20 | 4.689 ± 0.049 | 11 |
| 19.10 ± 0.20 | 4.643 ± 0.048 | 10 |
| 19.47 ± 0.20 | 4.556 ± 0.046 | 6 |
| 20.04 ± 0.20 | 4.427 ± 0.044 | 4 |
| 20.87 ± 0.20 | 4.254 ± 0.040 | 33 |
| 21.05 ± 0.20 | 4.218 ± 0.040 | 18 |
| 22.16 ± 0.20 | 4.008 ± 0.036 | 16 |
| 22.36 ± 0.20 | 3.973 ± 0.035 | 27 |
| 22.93 ± 0.20 | 3.876 ± 0.033 | 9 |
| 23.71 ± 0.20 | 3.750 ± 0.031 | 38 |
| 24.55 ± 0.20 | 3.623 ± 0.029 | 7 |
| 25.33 ± 0.20 | 3.513 ± 0.027 | 8 |
| 26.02 ± 0.20 | 3.421 ± 0.026 | 7 |
| 26.40 ± 0.20 | 3.373 ± 0.025 | 5 |
| 27.09 ± 0.20 | 3.289 ± 0.024 | 5 |
| 27.33 ± 0.20 | 3.260 ± 0.023 | 6 |
| 27.97 ± 0.20 | 3.187 ± 0.022 | 4 |
| 28.32 ± 0.20 | 3.149 ± 0.022 | 6 |
| 28.68 ± 0.20 | 3.110 ± 0.021 | 4 |
| 28.98 ± 0.20 | 3.078 ± 0.021 | 5 |

The XRPD pattern was successfully indexed.

### Unit cell data for Compound 1 Camsylate Form A

| Bravais Type | Primitive Monoclinic |
|---|---|
| a [Å] | 11.413 |
| b [Å] | 31.799 |
| c [Å] | 11.622 |
| α [deg] | 90 |
| β [deg] | 101.91 |
| γ [deg] | 90 |
| Volume [Å³/cell] | 4,127.1 |
| Chiral Contents? | Chiral |
| Extinction Symbol | P 1 2₁ 1 |
| Space Group(s) | P2₁ (4) |

A proton NMR spectrum for Compound 1 Camsylate Form A is consistent with the chemical structure of Compound 1 with 1 molar equivalent of acetone present.

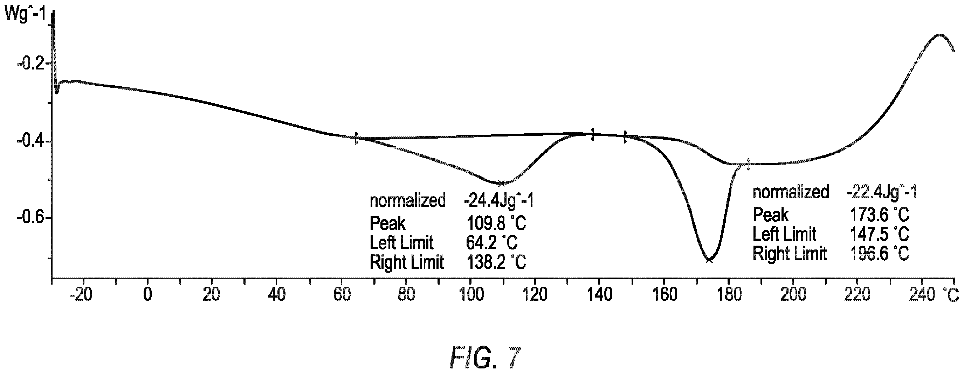

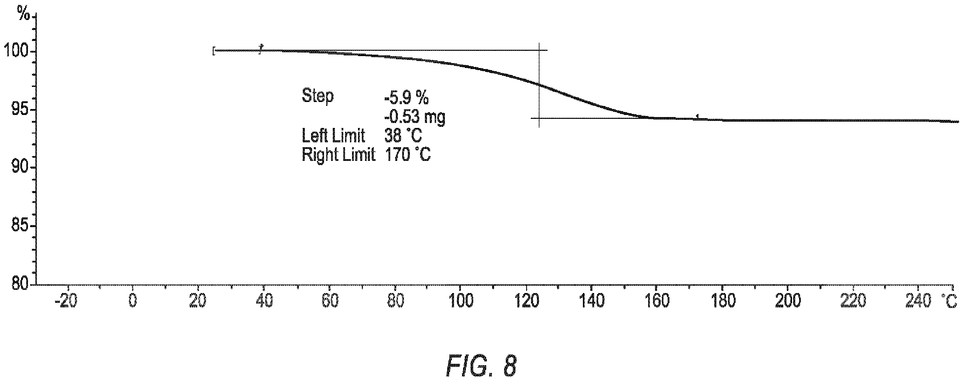

TGA analysis indicates that the material readily desolvates upon heating, losing 5.9 wt% between 38 °C and 170 °C (Figure 8), which corresponds to one molar equivalent of acetone. DSC thermogram shows two broad endotherms at 110 °C and 174 °C (Figure 7).

### Compound 1 Camsylate Form B

Compound 1 Camsylate Form B is the resulting material after desolvation of Compound 1 Camsylate Form A.

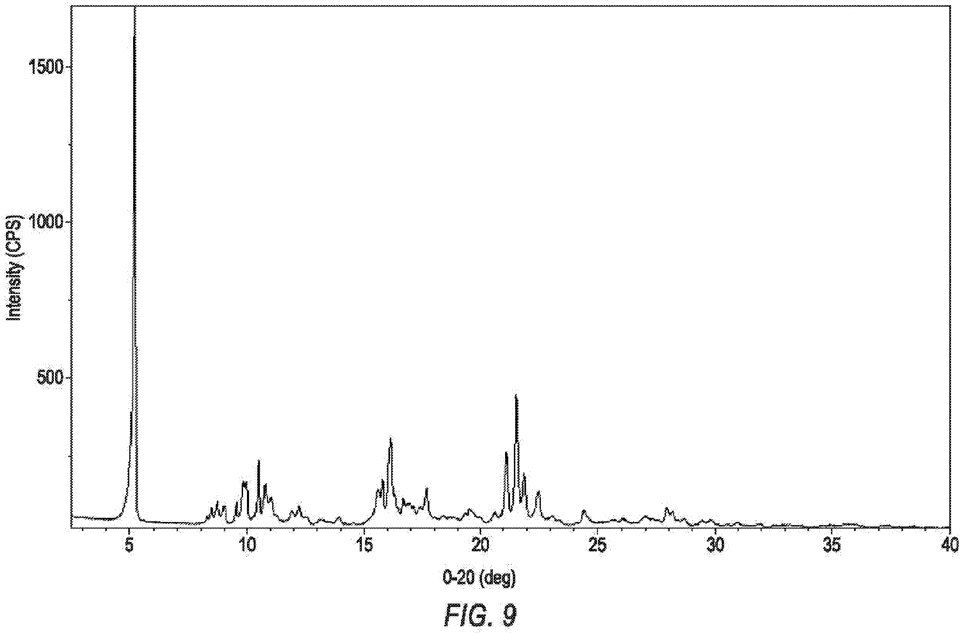

The XRPD pattern for Compound 1 Camsylate Form B is provided in Figure 9, and a list of peaks from the pattern is provided in Table 3 below.

**Table 3: XRPD peaks of Compound 1 Camsylate Form B**

| **2*θ* (°)** | ***d*-spacing (Å)** | **Intensity (%)** |
|---|---|---|
| 5.25 ± 0.20 | 16.830 ± 0.641 | 100 |
| 8.32 ± 0.20 | 10.620 ± 0.255 | 3 |
| 8.51 ± 0.20 | 10.376 ± 0.243 | 5 |
| 8.76 ± 0.20 | 10.084 ± 0.230 | 6 |
| 9.05 ± 0.20 | 9.765 ± 0.215 | 5 |
| 9.58 ± 0.20 | 9.221 ± 0.192 | 6 |
| 9.88 ± 0.20 | 8.942 ± 0.180 | 10 |
| 10.00 ± 0.20 | 8.838 ± 0.176 | 10 |
| 10.52 ± 0.20 | 8.406 ± 0.159 | 14 |
| 10.80 ± 0.20 | 8.182 ± 0.151 | 10 |
| 11.06 ± 0.20 | 7.997 ± 0.144 | 7 |
| 11.96 ± 0.20 | 7.395 ± 0.123 | 4 |
| 12.24 ± 0.20 | 7.225 ± 0.118 | 6 |
| 12.58 ± 0.20 | 7.029 ± 0.111 | 3 |
| 13.98 ± 0.20 | 6.331 ± 0.090 | 3 |
| 15.62 ± 0.20 | 5.669 ± 0.072 | 9 |
| 15.81 ± 0.20 | 5.602 ± 0.070 | 10 |
| 16.15 ± 0.20 | 5.483 ± 0.067 | 18 |
| 16.70 ± 0.20 | 5.303 ± 0.063 | 7 |
| 17.12 ± 0.20 | 5.175 ± 0.060 | 5 |
| 17.42 ± 0.20 | 5.086 ± 0.058 | 5 |
| 17.71 ± 0.20 | 5.005 ± 0.056 | 9 |
| 18.41 ± 0.20 | 4.816 ± 0.052 | 4 |
| 19.37 ± 0.20 | 4.579 ± 0.047 | 4 |
| 19.55 ± 0.20 | 4.538 ± 0.046 | 5 |
| 20.63 ± 0.20 | 4.302 ± 0.041 | 4 |
| 21.13 ± 0.20 | 4.200 ± 0.039 | 15 |
| 21.56 ± 0.20 | 4.118 ± 0.038 | 27 |
| 21.89 ± 0.20 | 4.057 ± 0.037 | 12 |
| 22.50 ± 0.20 | 3.949 ± 0.035 | 8 |
| 23.04 ± 0.20 | 3.857 ± 0.033 | 3 |
| 24.43 ± 0.20 | 3.641 ± 0.029 | 5 |
| 26.08 ± 0.20 | 3.413 ± 0.026 | 3 |
| 27.07 ± 0.20 | 3.292 ± 0.024 | 3 |
| 27.97 ± 0.20 | 3.187 ± 0.022 | 5 |
| 28.22 ± 0.20 | 3.160 ± 0.022 | 4 |
| 28.73 ± 0.20 | 3.105 ± 0.021 | 3 |

### Compound 1 Citrate Form A

Compound 1 Citrate Form A was crystallized from a 1:1 mixture of Compound 1 and citric acid in acetone.

The XRPD pattern for Compound 1 Citrate Form A shows that the material is a mixture with a free base form of Compound 1. The pattern is provided in Figure 10.

### Compound 1 Citrate Form B

Compound 1 Citrate Form B is a 1:1 salt, provided by adding 2 molar equivalents of citric acid in acetone to Compound 1 to provide a 1:1 salt of Compound 1 and citric acid.

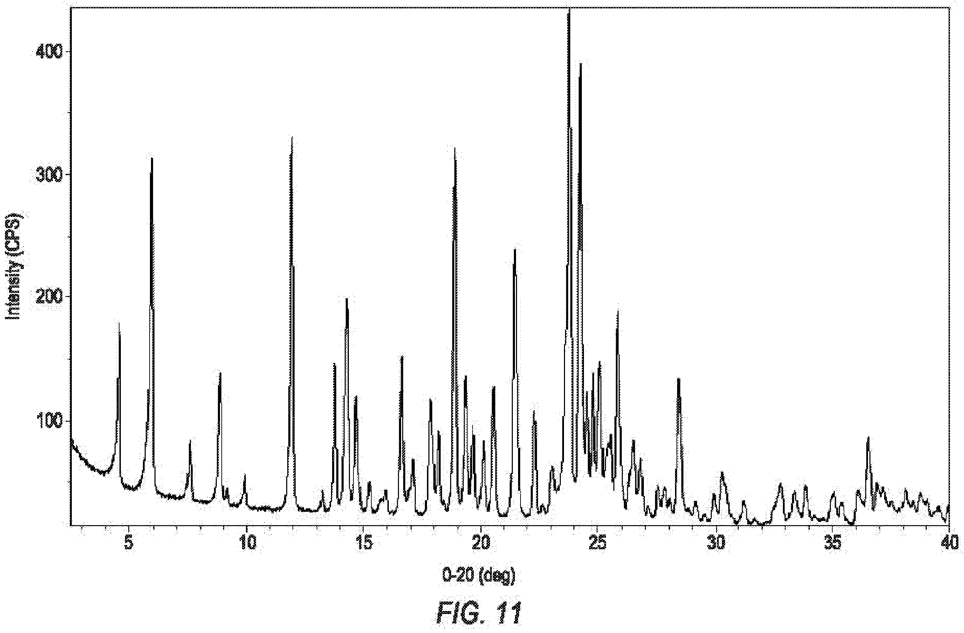

The XRPD pattern for Compound 1 Citrate Form B is provided in Figure 11, and the XRPD pattern was also successfully indexed. A list of peaks from the pattern is provided in Table 4 below.

**Table 4: XRPD peaks of Compound 1 Citrate Form B**

| **2*θ* (°)** | ***d*-spacing (Å)** | **Intensity (%)** |
|---|---|---|
| 4.58 ± 0.20 | 19.296 ± 0.843 | 40 |
| 5.95 ± 0.20 | 14.840 ± 0.498 | 72 |
| 7.61 ± 0.20 | 11.607 ± 0.305 | 19 |
| 8.88 ± 0.20 | 9.945 ± 0.223 | 32 |
| 9.19 ± 0.20 | 9.619 ± 0.209 | 10 |
| 9.94 ± 0.20 | 8.894 ± 0.179 | 12 |
| 11.93 ± 0.20 | 7.409 ± 0.124 | 76 |
| 13.26 ± 0.20 | 6.673 ± 0.100 | 10 |
| 13.79 ± 0.20 | 6.415 ± 0.093 | 33 |
| 14.29 ± 0.20 | 6.193 ± 0.086 | 46 |
| 14.68 ± 0.20 | 6.028 ± 0.082 | 27 |
| 15.26 ± 0.20 | 5.801 ± 0.076 | 11 |
| 15.95 ± 0.20 | 5.553 ± 0.069 | 10 |
| 16.64 ± 0.20 | 5.323 ± 0.064 | 35 |
| 17.11 ± 0.20 | 5.177 ± 0.060 | 16 |
| 17.87 ± 0.20 | 4.959 ± 0.055 | 27 |
| 18.21 ± 0.20 | 4.867 ± 0.053 | 21 |
| 18.43 ± 0.20 | 4.811 ± 0.052 | 8 |
| 18.90 ± 0.20 | 4.692 ± 0.049 | 74 |
| 19.36 ± 0.20 | 4.582 ± 0.047 | 31 |
| 19.67 ± 0.20 | 4.510 ± 0.045 | 22 |
| 20.14 ± 0.20 | 4.406 ± 0.043 | 19 |
| 20.57 ± 0.20 | 4.315 ± 0.042 | 29 |
| 21.47 ± 0.20 | 4.135 ± 0.038 | 55 |
| 22.30 ± 0.20 | 3.984 ± 0.035 | 25 |
| 22.64 ± 0.20 | 3.925 ± 0.034 | 7 |
| 23.06 ± 0.20 | 3.853 ± 0.033 | 14 |
| 23.80 ± 0.20 | 3.736 ± 0.031 | 100 |
| 24.25 ± 0.20 | 3.667 ± 0.030 | 89 |
| 24.55 ± 0.20 | 3.623 ± 0.029 | 28 |
| 24.81 ± 0.20 | 3.586 ± 0.028 | 32 |
| 25.10 ± 0.20 | 3.545 ± 0.028 | 34 |
| 25.45 ± 0.20 | 3.497 ± 0.027 | 18 |
| 25.54 ± 0.20 | 3.485 ± 0.027 | 20 |
| 25.85 ± 0.20 | 3.444 ± 0.026 | 43 |
| 26.35 ± 0.20 | 3.379 ± 0.025 | 13 |
| 26.51 ± 0.20 | 3.359 ± 0.025 | 19 |
| 26.83 ± 0.20 | 3.320 ± 0.024 | 16 |
| 27.16 ± 0.20 | 3.281 ± 0.024 | 7 |
| 27.57 ± 0.20 | 3.232 ± 0.023 | 11 |
| 27.85 ± 0.20 | 3.201 ± 0.023 | 10 |
| 28.08 ± 0.20 | 3.176 ± 0.022 | 8 |
| 28.47 ± 0.20 | 3.132 ± 0.022 | 31 |

### Unit cell data for Compound 1 Citrate Form B

| Bravais Type | Primitive Orthorhombic |
|---|---|
| a [Å] | 38.495 |
| b [Å] | 23.202 |
| c [Å] | 7.473 |
| a [deg] | 90 |
| β [deg] | 90 |
| γ [deg] | 90 |
| Volume [Å³/cell] | 6,674.6 |
| Chiral Contents? | Achiral |
| Extinction Symbol | P b c a |
| Space Group(s) | Pbca (61) |

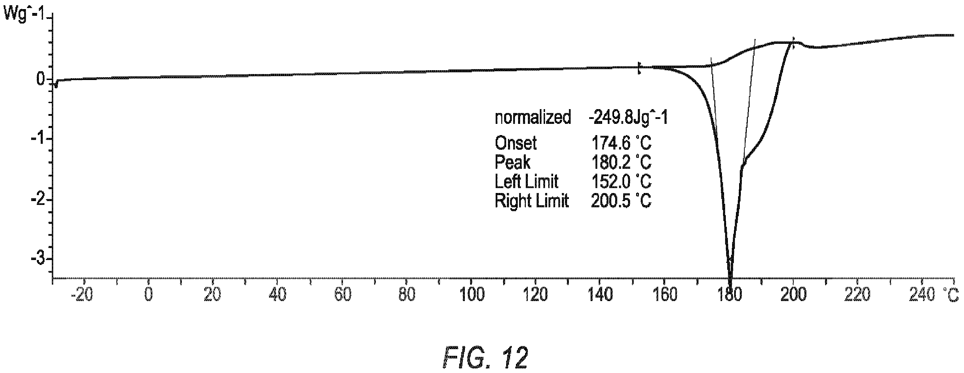

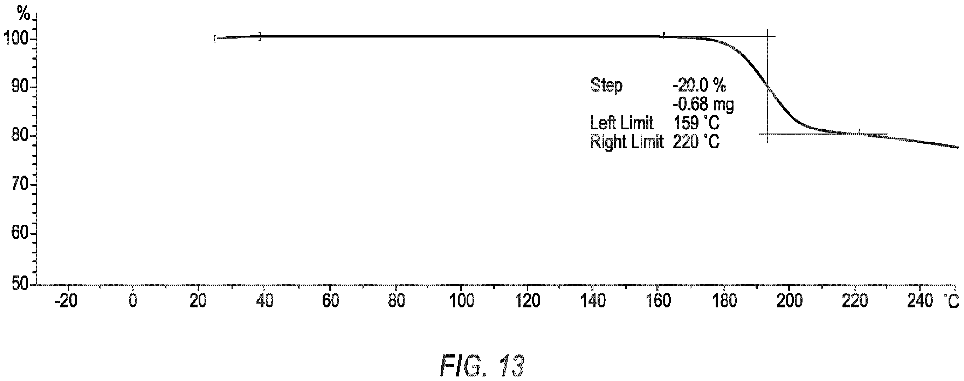

The TGA thermogram for Compound 1 Citrate Form B (Figure 13) shows negligible weight loss until decomposition beginning at about 159 °C and ending at about 220 °C. The DSC thermogram (Figure 12) also shows a sharp endotherm with an onset at ~175 °C, indicating decomposition.

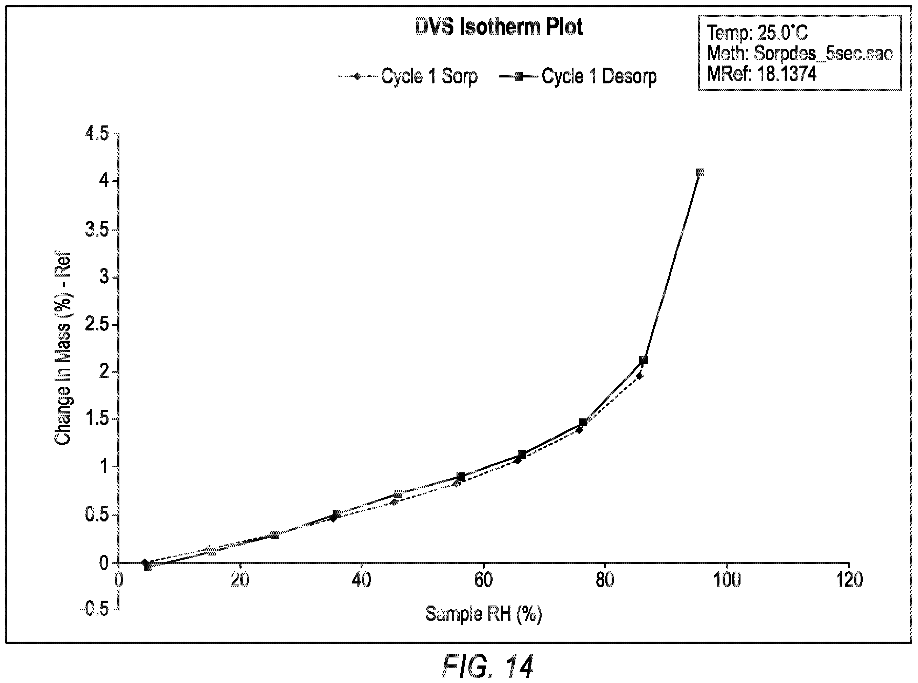

DVS analysis (Figure 14) provides that the sample of Compound 1 Citrate Form B experienced a ∼4.1% weight gain from 5% to 95% relative humidity. The water absorbed by the sample was released as the relative humidity was ramped down from 95% to 5%. XRPD analysis shows that the sample remained as Compound 1 Citrate Form B after DVS analysis.

### Compound 1 Esylate Form A

Compound 1 Esylate Form A is a 1:1 salt of Compound 1 and ethanesulfonic acid, and further includes one molar equivalent of acetone in the crystal lattice.

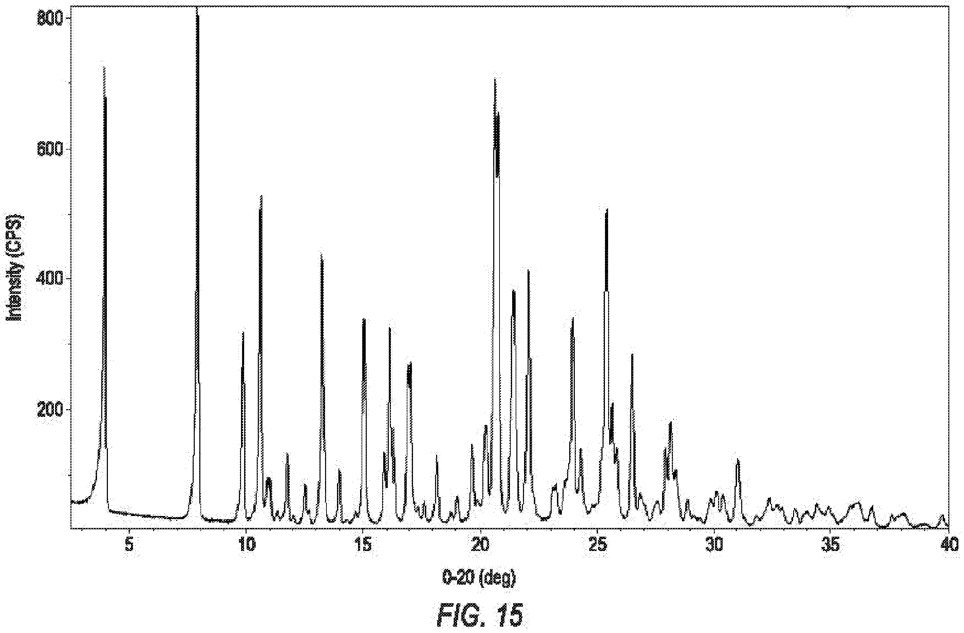

The XRPD pattern for Compound 1 Esylate Form A is provided in Figure 15, and a list of peaks from the pattern is provided in Table 5 below.

**Table 5: XRPD peaks of Compound 1 Esylate Form A**

| **2*θ* (°)** | ***d*-spacing (Å)** | **Intensity (%)** |
|---|---|---|
| 3.97 ± 0.20 | 22.255 ± 1.122 | 86 |
| 7.95 ± 0.20 | 11.119 ± 0.279 | 100 |
| 9.89 ± 0.20 | 8.937 ± 0.180 | 38 |
| 10.62 ± 0.20 | 8.322 ± 0.156 | 63 |
| 10.92 ± 0.20 | 8.096 ± 0.148 | 12 |
| 11.03 ± 0.20 | 8.012 ± 0.145 | 12 |
| 11.34 ± 0.20 | 7.798 ± 0.137 | 6 |
| 11.77 ± 0.20 | 7.516 ± 0.127 | 16 |
| 12.03 ± 0.20 | 7.348 ± 0.122 | 5 |
| 12.53 ± 0.20 | 7.057 ± 0.112 | 11 |
| 12.71 ± 0.20 | 6.960 ± 0.109 | 6 |
| 13.27 ± 0.20 | 6.665 ± 0.100 | 54 |
| 14.00 ± 0.20 | 6.321 ± 0.090 | 13 |
| 14.70 ± 0.20 | 6.021 ± 0.081 | 5 |
| 15.08 ± 0.20 | 5.870 ± 0.077 | 42 |
| 15.94 ± 0.20 | 5.554 ± 0.069 | 17 |
| 16.17 ± 0.20 | 5.477 ± 0.067 | 40 |
| 16.92 ± 0.20 | 5.236 ± 0.061 | 32 |
| 17.01 ± 0.20 | 5.209 ± 0.061 | 33 |
| 17.19 ± 0.20 | 5.155 ± 0.060 | 7 |
| 17.35 ± 0.20 | 5.108 ± 0.058 | 6 |
| 17.61 ± 0.20 | 5.033 ± 0.057 | 7 |
| 18.15 ± 0.20 | 4.883 ± 0.053 | 16 |
| 18.76 ± 0.20 | 4.727 ± 0.050 | 5 |
| 19.03 ± 0.20 | 4.661 ± 0.049 | 8 |
| 19.66 ± 0.20 | 4.511 ± 0.045 | 18 |
| 19.89 ± 0.20 | 4.461 ± 0.044 | 8 |
| 20.26 ± 0.20 | 4.380 ± 0.043 | 22 |
| 20.65 ± 0.20 | 4.297 ± 0.041 | 87 |
| 20.78 ± 0.20 | 4.272 ± 0.041 | 81 |
| 21.44 ± 0.20 | 4.141 ± 0.038 | 47 |
| 22.08 ± 0.20 | 4.022 ± 0.036 | 51 |
| 23.14 ± 0.20 | 3.840 ± 0.033 | 10 |
| 23.27 ± 0.20 | 3.820 ± 0.032 | 11 |
| 23.99 ± 0.20 | 3.706 ± 0.030 | 42 |
| 24.32 ± 0.20 | 3.657 ± 0.030 | 17 |
| 25.43 ± 0.20 | 3.500 ± 0.027 | 62 |
| 25.66 ± 0.20 | 3.469 ± 0.027 | 26 |
| 25.85 ± 0.20 | 3.444 ± 0.026 | 17 |
| 26.52 ± 0.20 | 3.358 ± 0.025 | 35 |
| 26.87 ± 0.20 | 3.315 ± 0.024 | 9 |
| 27.61 ± 0.20 | 3.228 ± 0.023 | 7 |
| 27.96 ± 0.20 | 3.189 ± 0.022 | 18 |
| 28.18 ± 0.20 | 3.164 ± 0.022 | 22 |
| 28.40 ± 0.20 | 3.140 ± 0.022 | 13 |
| 28.91 ± 0.20 | 3.085 ± 0.021 | 7 |

The XRPD pattern was successfully indexed.

### Unit cell data for Compound 1 Esylate Form A

| Bravais Type | Triclinic |
|---|---|
| a [Å] | 8.593 |
| b [Å] | 9.223 |
| c [Å] | 22,439 |
| α [deg] | 94.60 |
| β [deg] | 95,J3 |
| γ [deg] | 103.05 |
| Volume [Å³/cell] | 1.715.6 |
| Chiral Contents? | Not Specified |
| Extinction Symbol | P - |
| Space Group(s) | P1 (1), P1̅ (2) |

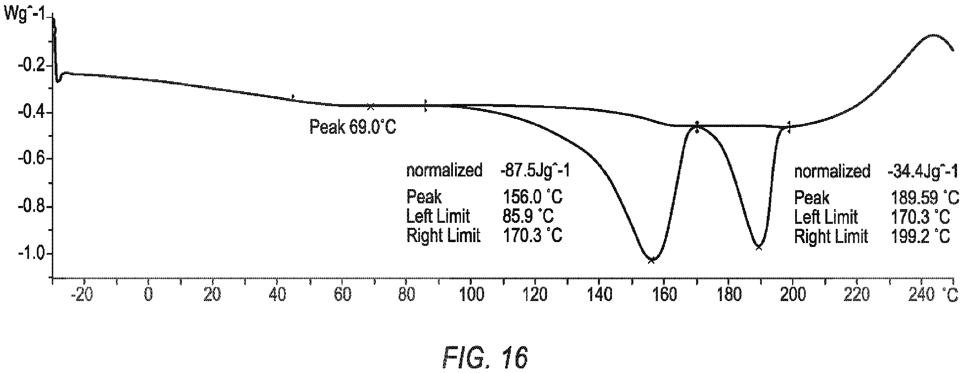

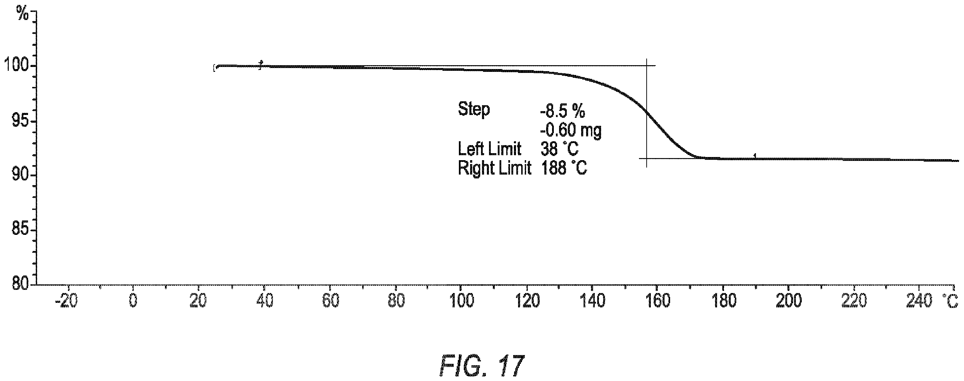

The DSC thermogram for Compound 1 Esylate Form A is provided in Figure 16, and shows endotherms at ~69 °C, ∼156 °C, and ~190 °C. The TGA thermogram (Figure 17) shows that the material undergoes a weight loss of -8.5% of the temperature range of 38-188 °C, which is consistent with the loss of one molar equivalent of acetone.

### Compound 1 Esylate Form B

Compound 1 Esylate Form B was formed by the desolvation of Compound 1 Esylate Form A, and was determined to be a mixture with Compound 1 Esylate Form A. This form was not characterized further.

The XRPD pattern for Compound 1 Esylate Form B is provided in Figure 18.

### Compound 1 HBr Form A

Compound 1 HBr Form A is a 1:1 salt, formed by the addition of HBr to Compound 1 in a hot acetone slurry.

The XRPD pattern for Compound 1 HBr Form A is provided in Figure 19.

### Compound 1 HBr Form B

Compound 1 HBr Form B is a 1:1 salt, formed by the addition of 1 molar equivalent of HBr to Compound 1 in a hot MEK slurry.

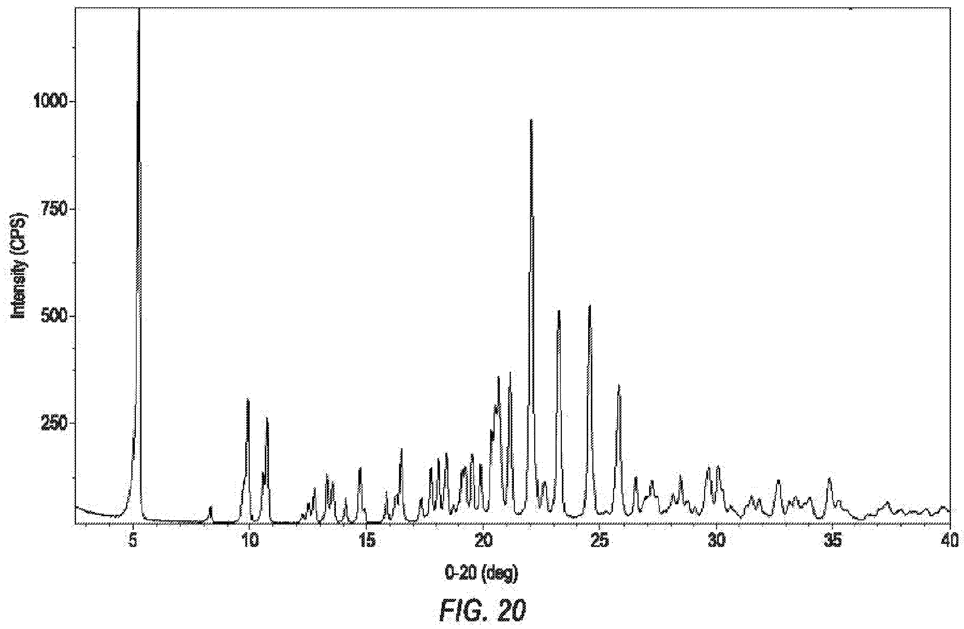

The XRPD pattern for Compound 1 HBr Form B is provided in Figure 20, and a list of peaks from the pattern is provided in Table 6 below.

**Table 6: XRPD peaks of Compound 1 HBr Form B**

| **2*θ* (°)** | ***d*-spacing (Å)** | **Intensity (%)** |
|---|---|---|
| 5.26 ± 0.20 | 16.777 ± 0.637 | 100 |
| 8.29 ± 0.20 | 10.661 ± 0.257 | 5 |
| 9.75 ± 0.20 | 9.068 ± 0.186 | 10 |
| 9.91 ± 0.20 | 8.916 ± 0.179 | 26 |
| 10.56 ± 0.20 | 8.367 ± 0.158 | 11 |
| 10.72 ± 0.20 | 8.243 ± 0.153 | 22 |
| 12.25 ± 0.20 | 7.219 ± 0.117 | 4 |
| 12.50 ± 0.20 | 7.075 ± 0.113 | 6 |
| 12.75 ± 0.20 | 6.938 ± 0.108 | 9 |
| 13.31 ± 0.20 | 6.648 ± 0.099 | 11 |
| 13.54 ± 0.20 | 6.535 ± 0.096 | 10 |
| 14.11 ± 0.20 | 6.270 ± 0.088 | 6 |
| 14.72 ± 0.20 | 6.011 ± 0.081 | 12 |
| 14.89 ± 0.20 | 5.943 ± 0.079 | 5 |
| 15.87 ± 0.20 | 5.581 ± 0.070 | 8 |
| 16.29 ± 0.20 | 5.438 ± 0.066 | 7 |
| 16.50 ± 0.20 | 5.368 ± 0.065 | 16 |
| 17.36 ± 0.20 | 5.104 ± 0.058 | 7 |
| 17.78 ± 0.20 | 4.984 ± 0.056 | 12 |
| 18.09 ± 0.20 | 4.899 ± 0.054 | 14 |
| 18.44 ± 0.20 | 4.808 ± 0.052 | 15 |
| 18.75 ± 0.20 | 4.728 ± 0.050 | 5 |
| 19.12 ± 0.20 | 4.637 ± 0.048 | 12 |
| 19.24 ± 0.20 | 4.609 ± 0.047 | 12 |
| 19.56 ± 0.20 | 4.535 ± 0.046 | 15 |
| 19.92 ± 0.20 | 4.453 ± 0.044 | 13 |
| 20.40 ± 0.20 | 4.350 ± 0.042 | 19 |
| 20.55 ± 0.20 | 4.319 ± 0.042 | 24 |
| 20.69 ± 0.20 | 4.290 ± 0.041 | 30 |
| 21.18 ± 0.20 | 4.191 ± 0.039 | 31 |
| 22.09 ±0.20 | 4.021 ± 0.036 | 80 |
| 22.64 ± 0.20 | 3.924 ± 0.034 | 10 |
| 23.24 ± 0.20 | 3.825 ± 0.032 | 43 |
| 24.56 ± 0.20 | 3.622 ± 0.029 | 44 |
| 25.80 ± 0.20 | 3.450 ± 0.026 | 28 |
| 26.52 ± 0.20 | 3.358 ± 0.025 | 11 |
| 27.23 ± 0.20 | 3.272 ± 0.024 | 10 |
| 27.43 ± 0.20 | 3.249 ± 0.023 | 7 |
| 28.13 ± 0.20 | 3.169 ± 0.022 | 7 |
| 28.46 ± 0.20 | 3.134 ± 0.022 | 11 |
| 28.76 ± 0.20 | 3.101 ± 0.021 | 6 |
| 29.08 ± 0.20 | 3.069 ± 0.021 | 5 |
| 29.66 ± 0.20 | 3.009 ± 0.020 | 13 |
| 30.07 ± 0.20 | 2.970 ± 0.019 | 13 |

The XRPD for Compound 1 HBr Form B was successfully indexed.

### Unit cell data for Compound 1 HBr Form B

| Bravais Type | Primitive Monoclinic |
|---|---|
| a [Å] | 10.652 |
| b [Å] | 9.460 |
| c [Å] | 33.496 |
| α [deg] | 90 |
| β [deg] | 91.13 |
| γ [deg] | 90 |
| Volume [Å³/cell] | 3,374.7 |
| Chiral Contents? | Achiral |
| Extinction Symbol | P 1 2₁ /c 1 |
| Space Group(s) | P2₁ /c (14) |

### Compound 1 Lactate Form A

Compound 1 Lactate Form A was formed by adding one molar equivalent of lactic acid to Compound 1 in a hot acetone slurry. The material was determined to be a mixture with a crystalline form of the free base of Compound 1.

The XRPD pattern for Compound 1 Lactate Form A is provided in Figure 21.

### Compound 1 Malate Form A

Compound 1 Malate Form A was made by adding L-malic acid to Compound 1 in acetone. The material was determined to be a mixture with a crystalline form of the free base of Compound 1.

The XRPD pattern for Compound 1 Malate Form A is provided in Figure 22.

### Compound 1 Maleate Form A

Compound 1 Maleate Form A is a 1:1 salt made by adding aqueous maleic acid to a slurry of Compound 1 in hot MEK or acetone.

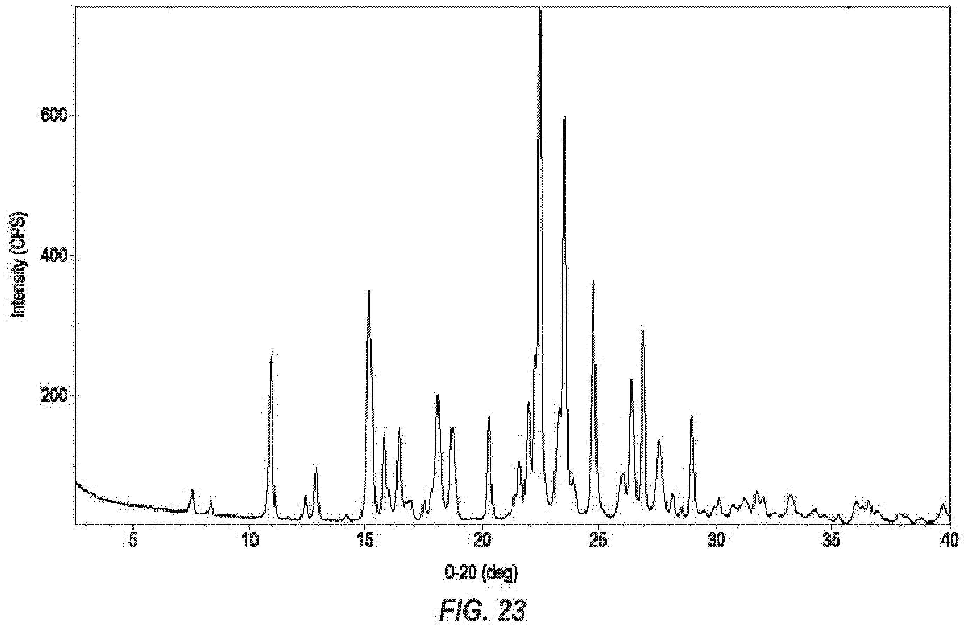

The XRPD pattern for Compound 1 Maleate Form A is provided in Figure 23, and a list of peaks from the pattern is provided in Table 7 below.

**Table 7: XRPD peaks of Compound 1 Maleate Form A**

| **2*θ* (°)** | ***d*-spacing (Å)** | **Intensity (%)** |
|---|---|---|
| 7.58 ± 0.20 | 11.659 ± 0.307 | 9 |
| 8.34 ± 0.20 | 10.593 ± 0.254 | 7 |
| 10.92 ± 0.20 | 8.094 ± 0.148 | 34 |
| 12.38 ± 0.20 | 7.141 ± 0.115 | 8 |
| 12.87 ± 0.20 | 6.874 ± 0.106 | 13 |
| 14.17 ± 0.20 | 6.244 ± 0.088 | 4 |
| 15.13 ± 0.20 | 5.852 ± 0.077 | 47 |
| 15.78 ± 0.20 | 5.612 ± 0.071 | 20 |
| 16.43 ± 0.20 | 5.389 ± 0.065 | 21 |
| 16.91 ± 0.20 | 5.238 ± 0.061 | 7 |
| 17.51 ± 0.20 | 5.060 ± 0.057 | 7 |
| 18.09 ± 0.20 | 4.900 ± 0.054 | 27 |
| 18.71 ± 0.20 | 4.738 ± 0.050 | 21 |
| 20.30 ± 0.20 | 4.372 ± 0.043 | 23 |
| 21.60 ± 0.20 | 4.111 ± 0.038 | 14 |
| 21.99 ± 0.20 | 4.039 ± 0.036 | 26 |
| 22.30 ± 0.20 | 3.983 ± 0.035 | 34 |
| 22.49 ± 0.20 | 3.951 ± 0.035 | 100 |
| 23.32 ± 0.20 | 3.811 ± 0.032 | 24 |
| 23.56 ± 0.20 | 3.773 ± 0.032 | 80 |
| 23.92 ± 0.20 | 3.718 ± 0.031 | 11 |
| 24.79 ± 0.20 | 3.588 ± 0.028 | 48 |
| 26.04 ± 0.20 | 3.419 ± 0.026 | 12 |
| 26.41 ± 0.20 | 3.372 ± 0.025 | 30 |
| 26.92 ± 0.20 | 3.309 ± 0.024 | 39 |
| 27.62 ± 0.20 | 3.228 ± 0.023 | 18 |
| 28.18 ± 0.20 | 3.164 ± 0.022 | 8 |
| 28.54 ± 0.20 | 3.125 ± 0.021 | 6 |
| 29.01 ± 0.20 | 3.075 ± 0.021 | 23 |

The XRPD pattern of Compound 1 Maleate Form A was successfully indexed, which indicates the material is composed of a single crystalline phase.

### Unit cell data for Compound 1 Maleate Form A

| Bravais Type | Triclinic |
|---|---|
| a [Å] | 6.264 |
| b [Å] | 15.319 |
| c [Å] | 16.232 |
| a [deg] | 95.43 |
| β [deg] | 90.51 |
| γ [deg] | 94.67 |
| Volume [Å³/cell] | 1,545.2 |
| Chiral Contents? | Achiral |
| Extinction Symbol | P - |
| Space Group(s) | P1 (1), P1̅ (2) |

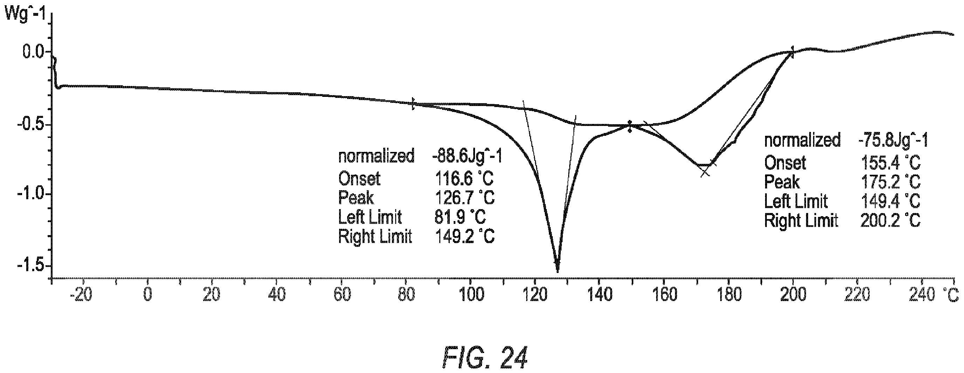

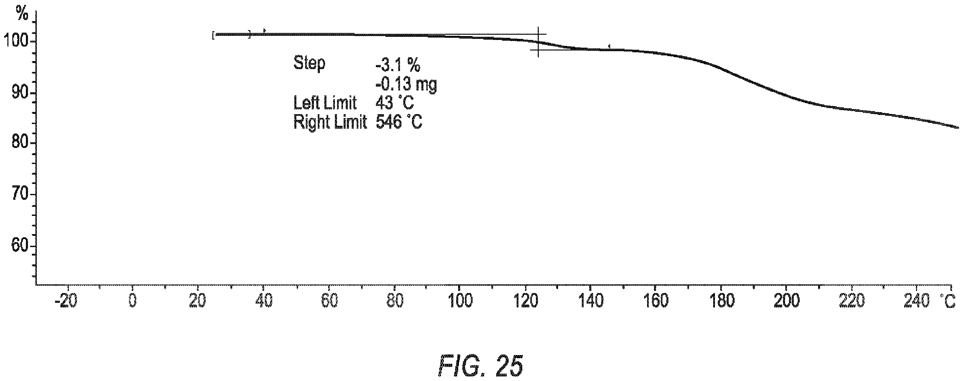

The curve of the TGA thermogram (Figure 25) shows a weight loss of -3.1% over the temperature range of 42-146 °C, mostly above ∼117 °C, likely due to decomposition of maleic acid. The curve of the DSC thermogram (Figure 24) exhibits a single broad endotherm with an onset of ∼117 °C.

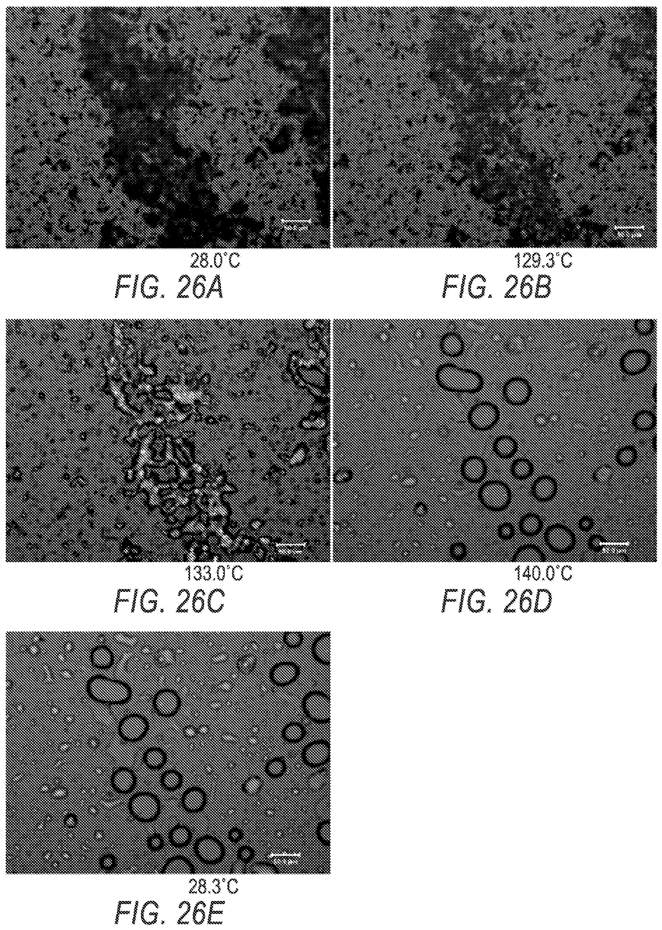

Hot stage images (Figures 26A - 26E) for Compound 1 Maleate Form A confirm the event as the melt.

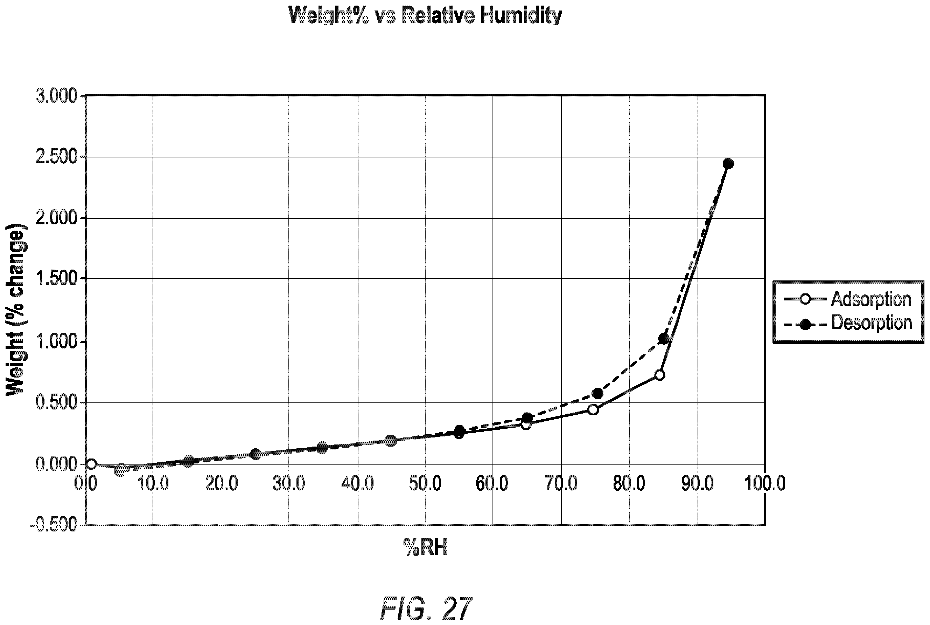

The DVS isotherm (Figure 27) indicates gains/losses of -2.5% weight through the sorption/desorption experiment (RH = 5% - 95%).

The material recovered from the experiment remained unchanged and identified as Compound 1 Maleate Form A by XRPD.

### Compound 1 Maleate Form B

Compound 1 Maleate Form B was produced in the scaled up procedure to product Compound 1 Maleate Form A in acetone. Compound 1 Maleate Form B was only made as a mixture with Compound 1 Maleate Form A.

The XRPD pattern for Compound 1 Maleate Form B is provided in Figure 28.

### Compound 1 Mesylate Form A

Compound 1 Mesylate Form A is a 1:1 salt, produced by slurrying Compound 1 and methanesulfonic acid in THF at room temperature.

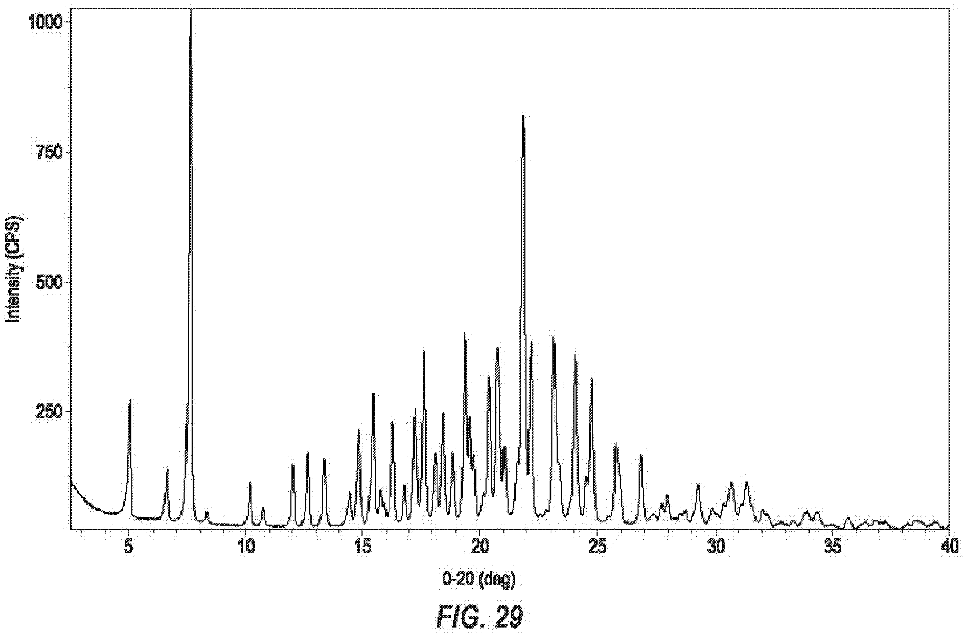

The XRPD pattern for Compound 1 Mesylate Form A is provided in Figure 29, and a list of peaks from the pattern is provided in Table 8 below.

**Table 8: XRPD peaks of Compound 1 Mesylate Form A**

| **2*θ* (°)** | ***d*-spacing (Å)** | **Intensity (%)** |
|---|---|---|
| 5.06 ± 0.20 | 17.449 ± 0.689 | 26 |
| 6.65 ± 0.20 | 13.274 ± 0.399 | 14 |
| 7.66 ± 0.20 | 11.534 ± 0.301 | 100 |
| 8.35 ± 0.20 | 10.587 ± 0.253 | 6 |
| 10.16 ± 0.20 | 8.701 ± 0.171 | 11 |
| 10.72 ± 0.20 | 8.247 ± 0.153 | 7 |
| 11.98 ± 0.20 | 7.385 ± 0.123 | 14 |
| 12.62 ± 0.20 | 7.010 ± 0.111 | 17 |
| 13.32 ± 0.20 | 6.642 ± 0.099 | 16 |
| 14.41 ± 0.20 | 6.143 ± 0.085 | 9 |
| 14.79 ± 0.20 | 5.984 ± 0.080 | 21 |
| 15.42 ± 0.20 | 5.742 ± 0.074 | 28 |
| 15.72 ± 0.20 | 5.631 ± 0.071 | 10 |
| 15.89 ± 0.20 | 5.573 ± 0.070 | 8 |
| 16.23 ± 0.20 | 5.457 ± 0.067 | 23 |
| 16.77 ± 0.20 | 5.284 ± 0.063 | 11 |
| 17.20 ± 0.20 | 5.151 ± 0.059 | 25 |
| 17.59 ± 0.20 | 5.039 ± 0.057 | 36 |
| 18.08 ± 0.20 | 4.903 ± 0.054 | 17 |
| 18.40 ± 0.20 | 4.819 ± 0.052 | 24 |
| 18.81 ± 0.20 | 4.713 ± 0.050 | 17 |
| 19.33 ± 0.20 | 4.588 ± 0.047 | 39 |
| 19.53 ± 0.20 | 4.541 ± 0.046 | 24 |
| 19.71 ± 0.20 | 4.501 ± 0.045 | 16 |
| 20.14 ± 0.20 | 4.405 ± 0.043 | 9 |
| 20.38 ± 0.20 | 4.354 ± 0.042 | 31 |
| 20.73 ± 0.20 | 4.280 ± 0.041 | 37 |
| 21.05 ± 0.20 | 4.218 ± 0.040 | 18 |
| 21.56 ± 0.20 | 4.118 ± 0.038 | 15 |
| 21.83 ± 0.20 | 4.067 ± 0.037 | 81 |
| 22.16 ± 0.20 | 4.008 ± 0.036 | 38 |
| 23.14 ± 0.20 | 3.841 ± 0.033 | 38 |
| 24.06 ± 0.20 | 3.695 ± 0.030 | 35 |
| 24.54 ± 0.20 | 3.624 ± 0.029 | 12 |
| 24.77 ± 0.20 | 3.591 ± 0.029 | 31 |
| 25.75 ± 0.20 | 3.457 ± 0.026 | 19 |
| 26.82 ± 0.20 | 3.322 ± 0.024 | 16 |
| 27.71 ± 0.20 | 3.217 ± 0.023 | 7 |
| 27.94 ± 0.20 | 3.191 ± 0.022 | 9 |
| 28.71 ± 0.20 | 3.107 ± 0.021 | 6 |
| 29.27 ± 0.20 | 3.049 ± 0.020 | 11 |
| 29.85 ± 0.20 | 2.990 ± 0.020 | 6 |

The XRPD pattern of Compound 1 Mesylate Form A was successfully indexed, which indicates the material is composed of a single crystalline phase.

### Unit cell data for Compound 1 Mesylate Form A

| Bravais Type | Primitive Orthorhembic |
|---|---|
| a [Å] | 6.369 |
| b [Å] | 23.054 |
| c [Å] | 26.467 |
| α [deg] | 90 |
| β [deg] | 90 |
| γ [deg] | 90 |
| Volume [Å³/cell] | 3,886.2 |
| Chiral Contents? | Achiral |
| Extinction Symbol | P 2₁ 2₁ 2₁ |
| Space Group(s) | P2₁2₁2₁ (19) |

### Compound 1 Mesylate Form B

Compound 1 Mesylate Form B is a 1:1 salt, produced by mixing Compound 1 and methanesulfonic acid in hot chloroform. The material also includes about ~1.5 moles of chloroform per mole of Compound 1.

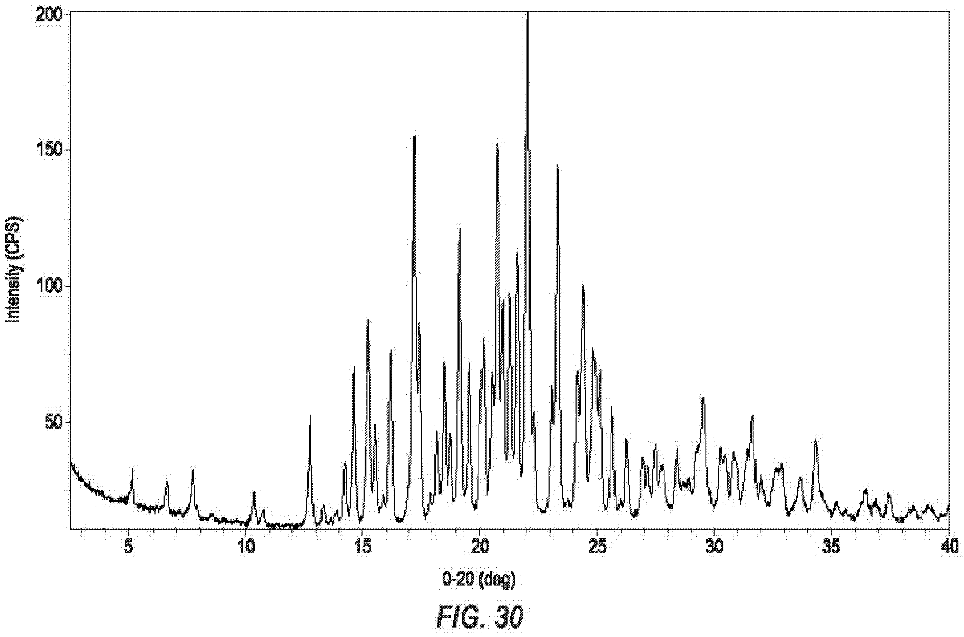

The XRPD pattern for Compound 1 Mesylate Form B is provided in Figure 30, and a list of peaks from the pattern is provided in Table 9 below.

**Table 9: XRPD peaks of Compound 1 Mesylate Form B**

| **2*θ* (°)** | ***d*-spacing (Å)** | **Intensity (%)** |
|---|---|---|
| 5.14 ± 0.20 | 17.169 ± 0.667 | 16 |
| 6.62 ± 0.20 | 13.336 ± 0.402 | 14 |
| 7.72 ± 0.20 | 11.441 ± 0.296 | 16 |
| 7.89 ± 0.20 | 11.193 ± 0.283 | 10 |
| 10.33 ± 0.20 | 8.556 ± 0.165 | 12 |
| 10.73 ± 0.20 | 8.240 ± 0.153 | 8 |
| 12.74 ± 0.20 | 6.945 ± 0.109 | 26 |
| 13.31 ± 0.20 | 6.646 ± 0.099 | 10 |
| 13.88 ± 0.20 | 6.377 ± 0.091 | 8 |
| 14.23 ± 0.20 | 6.220 ± 0.087 | 17 |
| 14.61 ± 0.20 | 6.057 ± 0.082 | 35 |
| 15.21 ± 0.20 | 5.822 ± 0.076 | 44 |
| 15.52 ± 0.20 | 5.704 ± 0.073 | 24 |
| 15.87 ± 0.20 | 5.580 ± 0.070 | 12 |
| 16.17 ± 0.20 | 5.477 ± 0.067 | 38 |
| 17.18 ± 0.20 | 5.157 ± 0.060 | 78 |
| 17.41 ± 0.20 | 5.090 ± 0.058 | 42 |
| 17.88 ± 0.20 | 4.956 ± 0.055 | 12 |
| 18.15 ± 0.20 | 4.883 ± 0.053 | 23 |
| 18.46 ± 0.20 | 4.802 ± 0.052 | 36 |
| 18.73 ± 0.20 | 4.733 ± 0.050 | 23 |
| 19.13 ± 0.20 | 4.636 ± 0.048 | 60 |
| 19.54 ± 0.20 | 4.539 ± 0.046 | 35 |
| 20.15 ± 0.20 | 4.403 ± 0.043 | 40 |
| 20.53 ± 0.20 | 4.323 ± 0.042 | 34 |
| 20.75 ± 0.20 | 4.278 ± 0.041 | 75 |
| 20.98 ± 0.20 | 4.230 ± 0.040 | 47 |
| 21.27 ± 0.20 | 4.173 ± 0.039 | 49 |
| 21.60 ± 0.20 | 4.110 ± 0.038 | 55 |
| 22.03 ± 0.20 | 4.032 ± 0.036 | 100 |
| 22.28 ± 0.20 | 3.986 ± 0.035 | 27 |
| 23.07 ± 0.20 | 3.852 ± 0.033 | 31 |
| 23.32 ± 0.20 | 3.811 ± 0.032 | 72 |
| 24.16 ± 0.20 | 3.681 ± 0.030 | 34 |
| 24.42 ± 0.20 | 3.642 ± 0.029 | 50 |
| 24.87 ± 0.20 | 3.577 ± 0.028 | 37 |
| 25.13 ± 0.20 | 3.541 ± 0.028 | 34 |
| 25.64 ± 0.20 | 3.472 ± 0.027 | 26 |
| 26.24 ± 0.20 | 3.393 ± 0.025 | 22 |
| 26.95 ± 0.20 | 3.306 ± 0.024 | 18 |
| 27.17 ± 0.20 | 3.279 ± 0.024 | 17 |
| 27.49 ± 0.20 | 3.242 ± 0.023 | 21 |
| 27.77 ± 0.20 | 3.210 ± 0.023 | 17 |
| 28.42 ± 0.20 | 3.138 ± 0.022 | 19 |
| 29.54 ± 0.20 | 3.022 ± 0.020 | 30 |

The XRPD pattern of Compound 1 Mesylate Form B was successfully indexed, which indicates the material is composed of a single crystalline phase.

### Unit cell data for Compound 1 Mesylate Form B

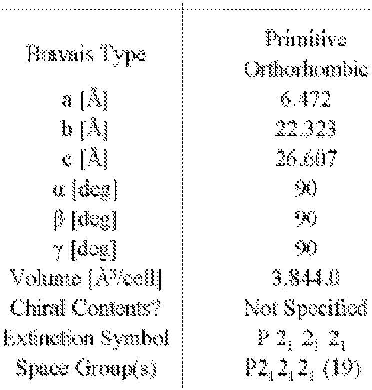

### Compound 1 Mesylate Form C

Compound 1 Mesylate Form C is a 1:1 salt produced by mixing Compound 1 and methanesulfonic acid in hot methanol. The material also had ~0.1 molar equivalents of methanol.

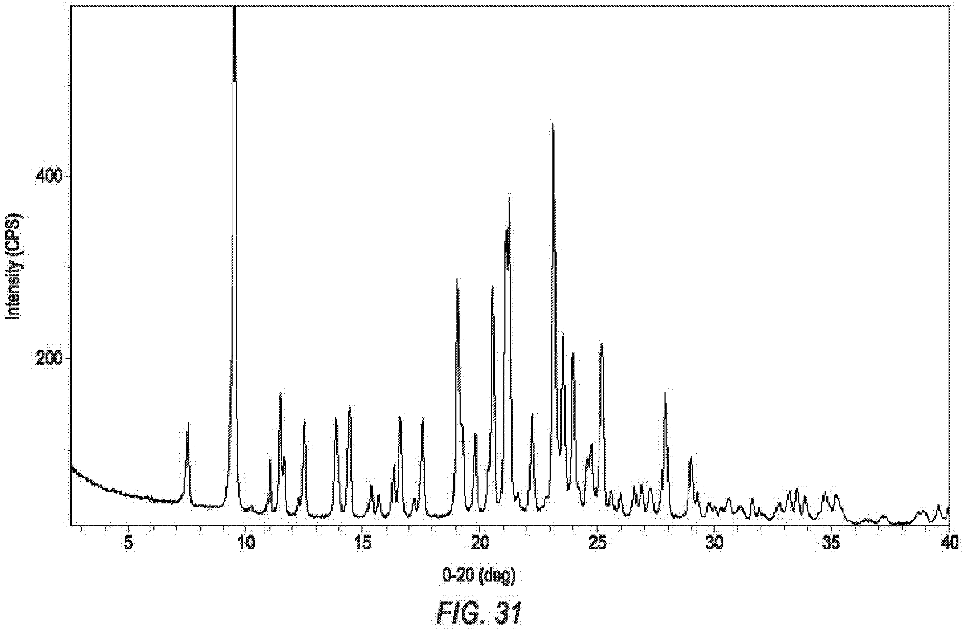

The XRPD pattern for Compound 1 Mesylate Form C is provided in Figure 31, and a list of peaks from the pattern is provided in Table 10 below.

**Table 10: XRPD peaks of Compound 1 Mesylate Form C**

| **2*θ* (°)** | ***d*-spacing (Å)** | **Intensity (%)** |
|---|---|---|
| 7.55 ± 0.20 | 11.702 ± 0.310 | 22 |
| 9.53 ± 0.20 | 9.278 ± 0.194 | 100 |
| 10.25 ± 0.20 | 8.621 ± 0.168 | 7 |
| 11.04 ± 0.20 | 8.008 ± 0.145 | 15 |
| 11.49 ± 0.20 | 7.692 ± 0.133 | 28 |
| 11.65 ± 0.20 | 7.587 ± 0.130 | 16 |
| 12.24 ± 0.20 | 7.223 ± 0.118 | 8 |
| 12.51 ± 0.20 | 7.072 ± 0.113 | 23 |
| 13.88 ± 0.20 | 6.373 ± 0.091 | 23 |
| 14.44 ± 0.20 | 6.129 ± 0.084 | 25 |
| 15.36 ± 0.20 | 5.765 ± 0.075 | 11 |
| 15.66 ± 0.20 | 5.653 ± 0.072 | 9 |
| 16.29 ± 0.20 | 5.437 ± 0.066 | 14 |
| 16.58 ± 0.20 | 5.342 ± 0.064 | 23 |
| 17.16 ± 0.20 | 5.164 ± 0.060 | 8 |
| 17.54 ± 0.20 | 5.053 ± 0.057 | 23 |
| 19.04 ± 0.20 | 4.659 ± 0.048 | 49 |
| 19.20 ± 0.20 | 4.618 ± 0.048 | 22 |
| 19.79 ± 0.20 | 4.482 ± 0.045 | 20 |
| 20.54 ± 0.20 | 4.322 ± 0.042 | 47 |
| 21.12 ± 0.20 | 4.203 ± 0.039 | 58 |
| 21.24 ± 0.20 | 4.179 ± 0.039 | 64 |
| 21.62 ± 0.20 | 4.106 ± 0.038 | 9 |
| 22.22 ± 0.20 | 3.998 ± 0.036 | 24 |
| 23.15 ± 0.20 | 3.839 ± 0.033 | 78 |
| 23.57 ± 0.20 | 3.772 ± 0.032 | 39 |
| 23.99 ± 0.20 | 3.706 ± 0.030 | 35 |
| 24.61 ± 0.20 | 3.615 ± 0.029 | 15 |
| 24.77 ± 0.20 | 3.591 ± 0.029 | 18 |
| 25.22 ± 0.20 | 3.528 ± 0.028 | 37 |
| 25.61 ± 0.20 | 3.475 ± 0.027 | 10 |
| 25.99 ± 0.20 | 3.426 ± 0.026 | 9 |
| 26.60 ± 0.20 | 3.348 ± 0.025 | 10 |
| 26.89 ± 0.20 | 3.312 ± 0.024 | 11 |
| 27.29 ± 0.20 | 3.265 ± 0.023 | 10 |
| 27.91 ± 0.20 | 3.194 ± 0.022 | 28 |
| 29.03 ± 0.20 | 3.074 ± 0.021 | 16 |
| 29.29 ± 0.20 | 3.047 ± 0.020 | 9 |

The XRPD pattern of Compound 1 Mesylate Form C was successfully indexed, which indicates the material is composed of a single crystalline phase.

### Unit cell data for Compound 1 Mesylate Form C

| Bravais Type | Triclinic |
|---|---|
| a[Å] | 8.884 |
| b [Å] | 11.922 |
| c [Å] | 15.312 |
| α [deg] | 88.79 |
| β [deg] | 81.68 |
| 7 [deg] | 78.70 |
| Volume [Å³/cell] | 1,573.6 |
| Chiral Contents? | Not Specified |
| Extinetion Symbol | P - |
| Space Group(s) | P1 (1), P1̅ (2) |

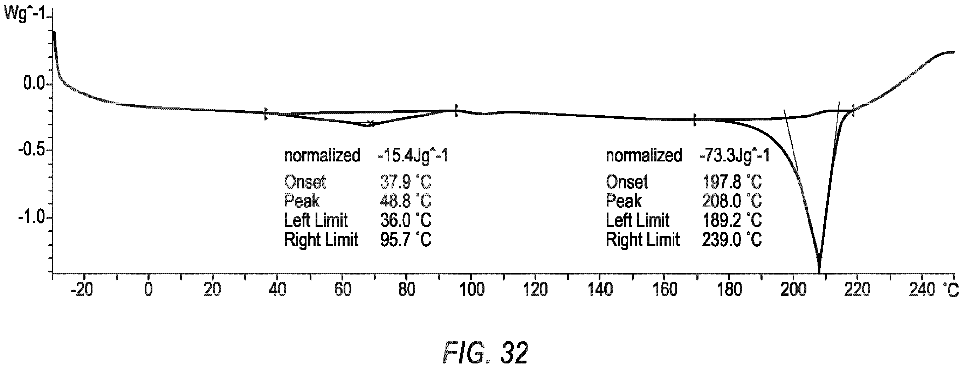

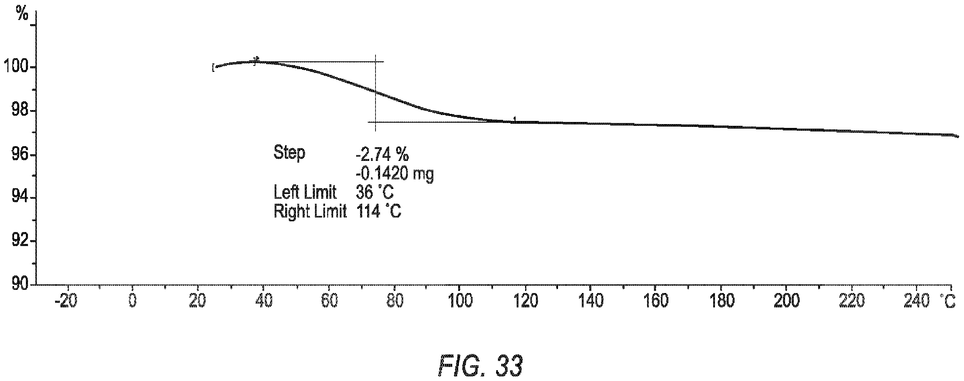

The DSC thermogram for Compound 1 Mesylate Form C is provided in Figure 32 and shows a broad endotherm with a peak at about 69 °C, wherein the endotherm has a lower limit of about 36 and an upper limit of about 96 °C. The thermogram also shows another sharp endotherm with a peak at about 208 °C, wherein the endotherm has a lower limit of about 169 and an upper limit of about 219 °C. TGA, Figure 33, indicates a -2.3% weight loss over the temperature range of 38-115 °C, corresponding to ~0.08 molar equivalents of methanol.

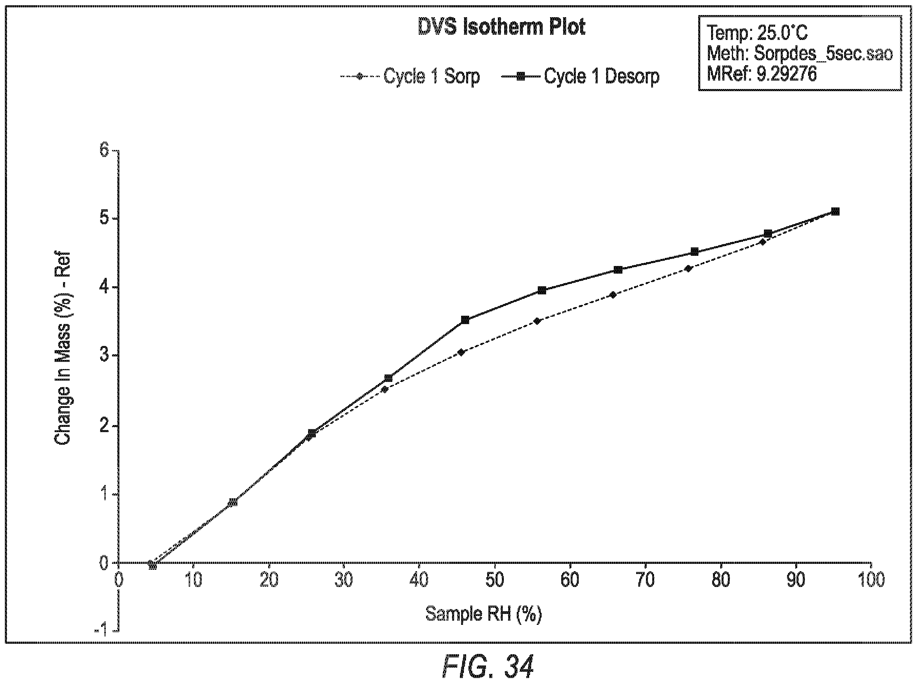

The DVS isotherm (Figure 34) indicates gains/losses of about 5.1% weight through the sorption/desorption experiment (RH = 5% - 95%).

### Compound 1 Oxalate Form A

Compound 1 Oxalate Form A is a 1:1 salt produced by adding oxalic acid in hot acetone to Compound 1 and forming a slurry.

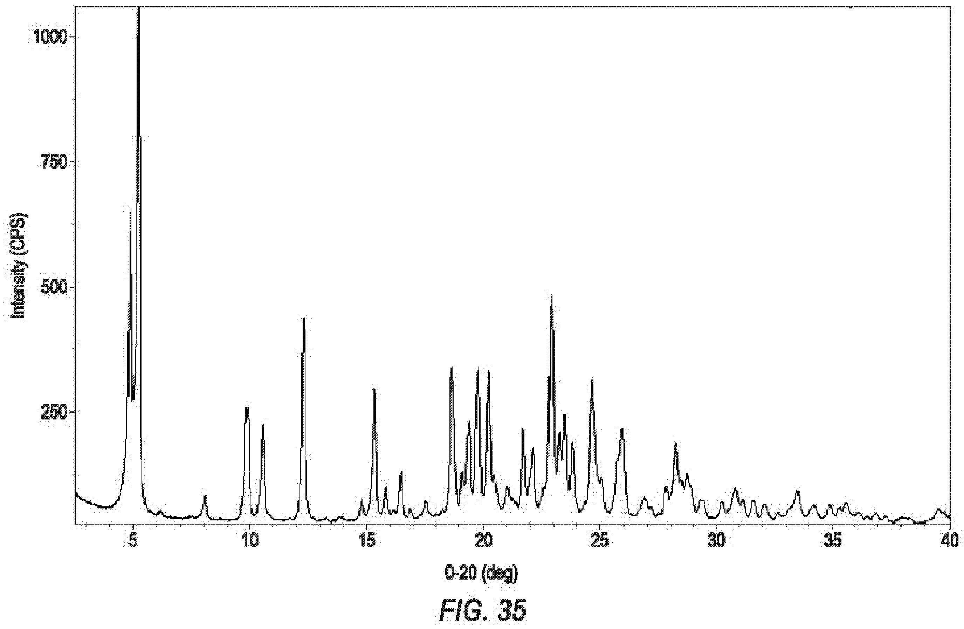

The XRPD pattern for Compound 1 Oxalate Form A is provided in Figure 35, and a list of peaks from the pattern is provided in Table 11 below.

**Table 11: XRPD peaks of Compound 1 Oxalate Form A**

| **2*θ* (°)** | ***d*-spacing (Å)** | **Intensity (%)** |
|---|---|---|
| 4.90 ± 0.20 | 18.008 ± 0.734 | 62 |
| 5.25 ± 0.20 | 16.811 ± 0.640 | 100 |
| 6.20 ± 0.20 | 14.248 ± 0.459 | 5 |
| 8.05 ± 0.20 | 10.969 ± 0.272 | 8 |
| 9.87 ± 0.20 | 8.951 ± 0.181 | 24 |
| 10.54 ± 0.20 | 8.389 ± 0.159 | 21 |
| 12.29 ± 0.20 | 7.195 ± 0.117 | 41 |
| 14.79 ± 0.20 | 5.985 ± 0.080 | 7 |
| 15.35 ± 0.20 | 5.768 ± 0.075 | 28 |
| 15.84 ± 0.20 | 5.589 ± 0.070 | 9 |
| 16.49 ± 0.20 | 5.371 ± 0.065 | 12 |
| 16.89 ± 0.20 | 5.246 ± 0.062 | 5 |
| 17.55 ± 0.20 | 5.049 ± 0.057 | 7 |
| 18.27 ± 0.20 | 4.852 ± 0.053 | 5 |
| 18.67 ± 0.20 | 4.750 ± 0.050 | 32 |
| 19.12 ± 0.20 | 4.638 ± 0.048 | 12 |
| 19.40 ± 0.20 | 4.572 ± 0.047 | 22 |
| 19.81 ± 0.20 | 4.478 ± 0.045 | 32 |
| 20.26 ± 0.20 | 4.380 ± 0.043 | 31 |
| 20.50 ± 0.20 | 4.329 ± 0.042 | 12 |
| 21.06 ± 0.20 | 4.215 ± 0.040 | 10 |
| 21.74 ± 0.20 | 4.084 ± 0.037 | 21 |
| 22.17 ± 0.20 | 4.007 ± 0.036 | 17 |
| 22.93 ± 0.20 | 3.876 ± 0.033 | 46 |
| 23.26 ± 0.20 | 3.822 ± 0.032 | 20 |
| 23.50 ± 0.20 | 3.782 ± 0.032 | 23 |
| 23.81 ± 0.20 | 3.734 ± 0.031 | 18 |
| 24.66 ± 0.20 | 3.607 ± 0.029 | 30 |
| 25.06 ± 0.20 | 3.550 ± 0.028 | 11 |
| 25.95 ± 0.20 | 3.431 ± 0.026 | 20 |
| 26.90 ± 0.20 | 3.312 ± 0.024 | 7 |
| 27.82 ± 0.20 | 3.204 ± 0.023 | 9 |
| 28.24 ± 0.20 | 3.157 ± 0.022 | 17 |

### Compound 1 Oxalate Form B

Compound 1 Oxalate Form B is a 1:1 salt produced by adding aqueous oxalic acid to Compound 1 in hot MEK and forming a slurry.

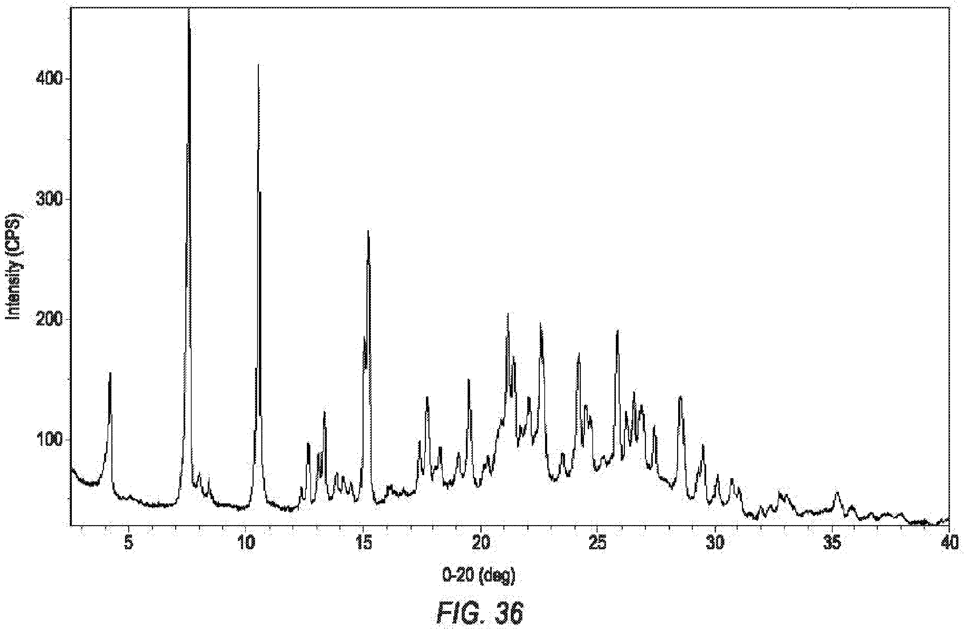

The XRPD pattern for Compound 1 Oxalate Form B is provided in Figure 36, and a list of peaks from the pattern is provided in Table 12 below.

**Table 12: XRPD peaks of Compound 1 Oxalate Form B.**

| **2*θ* (°)** | ***d*-spacing (Å)** | **Intensity (%)** |
|---|---|---|
| 4.20 ± 0.20 | 21.026 ± 1.001 | 34 |
| 7.59 ± 0.20 | 11.640 ± 0.306 | 100 |
| 8.02 ± 0.20 | 11.013 ± 0.274 | 16 |
| 8.42 ± 0.20 | 10.496 ± 0.249 | 15 |
| 10.53 ± 0.20 | 8.391 ± 0.159 | 90 |
| 12.36 ± 0.20 | 7.154 ± 0.115 | 13 |
| 12.66 ± 0.20 | 6.989 ± 0.110 | 21 |
| 13.09 ± 0.20 | 6.759 ± 0.103 | 19 |
| 13.35 ± 0.20 | 6.629 ± 0.099 | 27 |
| 13.88 ± 0.20 | 6.377 ± 0.091 | 16 |
| 14.15 ± 0.20 | 6.254 ± 0.088 | 15 |
| 14.46 ± 0.20 | 6.119 ± 0.084 | 14 |
| 15.06 ± 0.20 | 5.878 ± 0.078 | 40 |
| 15.23 ± 0.20 | 5.815 ± 0.076 | 59 |
| 17.42 ± 0.20 | 5.088 ± 0.058 | 21 |
| 17.75 ± 0.20 | 4.994 ± 0.056 | 29 |
| 18.28 ± 0.20 | 4.850 ± 0.053 | 20 |
| 19.07 ± 0.20 | 4.649 ± 0.048 | 19 |
| 19.52 ± 0.20 | 4.544 ± 0.046 | 32 |
| 20.33 ± 0.20 | 4.365 ± 0.042 | 19 |
| 21.18 ± 0.20 | 4.190 ± 0.039 | 44 |
| 21.43 ± 0.20 | 4.143 ± 0.038 | 37 |
| 21.74 ± 0.20 | 4.085 ± 0.037 | 24 |
| 22.08 ± 0.20 | 4.022 ± 0.036 | 29 |
| 22.59 ± 0.20 | 3.932 ± 0.034 | 43 |
| 23.53 ± 0.20 | 3.778 ± 0.032 | 19 |
| 24.21 ± 0.20 | 3.673 ± 0.030 | 37 |
| 24.52 ± 0.20 | 3.627 ± 0.029 | 28 |
| 24.71 ± 0.20 | 3.600 ± 0.029 | 25 |
| 25.24 ± 0.20 | 3.525 ± 0.027 | 19 |
| 25.85 ± 0.20 | 3.444 ± 0.026 | 41 |
| 26.24 ± 0.20 | 3.393 ± 0.025 | 26 |
| 26.55 ± 0.20 | 3.355 ± 0.025 | 30 |
| 26.90 ± 0.20 | 3.312 ± 0.024 | 28 |
| 27.43 ± 0.20 | 3.249 ± 0.023 | 24 |
| 28.56 ± 0.20 | 3.123 ± 0.021 | 29 |
| 29.30 ± 0.20 | 3.045 ± 0.020 | 17 |
| 29.51 ± 0.20 | 3.024 ± 0.020 | 21 |

### Compound 1 Oxalate Form C

Compound 1 Oxalate Form C is a 1:1 salt produced by adding oxalic acid in hot TFE to Compound 1 and forming a slurry.

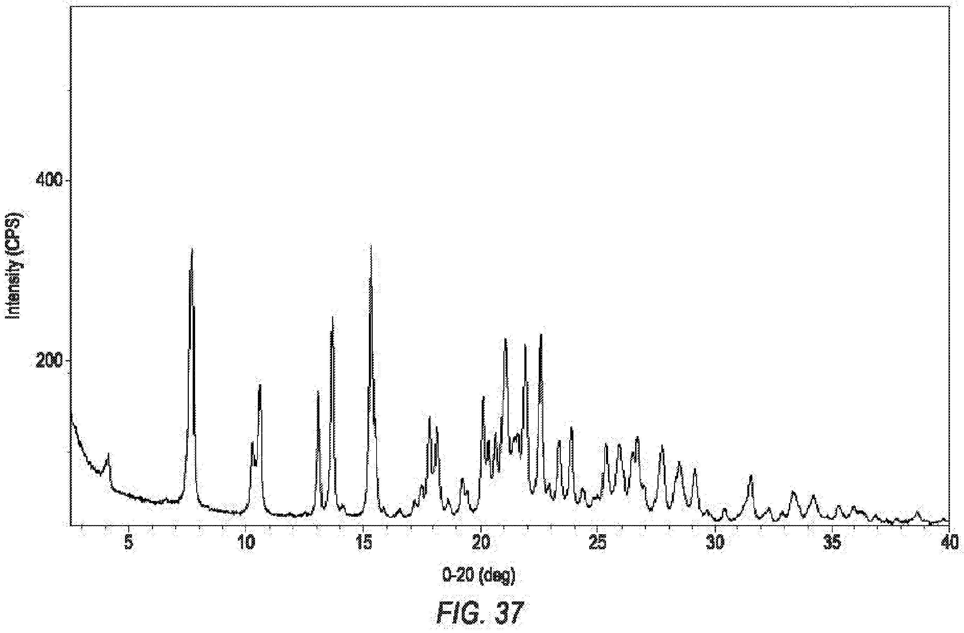

The XRPD pattern for Compound 1 Oxalate Form C is provided in Figure 37, and a list of peaks from the pattern is provided in Table 13 below.

**Table 13: XRPD peaks of Compound 1 Oxalate Form C.**

| **2*θ* (°)** | ***d*-spacing (Å)** | **Intensity (%)** |
|---|---|---|
| 4.13 ± 0.20 | 21.391 ± 1.036 | 30 |
| 7.70 ± 0.20 | 11.473 ± 0.298 | 98 |
| 10.29 ± 0.20 | 8.594 ± 0.167 | 34 |
| 10.59 ± 0.20 | 8.351 ± 0.157 | 53 |
| 13.09 ± 0.20 | 6.760 ± 0.103 | 51 |
| 13.70 ± 0.20 | 6.459 ± 0.094 | 76 |
| 14.13 ± 0.20 | 6.261 ± 0.088 | 13 |
| 15.33 ± 0.20 | 5.774 ± 0.075 | 100 |
| 15.87 ± 0.20 | 5.580 ± 0.070 | 12 |
| 16.57 ± 0.20 | 5.346 ± 0.064 | 12 |
| 17.19 ± 0.20 | 5.154 ± 0.060 | 15 |
| 17.50 ± 0.20 | 5.063 ± 0.057 | 19 |
| 17.83 ± 0.20 | 4.971 ± 0.055 | 42 |
| 18.15 ± 0.20 | 4.883 ± 0.053 | 39 |
| 18.61 ± 0.20 | 4.764 ± 0.051 | 14 |
| 19.24 ± 0.20 | 4.610 ± 0.047 | 22 |
| 19.42 ± 0.20 | 4.567 ± 0.047 | 18 |
| 20.11 ± 0.20 | 4.412 ± 0.043 | 49 |
| 20.34 ± 0.20 | 4.362 ± 0.042 | 34 |
| 20.64 ± 0.20 | 4.299 ± 0.041 | 37 |
| 21.07 ± 0.20 | 4.212 ± 0.040 | 69 |
| 21.47 ± 0.20 | 4.135 ± 0.038 | 35 |
| 21.59 ± 0.20 | 4.112 ± 0.038 | 36 |
| 21.91 ± 0.20 | 4.053 ± 0.037 | 67 |
| 22.56 ± 0.20 | 3.937 ± 0.034 | 69 |
| 22.92 ± 0.20 | 3.877 ± 0.033 | 20 |
| 23.37 ± 0.20 | 3.804 ± 0.032 | 34 |
| 23.87 ± 0.20 | 3.725 ± 0.031 | 39 |
| 24.35 ± 0.20 | 3.653 ± 0.030 | 18 |
| 25.35 ± 0.20 | 3.511 ± 0.027 | 33 |
| 25.91 ± 0.20 | 3.436 ± 0.026 | 33 |
| 26.47 ± 0.20 | 3.364 ± 0.025 | 31 |
| 26.68 ± 0.20 | 3.338 ± 0.025 | 36 |
| 27.74 ± 0.20 | 3.213 ± 0.023 | 32 |
| 28.47 ± 0.20 | 3.132 ± 0.022 | 27 |
| 29.14 ± 0.20 | 3.062 ± 0.021 | 25 |

### Compound 1 Propionate Form A

Compound 1 Propionate Form A is a 1:1 salt produced by adding propionic acid to Compound 1 and slurrying in hot acetone. Compound 1 Propionate Form A was only formed as a minor part of a mixture with a free base form of Compound 1.

The XRPD pattern for Compound 1 Propionate Form A is provided in Figure 38.

### Compound 1 Succinate Form A

Compound 1 Succinate Form A is a salt produced by adding one or two molar equivalent of succinic acid to Compound 1 and slurrying in hot acetone. Compound 1 Succinate Form A was only formed as a mixture with a free base form of Compound 1.

The XRPD pattern for Compound 1 Succinate Form A is provided in Figure 39.

### Compound 1 Sulfate Form A

Compound 1 Sulfate Form A is a 1:1 salt produced by adding one molar equivalents of sulfuric acid to Compound 1 in THF.

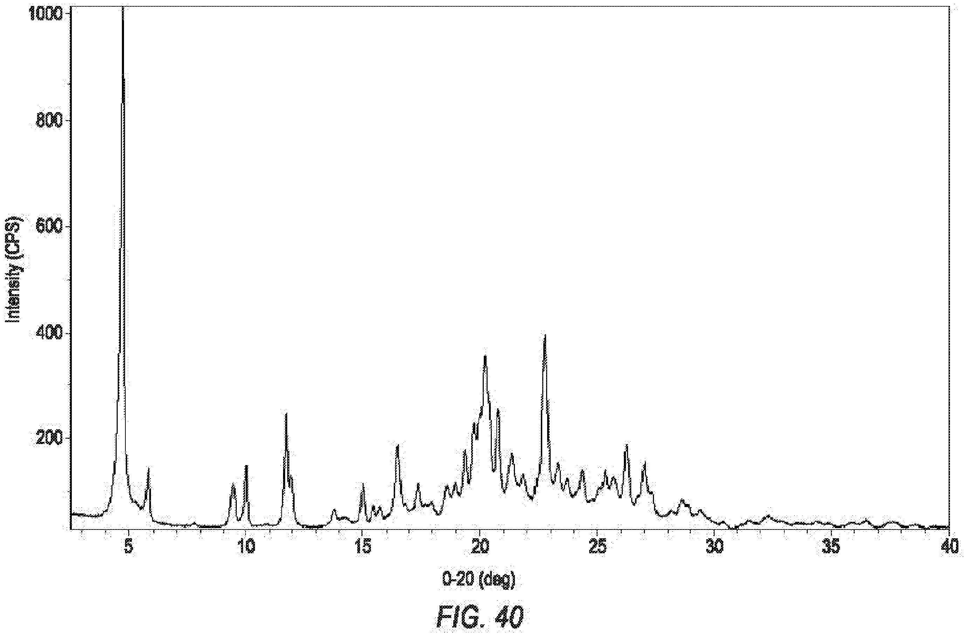

The XRPD pattern for Compound 1 Sulfate Form A is provided in Figure 40, and a list of peaks from the pattern is provided in Table 14 below.

**Table 14: XRPD peaks of Compound 1 Sulfate Form A.**

| **2*θ* (°)** | ***d*-spacing (Å)** | **Intensity (%)** |
|---|---|---|
| 4.74 ± 0.20 | 18.617 ± 0.785 | 100 |
| 5.85 ± 0.20 | 15.104 ± 0.516 | 14 |
| 9.47 ± 0.20 | 9.327 ± 0.196 | 11 |
| 10.04 ± 0.20 | 8.804 ± 0.175 | 15 |
| 11.74 ± 0.20 | 7.534 ± 0.128 | 24 |
| 11.95 ± 0.20 | 7.397 ± 0.123 | 13 |
| 13.77 ± 0.20 | 6.428 ± 0.093 | 7 |
| 14.99 ± 0.20 | 5.905 ± 0.078 | 12 |
| 15.42 ± 0.20 | 5.742 ± 0.074 | 7 |
| 15.71 ± 0.20 | 5.637 ± 0.071 | 7 |
| 16.48 ± 0.20 | 5.374 ± 0.065 | 19 |
| 17.36 ± 0.20 | 5.105 ± 0.058 | 11 |
| 17.66 ± 0.20 | 5.019 ± 0.056 | 8 |
| 17.92 ± 0.20 | 4.946 ± 0.055 | 8 |
| 18.59 ± 0.20 | 4.768 ± 0.051 | 11 |
| 18.95 ± 0.20 | 4.680 ± 0.049 | 12 |
| 19.36 ± 0.20 | 4.581 ± 0.047 | 18 |
| 19.74 ± 0.20 | 4.493 ± 0.045 | 23 |
| 20.22 ± 0.20 | 4.388 ± 0.043 | 35 |
| 20.77 ± 0.20 | 4.272 ± 0.041 | 25 |
| 21.38 ± 0.20 | 4.153 ± 0.038 | 17 |
| 21.86 ± 0.20 | 4.063 ± 0.037 | 13 |
| 22.74 ± 0.20 | 3.907 ± 0.034 | 39 |
| 23.30 ± 0.20 | 3.814 ± 0.032 | 15 |
| 23.70 ± 0.20 | 3.751 ± 0.031 | 12 |
| 24.33 ± 0.20 | 3.655 ± 0.030 | 14 |
| 25.07 ± 0.20 | 3.549 ± 0.028 | 11 |
| 25.32 ± 0.20 | 3.515 ± 0.027 | 14 |
| 25.67 ± 0.20 | 3.467 ± 0.027 | 13 |
| 26.23 ± 0.20 | 3.395 ± 0.025 | 19 |
| 27.00 ± 0.20 | 3.299 ± 0.024 | 15 |

Compound 1 Sulfate Form B

Compound 1 Sulfate Form B is a 1:1 salt produced by adding one molar equivalent of sulfuric acid to Compound 1 in hot methanol.

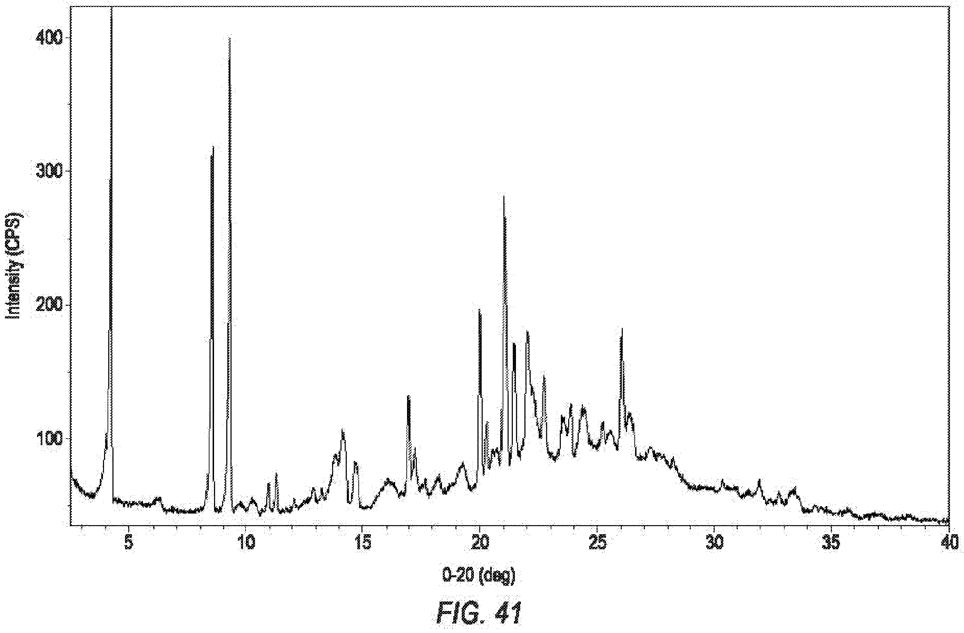

The XRPD pattern for Compound 1 Sulfate Form B is provided in Figure 41, and a list of peaks from the pattern is provided in Table 15 below.

**Table 15: XRPD peaks of Compound 1 Sulfate Form B.**

| **2*θ* (°)** | ***d*-spacing (Å)** | **Intensity (%)** |
|---|---|---|
| 4.27 ± 0.20 | 20.683 ± 0.969 | 100 |
| 8.57 ± 0.20 | 10.313 ± 0.240 | 75 |
| 9.32 ± 0.20 | 9.481 ± 0.203 | 94 |
| 10.95 ± 0.20 | 8.071 ± 0.147 | 16 |
| 11.29 ± 0.20 | 7.834 ± 0.138 | 18 |
| 12.07 ± 0.20 | 7.329 ± 0.121 | 13 |
| 12.87 ± 0.20 | 6.875 ± 0.106 | 15 |
| 13.25 ± 0.20 | 6.678 ± 0.100 | 15 |
| 13.82 ± 0.20 | 6.404 ± 0.092 | 21 |
| 14.13 ± 0.20 | 6.264 ± 0.088 | 25 |
| 14.67 ± 0.20 | 6.032 ± 0.082 | 20 |
| 16.94 ± 0.20 | 5.230 ± 0.061 | 32 |
| 17.21 ± 0.20 | 5.147 ± 0.059 | 23 |
| 17.64 ± 0.20 | 5.024 ± 0.057 | 17 |
| 19.99 ± 0.20 | 4.439 ± 0.044 | 48 |
| 20.26 ± 0.20 | 4.380 ± 0.043 | 26 |
| 21.03 ± 0.20 | 4.221 ± 0.040 | 67 |
| 21.44 ± 0.20 | 4.141 ± 0.038 | 41 |
| 22.00 ± 0.20 | 4.036 ± 0.036 | 43 |
| 22.71 ± 0.20 | 3.913 ± 0.034 | 35 |
| 23.85 ± 0.20 | 3.728 ± 0.031 | 30 |
| 25.20 ± 0.20 | 3.531 ± 0.028 | 27 |
| 26.02 ± 0.20 | 3.422 ± 0.026 | 44 |

### Compound 1 Sulfate Form C

Compound 1 Sulfate Form C is a 1:1 salt produced by adding one molar equivalent of sulfuric acid to Compound 1 in hot chloroform.

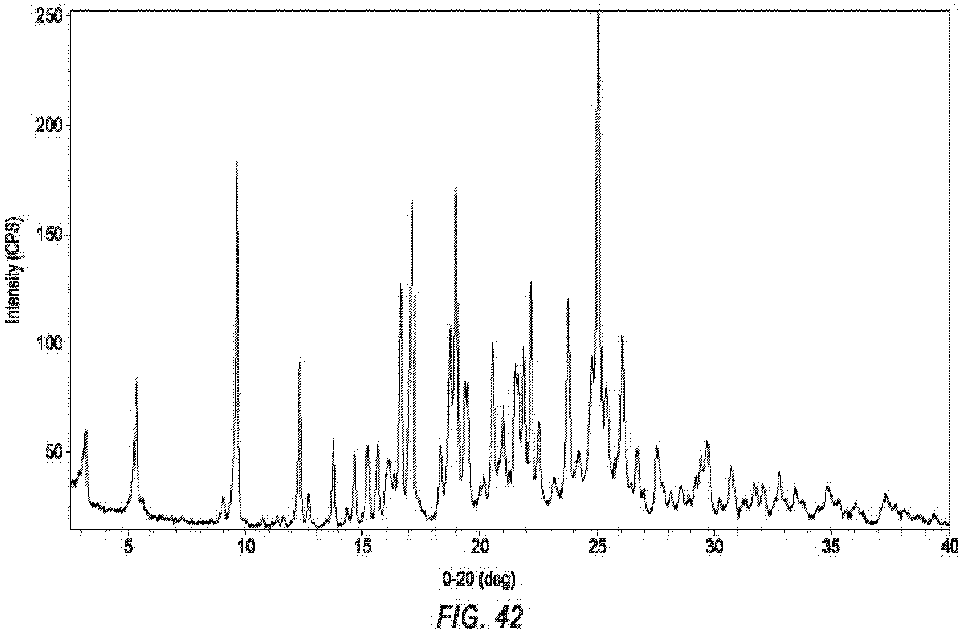

The XRPD pattern for Compound 1 Sulfate Form C is provided in Figure 42, and a list of peaks from the pattern is provided in Table 16 below.

**Table 16: XRPD peaks of Compound 1 Sulfate Form B.**

| **2*θ* (°)** | ***d*-spacing (Å)** | **Intensity (%)** |
|---|---|---|
| 3.19 ± 0.20 | 27.658 ± 1.733 | 23 |
| 5.35 ± 0.20 | 16.519 ± 0.618 | 33 |
| 9.04 ± 0.20 | 9.776 ± 0.216 | 12 |
| 9.62 ± 0.20 | 9.182 ± 0.190 | 72 |
| 10.73 ± 0.20 | 8.239 ± 0.153 | 8 |
| 11.31 ± 0.20 | 7.820 ± 0.138 | 8 |
| 11.60 ± 0.20 | 7.619 ± 0.131 | 8 |
| 12.27 ± 0.20 | 7.208 ± 0.117 | 36 |
| 12.67 ± 0.20 | 6.978 ± 0.110 | 12 |
| 13.76 ± 0.20 | 6.432 ± 0.093 | 22 |
| 14.31 ± 0.20 | 6.183 ± 0.086 | 10 |
| 14.65 ± 0.20 | 6.040 ± 0.082 | 20 |
| 15.21 ± 0.20 | 5.820 ± 0.076 | 21 |
| 15.63 ± 0.20 | 5.664 ± 0.072 | 21 |
| 16.11 ± 0.20 | 5.498 ± 0.068 | 18 |
| 16.33 ± 0.20 | 5.424 ± 0.066 | 16 |
| 16.63 ± 0.20 | 5.327 ± 0.064 | 51 |
| 17.11 ± 0.20 | 5.177 ± 0.060 | 66 |
| 18.32 ± 0.20 | 4.839 ± 0.052 | 21 |
| 18.75 ± 0.20 | 4.730 ± 0.050 | 42 |
| 18.99 ± 0.20 | 4.670 ± 0.049 | 68 |
| 19.36 ± 0.20 | 4.582 ± 0.047 | 32 |
| 19.47 ± 0.20 | 4.555 ± 0.046 | 32 |
| 20.16 ± 0.20 | 4.401 ± 0.043 | 15 |
| 20.55 ± 0.20 | 4.318 ± 0.042 | 39 |
| 21.01 ± 0.20 | 4.224 ± 0.040 | 28 |
| 21.52 ± 0.20 | 4.125 ± 0.038 | 36 |
| 21.62 ± 0.20 | 4.107 ± 0.038 | 34 |
| 21.89 ± 0.20 | 4.056 ± 0.037 | 39 |
| 22.19 ± 0.20 | 4.004 ± 0.036 | 50 |
| 22.53 ± 0.20 | 3.943 ± 0.035 | 25 |
| 23.19 ± 0.20 | 3.833 ± 0.033 | 15 |
| 23.79 ± 0.20 | 3.737 ± 0.031 | 47 |
| 24.23 ± 0.20 | 3.670 ± 0.030 | 20 |
| 24.81 ± 0.20 | 3.586 ± 0.028 | 37 |
| 25.07 ± 0.20 | 3.549 ± 0.028 | 100 |
| 25.41 ± 0.20 | 3.502 ± 0.027 | 31 |
| 26.06 ± 0.20 | 3.416 ± 0.026 | 41 |
| 26.73 ± 0.20 | 3.332 ± 0.024 | 20 |
| 27.58 ± 0.20 | 3.231 ± 0.023 | 21 |
| 28.16 ± 0.20 | 3.167 ± 0.022 | 13 |
| 28.63 ± 0.20 | 3.116 ± 0.021 | 13 |
| 28.90 ± 0.20 | 3.087 ± 0.021 | 12 |
| 29.22 ± 0.20 | 3.054 ± 0.020 | 16 |
| 29.47 ± 0.20 | 3.029 ± 0.020 | 19 |
| 29.73 ± 0.20 | 3.003 ± 0.020 | 21 |

### Compound 1 Tartrate Form A

Compound 1 Tartrate Form A is a 1:1 salt produced by adding one molar equivalent of L-tartaric acid to Compound 1 in THF. Compound 1 Tartrate Form A was only formed as a mixture with a free base form of Compound 1.

The XRPD pattern for Compound 1 Tartrate Form A is provided in Figure 43.

### Compound 1 Hemitartrate Form B

Compound 1 Hemitartrate Form B is a 2:1 salt produced by adding one molar equivalent of L-tartaric acid to Compound 1 in acetone. The material also contains about 0.7 molar equivalents of acetone.

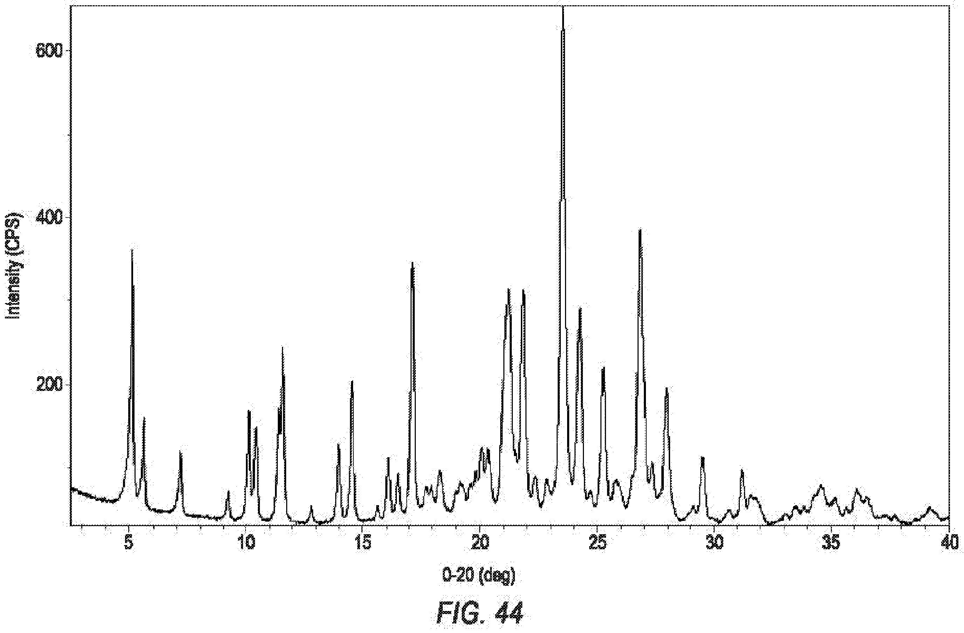

The XRPD pattern for Compound 1 Hemitartrate Form B is provided in Figure 44 and a list of peaks from the pattern is provided in Table 17 below.

**Table 17: XRPD peaks of Compound 1 Hemitartrate Form B**

| **2*θ* (°)** | ***d*-spacing (Å)** | **Intensity (%)** |
|---|---|---|
| 5.17 ± 0.20 | 17.079 ± 0.660 | 55 |
| 5.66 ± 0.20 | 15.592 ± 0.550 | 24 |
| 7.22 ± 0.20 | 12.229 ± 0.338 | 18 |
| 9.27 ± 0.20 | 9.534 ± 0.205 | 11 |
| 10.10 ± 0.20 | 8.752 ± 0.173 | 26 |
| 10.40 ± 0.20 | 8.503 ± 0.163 | 23 |
| 11.37 ± 0.20 | 7.776 ± 0.136 | 26 |
| 11.55 ± 0.20 | 7.659 ± 0.132 | 37 |
| 12.77 ± 0.20 | 6.929 ± 0.108 | 8 |
| 13.94 ± 0.20 | 6.346 ± 0.091 | 19 |
| 14.51 ± 0.20 | 6.100 ± 0.084 | 31 |
| 15.60 ± 0.20 | 5.676 ± 0.072 | 8 |
| 16.06 ± 0.20 | 5.514 ± 0.068 | 17 |
| 16.48 ± 0.20 | 5.374 ± 0.065 | 14 |
| 17.09 ± 0.20 | 5.184 ± 0.060 | 53 |
| 17.69 ± 0.20 | 5.011 ± 0.056 | 12 |
| 17.90 ± 0.20 | 4.951 ± 0.055 | 12 |
| 18.27 ± 0.20 | 4.851 ± 0.053 | 15 |
| 18.94 ± 0.20 | 4.681 ± 0.049 | 11 |
| 19.15 ± 0.20 | 4.630 ± 0.048 | 13 |
| 19.59 ± 0.20 | 4.529 ± 0.046 | 12 |
| 19.81 ± 0.20 | 4.478 ± 0.045 | 15 |
| 20.06 ± 0.20 | 4.423 ± 0.044 | 19 |
| 20.32 ± 0.20 | 4.367 ± 0.043 | 19 |
| 21.23 ± 0.20 | 4.182 ± 0.039 | 48 |
| 21.83 ± 0.20 | 4.068 ± 0.037 | 48 |
| 22.34 ± 0.20 | 3.976 ± 0.035 | 14 |
| 22.84 ± 0.20 | 3.891 ± 0.034 | 13 |
| 23.53 ± 0.20 | 3.778 ± 0.032 | 100 |
| 24.26 ± 0.20 | 3.666 ± 0.030 | 45 |
| 24.70 ± 0.20 | 3.601 ± 0.029 | 11 |
| 25.26 ± 0.20 | 3.523 ± 0.027 | 34 |
| 25.80 ± 0.20 | 3.450 ± 0.026 | 13 |
| 26.78 ± 0.20 | 3.326 ± 0.024 | 59 |
| 27.29 ± 0.20 | 3.266 ± 0.023 | 16 |
| 27.90 ± 0.20 | 3.195 ± 0.022 | 30 |
| 29.04 ± 0.20 | 3.073 ± 0.021 | 8 |
| 29.45 ± 0.20 | 3.030 ± 0.020 | 17 |

The XRPD pattern of Compound 1 Hemitartrate Form B was successfully indexed, which indicates the material is composed of a single crystalline phase.

### Unit cell data for Compound 1 Hemitartrate Form B

| Bravais Type | Primitive Monoclinic |
|---|---|
| a [Å] | 5.356 |
| b [Å] | 34.062 |
| c [Å] | 17.503 |
| a [deg] | 90 |
| β [deg] | 92.11 |
| γ [deg] | 90 |
| Volume [Å³/cell] | 3,191.0 |
| Chiral Contents? | Chiral |
| Extinction Symbol | P 1 2₁ 1 |
| Space Group(s) | P2₁ (4) |

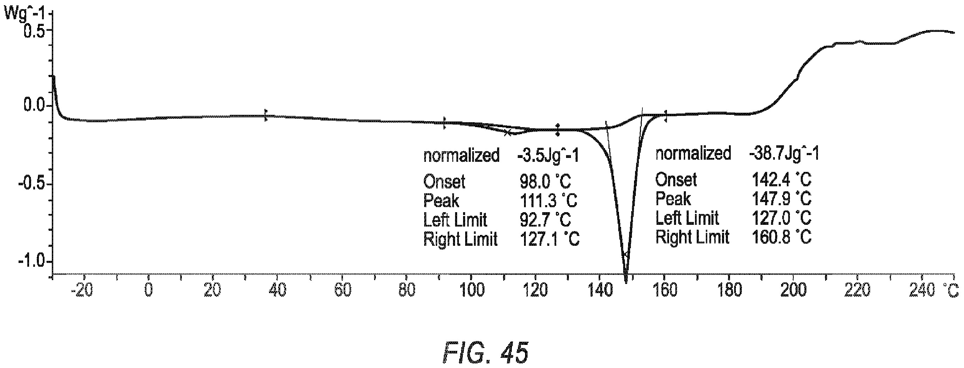

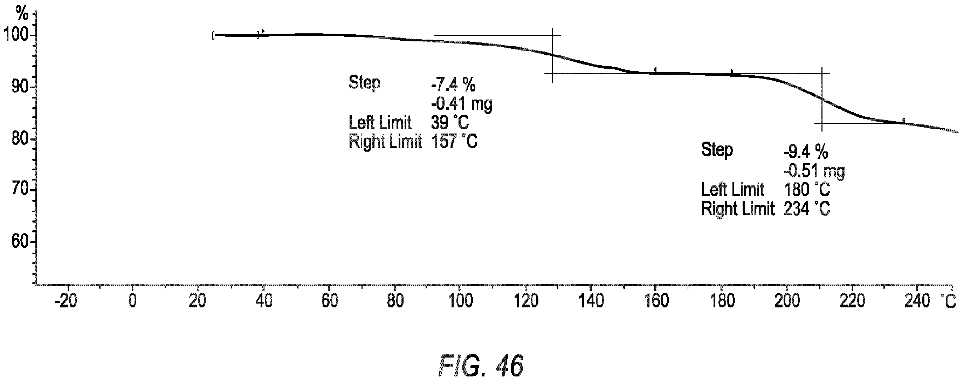

The DSC thermogram for Compound 1 Hemitartrate Form B is provided in Figure 45 and shows a broad endotherm at ∼111 °C, and another sharp endotherm at ∼148 °C. TGA, Figure 46, indicates a ~7.4% weight loss over the temperature range of 39-157 °C, corresponding to 0.8 molar equivalents of acetone.

### Compound 1 Tosylate Form A

Compound 1 Tosylate Form A is a 1:1 salt produced by adding one molar equivalent of p-toluenesulfonic acid to Compound 1 in hot acetone. The material also contains about ~0.2 molar equivalents of acetone.

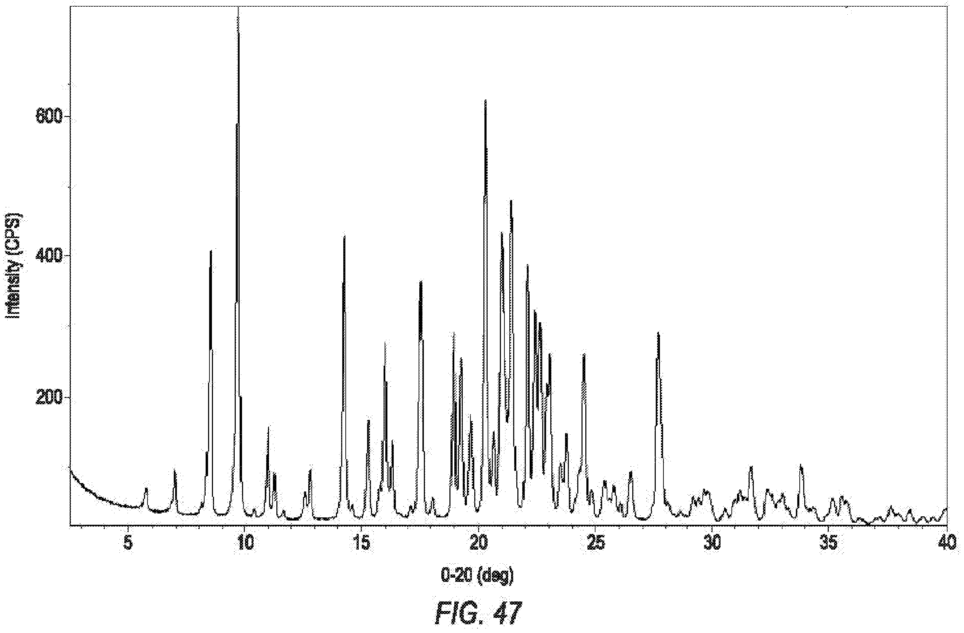

The XRPD pattern for Compound 1 Tosylate Form A is provided in Figure 47 and a list of peaks from the pattern is provided in Table 18 below.

**Table 18: XRPD peaks of Compound 1 Tosylate Form A**

| **2*θ* (°)** | ***d*-spacing (Å)** | **Intensity (%)** |
|---|---|---|
| 5.81 ± 0.20 | 15.205 ± 0.523 | 10 |
| 7.05 ± 0.20 | 12.532 ± 0.355 | 13 |
| 8.52 ± 0.20 | 10.364 ± 0.243 | 55 |
| 9.74 ± 0.20 | 9.076 ± 0.186 | 100 |
| 10.40 ± 0.20 | 8.497 ± 0.163 | 5 |
| 10.99 ± 0.20 | 8.042 ± 0.146 | 21 |
| 11.26 ± 0.20 | 7.849 ± 0.139 | 12 |
| 11.66 ± 0.20 | 7.582 ± 0.130 | 5 |
| 12.58 ± 0.20 | 7.030 ± 0.111 | 9 |
| 12.81 ± 0.20 | 6.904 ± 0.107 | 13 |
| 14.28 ± 0.20 | 6.199 ± 0.086 | 57 |
| 14.62 ± 0.20 | 6.053 ± 0.082 | 6 |
| 15.30 ± 0.20 | 5.787 ± 0.075 | 23 |
| 15.80 ± 0.20 | 5.606 ± 0.071 | 10 |
| 16.03 ± 0.20 | 5.524 ± 0.068 | 37 |
| 16.34 ± 0.20 | 5.420 ± 0.066 | 18 |
| 17.12 ± 0.20 | 5.174 ± 0.060 | 6 |
| 17.55 ± 0.20 | 5.050 ± 0.057 | 49 |
| 18.03 ± 0.20 | 4.917 ± 0.054 | 7 |
| 18.94 ± 0.20 | 4.682 ± 0.049 | 39 |
| 19.26 ± 0.20 | 4.605 ± 0.047 | 34 |
| 19.67 ± 0.20 | 4.509 ± 0.045 | 23 |
| 20.31 ± 0.20 | 4.370 ± 0.043 | 84 |
| 20.65 ± 0.20 | 4.297 ± 0.041 | 20 |
| 21.02 ± 0.20 | 4.222 ± 0.040 | 58 |
| 21.43 ± 0.20 | 4.143 ± 0.038 | 64 |
| 22.11 ± 0.20 | 4.018 ± 0.036 | 52 |
| 22.44 ± 0.20 | 3.960 ± 0.035 | 43 |
| 22.66 ± 0.20 | 3.921 ± 0.034 | 41 |
| 22.94 ± 0.20 | 3.874 ± 0.033 | 29 |
| 23.07 ± 0.20 | 3.851 ± 0.033 | 35 |
| 23.54 ± 0.20 | 3.776 ± 0.032 | 14 |
| 23.79 ± 0.20 | 3.737 ± 0.031 | 20 |
| 24.55 ± 0.20 | 3.623 ± 0.029 | 35 |
| 24.88 ± 0.20 | 3.576 ± 0.028 | 9 |
| 25.42 ± 0.20 | 3.501 ± 0.027 | 11 |
| 25.83 ± 0.20 | 3.446 ± 0.026 | 9 |
| 26.10 ± 0.20 | 3.412 ± 0.026 | 6 |
| 26.53 ± 0.20 | 3.357 ± 0.025 | 12 |
| 27.70 ± 0.20 | 3.218 ± 0.023 | 39 |
| 28.11 ± 0.20 | 3.172 ± 0.022 | 7 |
| 28.62 ± 0.20 | 3.116 ± 0.021 | 5 |
| 29.18 ± 0.20 | 3.058 ± 0.021 | 8 |
| 29.40 ± 0.20 | 3.036 ± 0.020 | 7 |
| 29.66 ± 0.20 | 3.010 ± 0.020 | 9 |
| 29.83 ± 0.20 | 2.993 ± 0.020 | 9 |

The XRPD pattern of Compound 1 Tosylate Form A was successfully indexed, which indicates the material is composed of a single crystalline phase.

### Unit cell data for Compound 1 Tosylate Form A

| Bravais Type | Triclinic |
|---|---|
| a [Å] | 8.824 |
| b [Å] | 13.010 |
| c [Å] | 15.364 |
| α [deg] | 96.03 |
| β [deg] | 95 62 |
| γ [deg] | 103.96 |
| Volume [Å⁵]/cell] | 1,688.2 |
| Chiral Contents? | Not Specified |
| Extinction Symbol | P - |
| Space Group(s) | P1 (1), P1̅ (2) |

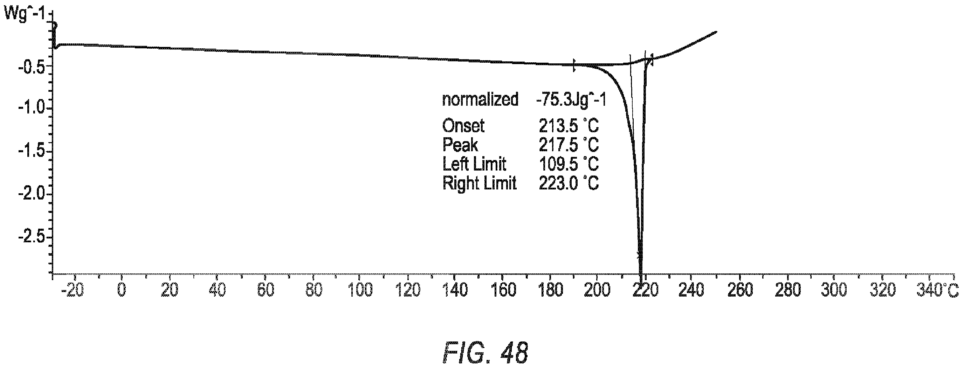

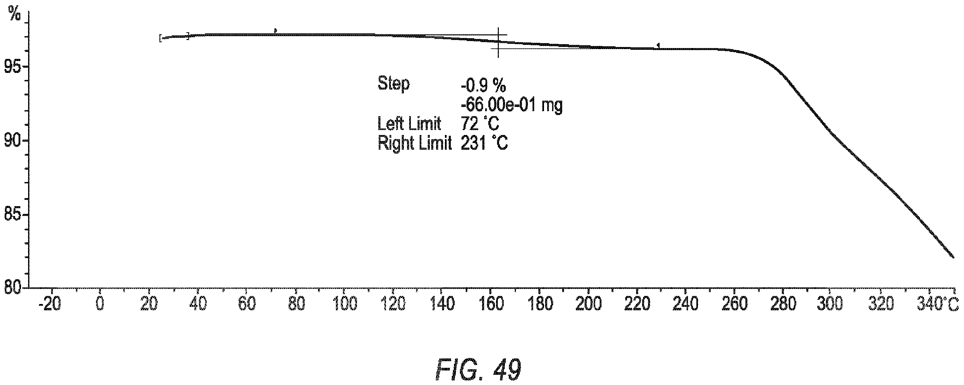

The DSC thermogram for Compound 1 Tosylate Form A is provided in Figure 48 and shows a sharp endotherm with an onset at about 214 °C. TGA, Figure 49, indicates a ∼0.9% weight loss over the temperature range of 72-231 °C, corresponding to 0.1 molar equivalents of acetone.

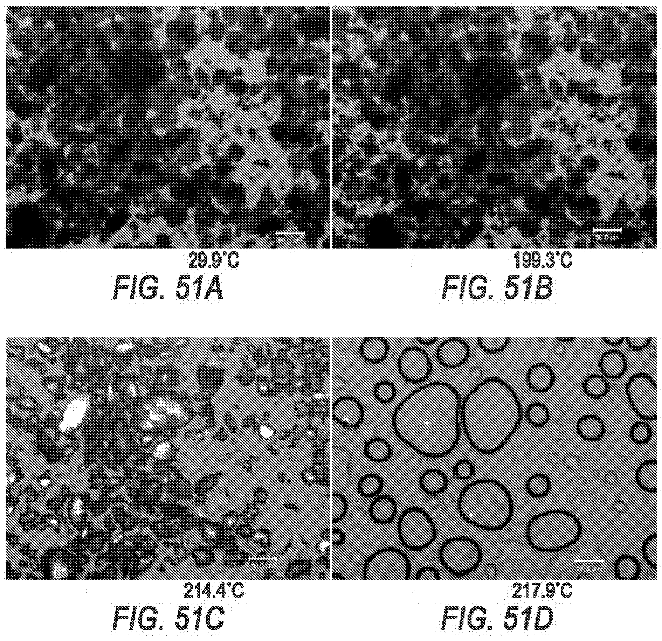

Hot stage photomicrographs, shown in Figures 51A - 51D confirm the melting temperature of Compound 1 Tosylate Form A to be about 214 °C.

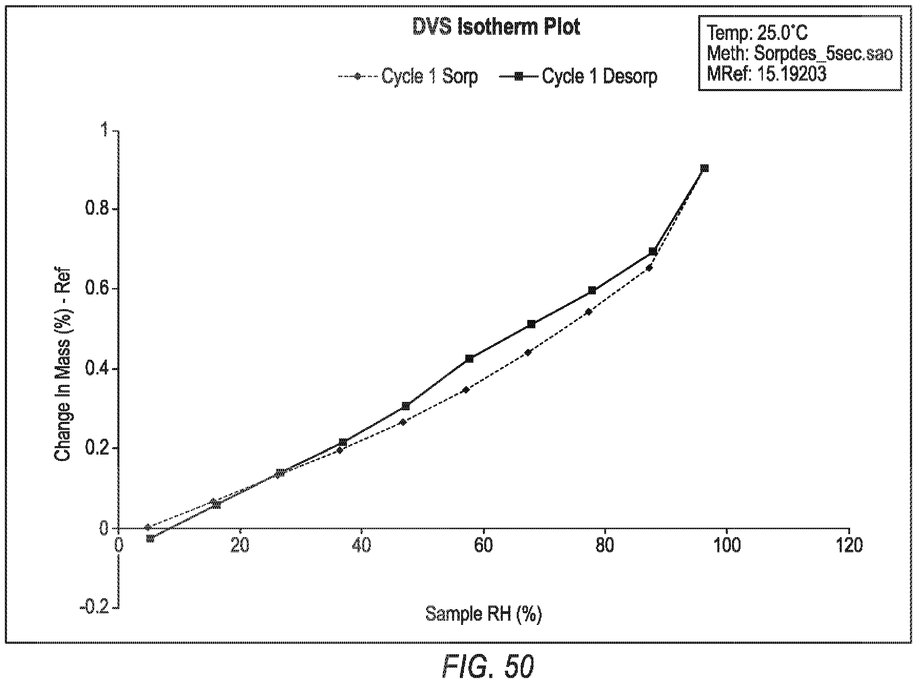

The DVS isotherm (Figure 50) indicates gains/losses of about 0.9% weight through the sorption/desorption experiment (RH = 5% - 95%).

### Compound 1 Tosylate Form B

Compound 1 Tosylate Form B is a 1:1 salt produced during the scaled up procedure of Compound 1 Tosylate Form A.

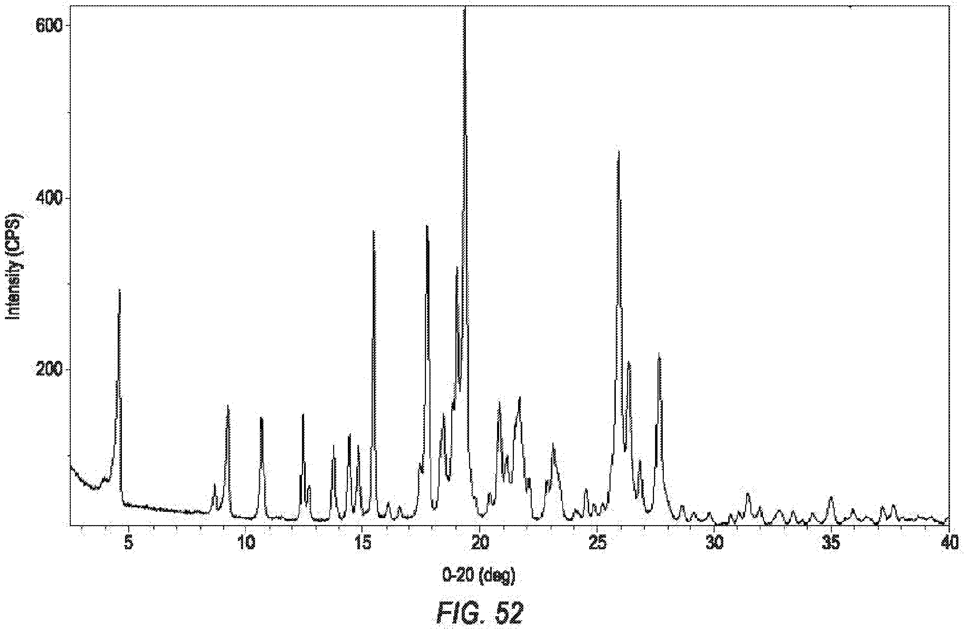

The XRPD pattern for Compound 1 Tosylate Form B is provided in Figure 52 and a list of peaks from the pattern is provided in Table 19 below.

**Table 19: XRPD peaks of Compound 1 Tosylate Form B**

| **2*θ* (°)** | ***d*-spacing (Å)** | **Intensity (%)** |
|---|---|---|
| 4.61 ± 0.20 | 19.171 ± 0.832 | 47 |
| 8.69 ± 0.20 | 10.171 ± 0.234 | 10 |
| 9.25 ± 0.20 | 9.550 ± 0.206 | 25 |
| 10.68 ± 0.20 | 8.274 ± 0.154 | 23 |
| 12.44 ± 0.20 | 7.107 ± 0.114 | 24 |
| 12.69 ± 0.20 | 6.972 ± 0.109 | 10 |
| 13.76 ± 0.20 | 6.429 ± 0.093 | 18 |
| 14.42 ± 0.20 | 6.138 ± 0.085 | 20 |
| 14.81 ± 0.20 | 5.975 ± 0.080 | 18 |
| 15.47 ± 0.20 | 5.725 ± 0.074 | 58 |
| 16.08 ± 0.20 | 5.509 ± 0.068 | 7 |
| 16.57 ± 0.20 | 5.345 ± 0.064 | 7 |
| 17.45 ± 0.20 | 5.078 ± 0.058 | 15 |
| 17.76 ± 0.20 | 4.991 ± 0.056 | 59 |
| 18.44 ± 0.20 | 4.808 ± 0.052 | 24 |
| 18.83 ± 0.20 | 4.709 ± 0.050 | 26 |
| 19.01 ± 0.20 | 4.665 ± 0.049 | 51 |
| 19.35 ± 0.20 | 4.584 ± 0.047 | 100 |
| 20.42 ± 0.20 | 4.345 ± 0.042 | 9 |
| 20.84 ± 0.20 | 4.259 ± 0.040 | 26 |
| 21.15 ± 0.20 | 4.198 ± 0.039 | 16 |
| 21.69 ± 0.20 | 4.095 ± 0.037 | 27 |
| 22.09 ± 0.20 | 4.021 ± 0.036 | 12 |
| 22.86 ± 0.20 | 3.887 ± 0.034 | 11 |
| 23.12 ± 0.20 | 3.843 ± 0.033 | 18 |
| 24.08 ± 0.20 | 3.692 ± 0.030 | 6 |
| 24.53 ± 0.20 | 3.626 ± 0.029 | 10 |
| 24.86 ± 0.20 | 3.579 ± 0.028 | 7 |
| 25.22 ± 0.20 | 3.528 ± 0.028 | 7 |
| 25.91 ± 0.20 | 3.436 ± 0.026 | 73 |
| 26.33 ± 0.20 | 3.382 ± 0.025 | 34 |
| 26.82 ± 0.20 | 3.321 ± 0.024 | 15 |
| 27.63 ± 0.20 | 3.226 ± 0.023 | 35 |
| 28.61 ± 0.20 | 3.118 ± 0.021 | 7 |
| 29.11 ± 0.20 | 3.066 ± 0.021 | 5 |
| 29.77 ± 0.20 | 2.999 ± 0.020 | 5 |

The XRPD pattern of Compound 1 Tosylate Form B was successfully indexed, which indicates the material is composed of a single crystalline phase.

### Unit cell data for Compound 1 Tosylate Form B

| Bravais Type | Triclinic |
|---|---|
| a [Å] | 8.501 |
| b [Å] | 10.393 |
| c [Å] | 19.667 |
| α [deg] | 98.40 |
| β [deg] | 99.75 |
| γ [deg] | 97.34 |
| Volume [Å³/cell] | 1,673.3 |
| Chiral Contents? | Achiral |
| Extinction Symbol | P - |
| Space Croup(s) | P1 (1), P1̅ (2) |

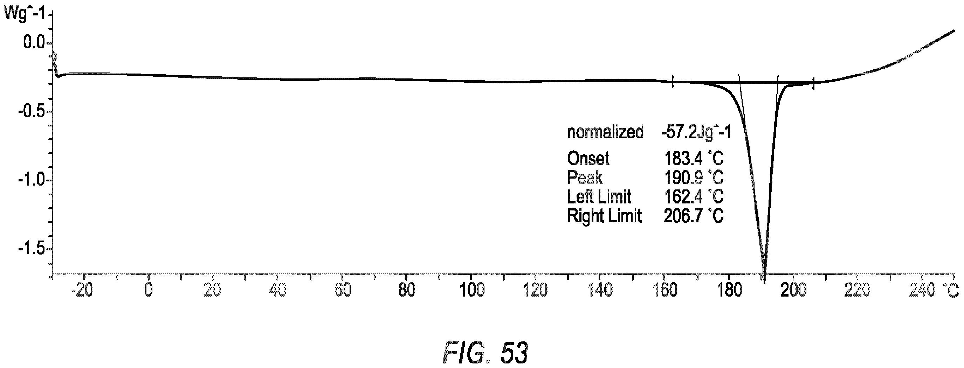

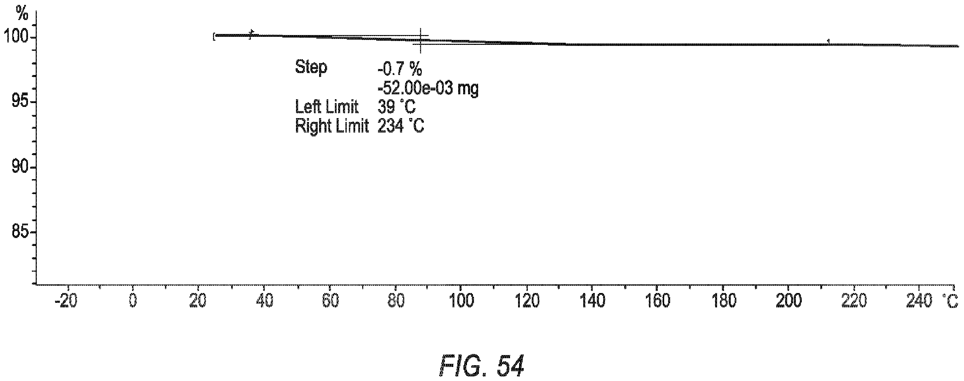

The DSC thermogram for Compound 1 Tosylate Form B is provided in Figure 53 and shows a sharp endotherm at ∼183 °C. TGA, Figure 54, indicates a ∼0.7% weight loss over the temperature range of 39-214 °C.

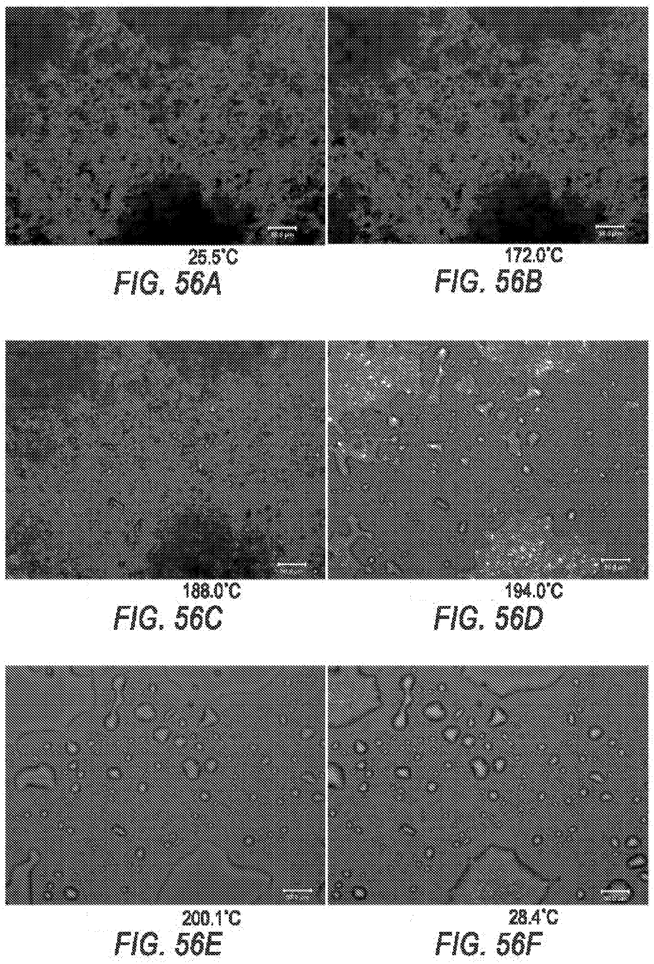

Hot stage photomicrographs, shown in Figures 56A - 56F shows that Compound 1 Tosylate Form B begins to melt at -183 °C, is still melting at -194 °C, and is not completely melted until 200 °C.

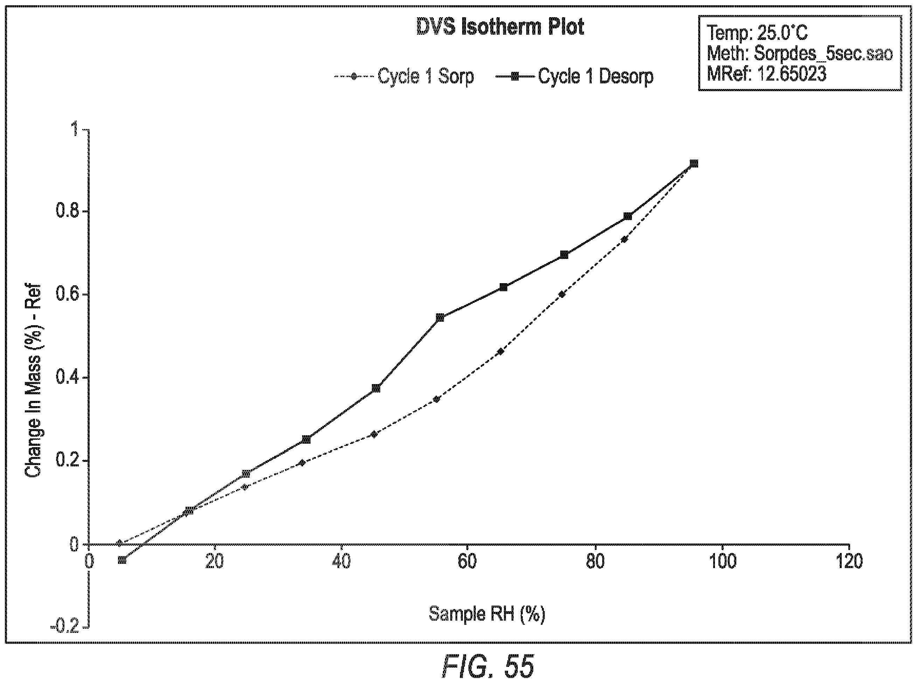

The DVS isotherm (Figure 55) indicates gains/losses of about ∼0.9% weight through the sorption/desorption experiment (RH = 5% - 95%).

### GENERAL ADMINISTRATION

The invention provides a pharmaceutical composition comprising a crystalline salt form of the invention and a pharmaceutically acceptable excipient. The invention also provides a crystalline salt form of the invention, or a pharmaceutical composition of the invention, for use in a method of treating cancer. Methods of treatment are disclosed herein for illustrative purposes, but are not claimed.

Administration of the crystalline salt forms of Compound 1, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally (intravenous, intramuscular, or subcutaneous), topically, transdermally, intravaginally, intravesically, intracistemally, or rectally, in the form of solid, semi-solid, lyophilized powder, or liquid dosage forms, such as, for example, tablets, suppositories, pills, soft elastic and hard gelatin capsules, powders, solutions, suspensions, aerosols, and the like, preferably in unit dosage forms suitable for simple administration of precise dosages.

The compositions will include a conventional pharmaceutical excipient and a crystalline salt form of Compound 1 as the/an active agent, and, in addition, may include other medicinal agents, pharmaceutical agents, adjuvants, etc. Compositions of the invention may be used in combination with anticancer or other agents that are generally administered to a patient being treated for cancer. Adjuvants include preserving, wetting, suspending, sweetening, flavoring, perfuming, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate, and gelatin.

If desired, a pharmaceutical composition of the invention may also contain minor amounts of auxiliary substances such as wetting or emulsifying agents, pH buffering agents, antioxidants, and the like, such as, for example, citric acid, sorbitan, monolaurate, triethanolamine oleate, butylalted hydroxytoluene, etc.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous excipients, diluents, solvents, or vehicles include water, ethanol, polyols (propyleneglycol, polyethyleneglycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions, and by the use of surfactants.

One preferable route of administration is oral, using a convenient daily dosage regimen that can be adjusted according to the degree of severity of the disease-state to be treated.

Solid dosage forms for oral administration include capsules, tablets, pills, powders, and granules. In such solid dosage forms, the active compound is admixed with at least one inert customary excipient such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate, and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid dosage forms as described above can be prepared with coatings and shells, such as enteric coatings and others well known in the art. They may contain pacifying agents and can also be of such composition that they release the active compound or compounds in a certain part of the intestinal tract in a delayed manner. Examples of embedded compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., a crystalline salt form of Compound 1, and optional pharmaceutical adjuvants in an excipient, such as, for example, water, saline, aqueous dextrose, glycerol, ethanol, and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, and dimethylformamide; oils, in particular, cottonseed oil, groundnut oil, corn germ oil, olive oil, castor oil, and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol, and sorbitan esters, microcrystalline cellulose, aluminum metahydroxide, bentonite, agar-agar, and tragacanth, or mixtures of these substances, and the like.

Compositions for rectal administrations are, for example, suppositories that can be prepared by mixing the compounds of the present invention with for example suitable nonirritating excipients or excipients such as cocoa butter, polyethyleneglycol, or a suppository wax, which are solid at ordinary temperatures but liquid at body temperature and therefore melt while in a suitable body cavity and release the active component therein.

Dosage forms for topical administration of a compound of this invention include ointments, powders, sprays, and inhalants. The active component is admixed under sterile conditions with a physiologically acceptable excipient and any preservatives, buffers, or propellants as may be required. Ophthalmic formulations, eye ointments, powders, and solutions are also contemplated as being within the scope of this invention.

Generally, depending on the intended mode of administration, the pharmaceutically acceptable compositions will contain about 1% to about 99% by weight of a crystalline salt form of Compound 1, and 99% to 1% by weight of a suitable pharmaceutical excipient. In one example, the composition will be between about 5% and about 75% by weight of a crystalline salt form of Compound 1, with the rest being suitable pharmaceutical excipients.

Actual methods of preparing such dosage forms are known, or will be apparent, to those skilled in this art; for example, see Remington's Pharmaceutical Sciences, 21st Ed., (Lippincott, Williams and Wilkins Philadelphia, PA, 2006). The composition to be administered will, in any event, contain a therapeutically effective amount of a crystalline salt form of Compound 1, for treatment of a disease-state in accordance with the teachings of this invention.

The crystalline salt forms of Compound 1, are administered in a therapeutically effective amount which will vary depending upon a variety of factors including the activity of Compound 1, the metabolic stability and length of action of Compound 1, the age, body weight, general health, sex, diet, mode, and time of administration, rate of excretion, drug combination, the severity of the particular disease-states, and the host undergoing therapy. The crystalline salt forms of Compound 1 can be administered to a patient at dosage levels in the range of about 0.1 to about 1,000 mg per day. For a normal human adult having a body weight of about 70 kilograms, a dosage in the range of about 0.01 to about 100 mg per kilogram of body weight per day is an example. The specific dosage used, however, can vary. For example, the dosage can depend on a number of factors including the requirements of the patient, the severity of the condition being treated, and the pharmacological activity of the compound being used. The determination of optimum dosages for a particular patient is well known to one of ordinary skill in the art.

### Combination Therapy

A crystalline salt form of Compound 1 as disclosed herein can be administered as a single therapy or in combination ("co-administered") with one or more additional therapies for the treatment of a disease or disorder, for instance a disease or disorder associated with hyper-proliferation such as cancer. Therapies that may be used in combination with a compound disclosed herein include: (i) surgery; (ii) radiotherapy (for example, gamma radiation, neutron beam radiotherapy, electron beam radiotherapy, proton therapy, brachytherapy, and systemic radioactive isotopes); (iii) endocrine therapy; (iv) adjuvant therapy, immunotherapy, CAR T-cell therapy; and (v) other chemotherapeutic agents.

The term "co-administered" ("co-administering") refers to either simultaneous administration, or any manner of separate sequential administration, of a crystalline salt form of Compound 1 as disclosed herein, and a further active pharmaceutical ingredient or ingredients, including cytotoxic agents and radiation treatment. If the administration is not simultaneous, the compounds are administered in a close time proximity to each other. Furthermore, it does not matter if the compounds are administered in the same dosage form, e.g. one compound may be administered topically and another compound may be administered orally.

Typically, any agent that has activity against a disease or condition being treated may be co-administered. Examples of such agents for cancer treatment can be found, for instance, at https://www.cancer.gov/about-cancer/treatment/drugs (last visited January 22, 2019) and in publically available sources such as Cancer Principles and Practice of Oncology by V. T. Devita and S. Hellman (editors), 11th edition (2018), Lippincott Williams & Wilkins Publishers. A person of ordinary skill in the art would be able to discern which combinations of agents would be useful based on the particular characteristics of the drugs and the disease involved.

In one instance, the treatment method includes the co-administration of a crystalline salt form of Compound 1 as disclosed herein, and at least one immunotherapy. Immunotherapy (also called biological response modifier therapy, biologic therapy, biotherapy, immune therapy, or biological therapy) is a treatment that uses parts of the immune system to fight disease. Immunotherapy can help the immune system recognize cancer cells, or enhance a response against cancer cells. Immunotherapies include active and passive immunotherapies. Active immunotherapies stimulate the body's own immune system while passive immunotherapies generally use immune system components created outside of the body.

Examples of active immunotherapies include, but are not limited to vaccines including cancer vaccines, tumor cell vaccines (autologous or allogeneic), dendritic cell vaccines, antigen vaccines, anti-idiotype vaccines, DNA vaccines, viral vaccines, or Tumor-Infiltrating Lymphocyte (TIL) Vaccine with Interleukin-2 (IL-2) or Lymphokine-Activated Killer (LAK) Cell Therapy.

Examples of passive immunotherapies include but are not limited to monoclonal antibodies and targeted therapies containing toxins. Monoclonal antibodies include naked antibodies and conjugated monoclonal antibodies (also called tagged, labeled, or loaded antibodies). Naked monoclonal antibodies do not have a drug or radioactive material attached whereas conjugated monoclonal antibodies are joined to, for example, a chemotherapy drug (chemolabeled), a radioactive particle (radiolabeled), or a toxin (immunotoxin). Examples of these naked monoclonal antibody drugs include, but are not limited to Rituximab (Rituxan), an antibody against the CD20 antigen used to treat, for example, B cell non-Hodgkin lymphoma; Trastuzumab (Herceptin), an antibody against the HER2 protein used to treat, for example, advanced breast cancer; Alemtuzumab (Campath), an antibody against the CD52 antigen used to treat, for example, B cell chronic lymphocytic leukemia (B-CLL); Cetuximab (Erbitux), an antibody against the EGFR protein used, for example, in combination with irinotecan to treat, for example, advanced colorectal cancer and head and neck cancers; and Bevacizumab (Avastin) which is an antiangiogenesis therapy that works against the VEGF protein and is used, for example, in combination with chemotherapy to treat, for example, metastatic colorectal cancer. Examples of the conjugated monoclonal antibodies include, but are not limited to Radiolabeled antibody Ibritumomab tiuxetan (Zevalin) which delivers radioactivity directly to cancerous B lymphocytes and is used to treat, for example, B cell non-Hodgkin lymphoma; radiolabeled antibody Tositumomab (Bexxar) which is used to treat, for example, certain types of non-Hodgkin lymphoma; and immunotoxin Gemtuzumab ozogamicin (Mylotarg) which contains calicheamicin and is used to treat, for example, acute myelogenous leukemia (AML). BL22 is a conjugated monoclonal antibody for treating, for example, hairy cell leukemia, immunotoxins for treating, for example, leukemias, lymphomas, and brain tumors, and radiolabeled antibodies such as OncoScint for example, for colorectal and ovarian cancers and ProstaScint for example, for prostate cancers.

Further examples of therapeutic antibodies that can be used include, but are not limited to, HERCEPTIN^{™} (Trastuzumab) (Genentech, Calif.) which is a humanized anti-HER2 monoclonal antibody for the treatment of patients with metastatic breast cancer; REOPRO.RTM. (abciximab) (Centocor) which is an anti-glycoprotein IIb/IIIa receptor on the platelets for the prevention of clot formation; ZENAPAX^{™} (daclizumab) (Roche Pharmaceuticals, Switzerland) which is an immunosuppressive, humanized anti-CD25 monoclonal antibody for the prevention of acute renal allograft rejection; PANOREX^{™} which is a murine anti-17-IA cell surface antigen IgG2a antibody (Glaxo Wellcome/Centocor); BEC2 which is a murine anti-idiotype (GD3epitope) IgG antibody (ImClone System); IMC-C225 which is a chimeric anti-EGFR IgG antibody (ImClone System); VITAXIN^{™} which is a humanized anti-alpha V beta 3 integrin antibody (Applied Molecular Evolution/Medlmmune); Campath 1H/LDP-03 which is a humanized anti CD52 IgG1 antibody (Leukosite); Smart M195 which is a humanized anti-CD33 IgG antibody (Protein Design Lab/Kanebo); RITUXAN^{™} which is a chimeric anti-CD20 IgG1 antibody (IDEC Pharm/Genentech, Roche/Zettyaku); LYMPHOCIDE^{™} which is a humanized anti-CD22 IgG antibody (Immunomedics); LYMPHOCIDE^{™} Y-90 (Immunomedics); Lymphoscan (Tc-99m-labeled; radioimaging; Immunomedics); Nuvion (against CD3; Protein Design Labs); CM3 is a humanized anti-ICAM3 antibody (ICOS Pharm); IDEC-114 is a primatized anti-CD80 antibody (IDEC Pharm/Mitsubishi); ZEVALIN^{™} is a radiolabelled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1. 1 is a humanized anti-complement factor 5 (C5) antibody (Alexion Pharm); D2E7 is a humanized anti-TNF-alpha antibody (CAT/BASF); CDP870 is a humanized anti-TNF-alpha. Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CD20-sreptdavidin (+biotin-yttrium 90; NeoRx); CDP571 is a humanized anti-TNF-alpha. IgG4 antibody (Celltech); LDP-02 is a humanized anti-alpha4 beta7 antibody (LeukoSite/Genentech); OrthoClone OKT4A is a humanized anti-CD4 IgG antibody (Ortho Biotech); ANTOVA^{™} is a humanized anti-CD40L IgG antibody (Biogen); ANTEGREN^{™} is a humanized anti-VLA-4 IgG antibody (Elan); and CAT-152 is a human anti-TGF-beta₂ antibody (Cambridge Ab Tech). Others are provided in later paragraphs.

Immunotherapies that can be used in combination with a crystalline salt form of Compound 1 as disclosed herein include adjuvant immunotherapies. Examples include cytokines, such as granulocyte-macrophage colony-stimulating factor (GM-CSF), granulocyte-colony stimulating factor (G-CSF), macrophage inflammatory protein (MIP)-1-alpha, interleukins (including IL-1, IL-2, IL-4, IL-6, IL-7, IL-12, IL-15, IL-18, IL-21, and IL-27), tumor necrosis factors (including TNF-alpha), and interferons (including IFN-alpha, IFN-beta, and IFN-gamma); aluminum hydroxide (alum); Bacille Calmette-Guerin (BCG); Keyhole limpet hemocyanin (KLH); Incomplete Freund's adjuvant (IFA); QS-21; DETOX; Levamisole; and Dinitrophenyl (DNP), and combinations thereof, such as combinations of interleukins, for example IL-2 with other cytokines, such as IFN-alpha.

In various instances, a crystalline salt form of Compound 1 can be combined with an immunological therapy and/or an immunological therapeutic agent. In various instances, an immunological therapy and/or an immunological therapeutic agent can include, one or more of the following: an adoptive cell transfer, an angiogenesis inhibitor, Bacillus Calmette-Guerin therapy, biochemotherapy, a cancer vaccine, a chimeric antigen receptor (CAR) T-cell therapy, a cytokine therapy, gene therapy, an immune checkpoint modulator, an immunoconjugate, a radioconjugate, an oncolytic virus therapy, or a targeted drug therapy. The immunological therapy or immunological therapeutic agent, is collectively referred to herein as an "immunotherapeutic agent".

The present disclosure provides a method for preventing, treating, reducing, inhibiting or controlling a neoplasia, a tumor or a cancer in a subject in need thereof, involving administering a therapeutically effective amount of a crystalline salt form of Compound 1 in combination with an immunotherapeutic agent. In one instance, the method comprises administering a therapeutically effective amount of a combination comprising a crystalline salt form of Compound 1 in combination with an immunotherapeutic agent. In various instances, the combination provides a cooperative effect, an additive effect, or a synergistic effect in reducing the number of cancer cells when treated with the combination as compared to each treatment alone. In some instances, administration of a therapeutically effective amount of a combination comprising a crystalline salt form of Compound 1 and an immunotherapeutic agent, results in synergistic anti-tumor activity and/or antitumor activity that is more potent than the additive effect of administration of a crystalline salt form of Compound 1 or immunotherapeutic agent alone.

Human cancers harbor numerous genetic and epigenetic alterations, generating neoantigens potentially recognizable by the immune system (Sjoblom et al. (2006) Science 314:268-74). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities.

The present disclosure provides a combination of a crystalline salt form of Compound 1 and an immunotherapeutic agent. These exemplified combinations can be used to treat a subject with a cancer. In various instances, immunotherapeutic agents that find utility in the present compositions, formulations, and methods can include one or more agents or therapies, including: an adoptive cell transfer, an angiogenesis inhibitor, Bacillus Calmette-Guerin therapy, biochemotherapy, a cancer vaccine, a chimeric antigen receptor (CAR) T-cell therapy, a cytokine therapy, gene therapy, an immune checkpoint modulator, for example an immune checkpoint inhibitor, an immunoconjugate, a radioconjugate, an oncolytic virus therapy, or a targeted drug therapy.

In certain instances of the present disclosure, a therapeutically effective combination comprises a crystalline salt form of Compound 1 and an immunotherapeutic agent. In various related instances, the crystalline salt form of Compound 1 enhances the activity of the immunotherapeutic agent.

In certain instances of each of the aforementioned aspects, as well as other aspects and instances described elsewhere herein, the immunotherapeutic agent enhances the activity of the crystalline salt form of Compound 1 of the present disclosure.

In certain instances of each of the aforementioned aspects, as well as other aspects and instances described elsewhere herein, the crystalline salt form of Compound 1 and the immunotherapeutic agent act synergistically. In various instances described herein, an exemplary immunotherapeutic agent is an immune cell (e.g. T-cell, dendritic cell, a natural killer cell and the like) modulator chosen from an agonist or an activator of a costimulatory molecule, wherein the modulator is a monoclonal antibody, a bispecific antibody comprising one or more immune checkpoint antigen binding moieties, a trispecific antibody, or an immune cell-engaging multivalent antibody/fusion protein/construct known in the art. In some instances, the immunotherapeutic agent can be an antibody that modulates a costimulatory molecule, bind to an antigen on the surface of an immune cell, or a cancer cell. In each of these different instances, the antibody modulator can be a monoclonal antibody, a polyclonal antibody, a bispecific antibody, a trispecific or multispecific format antibody, a fusion protein, or a fragment thereof, for example, a Diabody, a Single-chain (sc)-diabody (scFv)2, a Miniantibody, a Minibody, a Barnase-barstar, a scFv-Fc, a sc(Fab)2, a Trimeric antibody construct, a Triabody antibody construct, a Trimerbody antibody construct, a Tribody antibody construct, a Collabody antibody construct, a (scFv-TNFa)3, or a F(ab)3/DNL antibody construct.

In certain instances of each of the aforementioned aspects, as well as other aspects and instances described elsewhere herein, the immunotherapeutic agent is an agent that modulates immune responses, for example, a checkpoint inhibitor or a checkpoint agonist. In some instances, the immunotherapeutic agent is an agent that enhances anti-tumor immune responses. In some instances, the immunotherapeutic agent is an agent that increases cell-mediated immunity. In some instances, the immunotherapeutic agent is an agent that increases T-cell activity. In some instances, the immunotherapeutic agent is an agent that increases cytolytic T-cell (CTL) activity.

In some instances, the disclosed methods of treatment may include administering a crystalline salt form of Compound 1 together in combination with a molecule, for example, a binding agent, for example, an antibody or functional fragment thereof that modulates (activates or inhibits) a checkpoint protein. A checkpoint inhibitor can be any molecule, agent, treatment and/or method of inhibiting an immune checkpoint, and/or promoting an inhibitor of an immune checkpoint, e.g., by promoting an intrinsic immune checkpoint inhibitor; inhibiting a transcription factor involved in the expression of an immune checkpoint; and/or by acting in concert with some additional extrinsic factor. For example, a checkpoint inhibitor could include a treatment that inhibits transcription factors involved in the expression of immune checkpoint genes, or promotes the expression of transcription factors for tumor-suppressor genes, e.g., BACH2 (Luan et al., (2016). Transcription Factors and Checkpoint Inhibitor Expression with Age: Markers of Immunosenescence. Blood, 128(22), 5983). Moreover, a checkpoint inhibitor can inhibit the transcription of immune checkpoint genes; the modification and/or processing of immune checkpoint mRNA; the translation of immune checkpoint proteins; and/or molecules involved in immunity or the immune checkpoint pathway, e.g., PD-1 transcription factors such as HIF-1, STAT3, NF-κB, and AP-1, or the activation of common oncogenic pathways such as JAK/STAT, RAS/ERK, or PI3K/AKT/mTOR (Zerdes et al., Genetic, transcriptional and post-translational regulation of the programmed death protein ligand 1 in cancer: biology and clinical correlations, Oncogene volume 37, pages 4639-4661 (2018)).

Checkpoint inhibitors can include treatments, molecules, agents, and/or methods that regulate immune checkpoints at the transcriptional level, e.g., using the RNA-interference pathway co-suppression, and/or post-transcriptional gene silencing (PTGS) (e.g., microRNAs, miRNA; silencing-RNA, small-interfering-RNA, or short-interfering-RNA (siRNA). Transcriptional regulation of checkpoint molecules has been shown to involve mir-16, which has been shown to target the 3'UTR of the checkpoint mRNAs CD80, CD274 (PD-L1) and CD40 (Leibowitz et al., Post-transcriptional regulation of immune checkpoint genes by mir-16 in melanoma, Annals of Oncology (2017) 28; v428-v448). Mir-33a has also been shown to be involved in regulating the expression of PD-1 in cases of lung adenocarcinoma (Boldini et al., Role of microRNA-33a in regulating the expression of PD-1 in lung adenocarcinoma, Cancer Cell Int. 2017; 17: 105).

T-cell-specific aptamer-siRNA chimeras have been suggested as a highly specific method of inhibiting molecules in the immune checkpoint pathway (Hossain et al., The aptamer-siRNA conjugates: reprogramming T cells for cancer therapy, Ther. Deliv. 2015 Jan; 6(1): 1-4).

Alternatively, members of the immune checkpoint pathway can be inhibited using treatments that affect associated pathways, e.g., metabolism. For example, oversupplying the glycolytic intermediate pyruvate in mitochondria from CAD macrophages promoted expression of PD-L1 via induction of the bone morphogenetic protein 4/phosphorylated SMAD1/5/IFN regulatory factor 1 (BMP4/p-SMAD1/5/IRF1) signaling pathway. Accordingly, implementing treatments that modulate the metabolic pathway can result in subsequent modulation of the immunoinhibitory PD-1/PD-L1 checkpoint pathway (Watanabe et al., Pyruvate controls the checkpoint inhibitor PD-L1 and suppresses T cell immunity, J Clin Invest. 2017 Jun 30; 127(7): 2725-2738).

Checkpoint immunity can be regulated via oncolytic viruses that selectively replicate within tumor cells and induce acute immune responses in the tumor-micro-environment, i.e., by acting as genetic vectors that carry specific agents (e.g., antibodies, miRNA, siRNA, and the like) to cancer cells and effecting their oncolysis and secretion of cytokines and chemokines to synergize with immune checkpoint inhibition (Shi et al., Cancer Immunotherapy: A Focus on the Regulation of Immune Checkpoints, Int J Mol Sci. 2018 May; 19(5): 1389). Currently, there are clinical trials underway that utilize the following viruses as checkpoint inhibitors: poliovirus, measles virus, adenoviruses, poxviruses, herpes simplex virus (HSV), coxsackieviruses, reovirus, Newcastle disease virus (NDV), T-VEC (a herpes virus encoded with GM-CSF (granulocyte-macrophage colony stimulating factor)), and H101 (Shi et al., supra).

Checkpoint inhibitors can operate at the translational level of checkpoint immunity. The translation of mRNA into protein represents a key event in the regulation of gene expression, thus inhibition of immune checkpoint translation is a method in which the immune checkpoint pathway can be inhibited.

Inhibition of the immune checkpoint pathway can occur at any stage of the immune checkpoint translational process. For example, drugs, molecules, agents, treatments, and/or methods can inhibit the initiation process (whereby the 40S ribosomal subunit is recruited to the 5' end of the mRNA and scans the 5'UTR of the mRNA toward its 3' end. Inhibition can occur by targeting the anticodon of the initiator methionyl-transfer RNA (tRNA) (Met-tRNAi), its base-pairing with the start codon, or the recruitment of the 60S subunit to begin elongation and sequential addition of amino acids in the translation of immune-checkpoint-specific genes. Alternatively, a checkpoint inhibitor can inhibit checkpoints at the translational level by preventing the formation of the ternary complex (TC), i.e., eukaryotic initiation factor (eIF)2 (or one or more of its α, β, and γ subunits); GTP; and Met-tRNAi.

Checkpoint inhibition can occur via destabilization of eIF2α by precluding its phosphorylation via protein kinase R (PKR), PERK, GCN2, or HRI, or by precluding TCs from associating with the 40S ribosome and/or other initiation factors, thus preventing the preinitiation complex (PIC) from forming; inhibiting the eIF4F complex and/or its cap-binding protein eIF4E, the scaffolding protein eIF4G, or eIF4A helicase. Methods discussing the translational control of cancer are discussed in Truitt et al., New frontiers in translational control of the cancer genome, Nat Rev Cancer. 2016 Apr 26; 16(5): 288-304.

Checkpoint inhibitors can also include treatments, molecules, agents, and/or methods that regulate immune checkpoints at the cellular and/or protein level, e.g., by inhibiting an immune checkpoint receptor. Inhibition of checkpoints can occur via the use of antibodies, antibody fragments, antigen-binding fragments, small-molecules, and/or other drugs, agents, treatments, and/or methods.

Immune checkpoints refer to inhibitory pathways in the immune system that are responsible for maintaining self-tolerance and modulating the degree of immune system response to minimize peripheral tissue damage. However, tumor cells can also activate immune system checkpoints to decrease the effectiveness of immune response ('block' the immune response) against tumor tissues. In contrast to the majority of anti-cancer agents, checkpoint inhibitors do not target tumor cells directly, but rather target lymphocyte receptors or their ligands in order to enhance the endogenous antitumor activity of the immune system. (Pardoll, 2012, Nature Reviews Cancer 12:252-264).

In some instances, the immunotherapeutic agent is a modulator of PD-1 activity, a modulator of PD-L1 activity, a modulator of PD-L2 activity, a modulator of CTLA-4 activity, a modulator of CD28 activity, a modulator of CD80 activity, a modulator of CD86 activity, a modulator of 4-1BB activity, an modulator of OX40 activity, a modulator of KIR activity, a modulator of Tim-3 activity, a modulator of LAG3 activity, a modulator of CD27 activity, a modulator of CD40 activity, a modulator of GITR activity, a modulator of TIGIT activity, a modulator of CD20 activity, a modulator of CD96 activity, a modulator of IDO1 activity, a cytokine, a chemokine, an interferon, an interleukin, a lymphokine, a member of the tumor necrosis factor (TNF) family, or an immunostimulatory oligonucleotide. In some instances, the immune checkpoint modulator, i.e. is an inhibitor or antagonist, or is an activator or agonist, for example, a CD28 modulator, a 4-1BB modulator, an OX40 modulator, a CD27 modulator, a CD80 modulator, a CD86 modulator, a CD40 modulator, or a GITR modulator, a Lag-3 modulator, a 41BB modulator, a LIGHT modulator, a CD40 modulator, a GITR modulator, a TGF-beta modulator, a TIM-3 modulator, a SIRP-alpha modulator, a TIGIT modulator, a VSIG8 modulator, a BTLA modulator, a SIGLEC7 modulator, a SIGLEC9 modulator, a ICOS modulator, a B7H3 modulator, a B7H4 modulator, a FAS modulator, and/or a BTNL2 modulator. In some instances, the immunotherapeutic agent is an immune checkpoint modulator as described above (e.g., an immune checkpoint modulator antibody, which can be in the form of a monoclonal antibody, a bispecific antibody comprising one or more immune checkpoint antigen binding moieties, a trispecific antibody, or an immune cell-engaging multivalent antibody/fusion protein/construct known in the art).

In some instances, the immunotherapeutic agent is an agent that inhibits the activity of PD-1. In some instances, the immunotherapeutic agent is an agent that inhibits the activity of PD-L1 and/or PD-L2. In some instances, the immunotherapeutic agent is an agent that inhibits the activity of CTLA-4. In some instances, the immunotherapeutic agent is an agent that inhibits the activity of CD80 and/or CD86. In some instances, the immunotherapeutic agent is an agent that inhibits the activity of TIGIT. In some instances, the immunotherapeutic agent is an agent that inhibits the activity of KIR. In some instances, the immunotherapeutic agent is an agent that enhances or stimulates the activity of activating immune checkpoint receptors.

PD-1 (also known as Programmed Death 1, CD279, PDCD1) is a cell surface receptor with a critical role in regulating the balance between stimulatory and inhibitory signals in the immune system and maintaining peripheral tolerance (Ishida, Y et al. 1992 EMBO J. 11 3887; Kier, Mary E et al. 2008 Annu Rev Immunol 26 677-704; Okazaki, Taku et al. 2007 International Immunology 19 813-824). PD-1 is an inhibitory member of the immunoglobulin super-family with homology to CD28. The structure of PD-1 is a monomeric type 1 transmembrane protein, consisting of one immunoglobulin variable-like extracellular domain and a cytoplasmic domain containing an immunoreceptor tyrosine-based inhibitory motif (ITIM) and an immunoreceptor tyrosine-based switch motif (ITSM). Expression of PD-1 is inducible on T cells, B cells, natural killer (NK) cells and monocytes, for example upon lymphocyte activation via T cell receptor (TCR) or B cell receptor (BCR) signalling (Kier, Mary E et al. 2008 Annu Rev Immunol 26 677-704; Agata, Y et al 1996 Int Immunol 8 765-72). PD-1 is a receptor for the ligands CD80, CD86, PD-L1 (B7-H1, CD274) and PD-L2 (B7-DC, CD273), which are cell surface expressed members of the B7 family (Freeman, Gordon et al. 2000 J Exp Med 192 1027; Latchman, Y et al. 2001 Nat Immunol 2: 261). Upon ligand engagement, PD-1 recruits phosphatases such as SHP-1 and SHP-2 to its intracellular tyrosine motifs which subsequently dephosphorylate effector molecules activated by TCR or BCR signalling (Chemnitz, J et al. 2004 J Immunol 173: 945-954; Riley, James L 2009 Immunological Reviews 229: 114-125) In this way, PD-1 transduces inhibitory signals into T and B cells only when it is engaged simultaneously with the TCR or BCR.

PD-1 has been demonstrated to down-regulate effector T cell responses via both cell-intrinsic and cell-extrinsic functional mechanisms. Inhibitory signaling through PD-1 induces a state of unresponsiveness in T cells, resulting in the cells being unable to clonally expand or produce optimal levels of effector cytokines. PD-1 may also induce apoptosis in T cells via its ability to inhibit survival signals from co-stimulation, which leads to reduced expression of key anti-apoptotic molecules such as Bcl-XL (Kier, Mary E et al. 2008 Annu Rev Immunol 26: 677-704). In addition to these direct effects, recent publications have implicated PD-1 as being involved in the suppression of effector cells by promoting the induction and maintenance of regulatory T cells (TREG). For example, PD-L1 expressed on dendritic cells was shown to act in synergy with TGF-β to promote the induction of CD4+ FoxP3+TREG with enhanced suppressor function (Francisco, Loise M et al. 2009 J Exp Med 206: 3015-3029).

TIM-3 (also known as T-cell immunoglobulin and mucin-domain containing-3, TIM-3, Hepatitis A virus cellular receptor 2, HAVCR2, HAVcr-2, KIM-3, TIMD-3, TIMD3, Tim-3, and CD366) is a ~33.4-kDa single-pass type I membrane protein involved in immune responses (Sanchez-Fueyo et al., Tim-3 inhibits T helper type 1-mediated auto- and alloimmune responses and promotes immunological tolerance, Nat. Immunol. 4: 1093-1101(2003)).

TIM-3 is selectively expressed on Th1-cells, and phagocytic cells (e.g., macrophages and dendritic cells). The use of siRNA or a blocking antibody to reduce the expression of human TIM-3 resulted in increased secretion of interferon γ (IFN-γ) from CD4 positive T-cells, implicating the inhibitory role of TIM-3 in human T cells. Analysis of clinical samples from autoimmune disease patients showed no expression of TIM-3 in CD4 positive cells. In particular, expression level of TIM-3 is lower and secretion of IFN-γ is higher in T cell clones derived from the cerebrospinal fluid of patients with multiple sclerosis than those in clones derived from normal healthy persons (Koguchi K et al., J Exp Med. 203: 1413-8. (2006)).

TIM-3 is the receptor for the ligand Galectin-9, which is a member of galectin family, molecules ubiquitously expressed on a variety of cell types and which binds β-galactoside; Phospatidyl serine (PtdSer) (DeKryff et al., T cell/transmembrane, Ig, and mucin-3 allelic variants differentially recognize phosphatidylserine and mediate phagocytosis of apoptotic cells, J Immunol. 2010 Feb 15; 184(4): 1918-30); High Mobility Group Protein 1 (also known as HMGB1, HMG1, HMG3, SBP-1, HMG-1, and high mobility group box 1) Chiba et al., Tumor-infiltrating DCs suppress nucleic acid-mediated innate immune responses through interactions between the receptor TIM-3 and the alarmin HMGB1, Nat Immunol. 2012 Sep; 13(9): 832-42); and Carcinoembryonic Antigen Related Cell Adhesion Molecule 1 (also known as CEACAM1, BGP, BGP1, BGPI, carcinoembryonic antigen related cell adhesion molecule 1) (Huang et al., CEACAM1 regulates TIM-3-mediated tolerance and exhaustion, Nature. 2015 Jan 15; 517(7534): 386-90).

BTLA (also known as B- and T-lymphocyte attenuator, BTLA1, CD272, and B and T lymphocyte associated) is a ~27.3-kDa single-pass type I membrane protein involved in lymphocyte inhibition during immune response. BTLA is constitutively expressed in both B and T cells. BTLA interacts with HVEM (herpes virus-entry mediator), a member of the tumor-necrosis factor receptor (TNFR) family (Gonzalez et al., Proc. Natl. Acad. Sci. USA, 2005, 102: 1116-21). The interaction of BTLA, which belongs to the CD28 family of the immunoglobulin superfamily, and HVEM, a costimulatory tumor-necrosis factor (TNF) receptor (TNFR), is unique in that it defines a cross talk between these two families of receptors. BTLA contains a membrane proximal immunoreceptor tyrosine-based inhibitory motif (ITIM) and membrane distal immunoreceptor tyrosine-based switch motif (ITSM). Disruption of either the ITIM or ITSM abrogated the ability of BTLA to recruit either SHP1 or SHP2, suggesting that BTLA recruits SHP1 and SHP2 in a manner distinct from PD-1 and both tyrosine motifs are required to block T cell activation. The BTLA cytoplasmic tail also contains a third conserved tyrosine-containing motif within the cytoplasmic domain, similar in sequence to a Grb-2 recruitment site (YXN). Also, a phosphorylated peptide containing this BTLA N-terminal tyrosine motif can interact with GRB2 and the p85 subunit of PI3K in vitro, although the functional effects of this interaction remain unexplored in vivo (Gavrieli et al., Bioochem. Biophysi Res Commun, 2003, 312, 1236-43). BTLA is the receptor for the ligands PTPN6/SHP-1; PTPN11/SHP-2; TNFRSF14/HVEM; and B7H4.

VISTA (also known as V-domain Ig suppressor of T cell activation VSIR, B7-H5, B7H5, GI24, PP2135, SISP1, DD1alpha, VISTA, C10orf54, chromosome 10 open reading frame 54, PD-1H, and V-set immunoregulatory receptor) is a ~33.9-kDa single-pass type I membrane protein involved in T-cell inhibitory response, embryonic stem cells differentiation via BMP4 signaling inhibition, and MMP14-mediated MMP2 activation (Yoon et al., Control of signaling-mediated clearance of apoptotic cells by the tumor suppressor p53, Science. 2015 Jul 31; 349(6247): 1261669). VISTA interacts with the ligand VSIG-3 (Wang et al., VSIG-3 as a ligand of VISTA inhibits human T-cell function, Immunology. 2019 Jan; 156(1): 74-85)

LAG-3 (also known as Lymphocyte-activation gene 3, LAG3, CD223, and lymphocyte activating 3) is a ~57.4-kDa single-pass type I membrane protein involved in lymphocyte activation that also binds to HLA class-II antigens. LAG-3 is a member of the immunoglobulin supergene family, and is expressed on activated T cells (Huard et al., 1994, Immunogenetics 39: 213), NK cells (Triebel et al., 1990, J. Exp. Med. 171: 1393-1405), regulatory T cells (Huang et al., 2004, Immunity 21: 503-513; Camisaschi et al., 2010, J Immunol. 184: 6545-6551; Gagliani et al., 2013, Nat Med 19: 739-746), and plasmacytoid dendritic cells (DCs) (Workman et al., 2009, J Immunol 182: 1885-1891). LAG-3 is a membrane protein encoded by a gene located on chromosome 12, and is structurally and genetically related to CD4. Similar to CD4, LAG-3 can interact with MHC class II molecules on the cell surface (Baixeras et al., 1992, J. Exp. Med. 176: 327-337; Huard et al., 1996, Eur. J. Immunol. 26: 1180-1186). It has been suggested that the direct binding of LAG-3 to MHC class II plays a role in down-regulating antigen-dependent stimulation of CD4+ T lymphocytes (Huard et al., 1994, Eur. J. Immunol. 24: 3216-3221) and LAG-3 blockade has also been shown to reinvigorate CD8+ lymphocytes in both tumor or self-antigen (Gross et al., 2007, J Clin Invest. 117: 3383-3392) and viral models (Blackburn et al., 2009, Nat. Immunol. 10: 29-37). Further, the intra-cytoplasmic region of LAG-3 can interact with LAP (LAG-3-associated protein), which is a signal transduction molecule involved in the downregulation of the CD3/TCR activation pathway (Iouzalen et al., 2001, Eur. J. Immunol. 31: 2885-2891). Moreover, CD4+CD25+ regulatory T cells (Treg) have been shown to express LAG-3 upon activation, which contributes to the suppressor activity of Treg cells (Huang, C. et al., 2004, Immunity 21: 503-513). LAG-3 can also negatively regulate T cell homeostasis by Treg cells in both T cell-dependent and independent mechanisms (Workman, C. J. and Vignali, D. A., 2005, J. Immunol. 174: 688-695).

LAG-3 has been shown to interact with MHC class II molecules (Huard et al., CD4/major histocompatibility complex class II interaction analyzed with CD4- and lymphocyte activation gene-3 (LAG-3)-Ig fusion proteins, Eur J Immunol. 1995 Sep; 25(9): 2718-21).

Additionally, several kinases are known to be checkpoint inhibitors. For example, CHEK-1, CHEK-2, and A2aR.

CHEK-1 (also known as CHK 1 kinase, CHK1, and checkpoint kinase 1) is a ~54.4-kDa serine/threonine-protein kinase that is involved with checkpoint-mediated cell cycle arrest, and the activation of DNA repair in response to the DNA damage and/or unreplicated DNA.

CHEK-2 (also known as CHK2 kinase, CDS1, CHK2, HuCds1, LFS2, PP1425, RAD53, hCds1, and checkpoint kinase 2) is a ~60.9-kDa. serine/threonine-protein kinase involved in checkpoint-mediated cell cycle arrest, DNA-repair activation, and double-strand break-mediated apoptosis.

A2aR (also known as adenosine A2A receptor, ADORA2A, adenosine A2a receptor, A2aR, ADORA2, and RDC8) is a ~44.7-kDa multi-pass membrane receptor for adenosine and other ligands.

In some instances, illustrative immunotherapeutic agents can include one or more antibody modulators that target PD-1, PD-L1, PD-L2, CEACAM (e.g., CEACAM-1, -3 and/or - 5), CTLA-4, TIM-3, LAG-3, VISTA, BTLA, TIGIT, LAIR1, CD160, 2B4, TGF beta, OX40, 41BB, LIGHT, CD40, GITR, TGF-beta, TIM-3, SIRP-alpha, VSIG8, BTLA, SIGLEC7, SIGLEC9, ICOS, B7H3, B7H4, FAS, and/or BTNL2 among others known in the art, . In some instances, the immunotherapeutic agent is an agent that increases natural killer (NK) cell activity. In some instances, the immunotherapeutic agent is an agent that inhibits suppression of an immune response. In some instances, the immunotherapeutic agent is an agent that inhibits suppressor cells or suppressor cell activity. In some instances, the immunotherapeutic agent is an agent or therapy that inhibits Treg activity. In some instances, the immunotherapeutic agent is an agent that inhibits the activity of inhibitory immune checkpoint receptors.

In some instances, the combination of the present disclosure comprises a crystalline salt form of Compound 1 and an immunotherapeutic agent, wherein the immunotherapeutic agent includes a T cell modulator chosen from an agonist or an activator of a costimulatory molecule. In one instance, the agonist of the costimulatory molecule is chosen from an agonist (e.g., an agonistic antibody or antigen-binding fragment thereof, or a soluble fusion) of GITR, OX40, SLAM (e.g., SLAMF7), HVEM, LIGHT, CD2, CD27, CD28, CDS, ICAM-1, LFA-1 (CD11a/CD18), ICOS (CD278), 4-1BB (CD137), CD30, CD40, BAFFR, CD7, NKG2C, NKp80, CD160, B7-H3, or CD83 ligand. In other instances, the effector cell combination includes a bispecific T cell engager (e.g., a bispecific antibody molecule that binds to CD3 and a tumor antigen (e.g., EGFR, PSCA, PSMA, EpCAM, HER2 among others).

In some instances, the immunotherapeutic agent is a modulator of PD-1 activity, a modulator of PD-L1 activity, a modulator of PD-L2 activity, a modulator of CTLA-4 activity, a modulator of CD28 activity, a modulator of CD80 activity, a modulator of CD86 activity, a modulator of 4-1BB activity, an modulator of OX40 activity, a modulator of KIR activity, a modulator of Tim-3 activity, a modulator of LAG3 activity, a modulator of CD27 activity, a modulator of CD40 activity, a modulator of GITR activity, a modulator of TIGIT activity, a modulator of CD20 activity, a modulator of CD96 activity, a modulator of IDO1 activity, a modulator of SIRP-alpha activity, a modulator of TIGIT activity, a modulator of VSIG8 activity, a modulator of BTLA activity, a modulator of SIGLEC7 activity, a modulator of SIGLEC9 activity, a modulator of ICOS activity, a modulator of B7H3 activity, a modulator of B7H4 activity, a modulator of FAS activity, a modulator of BTNL2 activity, a cytokine, a chemokine, an interferon, an interleukin, a lymphokine, a member of the tumor necrosis factor (TNF) family, or an immunostimulatory oligonucleotide.

In some instances, the immunotherapeutic agent is an immune checkpoint modulator (e.g., an immune checkpoint inhibitor e.g. an inhibitor of PD-1 activity, a modulator of PD-L1 activity, a modulator of PD-L2 activity, a modulator of CTLA-4, or a CD40 agonist (e.g., an anti-CD40 antibody molecule), (xi) an OX40 agonist (e.g., an anti-OX40 antibody molecule), or (xii) a CD27 agonist (e.g., an anti-CD27 antibody molecule). In one instance, the immunotherapeutic agent is an inhibitor of: PD-1, PD-L1, PD-L2, CTLA-4, TIM-3, LAG-3, CEACAM (e.g., CEACAM-1, -3 and/or -5), VISTA, BTLA, TIGIT, LAIR1, CD160, 2B4 and/or TGF beta, Galectin 9, CD69, Galectin-1, CD113, GPR56, CD48, GARP, PD1H, LAIR1, TIM-1, and TIM-4. In one instance, the inhibitor of an immune checkpoint molecule inhibits PD-1, PD-L1, LAG-3, TIM-3, CEACAM (e.g., CEACAM-1, -3 and/or -5), CTLA-4, or any combination thereof.

In one instance, the immunotherapeutic agent is an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

In some instances, the immunotherapeutic agent used in the combinations disclosed herein (e.g., in combination with a crystalline salt form of Compound 1 of the present disclosure) is an activator or agonist of a costimulatory molecule. In one instance, the agonist of the costimulatory molecule is chosen from an agonist (e.g., an agonistic antibody or antigen-binding fragment thereof, or a soluble fusion) of CD2, CD28, CDS, ICAM-1, LFA-1 (CD11a/CD18), ICOS (CD278), 4-1BB (CD137), GITR, CD30, BAFFR, HVEM, CD7, LIGHT, NKG2C, SLAMF7, NKp80, CD160, B7-H3, or CD83 ligand.

Inhibition of an inhibitory molecule can be performed at the DNA, RNA or protein level. In instances, an inhibitory nucleic acid (e.g., a dsRNA, siRNA or shRNA), can be used to inhibit expression of an inhibitory molecule. In other instances, the inhibitor of an inhibitory signal is, a polypeptide e.g., a soluble ligand (e.g., PD-1-Ig or CTLA-4 Ig), or an antibody or antigen-binding fragment thereof, for example, a monoclonal antibody, a bispecific antibody comprising one or more immune checkpoint antigen binding moieties, a trispecific antibody, or an immune cell-engaging multivalent antibody/fusion protein/construct known in the art that binds to the inhibitory molecule; e.g., an antibody or fragment thereof (also referred to herein as "an antibody molecule") that binds to PD-1, PD-L1, PD-L2, CTLA-4, TIM-3, LAG-3, CEACAM (e.g., CEACAM-1, -3 and/or -5), VISTA, BTLA, TIGIT, LAIR1, CD160, 2B4 and/or TGF beta, Galectin 9, CD69, Galectin-1, CD113, GPR56, CD48, GARP, PD1H, LAIR1, TIM-1, TIM-4, or a combination thereof.

In some instances, where the combination comprises a crystalline salt form of Compound 1 and an immunotherapeutic agent, wherein the immunotherapeutic agent is a monoclonal antibody or a bispecific antibody. For example, the monoclonal or bispecific antibody may specifically bind a member of the c-Met pathway and/or an immune checkpoint modulator (e.g., the bispecific antibody binds to both a hepatocyte growth factor receptor (HGFR) and an immune checkpoint modulator described herein, such as an antibody that binds PD-1, PD-L1, PD-L2, or CTLA-4, LAG-3, OX40, 41BB, LIGHT, CD40, GITR, TGF-beta, TIM-3, SIRP-alpha, TIGIT, VSIG8, BTLA, SIGLEC7, SIGLEC9, ICOS, B7H3, B7H4, FAS, BTNL2 or CD27). In particular instances, the bispecific antibody specifically binds a human HGFR protein and one of PD-1, PD-L1, and CTLA-4.

In some of the instances of the methods described herein, the immunotherapeutic agent is a PD-1 antagonist, a PD-L1 antagonist, a PD-L2 antagonist, a CTLA-4 antagonist, a CD80 antagonist, a CD86 antagonist, a KIR antagonist, a Tim-3 antagonist, a LAG3 antagonist, a TIGIT antagonist, a CD20 antagonist, a CD96 antagonist, or an IDO1 antagonist.

In some instances, the PD-1 antagonist is an antibody that specifically binds PD-1. In some instances, the antibody that binds PD-1 is pembrolizumab (KEYTRUDA^{®}, MK-3475; Merck), pidilizumab (CT-011; Curetech Ltd.), nivolumab (OPDIVO^{®}, BMS-936558, MDX-1106; Bristol Myer Squibb), MEDI0680 (AMP-514; AstraZenenca/MedImmune), REGN2810 (Regeneron Pharmaceuticals), BGB-A317 (BeiGene Ltd.), PDR-001 (Novartis), or STI-A1110 (Sorrento Therapeutics). In some instances, the antibody that binds PD-1 is described in PCT Publication WO 2014/179664, for example, an antibody identified as APE2058, APE1922, APE1923, APE1924, APE 1950, or APE1963 (Anaptysbio), or an antibody containing the CDR regions of any of these antibodies. In other instances, the PD-1 antagonist is a fusion protein that includes the extracellular domain of PD-L1 or PD-L2, for example, AMP-224 (AstraZeneca/MedImmune). In other instances, the PD-1 antagonist is a peptide inhibitor, for example, AUNP-12 (Aurigene).

In some instances, the PD-L1 antagonist is an antibody that specifically binds PD-L1. In some instances, the antibody that binds PD-L1 is atezolizumab (RG7446, MPDL3280A; Genentech), MEDI4736 (AstraZeneca/MedImmune), BMS-936559 (NMX-1105; Bristol Myers Squibb), avelumab (MSB0010718C; Merck KGaA), KD033 (Kadmon), the antibody portion of KD033, or STI-A1014 (Sorrento Therapeutics). In some instances, the antibody that binds PD-L1 is described in PCT Publication WO 2014/055897, for example, Ab-14, Ab-16, Ab-30, Ab-31, Ab-42, Ab-50, Ab-52, or Ab-55, or an antibody that contains the CDR regions of any of these antibodies.

In some instances, the CTLA-4 antagonist is an antibody that specifically binds CTLA-4. In some instances, the antibody that binds CTLA-4 is ipilimumab (YERVOY^{®}; Bristol Myer Squibb) or tremelimumab (CP-675, 206; Pfizer). In some instances, the CTLA-4 antagonist a CTLA-4 fusion protein or soluble CTLA-4 receptor, for example, KARR-102 (Kahr Medical Ltd.).

In some instances, the LAG3 antagonist is an antibody that specifically binds LAG3. In some instances, the antibody that binds LAG3 is IMP701 (Prima BioMed), IMP731 (Prima BioMed/GlaxoSmithKline), BMS-986016 (Bristol Myer Squibb), LAG525 (Novartis), and GSK2831781 (GlaxoSmithKline). In some instances, the LAG3 antagonist includes a soluble LAG3 receptor, for example, IMP321 (Prima BioMed).

In some instances, the KIR antagonist is an antibody that specifically binds KIR. In some instances, the antibody that binds KIR is lirilumab (Bristol Myer Squibb/Innate Pharma).

In some instances, the immunotherapeutic agent is a cytokine, for example, a chemokine, an interferon, an interleukin, lymphokine, or a member of the tumor necrosis factor family. In some instances, the cytokine is IL-2, IL15, or interferon-gamma.

In some instances of any of the above aspects or those described elsewhere herein, the cancer is selected from the group consisting of lung cancer (e.g., a non-small cell lung cancer (NSCLC)), a kidney cancer (e.g., a kidney urothelial carcinoma), a bladder cancer (e.g., a bladder urothelial (transitional cell) carcinoma), a breast cancer, a colorectal cancer (e.g., a colon adenocarcinoma), an ovarian cancer, a pancreatic cancer, a gastric carcinoma, an esophageal cancer, a mesothelioma, a melanoma (e.g., a skin melanoma), a head and neck cancer (e.g., a head and neck squamous cell carcinoma (HNSCC)), a thyroid cancer, a sarcoma (e.g., a soft-tissue sarcoma, a fibrosarcoma, a myxosarcoma, a liposarcoma, an osteogenic sarcoma, an osteosarcoma, a chondrosarcoma, an angiosarcoma, an endotheliosarcoma, a lymphangiosarcoma, a lymphangioendotheliosarcoma, a leiomyosarcoma, or a rhabdomyosarcoma), a prostate cancer, a glioblastoma, a cervical cancer, a thymic carcinoma, a leukemia (e.g., an acute lymphocytic leukemia (ALL), an acute myelocytic leukemia (AML), a chronic myelocytic leukemia (CML), a chronic eosinophilic leukemia, or a chronic lymphocytic leukemia (CLL)), a lymphoma (e.g., a Hodgkin lymphoma or a non-Hodgkin lymphoma (NHL)), a myeloma (e.g., a multiple myeloma (MM)), a mycoses fungoides, a merkel cell cancer, a hematologic malignancy, a cancer of hematological tissues, a B cell cancer, a bronchus cancer, a stomach cancer, a brain or central nervous system cancer, a peripheral nervous system cancer, a uterine or endometrial cancer, a cancer of the oral cavity or pharynx, a liver cancer, a testicular cancer, a biliary tract cancer, a small bowel or appendix cancer, a salivary gland cancer, an adrenal gland cancer, adrenal cortex carcinoma, an adenocarcinoma, an inflammatory myofibroblastic tumor, a gastrointestinal stromal tumor (GIST), a colon cancer, a myelodysplastic syndrome (MDS), a myeloproliferative disorder (MPD), a polycythemia Vera, a chordoma, a synovioma, an Ewing's tumor, a squamous cell carcinoma, a basal cell carcinoma, an adenocarcinoma, a sweat gland carcinoma, a sebaceous gland carcinoma, a papillary carcinoma, a papillary adenocarcinoma, a medullary carcinoma, a bronchogenic carcinoma, a renal cell carcinoma, a hepatoma, a bile duct carcinoma, a choriocarcinoma, a seminoma, an embryonal carcinoma, a Wilms' tumor, a bladder carcinoma, an epithelial carcinoma, a glioma, anaplastic astrocytoma, an astrocytoma, a medulloblastoma, a craniopharyngioma, an ependymoma, a pinealoma, a hemangioblastoma, an acoustic neuroma, an oligodendroglioma, a meningioma, a neuroblastoma, a retinoblastoma, a follicular lymphoma, a diffuse large B-cell lymphoma, a mantle cell lymphoma, a hepatocellular carcinoma, a thyroid cancer, a small cell cancer, an essential thrombocythemia, an agnogenic myeloid metaplasia, a hypereosinophilic syndrome, a systemic mastocytosis, a familiar hypereosinophilia, a neuroendocrine cancer, or a carcinoid tumor.

In some instances of any of the above aspects or those described elsewhere herein, the subject's cancer or tumor does not respond to immune checkpoint inhibition (e.g., to any immune checkpoint inhibitor described herein, such as a PD-1 antagonist or PD-L1 antagonist) or the subject's cancer or tumor has progressed following an initial response to immune checkpoint inhibition (e.g., to any immune checkpoint inhibitor described herein, such as a PD-1 antagonist or PD-L1 antagonist).

In various instances, the immunotherapeutic agent can comprise an antibody or an antigen binding fragment thereof. Within this definition, immune checkpoint inhibitors include bispecific antibodies and immune cell-engaging multivalent antibody/fusion protein/constructs known in the art. In some instances, immunotherapeutic agents which comprise bispecific antibodies may include bispecific antibodies that are bivalent and bind either the same epitope of the immune checkpoint molecule, two different epitopes of the same immune checkpoint molecule or different epitopes of two different immune checkpoints.

Persons of ordinary skill in the art can implement several bispecific antibody formats known in the field to target one or more of CTLA4, PD1, PD-L1 TIM-3, LAG-3, various B-7 ligands, B7H3, B7H4, CHK 1 and CHK2 kinases, BTLA, A2aR, OX40, 41BB, LIGHT, CD40, GITR, TGF-beta, SIRP-alpha, TIGIT, VSIG8, SIGLEC7, SIGLEC9, ICOS, FAS, BTNL2 and other for use in the combination described herein.

In various instances, the immunotherapeutic agent can include am immune cell-engaging multivalent antibody/fusion protein/construct.

In an instance of the disclosure, the checkpoint inhibitor, in combination with a crystalline salt form of Compound 1, is used to reduce or inhibit metastasis of a primary tumor or cancer to other sites, or the formation or establishment of metastatic tumors or cancers at other sites distal from the primary tumor or cancer thereby inhibiting or reducing tumor or cancer relapse or tumor or cancer progression.

In a further instance of the disclosure, provided herein is a combination therapy for treating cancer, which comprises a crystalline salt form of Compound 1 and a checkpoint inhibitor with the potential to elicit potent and durable immune responses with enhanced therapeutic benefit and more manageable toxicity.

In a further instance of the disclosure, provided herein is a combination therapy for treating cancer, which comprises a crystalline salt form of Compound 1 and an immune checkpoint inhibitor. In an instance of the disclosure provided herein is a method for treating cancer and/or preventing the establishment of metastases by employing a crystalline salt form of Compound 1 of the present disclosure, which acts synergistically with a checkpoint inhibitor.

In further instances, the disclosure provides methods for one or more of the following: 1) reducing or inhibiting growth, proliferation, mobility or invasiveness of tumor or cancer cells that potentially or do develop metastases, 2) reducing or inhibiting formation or establishment of metastases arising from a primary tumor or cancer to one or more other sites, locations or regions distinct from the primary tumor or cancer; 3) reducing or inhibiting growth or proliferation of a metastasis at one or more other sites, locations or regions distinct from the primary tumor or cancer after a metastasis has formed or has been established, 4) reducing or inhibiting formation or establishment of additional metastasis after the metastasis has been formed or established, 5) prolonged overall survival, 6) prolonged progression free survival, or 7) disease stabilization. The methods include administering to a subject in need thereof a crystalline salt form of Compound 1 of the present disclosure, in combination with a check point inhibitor as described herein.

In an instance of the disclosure, administration of a crystalline salt form of Compound 1 in combination with the immunotherapeutic agent, provides a detectable or measurable improvement in a condition of a given subject, such as alleviating or ameliorating one or more adverse (physical) symptoms or consequences associated with the presence of a cell proliferative or cellular hyperproliferative disorder, neoplasia, tumor or cancer, or metastasis, i.e., a therapeutic benefit or a beneficial effect.

A therapeutic benefit or beneficial effect is any objective or subjective, transient, temporary, or long-term improvement in the condition or pathology, or a reduction in onset, severity, duration or frequency of adverse symptom associated with or caused by cell proliferation or a cellular hyperproliferative disorder such as a neoplasia, tumor or cancer, or metastasis. It may lead to improved survival. A satisfactory clinical endpoint of a treatment method in accordance with the disclosure is achieved, for example, when there is an incremental or a partial reduction in severity, duration or frequency of one or more associated pathologies, adverse symptoms or complications, or inhibition or reversal of one or more of the physiological, biochemical or cellular manifestations or characteristics of cell proliferation or a cellular hyperproliferative disorder such as a neoplasia, tumor or cancer, or metastasis. A therapeutic benefit or improvement therefore may be, but is not limited to destruction of target proliferating cells (e.g., neoplasia, tumor or cancer, or metastasis) or ablation of one or more, most or all pathologies, adverse symptoms or complications associated with or caused by cell proliferation or the cellular hyperproliferative disorder such as a neoplasia, tumor or cancer, or metastasis. However, a therapeutic benefit or improvement need not be a cure or complete destruction of all target proliferating cells (e.g., neoplasia, tumor or cancer, or metastasis) or ablation of all pathologies, adverse symptoms or complications associated with or caused by cell proliferation or the cellular hyperproliferative disorder such as a neoplasia, tumor or cancer, or metastasis. For example, partial destruction of a tumor or cancer cell mass, or a stabilization of the tumor or cancer mass, size or cell numbers by inhibiting progression or worsening of the tumor or cancer, can reduce mortality and prolong lifespan even if only for a few days, weeks or months, even though a portion or the bulk of the tumor or cancer mass, size or cells remain.

Specific non-limiting examples of therapeutic benefit include a reduction in neoplasia, tumor or cancer, or metastasis volume (size or cell mass) or numbers of cells; inhibiting or preventing an increase in neoplasia, tumor or cancer volume (e.g., stabilizing); slowing or inhibiting neoplasia, tumor or cancer progression, worsening or metastasis; or inhibiting neoplasia, tumor or cancer proliferation, growth or metastasis.

In an instance of the disclosure, administration of the immunotherapeutic agent, in combination therapy with a crystalline salt form of Compound 1, provides a detectable or measurable improvement or overall response according to the irRC (as derived from time-point response assessments and based on tumor burden), including one of more of the following: (i) irCR--complete disappearance of all lesions, whether measurable or not, and no new lesions (confirmation by a repeat, consecutive assessment no less than 4 weeks from the date first documented), (ii) irPR--decrease in tumor burden ≥50% relative to baseline (confirmed by a consecutive assessment at least 4 weeks after first documentation).

Optionally, any method described herein may not take effect immediately. For example, treatment may be followed by an increase in the neoplasia, tumor or cancer cell numbers or mass, but over time eventual stabilization or reduction in tumor cell mass, size or numbers of cells in a given subject may subsequently occur.

Additional adverse symptoms and complications associated with neoplasia, tumor, cancer and metastasis that can be inhibited, reduced, decreased, delayed or prevented include, for example, nausea, lack of appetite, lethargy, pain and discomfort. Thus, a partial or complete decrease or reduction in the severity, duration or frequency of adverse symptom or complication associated with or caused by a cellular hyperproliferative disorder, an improvement in the subject's quality of life and/or well-being, such as increased energy, appetite, psychological well-being, are all particular non-limiting examples of therapeutic benefit.

A therapeutic benefit or improvement therefore can also include a subjective improvement in the quality of life of a treated subject. In an additional instance, a method prolongs or extends lifespan (survival) of the subject. In a further instance, a method improves the quality of life of the subject.

In one instance, administration of the immunotherapeutic agent, in combination therapy with a crystalline salt form of Compound 1, results in a clinically relevant improvement in one or more markers of disease status and progression selected from one or more of the following: (i) overall survival, (ii) progression-free survival, (iii) overall response rate, (iv) reduction in metastatic disease, (v) circulating levels of tumor antigens such as carbohydrate antigen 19.9 (CA19.9) and carcinembryonic antigen (CEA) or others depending on tumor, (vii) nutritional status (weight, appetite, serum albumin), (viii) pain control or analgesic use, and (ix) CRP/albumin ratio.

Treatment with a crystalline salt form of Compound 1 in combination with an immunotherapeutic agent gives rise to more complex immunity including not only the development of innate immunity and type-1 immunity, but also immunoregulation which more efficiently restores appropriate immune functions.

In various exemplary methods, a checkpoint inhibitor antibody (monoclonal or polyclonal, bispecific, trispecific, or an immune cell-engaging multivalent antibody/fusion protein/construct) directed to a checkpoint molecule of interest (e.g., PD-1) may be sequenced and the polynucleotide sequence may then be cloned into a vector for expression or propagation. The sequence encoding the antibody or antigen-binding fragment thereof of interest may be maintained in vector in a host cell and the host cell can then be expanded and frozen for future use. Production of recombinant monoclonal antibodies in cell culture can be carried out through cloning of antibody genes from B cells by means known in the art. See, e.g. Tiller et al., 2008, J. Immunol. Methods 329: 112; U.S. Pat. No. 7,314,622.

Pharmaceutical compositions containing a crystalline salt form of Compound 1 according to the present disclosure will comprise an effective amount of a crystalline salt form of Compound 1, an immunotherapeutic agent, and/or both, typically dispersed in a pharmaceutically acceptable excipient. The phrases "pharmaceutically or pharmacologically acceptable" refers to molecular entities and compositions that do not produce adverse, allergic or other untoward reaction when administered to animal, such as, for example, a human, as appropriate. The preparation of an pharmaceutical composition that contains a crystalline salt form of Compound 1 will be known to those of skill in the art in light of the present disclosure, as exemplified by Remington's Pharmaceutical Sciences, 21st Ed., (Lippincott, Williams and Wilkins Philadelphia, PA, 2006). Moreover, for animal (e.g., human) administration, it will be understood that preparations should meet sterility, pyrogenicity, general safety and purity standards. A specific example of a pharmacologically acceptable excipient for a combination composition, containing a crystalline salt form of Compound 1 in admixture with an immunotherapeutic agent as described herein is borate buffer or sterile saline solution (0.9% NaCl).

Formulations of the an immunotherapeutic agent, for example an immune checkpoint modulator antibody used in accordance with the present disclosure can be prepared for storage by mixing an antibody having the desired degree of purity with optional pharmaceutically acceptable excipients or stabilizers as amply described and illustrated in Remington's Pharmaceutical Sciences 21st Ed., (Lippincott, Williams and Wilkins Philadelphia, PA, 2006), in the form of lyophilized formulations or aqueous solutions and/or suspensions. Acceptable excipients, buffers or stabilizers are nontoxic to recipients at the dosages and concentrations employed, and include suitable aqueous and/or non-aqueous excipients that may be employed in the pharmaceutical compositions of the disclosure, for example, water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants, buffers such as phosphate, citrate, and other organic acids. Antioxidants may be included, for example, (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like; preservatives (such as octade-cyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues). Other exemplary pharmaceutically acceptable excipients may include polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG).

In one illustrative embodiment, the pharmaceutical compositions can optionally contain pharmaceutically acceptable auxiliary substances as required to approximate physiological conditions such as pH adjusting and buffering agents and toxicity adjusting agents, for example, sodium acetate, sodium chloride, potassium chloride, calcium chloride and sodium lactate. In some embodiments, the checkpoint inhibitor antibodies or antigen-binding fragments thereof of the present disclosure are formulated for and can be lyophilized for storage and reconstituted in a suitable excipient prior to use according to art-known lyophilization and reconstitution techniques. In one exemplary pharmaceutical composition containing one or more checkpoint inhibitor antibodies or antigen-binding fragment thereof, the composition is formulated as a sterile, preservative-free solution of one or more checkpoint inhibitor antibodies or antigen-binding fragment thereof for intravenous or subcutaneous administration. The formulation can be supplied as either a single-use, prefilled pen, as a single-use, for example containing about 1 mL prefilled glass syringe, or as a single-use institutional use vial. Preferably, the pharmaceutical composition containing the checkpoint inhibitor antibody or antigen-binding fragment thereof is clear and colorless, with a pH of about 6.9-5.0, preferably a pH of 6.5-5.0, and even more preferably a pH ranging from about 6.0 to about 5.0. In various embodiments, the formulations comprising the pharmaceutical compositions can contain from about 500 mg to about 10 mg, or from about 400 mg to about 20 mg, or from about 300 mg to about 30 mg or from about 200 mg to about 50 mg of the checkpoint inhibitor antibody or antigen-binding fragment thereof per mL of solution when reconstituted and administered to the subject. Exemplary injection or infusion excipients can include mannitol, citric acid monohydrate, dibasic sodium phosphate dihydrate, monobasic sodium phosphate dihydrate, polysorbate 80, sodium chloride, sodium citrate and water for parenteral administration, for example, intravenously, intramuscularly, intraperitoneally, or subcutaneous administration.

In another exemplary embodiment, one or more immunotherapeutic agents, or an antigen-binding fragment thereof is formulated for intravenous or subcutaneous administration as a sterile aqueous solution containing 1-75 mg/mL, or more preferably, about 5-60 mg/mL, or yet more preferably, about 10-50 mg/mL, or even more preferably, about 10-40 mg/mL of antibody, with sodium acetate, polysorbate 80, and sodium chloride at a pH ranging from about 5 to 6. Preferably, the intravenous or subcutaneous formulation is a sterile aqueous solution containing 5, 10, 15, 20, 25, 30, 35, 40, 45, or 50 mg/mL of the immunotherapeutic agent, for example, an immune checkpoint inhibitor antibody or an antigen-binding fragment thereof, with 20 mM sodium acetate, 0.2 mg/mL polysorbate 80, and 140 mM sodium chloride at pH 5.5. Further, a solution comprising a checkpoint inhibitor antibody or an antigen-binding fragment thereof, can comprise, among many other compounds, histidine, mannitol, sucrose, trehalose, glycine, poly(ethylene)glycol, EDTA, methionine, and any combination thereof, and many other compounds known in the relevant art.

In one embodiment, a pharmaceutical composition of the present disclosure comprises the following components: 5-500 mg of an immunotherapeutic agent or antigen-binding fragment thereof of the present disclosure, 10 mM histidine, 5% sucrose, and 0.01% polysorbate 80 at pH 5.8, with a crystalline salt form of Compound 1. This composition may be provided as a lyophilized powder. When the powder is reconstituted at full volume, the composition retains the same formulation. Alternatively, the powder may be reconstituted at half volume, in which case the composition comprises 10-500 mg of an immunotherapeutic agent or antigen-binding fragment thereof of the present disclosure, 20 mM histidine, 10% sucrose, and 0.02% polysorbate 80 at pH 5.8.

In one instance, part of the dose is administered by an intravenous bolus and the rest by infusion of the immunotherapeutic agent formulation. For example, from about 0.001 to about 200 mg/kg, for example, from about 0.001 mg/kg to about 100 mg/kg, or from about 0.001 mg/kg to about 50 mg/kg, or from about 0.001 mg/kg to about 10 mg/kg intravenous injection of the immunotherapeutic agent, or antigen-binding fragment thereof, may be given as a bolus, and the rest of the antibody dose may be administered by intravenous injection. A predetermined dose of the immunotherapeutic agent, or antigen-binding fragment thereof, may be administered, for example, over a period of an hour to two hours to five hours.

In a further instance, part of the dose is administered by a subcutaneous injection and/or infusion in the form of a bolus and the rest by infusion of the immunotherapeutic agent formulation. In some exemplary doses, the immunotherapeutic agent formulation can be administered subcutaneously in a dose ranging from about 0.001 to about 200 mg/kg, for example, from about 0.001 mg/kg to about 100 mg/kg, or from about 0.001 mg/kg to about 50 mg/kg, or from about 0.001 mg/kg to about 10 mg/kg intravenous injection of the immunotherapeutic agent, or antigen-binding fragment thereof. In some instances the dose may be given as a bolus, and the rest of the immunotherapeutic agent dose may be administered by subcutaneous or intravenous injection. A predetermined dose of the immunotherapeutic agent, or antigen-binding fragment thereof, may be administered, for example, over a period of an hour to two hours to five hours.

The formulation herein may also contain more than one active compound as necessary for the particular indication being treated, preferably those with complementary activities that do not adversely affect each other. For example, it may be desirable to provide one or more immunotherapeutic agents with other specificities. Alternatively, or in addition, the composition may comprise an anti-inflammatory agent, a chemotherapeutic agent, a cytotoxic agent, a cytokine, a growth inhibitory agent and/or a small molecule antagonist. Such molecules are suitably present in combination in amounts that are effective for the purpose intended.

The formulations to be used for in vivo administration should be sterile, or nearly so. This is readily accomplished by filtration through sterile filtration membranes.

In various instances, illustrative formulations of the pharmaceutical compositions described herein can be prepared using methods widely known in the field of pharmaceutical formulations. In general, such preparatory methods can include the step of bringing the active ingredient into association with a excipient or one or more other accessory ingredients, and then, if desirable, packaging the product into a desired single-or multi-dose unit.

In some instances, the composition comprising a crystalline salt form of Compound 1 can be also delivered in a vesicle, and the immunotherapeutic agent can be delivered in the same liposome formulation, or in a separate formulation that is compatible with the liposomal formulation containing the crystalline salt form of Compound 1. In some illustrative examples, a liposome containing one or more liposomal surface moieties for example, polyethylene glycol, antibodies and antibody fragments thereof that target a desired tumor surface antigen, receptor, growth factor, glycoprotein, glycolipid or neoantigen, which are selectively transported into specific cells or organs, thus enhance targeted drug delivery.

In another instance, a crystalline salt form of Compound 1 can be delivered in a vesicle, in particular a liposome (see Langer, Science 249: 1527-1533 (1990); Treat et al., in

LIPOSOMES IN THE THERAPY OF INFECTIOUS DISEASE AND CANCER, Lopez-Berestein and Fidler (eds.), Liss, N.Y., pp. 353-365 (1989); Lopez-Berestein, ibid., pp. 317-327; see generally ibid.).

In yet another instance, a crystalline salt form of Compound 1, or the composition containing the combination, or a composition containing the immunotherapeutic agent, can be delivered in a controlled release system. In one instance, a pump can be used (see Langer, supra; Sefton, CRC Crit. Ref. Biomed. Eng. 14: 201 (1987); Buchwald et al., Surgery 88: 507 (1980); Saudek et al., N. Engl. J. Med. 321: 574 (1989)). In another instance, controlled release of the crystalline salt form of Compound 1 can comprise polymeric materials to provide sustained, intermediate, pulsatile, or alternate release (see MEDICAL APPLICATIONS OF CONTROLLED RELEASE, Langer and Wise (eds.), CRC Pres., Boca Raton, Fla. (1974); CONTROLLED DRUG BIOAVAILABILITY, DRUG PRODUCT DESIGN AND PERFORMANCE, Smolen and Ball (eds.), Wiley, New York (1984); Ranger and Peppas, J. Macromol. Sci. Rev. Macromol. Chem. 23: 61 (1983); see also Levy et al., Science 228: 190 (1985); During et al., Ann. Neurol. 25: 351(1989); Howard et al., J. Neurosurg. 71: 105 (1989)). Other controlled-release systems discussed in the review by Langer (Science 249: 1527-1533 (1990)) can be used.

The optimum concentration of the active ingredient(s) in the chosen medium can be determined empirically, according to procedures well known to the skilled artisan, and will depend on the ultimate pharmaceutical formulation desired and the use to be employed.

The present disclosure also provides a pharmaceutical pack or kit comprising one or more containers filled with one or more of the ingredients of the pharmaceutical compositions of the disclosure, which at minimum will include a crystalline salt form of Compound 1 and one or more checkpoint inhibitor antibodies or antigen-binding fragment thereof as described herein. In other instances, the kit may contain one or more further containers providing a pharmaceutically acceptable excipient, for example a diluent. In one instance a kit may comprise at least one container, wherein the container can include a crystalline salt form of Compound 1, a checkpoint inhibitor antibody or an antigen-binding fragment thereof of the present disclosure. The kit may also include a set of instructions for preparing and administering the final pharmaceutical composition to the subject in need thereof, for the treatment of a checkpoint molecule-mediated disease or disorder.

In some instances of the present disclosure, the immunotherapeutic agent is a population of immune cells, which can be administered in combination with a crystalline salt form of Compound 1 to treat a subject with cancer. In some instances, the immunotherapeutic agent is a population of immune cells, such as leukocytes (nucleated white blood cells), comprising (e.g., expressing) a receptor that binds to an antigen of interest. A leukocyte of the present disclosure may be, for example, a neutrophil, eosinophil, basophil, lymphocyte or a monocyte. In some instances, a leukocyte is a lymphocyte. Examples of lymphocytes include T cells, B cells, Natural Killer (NK) cells or NKT cells. In some instances, a T-cell is a CD4+ Th (T helper) cell, a CD8+ cytotoxic T cell, a γδT cell or a regulatory (suppressor) T cell. In some instances, an immune cell is a dendritic cell.

Immune cells of the present disclosure, in some instances, are genetically engineered to express an antigen-binding receptor. A cell is considered "engineered" if it contains an engineered (exogenous) nucleic acid. Engineered nucleic acids of the present disclosure may be introduced into a cell by any known (e.g., conventional) method. For example, an engineered nucleic acid may be introduced into a cell by electroporation (see, e.g., Heiser W. C. Transcription Factor Protocols: Methods in Molecular Biology.TM. 2000; 130: 117-134), chemical (e.g., calcium phosphate or lipid), transfection (see, e.g., Lewis W. H., et al., Somatic Cell Genet. 1980 May; 6(3): 333-47; Chen C., et al., Mol Cell Biol. 1987 August; 7(8): 2745-2752), fusion with bacterial protoplasts containing recombinant plasmids (see, e.g., Schaffner W. Proc Natl Acad Sci USA. 1980 April; 77(4): 2163-7), microinjection of purified DNA directly into the nucleus of the cell (see, e.g., Capecchi M. R. Cell. 1980 November; 22(2 Pt 2): 479-88), or retrovirus transduction.

Some aspects of the present disclosure provide an "adoptive cell" approach, which involves isolating immune cells (e.g., T-cells) from a subject with cancer, genetically engineering the immune cells (e.g., to express an antigen-binding receptor, such as a chimeric antigen receptor), expanding the cells ex vivo, and then re-introducing the immune cells into the subject. This method results in a greater number of engineered immune cells in the subject relative to what could be achieved by conventional gene delivery and vaccination methods. In some instances, immune cells are isolated from a subject, expanded ex vivo without genetic modification, and then re-introduced into the subject.

Immune cells of the present disclosure comprise receptors that bind to antigens, such as an antigen encoded by an exogenously delivered nucleic acid, as provided herein. In some instances, a leukocyte is modified (e.g., genetically modified) to express a receptor that binds to an antigen. The receptor may be, in some instances, a naturally-occurring antigen receptor (normally expressed on the immune cell), recombinant antigen receptor (not normally expressed on the immune cell) or a chimeric antigen receptor (CAR). Naturally-occurring and recombinant antigen receptors encompassed by the present disclosure include T cell receptors, B cell receptors, NK cell receptors, NKT cell receptors and dendritic cell receptors. A "chimeric antigen receptor" refers to an artificial immune cell receptor that is engineered to recognize and bind to an antigen expressed by tumor cells. Generally, a CAR is designed for a T cell and is a chimera of a signaling domain of the T-cell receptor (TcR) complex and an antigen-recognizing domain (e.g., a single chain fragment (scFv) of an antibody) (Enblad et al., Human Gene Therapy. 2015; 26(8): 498-505).

In some instances, an antigen binding receptor is a chimeric antigen receptor (CAR). A T cell that expresses a CAR is referred to as a "CAR T cell." A CAR T cell receptor, in some instances, comprises a signaling domain of the T-cell receptor (TcR) complex and an antigen-recognizing domain (e.g., a single chain fragment (scFv) of an antibody) (Enblad et al., Human Gene Therapy. 2015; 26(8): 498-505).

There are four generations of CARs, each of which contains different components. First generation CARs join an antibody-derived scFv to the CD3zeta (zeta. or z) intracellular signaling domain of the T-cell receptor through hinge and transmembrane domains. Second generation CARs incorporate an additional domain, e.g., CD28, 4-1BB (41BB), or ICOS, to supply a costimulatory signal. Third-generation CARs contain two costimulatory domains fused with the TcR CD3-zeta chain. Third-generation costimulatory domains may include, e.g., a combination of CD3z, CD27, CD28, 4-1BB, ICOS, or OX40. CARs, in some instances, contain an ectodomain (e.g., CD3), commonly derived from a single chain variable fragment (scFv), a hinge, a transmembrane domain, and an endodomain with one (first generation), two (second generation), or three (third generation) signaling domains derived from CD3Z and/or costimulatory molecules (Maude et al., Blood. 2015; 125(26): 4017-4023; Kakarla and Gottschalk, Cancer J. 2014; 20(2): 151-155).

In some instances, the chimeric antigen receptor (CAR) is a T-cell redirected for universal cytokine killing (TRUCK), also known as a fourth generation CAR. TRUCKs are CAR-redirected T-cells used as vehicles to produce and release a transgenic cytokine that accumulates in the targeted tissue, e.g., a targeted tumor tissue. The transgenic cytokine is released upon CAR engagement of the target. TRUCK cells may deposit a variety of therapeutic cytokines in the target. This may result in therapeutic concentrations at the targeted site and avoid systemic toxicity.

CARs typically differ in their functional properties. The CD3zeta signaling domain of the T-cell receptor, when engaged, will activate and induce proliferation of T-cells but can lead to anergy (a lack of reaction by the body's defense mechanisms, resulting in direct induction of peripheral lymphocyte tolerance). Lymphocytes are considered anergic when they fail to respond to a specific antigen. The addition of a costimulatory domain in second-generation CARs improved replicative capacity and persistence of modified T-cells. Similar antitumor effects are observed in vitro with CD28 or 4-1BB CARs, but preclinical in vivo studies suggest that 4-1BB CARs may produce superior proliferation and/or persistence. Clinical trials suggest that both of these second-generation CARs are capable of inducing substantial T-cell proliferation in vivo, but CARs containing the 4-1BB costimulatory domain appear to persist longer. Third generation CARs combine multiple signaling domains (costimulatory) to augment potency. Fourth generation CARs are additionally modified with a constitutive or inducible expression cassette for a transgenic cytokine, which is released by the CAR T-cell to modulate the T-cell response. See, for example, Enblad et al., Human Gene Therapy. 2015; 26(8): 498-505; Chmielewski and Hinrich, Expert Opinion on Biological Therapy. 2015; 15(8): 1145-1154.

In some instances, an illustrative immunotherapeutic agent is a first generation chimeric antigen receptor CAR. In some instances, a chimeric antigen receptor is a second generation CAR. In some instances, a chimeric antigen receptor is a third generation CAR. In some instances, the chimeric antigen receptor is a fourth generation CAR or a T-cell redirected for universal cytokine killing (TRUCK).

In some instances, a chimeric antigen receptor (CAR) comprises an extracellular domain comprising an antigen binding domain, a transmembrane domain, and a cytoplasmic domain. In some instances, a CAR is fully human. In some instances, the antigen binding domain of a CAR is specific for one or more antigens. In some instances, a "spacer" domain or "hinge" domain is located between an extracellular domain (comprising the antigen binding domain) and a transmembrane domain of a CAR, or between a cytoplasmic domain and a transmembrane domain of the CAR. A "spacer domain" refers to any oligopeptide or polypeptide that functions to link the transmembrane domain to the extracellular domain and/or the cytoplasmic domain in the polypeptide chain. A "hinge domain" refers to any oligopeptide or polypeptide that functions to provide flexibility to the CAR, or domains thereof, or to prevent steric hindrance of the CAR, or domains thereof. In some instances, a spacer domain or hinge domain may comprise up to 300 amino acids (e.g., 10 to 100 amino acids, or 5 to 20 amino acids). In some instances, one or more spacer domain(s) may be included in other regions of a CAR.

In some instances, a CAR of the disclosure comprises an antigen binding domain, such as a single chain Fv (scFv) specific for a tumor antigen. The choice of binding domain depends upon the type and number of ligands that define the surface of a target cell. For example, the antigen binding domain may be chosen to recognize a ligand that acts as a cell surface marker on target cells associated with a particular disease state, such as cancer or an autoimmune disease. Thus, examples of cell surface markers that may act as ligands for the antigen binding domain in the CAR of the present disclosure include those associated with cancer cells and/or other forms of diseased cells. In some instances, a CAR is engineered to target a tumor antigen of interest by way of engineering a desired antigen binding domain that specifically binds to an antigen on a tumor cell encoded by an engineered nucleic acid, as provided herein.

An antigen binding domain (e.g., an scFv) that "specifically binds" to a target or an epitope is a term understood in the art, and methods to determine such specific binding are also known in the art. A molecule is said to exhibit "specific binding" if it reacts or associates more frequently, more rapidly, with greater duration and/or with greater affinity with a particular target antigen than it does with alternative targets. An antigen binding domain (e.g., an scFv) that specifically binds to a first target antigen may or may not specifically bind to a second target antigen. As such, "specific binding" does not necessarily require (although it can include) exclusive binding.

In some instances, immune cells expressing a CAR are genetically modified to recognize multiple targets or antigens, which permits the recognition of unique target or antigen expression patterns on tumor cells. Examples of CARs that can bind multiple targets include: "split signal CARs," which limit complete immune cell activation to tumors expressing multiple antigens; "tandem CARs" (TanCARs), which contain ectodomains having two scFvs; and "universal ectodomain CARs," which incorporate avidin or a fluorescein isothiocyanate (FITC)-specific scFv to recognize tumor cells that have been incubated with tagged monoclonal antibodies (Mabs).

A CAR is considered "bispecific" if it recognizes two distinct antigens (has two distinct antigen recognition domains). In some instances, a bispecific CAR is comprised of two distinct antigen recognition domains present in tandem on a single transgenic receptor (referred to as a TanCAR; see, e.g., Grada Z et al. Molecular Therapy Nucleic Acids 2013; 2: e105). Thus, methods, in some instances, comprise delivering to a tumor a combination comprising a crystalline salt form of Compound 1 and an immunotherapeutic agent, wherein the immunotherapeutic agent is an engineered nucleic acid that encodes an antigen, or delivering to a tumor an engineered nucleic acid that induces expression of a self-antigen, and delivering to the tumor an immune cell expressing a bispecific CAR that binds to two antigens, one of which is encoded by the engineered nucleic acid.

In some instances, a CAR is an antigen-specific inhibitory CAR (iCAR), which may be used, for example, to avoid off-tumor toxicity (Fedorov, V D et al. Sci. Transl. Med. published online Dec. 11, 2013). iCARs contain an antigen-specific inhibitory receptor, for example, to block nonspecific immunosuppression, which may result from extra tumor target expression. iCARs may be based, for example, on inhibitory molecules CTLA-4 or PD-1. In some instances, these iCARs block T cell responses from T cells activated by either their endogenous T cell receptor or an activating CAR. In some instances, this inhibiting effect is temporary.

In some instances, CARs may be used in adoptive cell transfer, wherein immune cells are removed from a subject and modified so that they express receptors specific to an antigen, e.g., a tumor-specific antigen. The modified immune cells, which may then recognize and kill the cancer cells, are reintroduced into the subject (Pule, et al., Cytotherapy. 2003; 5(3): 211-226; Maude et al., Blood. 2015; 125(26): 4017-4023).

According to other aspects of the disclosure, the tumor antigenic component in the vaccine of the disclosure is any natural or synthetic tumor-associated protein or peptide or combination of tumor-associated proteins and/or peptides or glycoproteins or glycopeptides. In still yet other aspects, the antigenic component can be patient-specific or common to many or most patients with a particular type of cancer. According to one aspect, the antigenic component consists of a cell lysate derived from tumor tissue removed from the patient being treated. In another aspect, the lysate can be engineered or synthesized from exosomes derived from tumor tissue. In yet another aspect, the antigenic component consists of a cell lysate derived from tumor tissue extracted from one or more unrelated individuals or from tumor-cell lines.

In various instances, an illustrative immunotherapeutic agent comprises one or more cancer vaccines, for use in combination with a crystalline salt form of Compound 1. The tumor-associated antigen component of the vaccine may be manufactured by any of a variety of well-known techniques. For individual protein components, the antigenic protein is isolated from tumor tissue or a tumor-cell line by standard chromatographic means such as high-pressure liquid chromatography or affinity chromatography or, alternatively, it is synthesized by standard recombinant DNA technology in a suitable expression system, such as E. coli, yeast or plants. The tumor-associated antigenic protein is then purified from the expression system by standard chromatographic means. In the case of peptide antigenic components, these are generally prepared by standard automated synthesis. Proteins and peptides can be modified by addition of amino acids, lipids and other agents to improve their incorporation into the delivery system of the vaccine (such as a multilamellar liposome). For a tumor-associated antigenic component derived from the patient's own tumor, or tumors from other individuals, or cell lines, the tumor tissue, or a single cell suspension derived from the tumor tissue, is typically homogenized in a suitable buffer. The homogenate can also be fractionated, such as by centrifugation, to isolate particular cellular components such as cell membranes or soluble material. The tumor material can be used directly or tumor-associated antigens can be extracted for incorporation in the vaccine using a buffer containing a low concentration of a suitable agent such as a detergent. An example of a suitable detergent for extracting antigenic proteins from tumor tissue, tumor cells, and tumor-cell membranes is diheptanoyl phosphatidylcholine. Exosomes derived from tumor tissue or tumor cells, whether autologous or heterologous to the patient, can be used for the antigenic component for incorporation in the vaccine or as a starting material for extraction of tumor-associated antigens.

In some instances of the present disclosure, a combination therapy comprises a crystalline salt form of Compound 1 in combination with a cancer vaccine immunotherapeutic agent. In various examples, the cancer vaccine includes at least one tumor-associated antigen, at least one immunostimulant, and optionally, at least one cell-based immunotherapeutic agent. In some instances, the immunostimulant component in the cancer vaccine of the disclosure is any Biological Response Modifier (BRM) with the ability to enhance the therapeutic cancer vaccine's effectiveness to induce humoral and cellular immune responses against cancer cells in a patient. According to one aspect, the immunostimulant is a cytokine or combination of cytokines. Examples of such cytokines include the interferons, such as IFN-gamma, the interleukins, such as IL-2, IL-15 and IL-23, the colony stimulating factors, such as M-CSF and GM-CSF, and tumor necrosis factor. According to another aspect, the immunostimulant component of the disclosed cancer vaccine includes one or more adjuvant-type immunostimulatory agents such as APC Toll-like Receptor agonists or costimulatory/cell adhesion membrane proteins, with or without immunostimulatory cytokines. Examples of Toll-like Receptor agonists include lipid A and CpG, and costimulatory/adhesion proteins such as CD80, CD86, and ICAM-1.

In some instances, the immunostimulant is selected from the group consisting of IFN-gamma (IFN-γ), IL-2, IL-15, IL-23, M-CSF, GM-CSF, tumor necrosis factor, lipid A, CpG, CD80, CD86, and ICAM-1, or combinations thereof. According to other aspects, the cell-based immunotherapeutic agent is selected from the group consisting of dendritic cells, tumor-infiltrating T lymphocytes, chimeric antigen receptor-modified T effector cells directed to the patient's tumor type, B lymphocytes, natural killer cells, bone marrow cells, and any other cell of a patient's immune system, or combinations thereof. In one aspect, the cancer vaccine immunostimulant includes one or more cytokines, such as interleukin 2 (IL-2), GM-CSF, M-CSF, and interferon-gamma (IFN-γ), one or more Toll-like Receptor agonists and/or adjuvants, such as monophosphoryl lipid A, lipid A, muramyl dipeptide (MDP) lipid conjugate and double stranded RNA, or one or more costimulatory membrane proteins and/or cell adhesion proteins, such CD80, CD86 and ICAM-1, or any combination of the above. In one aspect, the cancer vaccine includes an immunostimulant that is a cytokine selected from the group consisting of interleukin 2 (IL-2), GM-CSF, M-CSF, and interferon-gamma (IFN-γ). In another aspect, the cancer vaccine includes an immunostimulant that is a Toll-like Receptor agonist and/or adjuvant selected from the group consisting of monophosphoryl lipid A, lipid A, and muramyl dipeptide (MDP) lipid conjugate and double stranded RNA. In yet another aspect, the cancer vaccine includes an immunostimulant that is a costimulatory membrane protein and/or cell adhesion protein selected from the group consisting of CD80, CD86, and ICAM-1.

In various instances, an immunotherapeutic agent can include a cancer vaccine, wherein the cancer vaccine incorporates any tumor antigen that can be potentially used to construct a fusion protein according to the disclosure and particularly the following: (a) cancer-testis antigens including NY-ESO-1, SSX2, SCP1 as well as RAGE, BAGE, GAGE and MAGE family polypeptides, for example, GAGE-1, GAGE-2, MAGE-1 MAGE-2, MAGE-3, MAGE-4, MAGE-5, MAGE-6, and MAGE-12, which can be used, for example, to address melanoma, lung, head and neck, NSCLC, breast, gastrointestinal, and bladder tumors; (b) mutated antigens, including p53, associated with various solid tumors, e.g., colorectal, lung, head and neck cancer; p21/Ras associated with, e.g., melanoma, pancreatic cancer and colorectal cancer; CDK4, associated with, e.g., melanoma; MUM1 associated with, e.g., melanoma; caspase-8 associated with, e.g., head and neck cancer; CIA 0205 associated with, e.g., bladder cancer; HLA-A2-R1701, beta catenin associated with, e.g., melanoma; TCR associated with, e.g., T-cell non-Hodgkin lymphoma; BCR-abl associated with, e.g., chronic myelogenous leukemia; triosephosphate isomerase; KIA 0205; CDC-27, and LDLR-FUT; (c) over-expressed antigens, including, Galectin 4 associated with, e.g., colorectal cancer; Galectin 9 associated with, e.g., Hodgkin's disease; proteinase 3 associated with, e.g., chronic myelogenous leukemia; WT 1 associated with, e.g., various leukemias; carbonic anhydrase associated with, e.g., renal cancer; aldolase A associated with, e.g., lung cancer; PRAME associated with, e.g., melanoma; HER-2/neu associated with, e.g., breast, colon, lung and ovarian cancer; mammaglobin, alpha-fetoprotein associated with, e.g., hepatoma; KSA associated with, e.g., colorectal cancer; gastrin associated with, e.g., pancreatic and gastric cancer; telomerase catalytic protein, MUC-1 associated with, e.g., breast and ovarian cancer; G-250 associated with, e.g., renal cell carcinoma; p53 associated with, e.g., breast, colon cancer; and carcinoembryonic antigen associated with, e.g., breast cancer, lung cancer, and cancers of the gastrointestinal tract such as colorectal cancer; (d) shared antigens, including melanoma-melanocyte differentiation antigens such as MART-1/Melan A; gp100; MC1R; melanocyte-stimulating hormone receptor; tyrosinase; tyrosinase related protein-1/TRP1 and tyrosinase related protein-2/TRP2 associated with, e.g., melanoma; (e) prostate associated antigens including PAP, PSA, PSMA, PSH-P1, PSM-P1, PSM-P2, associated with e.g., prostate cancer; (f) immunoglobulin idiotypes associated with myeloma and B cell lymphomas. In certain instances, the one or more TAA can be selected from pi 5, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, pl85erbB2, pl 80erbB-3, c-met, mn-23H1, TAG-72-4, CA 19-9, CA 72-4, CAM 17.1, NuMa, K-ras, pi 6, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, TPS or any combinations thereof.

In some instances, the present disclosure provides a crystalline salt form of Compound 1 for use in combination with a cancer vaccine which can include a tumor antigen comprising the entire amino acid sequence, a portion of it, or specific immunogenic epitopes of a human protein.

In various instances, an illustrative immunotherapeutic agent may include an mRNA operable to encode any one or more of the aforementioned cancer antigens useful for synthesizing a cancer vaccine. In some illustrative instances, the mRNA based cancer vaccine may have one or more of the following properties: a) the mRNA encoding each cancer antigen is interspersed by cleavage sensitive sites; b) the mRNA encoding each cancer antigen is linked directly to one another without a linker; c) the mRNA encoding each cancer antigen is linked to one another with a single nucleotide linker; d) each cancer antigen comprises a 20-40 amino acids and includes a centrally located SNP mutation; e) at least 40% of the cancer antigens have a highest affinity for class I MHC molecules from the subject; f) at least 40% of the cancer antigens have a highest affinity for class II MHC molecules from the subject; g) at least 40% of the cancer antigens have a predicted binding affinity of IC>500 nM for HLA-A, HLA-B and/or DRB1; h) the mRNA encodes 1 to 15 cancer antigens; i) 10-60% of the cancer antigens have a binding affinity for class I MHC and 10-60% of the cancer antigens have a binding affinity for class II MHC; and/or j) the mRNA encoding the cancer antigens is arranged such that the cancer antigens are ordered to minimize pseudo-epitopes.

In various instances, the combination comprising a crystalline salt form of Compound 1 and a cancer vaccine immunotherapeutic agent as disclosed herein can be used to illicit an immune response in a subject against a cancer antigen. The method involves administering to the subject a RNA vaccine comprising at least one RNA polynucleotide having an open reading frame encoding at least one antigenic polypeptide or an immunogenic fragment thereof, thereby inducing in the subject an immune response specific to the antigenic polypeptide or an immunogenic fragment thereof, in combination with administering a crystalline salt form of Compound 1 either in the same composition or a separate composition, administered at the same time, or sequentially dosed, wherein the anti-antigenic polypeptide antibody titer in the subject is increased following vaccination relative to anti-antigenic polypeptide antibody titer in a subject vaccinated with a prophylactically effective dose of a traditional vaccine against the cancer. An "anti-antigenic polypeptide antibody" is a serum antibody the binds specifically to the antigenic polypeptide.

A prophylactically effective dose is a therapeutically effective dose that prevents advancement of cancer at a clinically acceptable level. In some instances the therapeutically effective dose is a dose listed in a package insert for the vaccine. A traditional vaccine, as used herein, refers to a vaccine other than the mRNA vaccines of the disclosure. For instance, a traditional vaccine includes but is not limited to live microorganism vaccines, killed microorganism vaccines, subunit vaccines, protein antigen vaccines, DNA vaccines, and the like. In exemplary instances, a traditional vaccine is a vaccine that has achieved regulatory approval and/or is registered by a national drug regulatory body, for example the Food and Drug Administration (FDA) in the United States or the European Medicines Agency (EMA.)

In some instances the anti-antigenic polypeptide antibody titer in the subject is increased 1 log to 10 log following vaccination relative to anti-antigenic polypeptide antibody titer in a subject vaccinated with a prophylactically effective dose of a traditional vaccine against the cancer. In some instances the anti-antigenic polypeptide antibody titer in the subject is increased 1 log following vaccination relative to anti-antigenic polypeptide antibody titer in a subject vaccinated with a prophylactically effective dose of a traditional vaccine against the cancer. In some instances the anti-antigenic polypeptide antibody titer in the subject is increased 2 log following vaccination relative to anti-antigenic polypeptide antibody titer in a subject vaccinated with a prophylactically effective dose of a traditional vaccine against the cancer.

Aspects of the disclosure provide nucleic acid vaccines comprising one or more RNA polynucleotides having an open reading frame encoding a first antigenic polypeptide, wherein the RNA polynucleotide is present in the formulation for in vivo administration to a host, which confers an antibody titer superior to the criterion for sero-protection for the first antigen for an acceptable percentage of human subjects. In some instances, the antibody titer produced by the mRNA vaccines of the disclosure is a neutralizing antibody titer. In some instances the neutralizing antibody titer is greater than a protein vaccine. In other instances the neutralizing antibody titer produced by the mRNA vaccines of the disclosure is greater than an adjuvanted protein vaccine. In yet other instances the neutralizing antibody titer produced by the mRNA vaccines of the disclosure is 1,000-10,000, 1,200-10,000, 1,400-10,000, 1,500-10,000, 1,000-5,000, 1,000-4,000, 1,800-10,000, 2000-10,000, 2,000-5,000, 2,000-3,000, 2,000-4,000, 3,000-5,000, 3,000-4,000, or 2,000-2,500. A neutralization titer is typically expressed as the highest serum dilution required to achieve a 50% reduction in the number of plaques.

In preferred aspects, RNA vaccine immunotherapeutic agents of the present disclosure (e.g., mRNA vaccines) produce prophylactically- and/or therapeutically-efficacious levels, concentrations and/or titers of antigen-specific antibodies in the blood or serum of a vaccinated subject. As defined herein, the term antibody titer refers to the amount of antigen-specific antibody produced in a subject, e.g., a human subject. In exemplary instances, antibody titer is expressed as the inverse of the greatest dilution (in a serial dilution) that still gives a positive result. In exemplary instances, antibody titer is determined or measured by enzyme-linked immunosorbent assay (ELISA). In exemplary instances, antibody titer is determined or measured by neutralization assay, e.g., by microneutralization assay. In certain aspects, antibody titer measurement is expressed as a ratio, such as 1:40, 1:100, and the like.

In exemplary instances of the disclosure, an efficacious vaccine produces an antibody titer of greater than 1:40, greater that 1:100, greater than 1:400, greater than 1: 1000, greater than 1:2000, greater than 1:3000, greater than 1:4000, greater than 1:500, greater than 1:6000, greater than 1:7500, greater than 1: 10000. In exemplary instances, the antibody titer is produced or reached by 10 days following vaccination, by 20 days following vaccination, by 30 days following vaccination, by 40 days following vaccination, or by 50 or more days following vaccination. In exemplary instances, the titer is produced or reached following a single dose of vaccine administered to the subject. In other instances, the titer is produced or reached following multiple doses, e.g., following a first and a second dose (e.g., a booster dose.) In exemplary aspects of the disclosure, antigen-specific antibodies are measured in units of g/ml or are measured in units of IU/L (International Units per liter) or mIU/ml (milli International Units per ml). In exemplary instances of the disclosure, an efficacious vaccine produces >0.5 µg/mL, >0.1 µg/mL, >0.2 µg/mL, >0.35 µg/mL, >0.5 µg/mL, >1 µg/mL, >2 µg/mL, >5 µg/mL or >10 µg/mL. In exemplary instances of the disclosure, an efficacious vaccine produces >10 mIU/ mL, >20 mIU/ mL, >50 mIU/ mL, >100 mIU/ mL, >200 mIU/ mL, >500 mIU/ml or >1000 mIU/ml. In exemplary instances, the antibody level or concentration is produced or reached by 10 days following vaccination, by 20 days following vaccination, by 30 days following vaccination, by 40 days following vaccination, or by 50 or more days following vaccination. In exemplary instances, the level or concentration is produced or reached following a single dose of vaccine administered to the subject. In other instances, the level or concentration is produced or reached following multiple doses, e.g., following a first and a second dose (e.g., a booster dose.) In exemplary instances, antibody level or concentration is determined or measured by enzyme-linked immunosorbent assay (ELISA). In exemplary instances, antibody level or concentration is determined or measured by neutralization assay, e.g., by microneutralization assay. Also provided are nucleic acid vaccines comprising one or more RNA polynucleotides having an open reading frame encoding a first antigenic polypeptide or a concatemeric polypeptide, wherein the RNA polynucleotide is present in a formulation for in vivo administration to a host for eliciting a longer lasting high antibody titer than an antibody titer elicited by an mRNA vaccine having a stabilizing element or formulated with an adjuvant and encoding the first antigenic polypeptide. In some instances, the RNA polynucleotide is formulated to produce neutralizing antibodies within one week of a single administration. In some instances, the adjuvant is selected from a cationic peptide and an immunostimulatory nucleic acid. In some instances, the cationic peptide is protamine.

Immunotherapeutic agents comprising a nucleic acid vaccine comprising one or more RNA polynucleotides having an open reading frame comprising at least one chemical modification or optionally no nucleotide modification, the open reading frame encoding a first antigenic polypeptide or a concatemeric polypeptide, wherein the RNA polynucleotide is present in the formulation for in vivo administration to a host such that the level of antigen expression in the host significantly exceeds a level of antigen expression produced by an mRNA vaccine having a stabilizing element or formulated with an adjuvant and encoding the first antigenic polypeptide.

Other aspects provide nucleic acid vaccines comprising one or more RNA polynucleotides having an open reading frame comprising at least one chemical modification or optionally no nucleotide modification, the open reading frame encoding a first antigenic polypeptide or a concatemeric polypeptide, wherein the vaccine has at least 10 fold less RNA polynucleotide than is required for an unmodified mRNA vaccine to produce an equivalent antibody titer. In some instances, the RNA polynucleotide is present in a dosage of 25-100 micrograms.

Aspects of the disclosure also provide a unit of use vaccine, comprising between 10 µg and 400 µg of one or more RNA polynucleotides having an open reading frame comprising at least one chemical modification or optionally no nucleotide modification, the open reading frame encoding a first antigenic polypeptide or a concatemeric polypeptide, and a pharmaceutically acceptable excipient, formulated for delivery to a human subject. In some instances, the vaccine further comprises a cationic lipid nanoparticle.

Aspects of the disclosure provide methods of creating, maintaining or restoring antigenic memory to a tumor in an individual or population of individuals comprising administering to said individual or population an antigenic memory booster nucleic acid vaccine comprising (a) at least one RNA polynucleotide, said polynucleotide comprising at least one chemical modification or optionally no nucleotide modification and two or more codon-optimized open reading frames, said open reading frames encoding a set of reference antigenic polypeptides, and (b) optionally a pharmaceutically acceptable excipient. In some instances, the vaccine is administered to the individual via a route selected from the group consisting of intramuscular administration, intradermal administration and subcutaneous administration. In some instances, the administering step comprises contacting a muscle tissue of the subject with a device suitable for injection of the composition. In some instances, the administering step comprises contacting a muscle tissue of the subject with a device suitable for injection of the composition in combination with electroporation.

Aspects of the disclosure provide methods of vaccinating a subject comprising administering to the subject a single dosage of between 25 µg /kg and 400 µg /kg of a nucleic acid vaccine comprising one or more RNA polynucleotides having an open reading frame encoding a first antigenic polypeptide or a concatemeric polypeptide in an effective amount to vaccinate the subject.

Other aspects provide nucleic acid vaccines comprising one or more RNA polynucleotides having an open reading frame comprising at least one chemical modification, the open reading frame encoding a first antigenic polypeptide or a concatemeric polypeptide, wherein the vaccine has at least 10 fold less RNA polynucleotide than is required for an unmodified mRNA vaccine to produce an equivalent antibody titer. In some instances, the RNA polynucleotide is present in a dosage of 25-100 micrograms.

In some instances, a crystalline salt form of Compound 1 can be used in combination with a bispecific antibody immunotherapeutic agent. The bispecific antibody can include a protein construct having a first antigen binding moiety and a second antigen binding site that binds to a cytotoxic immune cell. The first antigen binding site can bind to a tumor antigen that is specifically being treated with the combination of the present disclosure. For example, the first antigen binding moiety may bind to a non-limiting example of tumor antigens selected from: EGFR, HGFR, Her2, Ep-CAM, CD20, CD30, CD33, CD47, CD52, CD133, CEA, gpA33, Mucins, TAG-72, CIX, PSMA, folate-binding protein, GD2, GD3, GM2, VEGF. VEGFR, Integrin αYβ3, Integrin α5β1, MUC1, ERBB2, ERBB3, MET, IGF1R, EPHA3, TRAILR1, TRAILR2, RANKL, FAP and Tenascin among others. In some instances, the first antigen binding moiety has specificity to a protein or a peptide that is overexpressed on a tumor cell as compared to a corresponding non-tumor cell. In some instances, the first antigen binding moiety has specificity to a protein that is overexpressed on a tumor cell as compared to a corresponding non-tumor cell. A "corresponding non-tumor cell" as used here, refers to a non-tumor cell that is of the same cell type as the origin of the tumor cell. It is noted that such proteins are not necessarily different from tumor antigens. Non-limiting examples include carcinoembryonic antigen (CEA), which is overexpressed in most colon, rectum, breast, lung, pancreas and gastrointestinal tract carcinomas; heregulin receptors (HER-2, neu or c-erbB-2), which is frequently overexpressed in breast, ovarian, colon, lung, prostate and cervical cancers; epidermal growth factor receptor (EGFR), which is highly expressed in a range of solid tumors including those of the breast, head and neck, non-small cell lung and prostate; asialoglycoprotein receptor; transferrin receptor; serpin enzyme complex receptor, which is expressed on hepatocytes; fibroblast growth factor receptor (FGFR), which is overexpressed on pancreatic ductal adenocarcinoma cells; vascular endothelial growth factor receptor (VEGFR), for anti-angiogenesis gene therapy; folate receptor, which is selectively overexpressed in 90% of nonmucinous ovarian carcinomas; cell surface glycocalyx; carbohydrate receptors; and polymeric immunoglobulin receptor.

The second antigen-binding moiety is any molecule that specifically binds to an antigen or protein or polypeptide expressed on the surface of a cytotoxic immune cell (a CIK cell). Exemplary non-limiting antigens expressed on the surface of the cytotoxic immune cells suitable for use with the present disclosure may include CD2, CD3, CD4, CD5, CD8, CD11a, CD11 b, CD14, CD16a, CD27, CD28, CD45, CD45RA, CD56, CD62L, the Fc receptor, LFA, LFA-1, TCRαβ, CCR7, macrophage inflammatory protein 1a, perforin, PD-1, PD-L1, PD-L2, or CTLA-4, LAG-3, OX40, 41BB, LIGHT, CD40, GITR, TGF-beta, TIM-3, SIRP-alpha, TIGIT, VSIG8, BTLA, SIGLEC7, SIGLEC9, ICOS, B7H3, B7H4, FAS, BTNL2, CD27 and Fas ligand. In some instances, the second antigen binding moiety binds to CD3 of the cytotoxic immune cell, e.g., CIK cell. In some instances, the second antigen binding moiety binds to CD56 of the cytotoxic immune cell. In some instances, the second antigen binding moiety binds to the Fc receptor of the cytotoxic immune cell. In some instances, the Fc region of the bispecific antibody binds to the Fc receptor of the cytotoxic immune cell. In some instances, a second antigen-binding moiety is any molecule that specifically binds to an antigen expressed on the surface of a cytotoxic immune cell (e.g., a CIK cell). The second antigen binding moiety is specific for an antigen on a cytotoxic immune cell. Exemplary cytotoxic immune cells include, but are not limited to CIK cells, T-cells, CD8+ T cells, activated T-cells, monocytes, natural killer (NK) cells, NK T cells, lymphokine-activated killer (LAK) cells, macrophages, and dendritic cells. The second antigen binding moiety specifically binds to an antigen expressed on the surface of a cytotoxic immune cell. Exemplary non-limiting antigens expressed on the surface of the cytotoxic immune cells suitable for modulation with the present disclosure may include CD2, CD3, CD4, CD5, CD8, CD1 1a, CD11 b, CD14, CD16a, CD27, CD28, CD45, CD45RA, CD56, CD62L, the Fc receptor, LFA, LFA-1, TCRαβ, CCR7, macrophage inflammatory protein 1a, perforin, PD-1, PD-L1, PD-L2, or CTLA-4, LAG-3, OX40, 41BB, LIGHT, CD40, GITR, TGF-beta, TIM-3, SIRP-alpha, TIGIT, VSIG8, BTLA, SIGLEC7, SIGLEC9, ICOS, B7H3, B7H4, FAS, BTNL2, CD27 and Fas ligand. In other instances, the bispecific antibody modulator is an activator of a costimulatory molecule (e.g., an OX40 agonist). In one instance, the OX40 agonist is a bispecific antibody molecule to OX40 and another tumor antigen or a costimulatory antigen. The OX40 agonist can be administered alone, or in combination with other immunomodulators, e.g., in combination with an inhibitor (for example an antibody construct) of PD-1, PD-L1, CTLA-4, CEACAM (e.g., CEACAM-1, -3 and/or -5), TIM-3 or LAG-3. In some instances, the anti-OX40 antibody molecule is a bispecific antibody that binds to GITR and PD-1, PD-L1, CTLA-4, CEACAM (e.g., CEACAM-1, -3 and/or -5), TIM-3 or LAG-3. In one exemplary instance, an OX40 antibody molecule is administered in combination with an anti-PD-1 antibody molecule (e.g., an anti-PD-1 molecule as described herein). The OX40 antibody molecule and the anti-PD-1 antibody molecule may be in the form of separate antibody composition, or as a bispecific antibody molecule. In other instances, the OX40 agonist can be administered in combination with other costimulatory molecule, e.g., an agonist of GITR, CD2, CD27, CD28, CDS, ICAM-1, LFA-1 (CD11a/CD18), ICOS (CD278), 4-1BB (CD137), CD30, CD40, BAFFR, HVEM, CD7, LIGHT, NKG2C, SLAMF7, NKp80, CD160, B7-H3, or CD83 ligand. In some instances, the second antigen binding moiety binds to the Fc receptor on the cytotoxic immune cell, e.g., CIK cell.

In some instances, the bispecific antibody immunotherapeutic agent has specificities for a tumor antigen and a CIK cell, which brings the tumor antigen expressing tumor cell in close proximity of the CIK cell, leading to the elimination of the tumor cell through anti-tumor cytotoxicity of CIK cell. In some instances, the bispecific antibody has specificity for a tumor antigen but does not have specificity for a CIK cell, however, the Fc region of the bispecific antibody can bind to the Fc receptor of the CIK cell, which in turn brings the tumor cell in close proximity of the CIK cell, leading to the elimination of the tumor cell through anti-tumor cytotoxicity of CIK cell. In some instances, the bispecific antibody has specificity for a CIK cell but does not have specificity for tumor cell, however, the Fc region of the bispecific antibody can bind to the Fc receptor of the tumor cell, which in turn brings the tumor cell in close proximity of the CIK cell, leading to the elimination of the tumor cell through anti-tumor cytotoxicity of CIK cell.

In some instances, a crystalline salt form of Compound 1 can be used in combination with an immune cell-engaging multivalent antibody/fusion protein/construct immunotherapeutic agent. In various instances, an exemplary immunotherapeutic agent can include immune cell-engaging multivalent antibody/fusion protein/construct which may comprise a recombinant structure, for example, all engineered antibodies that do not imitate the original IgG structure. Here, different strategies to multimerize antibody fragments are utilized. For example, shortening the peptide linker between the V domains forces the scFv to self-associate into a dimer (diabody; 55 kDa). Bispecific diabodies are formed by the noncovalent association of two VHA-VLB and VHB-VLA fragments expressed in the same cell. This leads to the formation of heterodimers with two different binding sites. Single-chain diabodies (sc-diabodies) are bispecific molecules where the VHA-VLB and VHB-VLA fragments are linked together by an additional third linker. Tandem-diabodies (Tandabs) are tetravalent bispecific antibodies generated by two scDiabodies.

Also included are the di-diabodies known in the art. This 130-kDa molecule is formed by the fusion of a diabody to the N-terminus of the CH3 domain of an IgG, resulting in an IgG-like structure. Further diabody derivatives are the triabody and the tetra-body, which fold into trimeric and tetrameric fragments by shortening the linker to <5 or 0-2 residues. Also exemplified are (scFv)₂ constructs known as 'bispecific T cell engager' (BITE). BITEs are bispecific single-chain antibodies consisting of two scFv antibody fragments, joined via a flexible linker, that are directed against a surface antigen on target cells and CD3 on T cells. Also exemplified are bivalent (Fab)2 and trivalent (Fab)3 antibody formats. Also exemplified are minibodies and trimerbodies generated from scFvs. Exemplary constructs useful to target tumor antigens as can include one or more of: Diabody, Single-chain (sc)-diabody (scFv)2, Miniantibody, Minibody, Barnase-barstar, scFv-Fc, sc(Fab)2, Trimeric antibody constructs, Triabody antibody constructs, Trimerbody antibody constructs, Tribody antibody constructs, Collabody antibody constructs, (scFv-TNFa)3, F(ab)3/DNL. Exemplary cytotoxic immune cells include, but are not limited to CIK cells, T-cells, CD8+ T cells, activated T-cells, monocytes, natural killer (NK) cells, NK T cells, lymphokine-activated killer (LAK) cells, macrophages, and dendritic cells.

In some instances, a crystalline salt form of Compound 1 can be used in combination with a radioconjugate immunotherapeutic agent.

In various instances, a radioconjugate is a small molecule or large molecule (herein referred to as a "cell targeting agent"), for example and polypeptide, an antibody or an antibody fragment thereof, that is coupled to or otherwise affixed to a radionuclide, or a plurality of radionuclides, such that the binding of the radioconjugate to its target (a protein or molecule on or in a cancer cell), will lead to the death or morbidity of said cancer cell. In various instances, the radioconjugate can be a cell targeting agent labelled with a radionuclide, or the cell targeting agent may be coupled or otherwise affixed to a particle, or microparticle, or nanoparticle containing a plurality of radionuclides, wherein the radionuclides are the same or different. Methods for synthesizing radioconjugates are known in the art, and may include the class of immunoglobulin or antigen binding parts thereof, that are conjugated to a toxic radionuclide.

In some instances, the molecule that binds to the cancer cell can be known as a "cell targeting agent". As used herein, an exemplary cell targeting agent can allow the drug-containing nanoparticles or radionuclide to target the specific types of cells of interest. Examples of cell targeting agents include, but are not limited to, small molecules (e.g., folate, adenosine, purine) and large molecule (e.g., peptide or antibody) that bind to or target a tumor associated antigen. Examples of tumor associated antigens include, but are not limited to, adenosine receptors, alpha v beta 3, aminopeptidase P, alpha fetoprotein, cancer antigen 125, carcinoembryonic antigen, cCaveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase, and tyrosine kinases. In some instances, the cell targeting agent is folate or a folate derivative that binds specifically to folate receptors (FRs). In some instances, the cell targeting agent is an antibody, a bispecific antibody, a trispecific antibody or an antigen binding construct thereof, that specifically binds to a cancer antigen selected from: EGFR, HGFR, Her2, Ep-CAM, CD20, CD30, CD33, CD47, CD52, CD133, CEA, gpA33, Mucins, TAG-72, CIX, PSMA, folate-binding protein, GD2, GD3, GM2, VEGF. VEGFR, Integrin αVβ3, Integrin α5β1, MUC1, ERBB2, ERBB3, MET, IGF1R, EPHA3, TRAILR1, TRAILR2, RANKL, FAP and Tenascin among others.

The use of folate as a targeting agent in the radioconjugate also allow both tumor cells and regulatory T (Treg) cells to be targeted for destruction. It is well accepted that high numbers of Treg cells suppress tumor immunity. Specifically, Treg cells suppress (foreign and self) reactive T cells without killing them through contact-dependent or cytokine (e.g., IL-10, TGF-beta., and the like) secretion. FR4 is selectively upregulated on Treg cells. It has been shown that antibody blockade of FR4 depleted Treg cells and provoked tumor immunity in tumor-bearing mice. Thus, folate-coated PBM nanoparticles carrying a cytotoxic agent would take FR-expressing cells for their destruction, which would both directly (i.e., BrCa cell) and indirectly (i.e., breast tumor associated and peripheral Treg cells) inhibit tumor progression.

In another further instance, the targeting agent is an antibody or peptide, or immune cell-engaging multivalent antibody/fusion protein/constructs capable of binding tumor associated antigens consisting of but not limited to: adenosine receptors, alpha v beta 3, aminopeptidase P, alpha fetoprotein, cancer antigen 125, carcinoembryonic antigen, caveolin-1, chemokine receptors, clusterin, oncofetal antigens, CD20, Human Growth Factor Receptor (HGFR), epithelial tumor antigen, melanoma associated antigen, MUC1, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyrosinase, tyrosine kinases, and the like.

In some instances, a crystalline salt form of Compound 1 as described herein can be used in combination with a vaccination protocol for the treatment of cancer. In some instances, a crystalline salt form of Compound 1 as described herein can be used in combination with an immunotherapeutic agent such as a vaccine. In various instances, exemplary vaccines include those used to stimulate the immune response to cancer antigens.

The amount of both the crystalline salt form of Compound 1 as disclosed herein and the additional one or more additional therapeutic agents (in those compositions which comprise an additional therapeutic agent as described above) that may be combined with excipient materials to produce a single dosage form will vary depending upon the host treated and the particular mode of administration. In certain instances, compositions of this disclosure are formulated such that a dosage of between 0.01-100 mg/kg body weight/day of an inventive can be administered.

The additional therapeutic agent and the crystalline salt form of Compound 1 as disclosed herein may act synergistically. Therefore, the amount of additional therapeutic agent in such compositions may be less than that required in a monotherapy utilizing only that therapeutic agent, or there may be fewer side effects for the patient given that a lower dose is used. In certain instances, in such compositions a dosage of between 0.01-10,000 µg/kg body weight/day of the additional therapeutic agent can be administered.

In some instances, the crystalline salt forms of compound 1 as disclosed herein can be combined with one or more inhibitors of the following kinases for the treatment of a disease disclosed herein such as cancer: Akt1, Akt2, Akt3, TGF-βR, PKA, PKG, PKC, CaM-kinase, phosphorylase kinase, MEKK, ERK, MAPK, mTOR, EGFR, HER2, HER3, HER4, 1NS-R, IGF-1R, IR-R, PDGFαR, PDGFβ/R, CSFIR, KIT, FLK-II, KDR/FLK-1, FLK-4, flt-1, FGFR1, FGFR2, FGFR3, FGFR4, Ron, Sea, TRKA, TRKB, TRKC, FLT3, VEGFR/Flt2, Flt4, EphAl, EphA2, EphA3, EphB2, EphB4, Tie2, Src, Fyn, Lck, Fgr, Btk, Fak, SYR, FRK, JAK, ABL, ALK, CDK7, CDK12, CDK13, KRAS, and B-Raf. In some instances, the crystalline forms or salt forms of compound 1 as disclosed herein can be combined with one or more inhibitors of CD47 and MALT1 proteins for the treatment of cancer.

In some instances, the crystalline salt forms of compound 1 as disclosed herein can be used in combination with one or more Poly ADP ribose polymerase (PARP) inhibitors for the treatment of a disease disclosed herein such as cancer. Exemplary PARP inhibitors include, but are not limited to, olaparib (Lynparza^{®}), rucaprib (Rubraca^{®}) niraparib (Zejula^{®}), talzoparib (Talzenna^{®}) and TPST-1120.

In some instances, the crystalline salt forms of compound 1 as disclosed herein can be used in combination therapy with any of the kinase inhibitors disclosed herein for the treatment of diseases such as cancer. Exemplary kinase inhibitors include imatinib, baricitinib gefitinib, erlotinib, sorafenib, dasatinib, sunitinib, lapatinib, nilotinib, pirfenidone, zanubrutinib, updacitinib, fedratinib, entrectinib, alpelisib, pazopanib, crizotinib, vemurafenib, vandetanib, ruxolitinib, axitinib, bosutinib, regorafenib, tofacitinib, cabozantinib, ponatinib, trametinib, dabrafenib, afatinib, ibrutinib, ceritinib, idelalisib, nintedanib, palbociclib, lenvatinib, cobimetinib, abemaciclib, acalabrutinib, alectinib, binimetinib, brigatinib, encorafenib, erdafitinib, everolimus, fostamatinib, gilter, larotrectinib, lorlatinib, netarsudil, osimertinib, pexidartinib, ribociclib, temsirolimus, XL-147, XL-765, XL-499, and XL-880. In some instances, a compound as described herein can be used in combination with a HSP90 inhibitor (e.g., XL888), liver X receptor (LXR) modulators, retinoid-related orphan receptor gamma (RORy) modulators, a CK1 inhibitor, a CKl-a inhibitor, a Wnt pathway inhibitor (e.g., SST-215), or a mineralocorticoid receptor inhibitor, (e.g., esaxerenone or XL- 550) for the treatment of a disease disclosed herein such as cancer.

In some instances, the crystalline salt forms of compound 1 as disclosed herein can be used in combination with polatuzumab vedotin for the treatment of a disease disclosed herein such as cancer.

### Labeled Compounds and Assay Methods

Another aspect, although not specifically claimed, relates to labeled crystalline salt forms of Compound 1 (radio-labeled, fluorescent-labeled, etc.) that would be useful not only in imaging techniques but also in assays, both *in vitro* and *in vivo*, for localizing and quantitating TAM kinases in tissue samples, including human, and for identifying TAM kinase ligands by inhibition binding of a labeled compound. Accordingly, the present disclosure includes TAM kinase assays that contain such labeled compounds.

The present disclosure further includes, but does not specifically claim, isotopically-labeled crystalline salt forms of Compound 1. An "isotopically" or "radio-labeled" compound is a crystalline salt form of Compound 1 where one or more atoms are replaced or substituted by an atom having an atomic mass or mass number different from the atomic mass or mass number typically found in nature (i.e., naturally occurring). Suitable radionuclides that may be incorporated in compounds of the present disclosure include but are not limited to ²H (also written as D for deuterium), ³H (also written as T for tritium), ¹¹C, ¹³C, ¹⁴C, ¹³N, ¹⁵N, ¹⁵O, ¹⁷O, ¹⁸O, ¹⁸F , ³⁵S, ³⁶Cl, ⁸²Br, ⁷⁵Br, ⁷⁶Br, ⁷⁷Br, ¹²³I, ¹²⁴I, ¹²⁵I, and ¹³¹I. The radionuclide that is incorporated in the instant radio-labeled compounds will depend on the specific application of that radio-labeled compound. For example, for *in vitro* metalloprotease labeling and competition assays, compounds that incorporate ³H, ¹⁴C, ⁸²Br, ¹²⁵I, ¹³¹I, or ³⁵S will generally be most useful. For radio-imaging applications ¹¹C, ¹⁸F, ¹²⁵I, ¹²³I, ¹²⁴I, ¹³¹I, ⁷⁵Br, ⁷⁶Br, or ⁷⁷Br will generally be most useful. In some instances, the crystalline forms or crystalline salt forms described herein in which one or more hydrogens is/are replaced by deuterium, such as hydrogen bonded to a carbon atom. Such compounds exhibit increased resistance to metabolism and are thus useful for increasing the half-life of any compound when administered to a mammal, particularly a human.

It is understood that a "radio-labeled" or "labeled compound" is a compound that has incorporated at least one radionuclide. In some instances, the radionuclide is selected from the group consisting of ³H, ¹⁴C, ¹²⁵I, ³⁵S, and ⁸²Br.

The present disclosure can further include synthetic methods for incorporating radioisotopes into crystalline salt forms of Compound 1. Synthetic methods for incorporating radioisotopes into organic compounds are well known in the art, and a person of ordinary skill in the art will readily recognize the methods applicable for the compounds of disclosure.

A labeled crystalline salt form of Compound 1 can be used in a screening assay to identify/evaluate compounds. For example, a newly synthesized or identified compound (i.e., test compound) which is labeled can be evaluated for its ability to bind a TAM by monitoring its concentration variation when contacting with the TAM kinases, through tracking of the labeling. For example, a test compound (labeled) can be evaluated for its ability to reduce binding of another compound which is known to bind to a TAM kinase (i.e., standard compound). Accordingly, the ability of a test compound to compete with the standard compound for binding to the TAM kinase directly correlates to its binding affinity. Conversely, in some other screening assays, the standard compound is labeled, and test compounds are unlabeled. Accordingly, the concentration of the labeled standard compound is monitored in order to evaluate the competition between the standard compound and the test compound, and the relative binding affinity of the test compound is thus ascertained.

### PREPARATIONS AND EXAMPLES

### General Experimental Techniques

*Aqueous Slurry Experiments:* Salts of Compound 1 that were determined to have aqueous solubility less than 1 mg/mL were slurried in 20 mL of water at ambient temperature for 1 day. Solids were then collected by vacuum filtration and analyzed by XRPD.

*Crash Cooling (CC):* Concentrated solutions of Compound 1 and various counterions were prepared in MeOH at elevated temperature with stirring. Capped vials containing hot solutions were transferred to the freezer (∼-20 °C) and rapidly cooled. Solids that were formed were collected. If no solids were present, additional crystallization techniques were employed.

*Crash Precipitation (CP):* Clear solutions of Compound 1 and coformer were prepared in various solvents at RT. Aliquots of various anti-solvents were added to the solution, slowly, with gentle stirring until solids crashed out of solution. Mixtures were allowed to stir for a specified period of time. Solids that formed were collected by positive-pressure filtration.

*Fast Cooling (FC):* Concentrated solutions of Compound 1 and various counterions were prepared in acetone or MeOH at elevated temperature with stirring. Capped vials containing hot solutions were transferred to the bench top at ambient temperature. Solids that were formed were collected. If no solids were present, additional crystallization techniques were employed.

*Fast Evaporation (FE)*: Clear solutions of Compound 1 and coformer were prepared in various solvents. Vials were left uncapped and solvent evaporated at ambient conditions.

*Interconversion Slurry:* A slurry of Compound 1 Form A was prepared by adding enough solids to a given solvent system at ambient conditions so that undissolved solids were present. The mixture was then agitated for an extended period of time to ensure saturation. Solids of the forms of interest were then added to an aliquot of the saturated solution (filtered through a 0.2-µm nylon filter) so that undissolved solids were present. The mixture was then agitated at ambient temperature for an extended period of time, and the solids were isolated.

*Isolation Techniques*: In general, isolation was done quickly after removing non-ambient samples from their respective temperature control devices to minimize equilibration to ambient temperature prior to isolation of the solids.

*Decanting Liquid Phase*: Some of the solids isolated from solution-based crystallization techniques were collected by centrifuging the suspension (if needed) and discarding the liquid phase, leaving behind damp solids. Solids were dried briefly (e.g. air dried or under nitrogen) unless specified as "analyzed damp" herein.

*Poitive-Pressure Filtration*: Solids were collected on 0.2-µm nylon or PTFE filters by pressing a slurry through a syringe and Swinnex filter holder assembly. In general, solids were dried briefly by blowing a 20-mL syringe of air over the filter. If designated as "analyzed damp" herein, solids were left damp with mother liquor. Some samples were additionally dried briefly under a gentle stream of nitrogen gas prior to analysis.

*Vacuum Filtration*: Solids were collected on paper or nylon filters by vacuum filtration and air dried on the filters under reduced pressure briefly before transferring to a vial.

*Reaction Crystallization (RC)*: A mixture of Compound 1 and various coformers were combined in an elevated temperature, acetone slurry, such that the molarity of coformer was 2-fold greater than the API. The solution stirred for a given period of time. Additional crystallization techniques were employed when clear solutions were observed.

*Stability Testing:* Various Compound 1 salts were placed in open vials within a 75% RH chamber (saturated sodium chloride solutiona). The RH chamber was placed in a 40 °C oven for 15-16 days. Samples were analyzed by PLM and XRPD upon the end of the duration.

*Slow Cooling (SC)*: Concentrated solutions of Compound 1 and various coformers were prepared in a variety of solvents at elevated temperatures with stirring. Vials were capped in the heated sample block and the hot plate was turned off, allowing the vials to gradually cool to ambient temperature in the heated vial block. Clear solutions, upon cooling to ambient, were further cooled in the refrigerator (5 to 7 °C) and/or the freezer (∼-20 °C). If no solids were present, additional crystallization techniques were employed.

*Slow Evaporation*: Solutions were prepared in various solvents with agitation and, typically, filtered through a 0.2-µm nylon or PTFE filter. Each solution was allowed to evaporate from a covered vial (such as loosely capped or covered with perforated aluminum foil) at ambient conditions, unless otherwise stated. Solutions were allowed to evaporate to dryness unless designated as partial evaporations (solid present with a small amount of solvent remaining), in which case solids were isolated as described herein.

*Solubility Estimation*: Aliquots of various solvents were added to measured amounts of Compound 1 with agitation (typically sonication) at stated temperatures until complete dissolution was achieved, as judged by visual observation. If dissolution occurred after the addition of the first aliquot, values are reported as ">." If dissolution did not occur, values are reported as "<."

*Aqueous Solubility Estimation*: Aliquots of water were added to measured amounts of various Compound 1 salts with sonication.

*Slurry Experiments*: Saturated solutions of Compound 1 and various coformers were prepared in a variety of solvents and solvent mixtures. Mixtures were stirred at ambient and elevated temperatures for the noted duration of time. Solids were collected by the stated technique and additional crystallization techniques were employed where appropriate.

*Vacuum Oven Desolvation*: Salts of Compound 1 that were determined to be solvates by various analytical methods underwent an attempted desolvation. Samples were placed in a vacuum oven at temperatures ranging from ambient to 80 °C for a given period of time. Samples were analyzed by XRPD and/or TGA for determination of desolvation success.

*Vapor Diffusion:* Concentrated solutions were prepared in various solvents and, typically, filtered through a 0.2-µm nylon or PTFE filter. The filtered solution was dispensed into a small vial, which was then placed inside a larger vial containing anti-solvent. The small vial was left uncapped and the larger vial was capped to allow vapor diffusion to occur. Any solids present were isolated as described herein.

*Vapor Stressing:* Select solids were transferred to a small vial, which was then placed inside a larger vial containing solvent. The small vial was left uncapped and the larger vial was capped to allow vapor stressing to occur at the stated temperature.

Coformer means one or more pharmaceutically acceptable bases and/or pharmaceutically acceptable acids disclosed herein in association with Compound 1. Exemplary coformers as used herein include benzenesulfonic acid, benzoic acid, camphorsulfonic acid, citric acid, ethanesulfonic acid, HBr, lactic acid, malic acid, maleic acid, methanesulfonic acid, oxalic acid, propionic acid, succinic acid, sulfuric acid, tartaric acid, and toluenesulfonic acid.

### Instrumental Techniques

*Differential Scanning Calorimetry (DSC):* DSC was performed using a Mettler-Toledo DSC3+ differential scanning calorimeter. Temperature calibration was performed using adamantane, phenyl salicylate, indium, tin, and zinc. The sample was placed into a hermetically sealed or an open aluminum DSC pan, and the weight was accurately recorded. A weighed aluminum pan configured as the sample pan was placed on the reference side of the cell. The samples were analyzed from -30 to 250 °C at a ramp rate of 10 °C/min. Although thermograms are plotted by reference temperature (x-axis), results are reported according to sample temperatures.

### Dynamic Vapor Sorption (DVS)

a. *VTI:* Automated vapor sorption (VS) data were collected on a VTI SGA-100 Vapor Sorption Analyzer. NaCl and PVP were used as calibration standards. Samples were dried prior to analysis. Sorption and desorption data were collected over a range from 5% to 95% RH at 10% RH increments under a nitrogen purge. The equilibrium criterion used for analysis was less than 0.0100% weight change in 5 minutes with a maximum equilibration time of 3 hours. Data were not corrected for the initial moisture content of the samples.
b. *Intrinsic:* Automated vapor sorption (VS) data were collected on a Surface Measurement System DVS Intrinsic instrument. Samples were not dried prior to analysis. Sorption and desorption data were collected over a range from 5% to 95% RH at 10% RH increments under a nitrogen purge. The equilibrium criterion used for analysis was less than 0.0100% weight change in 5 minutes with a maximum equilibration time of 3 hours. Data were not corrected for the initial moisture content of the samples.

*Hot stage Microscopy (HSM):* Hot stage microscopy was performed using a Linkam hot stage (FTIR 600) mounted on a Leica DM LP microscope equipped with a SPOT Insight^{™} color digital camera. Temperature calibrations were performed using USP melting point standards. Samples were placed on a cover glass, and a second cover glass was placed on top of the sample. As the stage was heated, each sample was visually observed using a 20x objective with crossed polarizers and a first order red compensator. Images were captured using SPOT software (v. 4.5.9).

*Optical Microscopy:* Samples were observed under a Motic or Wolfe optical microscope with crossed polarizers or under a Leica stereomicroscope with a first order red compensator with crossed polarizers.

*pKa and logP Determination:* pKa and logP determination were performed by Pion Inc./Sirius Analytical Instruments Ltd. in East Sussex, United Kingdom.

*Solution Proton Nuclear Magnetic Resonance Spectroscopy (¹HNMR):* The solution ¹H NMR spectra were acquired by Spectral Data Services of Champaign, IL. The samples were prepared by dissolving approximately 5-10 mg of sample in DMSO-d₆. The data acquisition parameters are displayed on the first page of each spectrum in the Data section of this report.

*Thermogravimetric Analysis (TGA):* Thermogravimetric analyses were performed using a Mettler Toledo TGA/DSC3+ analyzer. Temperature calibration was performed using phenyl salicylate, indium, tin, and zinc. The sample was placed in an aluminum pan. The open pan was inserted into the TG furnace. The furnace was heated under nitrogen. Each sample was heated from ambient temperature to 350 °C, at ramp rates of 2, 5, or 10 °C/min. Although thermograms are plotted by reference temperature (x-axis), results are reported according to sample temperatures.

### X-ray Powder Diffraction (XRPD)

a. *Reflection:* XRPD patterns were collected with a PANalytical X'Pert PRO MPD diffractometer using an incident beam of Cu Kα radiation produced using a long, fine-focus source and a nickel filter at room temperature (298 Kelvin). The diffractometer was configured using the symmetric Bragg-Brentano geometry. Prior to the analysis, a silicon specimen (NIST SRM 640e) was analyzed to verify the observed position of the Si 111 peak is consistent with the NIST-certified position. A specimen of the sample was packed in a well. Antiscatter slits (SS) were used to minimize the background generated by air. Soller slits for the incident and diffracted beams were used to minimize broadening from axial divergence. Diffraction patterns were collected using a scanning position-sensitive detector (X'Celerator) located 240 mm from the sample and Data Collector software v. 2.2b. The data acquisition parameters for each pattern are displayed above the image in the Data section of this report including the divergence slit (DS) and the incident-beam SS.
b. *Transmission:* XRPD patterns were collected with a PANalytical X'Pert PRO MPD diffractometer using an incident beam of Cu radiation produced using an Optix long, fine-focus source at room temperature (298 Kelvin). An elliptically graded multilayer mirror was used to focus Cu Kα X-rays through the specimen and onto the detector. Prior to the analysis, a silicon specimen (NIST SRM 640e) was analyzed to verify the observed position of the Si 111 peak is consistent with the NIST-certified position. A specimen of the sample was sandwiched between 3-µm-thick films and analyzed in transmission geometry. A beam-stop, short antiscatter extension, antiscatter knife edge, were used to minimize the background generated by air. Soller slits for the incident and diffracted beams were used to minimize broadening from axial divergence. Diffraction patterns were collected using a scanning position-sensitive detector (X'Celerator) located 240 mm from the specimen and Data Collector software v. 2.2b. The data acquisition parameters for each pattern are displayed above the image in the Data section of this report including the divergence slit (DS) before the mirror.

### XRPD Indexing

Indexing and structure refinement are computational studies. Within the figure referenced for a given indexed XRPD pattern, agreement between the allowed peak positions, marked with bars, and the observed peaks indicates a consistent unit cell determination. Successful indexing of a pattern indicates that the sample is composed primarily of a single crystalline phase unless otherwise stated. Space groups consistent with the assigned extinction symbol, unit cell parameters, and derived quantities are tabulated.

### Examples

### Preparative Example 1: Synthesis of Compound 1

### STEP 1: N-(4-Fluorophenyl)-N-(4-hydroxyphenyl)cyclopropane-1,1-dicarboxamide (4):

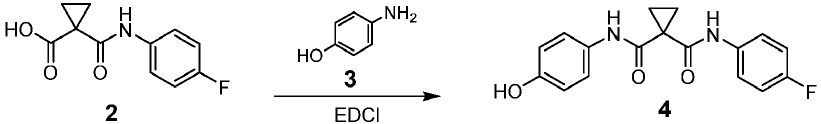

To a solution of Compound 2 (10 g, 44.80 mmol, 1 eq.) and Compound 3 (5.87 g, 53.8 mmol, 1.2 eq.) in dimethyl acetamide (DMA) (60 mL) was added 3-(ethyliminomethyleneamino)-N,N-dimethyl-propan-1-amine hydrochloride (EDCI) (10.31 g, 53.8 mmol, 1.2 eq.). The mixture was stirred vigorously at 20 °C until the reaction was complete. The mixture was poured into aqueous (aq) saturated NaHCO₃ (400 mL) and extracted with EtOAc (4 x 100 mL). The combined organic phases were washed with aqueous saturated NaCl (100 mL), dried over anhydrous (anhyd) Na₂SO₄, and concentrated. Compound 4 (21 g, crude) (50% purity) was obtained. ¹H NMR (400 MHz, DMSO-*d₆*) δ 10.16 (br s, 1H), 9.72 (br s, 1H), 7.61 (dd, 2H), 7.34 (d, 2H), 7.13 (t, 2H) 6.68 (d, 2H), 1.42 (s, 4H); MS (EI) for C₁₇H₁₅FN₂O₃, found 314.9 (MH+).

### STEP 2: Methyl 4-[4-[[1-[(4-fluorophenyl)carbamoyl]cyclopropane-carbonyl]amino]phenoxy]-7-methoxyquinoline-6-carboxylate (6):

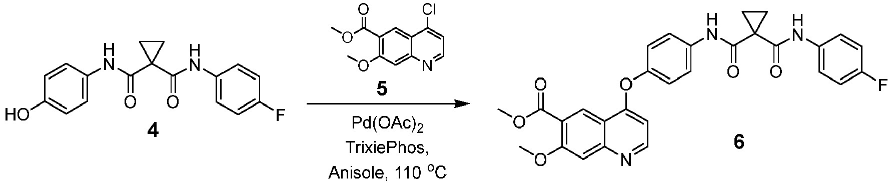

A mixture of Compound 4 (5.99 g, 9.5 mmol, 1.2 eq.), Compound 5 (2 g, 8.0 mmol, 1.0 eq.), Pd(OAc)₂ (89 mg, 397.4 µmol, 0.05 eq.), *rac*-2-(Di-*tert*-butylphosphino)-1,1'-binaphthyl (TrixiePhos, 316.71 mg, 794.7 µmol, 0.1 eq.), and K₃PO₄ (2.53 g, 11.9 mmol, 1.5 eq.) in anisole (50 mL) was stirred at 110 °C for 2 hours (h) under an atmosphere of nitrogen. The mixture was filtered, and the filtrate was concentrated. The residue was purified by flash silica gel chromatography (1:1 petroleum ether:EtOAc to 20:1 EtOAc:MeOH). Compound 6 was obtained (2.6 g, 61.8% yield). ¹H NMR (400 MHz, CDCl₃) δ 9.38 (s, 1H), 8.80 (s, 1H), 8.63 (d, 2H), 7.64 (d, 2H), 7.54-7.41 (m, 3H), 7.18 (d, 2H), 7.09-7.01 (m, 2H), 6.43 (d, 1H), 4.05 (s, 3H), 3.97 (s, 3H), 1.78-1.72 (m, 2H), 1.69-1.63 (m, 2H); MS (EI) for C₂₉H₂₄FN₃O₆, found 530.0 (MH+).

### STEP 3: 4-[4-[[1-[(4-Fluorophenyl)carbamoyl]cyclopropanecarbonyl]amino]phenoxy]-7-methoxyquinoline-6-carboxylic acid (7)

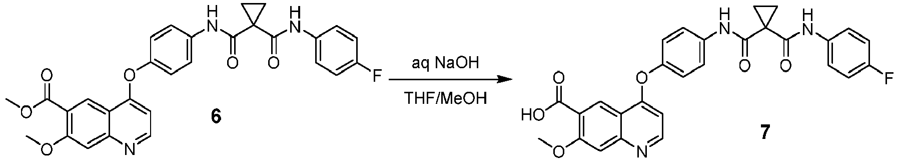

To a solution of Compound 6 (1.8 g, 3.4 mmol, 1 eq.) in tetrahydrofuran (THF) (15 mL) and MeOH (15 mL) was added 2 M aqueous NaOH (7 mL, 4.1 eq.). The mixture was stirred at 6-13 °C for 4 hours. The mixture was adjusted to a pH of approximately 8 with 1 M aqueous HCl and concentrated to remove solvent. Water (50 mL) was added, and the mixture was adjusted to a pH of approximately 6 with 1 M aqueous HCl. The resulting precipitate was filtered, washed with water (2 x 10 mL), and dried under vacuum. Compound 7 was obtained (1.7 g, 97.0% yield). ¹H NMR (400 MHz, DMSO-*d₆*) δ 10.22 (s, 1H), 10.08 (s, 1H), 8.65 (d, 1H), 8.48 (s, 1H), 7.77 (d, 2H), 7.64 (dd, 2H) 7.47 (s, 1H), 7.25 (d, 2H), 7.15 (t, 2H), 6.45 (d, 1H), 3.96 (s, 3H), 1.47 (s, 4H); MS (EI) for C₂₈H₂₂FN₃O₆, found 516.1 (MH+).

### STEP 4: 1-N'-(4-Fluorophenyl)-1-N-[4-[7-methoxy-6-(methylcarbamoyl)quinolin-4-yl]oxyphenyl]cyclopropane-1,1-dicarboxamide (1)

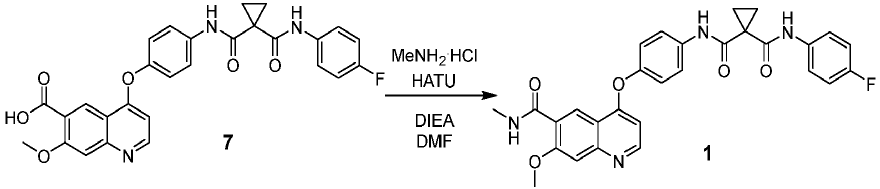

A solution of Compound 7 (300 mg, 582.0 µmol, 1 eq.), HATU (332 mg, 873.2 µmol, 1.5 eq.), and DIEA (301 mg, 2.3 mmol, 406 µL, 4 eq.) in DMF (10 mL) was stirred at 6-10 °C for 1 hour. Methanamine hydrochloride (79 mg, 1.2 mmol, 2.0 eq.) was added, and the mixture was stirred at 6-10 °C for 17 hours. The mixture was filtered, and the resulting filtrate purified by prep HPLC (Column: Waters Xbridge 150 mm*25 mm*5 µm, gradient: 33-63% of acetonitrile in 10 mM aqueous NH₄HCO₃, flow rate: 25 mL/min). Compound **1** was obtained (105.4 mg, 34.3% yield). ¹H NMR (400 MHz, DMSO-*d₆*) δ 10.20 (s, 1H), 10.06 (s, 1H), 8.65 (d, 1H), 8.61 (s, 1H), 8.42-8.33 (m, 1H), 7.77 (d, 2H), 7.68-7.61 (m, 2H), 7.51 (s, 1H), 7.25 (d, 2H), 7.19-7.11 (m, 2H), 6.46 (d, 1H), 4.02 (s, 3H), 2.84 (d, 3H) 1.47 (s, 4H); MS (EI) for C₂₉H₂₅FN₄O₅, found 529.1 (MH+).

### Example 1: Preparation of Compound 1 Besylate Form A

One molar equivalent of benzenesulfonic acid in hot acetone was added to Compound 1. The resulting slurry was then stirred at ~50 °C for 3 days. Solids were collected by positive pressure filtration.

### Example 2: Preparation of Compound 1 Besylate Form B

Compound 1 Besylate Form A was placed in a vacuum at 80 °C for 1 day to provide a mixture of Compound 1 Besylate Form A and Compound 1 Besylate Form B.

### Example 3: Preparation of Compound 1 Benzoate Form A

To one molar equivalent of benzoic acid and Compound 1 was added hot methanol. The resulting solution was stirred for 20 minutes at ~60 °C. The solution was then crash cooled to -20 °C and let stand for 1 day. The solids were filtered by vacuum filtration.

### Example 4: Preparation of Compound 1 Camsylate Form A

One molar equivalent of camphorsulfonic acid in hot acetone was added to Compound 1. The resulting solution was then stirred at ~50 °C for 3 days. Solids were collected by positive pressure filtration.

### Example 5: Preparation of Compound 1 Camsylate Form B

Compound 1 Camsylate Form A was placed in a vacuum at 80 °C for 1 day to provide Compound 1 Camsylate Form B.

### Example 6: Preparation of Compound 1 Citrate Form A

One molar equivalent of citric acid in acetone was added to Compound 1. The resulting solution was then stirred at ~50 °C for 4 days. The mixture was then slow cooled to room temperature over 2 hours, and 3 mL acetone was added and the solution was stirred for an additional day at room temperature. Solids were collected by positive pressure filtration.

### Example 7: Preparation of Compound 1 Citrate Form B

Two molar equivalents of citric acid in acetone was added to Compound 1. The resulting solution was then stirred at ~50 °C for 6 days. Solids were collected by positive pressure filtration of the hot solution.

### Example 8: Preparation of Compound 1 Esylate Form A

One molar equivalent of ethanesulfonic acid in hot acetone was added to Compound 1. The resulting solution was then stirred at ~50 °C for 3 days. Solids were collected by positive pressure filtration.

### Example 9: Preparation of Compound 1 Esylate Form B

Compound 1 Esylate Form A was placed in a vacuum at 80 °C for 1 day to provide Compound 1 Esylate Form B.

### Example 10: Preparation of Compound 1 HBr Form A

One molar equivalent of hydrobromic acid was added to a slurry of Compound 1 in hot acetone. The resulting solution was then stirred at ~50 °C for 3 days. Solids were collected by positive pressure filtration.

### Example 11: Preparation of Compound 1 HBr Form B

One molar equivalent of hydrobromic acid was added to a slurry of Compound 1 in hot MEK. The resulting solution was then stirred at ~55 °C for 1 day. Solution was then slow cooled.

### Example 12: Preparation of Compound 1 Lactate Form A

One molar equivalent of lactic acid was added to a slurry of Compound 1 in hot acetone. The resulting solution was then stirred at ~50 °C for 3 days. Solids were collected by positive pressure filtration.

### Example 13A: Preparation of Compound 1 Malate Form A

One molar equivalent of malic acid in acetone was added to Compound 1. The resulting solution was then stirred at ~50 °C for 4 days, slow cooled to room temperature over 2 hours, diluted with 3 mL acetone, and stirred at room temperature for 1 day. Solids were collected by positive pressure filtration.

### Example 13B: Preparation of Compound 1 Malate Form A

Two molar equivalents of malic acid in acetone was added to Compound 1. The resulting solution was then stirred at ~50 °C for 6 days. Solids were collected by positive pressure filtration of the hot solution.

### Example 14A: Preparation of Compound 1 Maleate Form A

One molar equivalent of aqueous maleic acid was added to a slurry of Compound 1 in MEK, as to provide a resulting solution of 1:7 water/MEK. The resulting solution was then stirred at ~55 °C for 5 minutes to 1 day, slow cooled, and the solvent partially evaporated (FE).

### Example 14B: Preparation of Compound 1 Maleate Form A

Two molar equivalents of maleic acid in hot acetone were added to Compound 1. The resulting solution was then stirred at ~50 °C for 1 day, and then the solution was fast cooled to room temperature. Solids were collected by positive pressure filtration of the hot solution.

### Example 15: Preparation of Compound 1 Maleate Form B

(scale up) Two molar equivalents of maleic acid and Compound 1 were slurried in hot acetone at ~50 °C, with seed crystals 2 days. Solids were collected by vacuum filtration to provide a mixture of Compound 1 Maleate Forms A and B.

### Example 16: Preparation of Compound 1 Mesylate Form A

One molar equivalent of methanesulfonic acid was added to a slurry of Compound 1 in THF at room temperature. The slurry was stirred at room temperature for 3 days, and the solids were collected by positive pressure filtration.

### Example 17: Preparation of Compound 1 Mesylate Form B

One molar equivalent of methanesulfonic acid was added to a slurry of Compound 1 in chloroform. The slurry was then stirred at ~50 °C for 3 days. The solution was then slow cooled to room temperature. Heptane was added so that the final solution was about 8:1 chloroform to heptane, and the solution was stirred for 5 hours. The solids were collected by positive pressure filtration.

### Example 18: Preparation of Compound 1 Mesylate Form C

One molar equivalent of methanesulfonic acid was added to a solution of Compound 1 in methanol at ~60 °C. The solution was then crash cooled to -20 °C, and let stand for 2 days. The solvent was partially evaporated and MTBE was added so that the final solution was about 2:5 methanol to MTBE, and the solution was stirred for 5 hours. The solids were collected by positive pressure filtration.

### Example 19: Preparation of Compound 1 Oxylate Form A

One molar equivalent of oxalic acid in hot acetone was added to Compound 1. The resulting solution was then stirred at ~50 °C for 3 days. Solids were collected by positive pressure filtration.

### Example 20: Preparation of Compound 1 Oxylate Form B

One molar equivalent of aqueous oxalic acid at 55 °C was added to a slurry of Compound 1 in MEK, as to provide a resulting solution of 1:7 water/MEK. The resulting solution was then stirred at ~55 °C for 1 day, slow cooled, and the solvent partially evaporated (FE).

### Example 21: Preparation of Compound 1 Oxylate Form C

One molar equivalent of oxalic acid in TFE at 55 °C was added to Compound 1. The resulting solution was then slow cooled to room temperature for 1 day, placed in the refrigerator at 2-8 °C for 2 hours, and then placed in the freezer at -20 °C for 6 days. The solution was then crash precipitated with 1 volume of MTBE, stirred at room temperature for 1 day, and filtered by positive pressure filtration.

### Example 22: Preparation of Compound 1 Propionate Form A

One molar equivalent of propionic acid was added to Compound 1 in hot acetone. The resulting solution was then stirred at ~50 °C for 3 days. Solids were collected by positive pressure filtration.

### Example 23A: Preparation of Compound 1 Succinate Form A

One molar equivalent of succinic acid was added to Compound 1 in acetone. The resulting solution was then stirred at ~50 °C for 4 days, then slow cooled to room temperature and stirred for another day. Solids were collected by positive pressure filtration.

### Example 23B: Preparation of Compound 1 Succinate Form A

Two molar equivalents of succinic acid was added to Compound 1 in acetone. The resulting solution was then stirred at ~50 °C for 6 days. Solids were collected by positive pressure filtration.

### Example 24: Preparation of Compound 1 Sulfate Form A

One molar equivalent of sulfuric acid was added to a slurry of Compound 1 in THF. The resulting solution was then stirred at room temperature for 3 days. Solids were collected by positive pressure filtration.

### Example 25: Preparation of Compound 1 Sulfate Form B

One molar equivalent of sulfuric acid was added to Compound 1 in methanol at ~60 °C. The resulting solution was then crash cooled to -20 °C and let stand for 2 days. The solvent was then evaporated (FE) to provide the title form.

### Example 26: Preparation of Compound 1 Sulfate Form C

One molar equivalent of sulfuric acid was added to Compound 1 in chloroform, and the resulting solution was stirred at ~50 °C for 3 days. The resulting solution was then slow cooled to room temperature and let stand for 1 day. Solids were collected by positive pressure filtration.

### Example 27: Preparation of Compound 1 Tartrate Form A

One molar equivalent of L-tartaric acid was added to Compound 1 in THF, and the resulting solution was stirred at room temperature for 3 days. Solids were collected by positive pressure filtration.

### Example 28: Preparation of Compound 1 Hemitartrate Form B

One molar equivalent of L-tartaric acid was added to Compound 1 in acetone, and the resulting solution was stirred at ~50 °C for 34days. The resulting solution was then slow cooled to room temperature and let stand for 1 day. Solids were collected by positive pressure filtration.

### Example 29: Preparation of Compound 1 Tosylate Form A

One molar equivalent of p-toluenesulfonic acid was added to Compound 1 in acetone, and the resulting solution was stirred at ~50 °C for 3 days. Solids were collected by positive pressure filtration.

### Example 30: Preparation of Compound 1 Tosylate Form B

(scale up) One molar equivalent of p-toluenesulfonic acid in hot acetone was added to Compound 1 in acetone at ~50 °C. Seed crystals were then added, and the resulting solution was stirred at ~50 °C for 2 days.

## Claims

1. A crystalline salt form of Compound 1 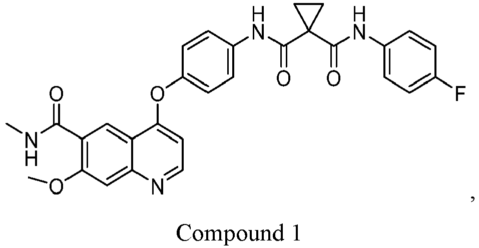 selected from the group consisting of Compound 1 Besylate Form A, Compound 1 Besylate Form B, Compound 1 Benzoate Form A, Compound 1 Camsylate Form A, Compound 1 Camsylate Form B, Compound 1 Citrate Form A, Compound 1 Citrate Form B, Compound 1 Esylate Form A, Compound 1 Esylate Form B, Compound 1 HBr Form A, Compound 1 HBr Form B, Compound 1 Lactate Form A, Compound 1 Malate Form A, Compound 1 Maleate Form A, Compound 1 Maleate Form B, Compound 1 Mesylate Form A, Compound 1 Mesylate Form B, Compound 1 Mesylate Form C, Compound 1 Oxalate Form A, Compound 1 Oxalate Form B, Compound 1 Oxalate Form C, Compound 1 Propionate Form A, Compound 1 Succinate Form A, Compound 1 Sulfate Form A, Compound 1 Sulfate Form B, Compound 1 Sulfate Form C, Compound 1 Tartrate Form A, Compound 1 Hemitartrate Form B, Compound 1 Tartrate Form C, Compound 1 Tosylate Form A, and Compound 1 Tosylate Form B, wherein:
Compound 1 Besylate Form A is **characterized by** one or more peaks in an XRPD pattern on a 2 Theta scale, wherein the one or more peaks is selected from 3.78, 7.56, 10.08, 10.42, 11.70, 11.78, 12.00, 12.71, 13.20, 13.92, 14.06, 14.44, 14.93, 15.18, 15.96, 16.21, 16.40, 16.73, 16.86, 17.38, 18.32, 19.07, 20.07, 20.26, 20.51, 20.94, 21.45, 21.60, 22.30, 22.90, 23.05, 23.28, 23.71, 24.28, 24.97, 25.30, 25.69, 26.33, 26.71, 27.29, 27.65, 28.07, 29.22, and 29.50,
Compound 1 Besylate Form B is **characterized by** the XRPD pattern provided in Figure 4;
Compound 1 Benzoate Form A is **characterized by** the XRPD pattern provided in Figure 5;
Compound 1 Camsylate Form A is **characterized by** one or more peaks in an XRPD pattern on a 2 Theta scale, wherein the one or more peaks is selected from 5.55, 7.90, 8.25, 8.38, 9.55, 9.87, 10.26, 11.12, 11.34, 12.50, 12.94, 13.40, 13.65, 14.79, 15.83, 16.16, 16.41, 16.54, 16.82, 17.10, 17.69, 18.02, 18.22, 18.47, 18.91, 19.10, 19.47, 20.04, 20.87, 21.05, 22.16, 22.36, 22.93, 23.71, 24.55, 25.33, 26.02, 26.40, 27.09, 27.33, 27.97, 28.32, 28.68, and 28.98;
Compound 1 Camsylate Form B is **characterized by** one or more peaks in an XRPD pattern on a 2 Theta scale, wherein the one or more peaks is selected from 5.25, 8.32, 8.51, 8.76, 9.05, 9.58, 9.88, 10.00, 10.52, 10.80, 11.06, 11.96, 12.24, 12.58, 13.98, 15.62, 15.81, 16.15, 16.70, 17.12, 17.42, 17.71, 18.41, 19.37, 19.55, 20.63, 21.13, 21.56, 21.89, 22.50, 23.04, 24.43, 26.08, 27.07, 27.97, 28.22, and 28.73;
Compound 1 Citrate Form A is **characterized by** the XRPD pattern provided in Figure 10;
Compound 1 Citrate Form B is **characterized by** one or more peaks in an XRPD pattern on a 2 Theta scale, wherein the one or more peaks is selected from 4.58, 5.95, 7.61, 8.88, 9.19, 9.94, 11.93, 13.26, 13.79, 14.29, 14.68, 15.26, 15.95, 16.64, 17.11, 17.87, 18.21, 18.43, 18.90, 19.36, 19.67, 20.14, 20.57, 21.47, 22.30, 22.64, 23.06, 23.80, 24.25, 24.55, 24.81, 25.10, 25.45, 25.54, 25.85, 26.35, 26.51, 26.83, 27.16, 27.57, 27.85, 28.08, and 28.47;
Compound 1 Esylate Form A is **characterized by** one or more peaks in an XRPD pattern on a 2 Theta scale, wherein the one or more peaks is selected from 3.97, 7.95, 9.89, 10.62, 10.92, 11.03, 11.34, 11.77, 12.03, 12.53, 12.71, 13.27, 14.00, 14.70, 15.08, 15.94, 16.17, 16.92, 17.01, 17.19, 17.35, 17.61, 18.15, 18.76, 19.03, 19.66, 19.89, 20.26, 20.65, 20.78, 21.44, 22.08, 23.14, 23.27, 23.99, 24.32, 25.43, 25.66, 25.85, 26.52, 26.87, 27.61, 27.96, 28.18, 28.40, and 28.91;
Compound 1 Esylate Form B is **characterized by** the XRPD pattern provided in Figure 18;
Compound 1 HBr Form A is **characterized by** the XRPD pattern provided in Figure 19;
Compound 1 HBr Form B is **characterized by** one or more peaks in an XRPD pattern on a 2 Theta scale, wherein the one or more peaks is selected from 5.26, 8.29, 9.75, 9.91, 10.56, 10.72, 12.25, 12.50, 12.75, 13.31, 13.54, 14.11, 14.72, 14.89, 15.87, 16.29, 16.50, 17.36, 17.78, 18.09, 18.44, 18.75, 19.12, 19.24, 19.56, 19.92, 20.40, 20.55, 20.69, 21.18, 22.09, 22.64, 23.24, 24.56, 25.80, 26.52, 27.23, 27.43, 28.13, 28.46, 28.76, 29.08, 29.66, and 30.07;
Compound 1 Lactate Form A is **characterized by** the XRPD pattern provided in Figure 21;
Compound 1 Malate Form A is **characterized by** the XRPD pattern provided in Figure 22;
Compound 1 Maleate Form A is **characterized by** one or more peaks in an XRPD pattern on a 2 Theta scale, wherein the one or more peaks is selected from 7.58, 8.34, 10.92, 12.38, 12.87, 14.17, 15.13, 15.78, 16.43, 16.91, 17.51, 18.09, 18.71, 20.30, 21.60, 21.99, 22.30, 22.49, 23.32, 23.56, 23.92, 24.79, 26.04, 26.41, 26.92, 27.62, 28.18, 28.54, and 29.01;
Compound 1 Maleate Form B is **characterized by** the XRPD pattern provided in Figure 28;
Compound 1 Mesylate Form A is **characterized by** one or more peaks in an XRPD pattern on a 2 Theta scale, wherein the one or more peaks is selected from 5.06, 6.65, 7.66, 8.35, 10.16, 10.72, 11.98, 12.62, 13.32, 14.41, 14.79, 15.42, 15.72, 15.89, 16.23, 16.77, 17.20, 17.59, 18.08, 18.40, 18.81, 19.33, 19.53, 19.71, 20.14, 20.38, 20.73, 21.05, 21.56, 21.83, 22.16, 23.14, 24.06, 24.54, 24.77, 25.75, 26.82, 27.71, 27.94, 28.71, 29.27, and 29.85;
Compound 1 Mesylate Form B is **characterized by** one or more peaks in an XRPD pattern on a 2 Theta scale, wherein the one or more peaks is selected from 5.14, 6.62, 7.72, 7.89, 10.33, 10.73, 12.74, 13.31, 13.88, 14.23, 14.61, 15.21, 15.52, 15.87, 16.17, 17.18, 17.41, 17.88, 18.15, 18.46, 18.73, 19.13, 19.54, 20.15, 20.53, 20.75, 20.98, 21.27, 21.60, 22.03, 22.28, 23.07, 23.32, 24.16, 24.42, 24.87, 25.13, 25.64, 26.24, 26.95, 27.17, 27.49, 27.77, 28.42, and 29.54;
Compound 1 Mesylate Form C is **characterized by** one or more peaks in an XRPD pattern on a 2 Theta scale, wherein the one or more peaks is selected from 7.55, 9.53, 10.25, 11.04, 11.49, 11.65, 12.24, 12.51, 13.88, 14.44, 15.36, 15.66, 16.29, 16.58, 17.16, 17.54, 19.04, 19.20, 19.79, 20.54, 21.12, 21.24, 21.62, 22.22, 23.15, 23.57, 23.99, 24.61, 24.77, 25.22, 25.61, 25.99, 26.60, 26.89, 27.29, 27.91, 29.03, and 29.29;
Compound 1 Oxalate Form A is **characterized by** one or more peaks in an XRPD pattern on a 2 Theta scale, wherein the one or more peaks is selected from 4.90, 5.25, 6.20, 8.05, 9.87, 10.54, 12.29, 14.79, 15.35, 15.84, 16.49, 16.89, 17.55, 18.27, 18.67, 19.12, 19.40, 19.81, 20.26, 20.50, 21.06, 21.74, 22.17, 22.93, 23.26, 23.50, 23.81, 24.66, 25.06, 25.95, 26.90, 27.82, and 28.24;
Compound 1 Oxalate Form B is **characterized by** one or more peaks in an XRPD pattern on a 2 Theta scale, wherein the one or more peaks is selected from 4.20, 7.59, 8.02, 8.42, 10.53, 12.36, 12.66, 13.09, 13.35, 13.88, 14.15, 14.46, 15.06, 15.23, 17.42, 17.75, 18.28, 19.07, 19.52, 20.33, 21.18, 21.43, 21.74, 22.08, 22.59, 23.53, 24.21, 24.52, 24.71, 25.24, 25.85, 26.24, 26.55, 26.90, 27.43, 28.56, 29.30, and 29.51;
Compound 1 Oxalate Form C is **characterized by** one or more peaks in an XRPD pattern on a 2 Theta scale, wherein the one or more peaks is selected from 4.13, 7.70, 10.29, 10.59, 13.09, 13.70, 14.13, 15.33, 15.87, 16.57, 17.19, 17.50, 17.83, 18.15, 18.61, 19.24, 19.42, 20.11, 20.34, 20.64, 21.07, 21.47, 21.59, 21.91, 22.56, 22.92, 23.37, 23.87, 24.35, 25.35, 25.91, 26.47, 26.68, 27.74, 28.47, and 29.14;
Compound 1 Propionate Form A is **characterized by** the XRPD pattern provided in Figure 38;
Compound 1 Succinate Form A is **characterized by** the XRPD pattern provided in Figure 39;
Compound 1 Sulfate Form A is **characterized by** one or more peaks in an XRPD pattern on a 2 Theta scale, wherein the one or more peaks is selected from 4.74, 5.85, 9.47, 10.04, 11.74, 11.95, 13.77, 14.99, 15.42, 15.71, 16.48, 17.36, 17.66, 17.92, 18.59, 18.95, 19.36, 19.74, 20.22, 20.77, 21.38, 21.86, 22.74, 23.30, 23.70, 24.33, 25.07, 25.32, 25.67, 26.23, and 27.00;
Compound 1 Sulfate Form B is **characterized by** one or more peaks in an XRPD pattern on a 2 Theta scale, wherein the one or more peaks is selected from 4.27, 8.57, 9.32, 10.95, 11.29, 12.07, 12.87, 13.25, 13.82, 14.13, 14.67, 16.94, 17.21, 17.64, 19.99, 20.26, 21.03, 21.44, 22.00, 22.71, 23.85, 25.20, and 26.02;
Compound 1 Sulfate Form C is **characterized by** one or more peaks in an XRPD pattern on a 2 Theta scale, wherein the one or more peaks is selected from 3.19, 5.35, 9.04, 9.62, 10.73, 11.31, 11.60, 12.27, 12.67, 13.76, 14.31, 14.65, 15.21, 15.63, 16.11, 16.33, 16.63, 17.11, 18.32, 18.75, 18.99, 19.36, 19.47, 20.16, 20.55, 21.01, 21.52, 21.62, 21.89, 22.19, 22.53, 23.19, 23.79, 24.23, 24.81, 25.07, 25.41, 26.06, 26.73, 27.58, 28.16, 28.63, 28.90, 29.22, 29.47, and 29.73;
Compound 1 Tartrate Form A is **characterized by** the XRPD pattern provided in Figure 43;
Compound 1 Hemitartrate Form B is **characterized by** one or more peaks in an XRPD pattern on a 2 Theta scale, wherein the one or more peaks is selected from 5.17, 5.66, 7.22, 9.27, 10.10, 10.40, 11.37, 11.55, 12.77, 13.94, 14.51, 15.60, 16.06, 16.48, 17.09, 17.69, 17.90, 18.27, 18.94, 19.15, 19.59, 19.81, 20.06, 20.32, 21.23, 21.83, 22.34, 22.84, 23.53, 24.26, 24.70, 25.26, 25.80, 26.78, 27.29, 27.90, 29.04, and 29.45;
Compound 1 Tartrate Form C is **characterized by** the XRPD pattern provided in Figure 69;
Compound 1 Tosylate Form A is **characterized by** one or more peaks in an XRPD pattern on a 2 Theta scale, wherein the one or more peaks is selected from 5.81, 7.05, 8.52, 9.74, 10.40, 10.99, 11.26, 11.66, 12.58, 12.81, 14.28, 14.62, 15.30, 15.80, 16.03, 16.34, 17.12, 17.55, 18.03, 18.94, 19.26, 19.67, 20.31, 20.65, 21.02, 21.43, 22.11, 22.44, 22.66, 22.94, 23.07, 23.54, 23.79, 24.55, 24.88, 25.42, 25.83, 26.10, 26.53, 27.70, 28.11, 28.62, 29.18, 29.40, 29.66, and 29.83; and
Compound 1 Tosylate Form B is **characterized by** one or more peaks in an XRPD pattern on a 2 Theta scale, wherein the one or more peaks is selected from 4.61, 8.69, 9.25, 10.68, 12.44, 12.69, 13.76, 14.42, 14.81, 15.47, 16.08, 16.57, 17.45, 17.76, 18.44, 18.83, 19.01, 19.35, 20.42, 20.84, 21.15, 21.69, 22.09, 22.86, 23.12, 24.08, 24.53, 24.86, 25.22, 25.91, 26.33, 26.82, 27.63, 28.61, 29.11, and 29.77.

2. The crystalline salt form of claim 1, wherein:
Compound 1 Besylate Form A is **characterized by** one or more peaks in an XRPD pattern on a 2 Theta scale, wherein the one or more peaks is selected from 3.78, 7.56, 10.42, 20.51, 20.94, 21.60, and 25.30;
Compound 1 Camsylate Form A is **characterized by** one or more peaks in an XRPD pattern on a 2 Theta scale, wherein the one or more peaks is selected from 5.55, 9.87, 10.26, 16.16, 16.82, 20.87, 22.36, and 23.71;
Compound 1 Camsylate Form B is **characterized by** one or more peaks in an XRPD pattern on a 2 Theta scale, wherein the one or more peaks is selected from 5.25, 10.52, 16.15, 21.13, 21.56, and 21.89;
Compound 1 Citrate Form B is **characterized by** one or more peaks in an XRPD pattern on a 2 Theta scale, wherein the one or more peaks is selected from 4.58, 5.95, 8.88, 11.93, 13.79, 14.29, 16.64, 18.90, 21.47, 23.80, 24.25, and 25.85;
Compound 1 Esylate Form A is **characterized by** one or more peaks in an XRPD pattern on a 2 Theta scale, wherein the one or more peaks is selected from 3.97, 7.95, 9.89, 10.62, 13.27, 15.08, 16.17, 16.92, 17.01, 20.65, 20.78, 21.44, 22.08, 23.99, 25.43, and 26.52;
Compound 1 HBr Form B is **characterized by** one or more peaks in an XRPD pattern on a 2 Theta scale, wherein the one or more peaks is selected from 5.26, 9.91, 10.72, 20.55, 20.69, 21.18, 22.09, 23.24, 24.56, and 25.80;
Compound 1 Maleate Form A is **characterized by** one or more peaks in an XRPD pattern on a 2 Theta scale, wherein the one or more peaks is selected from 10.92, 15.13, 15.78, 16.43, 18.09, 18.71, 20.30, 21.99, 22.30, 22.49, 23.32, 23.56, 24.79, 26.41, 26.92, and 29.01;
Compound 1 Mesylate Form A is **characterized by** one or more peaks in an XRPD pattern on a 2 Theta scale, wherein the one or more peaks is selected from 5.06, 7.66, 14.79, 15.42, 16.23, 17.20, 17.59, 18.40, 19.33, 19.53, 20.38, 20.73, 21.83, 22.16, 23.14, 24.06, and 24.77;
Compound 1 Mesylate Form B is **characterized by** one or more peaks in an XRPD pattern on a 2 Theta scale, wherein the one or more peaks is selected from 14.61, 15.21, 16.17, 17.18, 17.41, 18.46, 19.13, 19.54, 20.15, 20.53, 20.75, 20.98, 21.27, 21.60, 22.03, 23.32, 24.16, 24.42, 24.87, and 25.13;
Compound 1 Mesylate Form C is **characterized by** one or more peaks in an XRPD pattern on a 2 Theta scale, wherein the one or more peaks is selected from 7.55, 9.53, 11.49, 12.51, 14.44, 19.04, 20.54, 21.12, 21.24, and 23.15;
Compound 1 Oxalate Form A is **characterized by** one or more peaks in an XRPD pattern on a 2 Theta scale, wherein the one or more peaks is selected from 4.90, 5.25, 9.87, 10.54, 12.29, 15.35, 18.67, 19.40, 19.81, 20.26, 22.93, and 24.66;
Compound 1 Oxalate Form B is **characterized by** one or more peaks in an XRPD pattern on a 2 Theta scale, wherein the one or more peaks is selected from 4.20, 7.59, 10.53, 15.06, 15.23, 19.52, 21.18, 21.43, 22.59, 24.21, and 25.85;
Compound 1 Oxalate Form C is **characterized by** one or more peaks in an XRPD pattern on a 2 Theta scale, wherein the one or more peaks is selected from 7.70, 10.29, 10.59, 13.09, 13.70, 15.33, 17.83, 18.15, 20.11, 21.07, 21.91, and 22.56;
Compound 1 Sulfate Form A is **characterized by** one or more peaks in an XRPD pattern on a 2 Theta scale, wherein the one or more peaks is selected from 4.74, 5.85, 9.47, 10.04, 11.74, 11.95, 16.48, 20.22, and 22.74;
Compound 1 Sulfate Form B is **characterized by** one or more peaks in an XRPD pattern on a 2 Theta scale, wherein the one or more peaks is selected from 4.27, 8.57, 9.32, 16.94, 19.99, 21.03, 21.44, 22.00, and 26.02;
Compound 1 Sulfate Form C is **characterized by** one or more peaks in an XRPD pattern on a 2 Theta scale, wherein the one or more peaks is selected from 3.19, 5.35, 9.62, 12.27, 16.33, 17.11, 18.75, 18.99, 19.36, 19.47, 20.55, 21.01, 21.52, 21.62, 21.89, 22.19, 23.79, 25.07, and 26.06;
Compound 1 Hemitartrate Form B is **characterized by** one or more peaks in an XRPD pattern on a 2 Theta scale, wherein the one or more peaks is selected from 5.17, 5.66, 10.10, 10.40, 11.37, 11.55, 14.51, 17.09, 21.23, 21.83, 23.53, 24.26, 25.26, and 26.78;
Compound 1 Tosylate Form A is **characterized by** one or more peaks in an XRPD pattern on a 2 Theta scale, wherein the one or more peaks is selected from 8.52, 9.74, 14.28, 16.03, 17.55, 18.94, 19.26, 20.31, 21.02, 21.43, 22.11, 22.44, 22.66, 23.07, 24.55, and 27.70; and
Compound 1 Tosylate Form B is **characterized by** one or more peaks in an XRPD pattern on a 2 Theta scale, wherein the one or more peaks is selected from 4.61, 9.25, 12.44, 15.47, 17.76, 19.01, 19.35, 25.91, 26.33, and 27.63.

3. The crystalline salt form of claim 1 or 2, wherein:
Compound 1 Besylate Form A is **characterized by** all of the following peaks in an XRPD pattern on a 2 Theta scale, selected from 3.78, 7.56, 10.42, 20.51, 20.94, 21.60, and 25.30;
Compound 1 Camsylate Form A is **characterized by** all of the following peaks in an XRPD pattern on a 2 Theta scale, selected from 5.55, 9.87, 10.26, 16.16, 16.82, 20.87, 22.36, and 23.71;
Compound 1 Camsylate Form B is **characterized by** all of the following peaks in an XRPD pattern on a 2 Theta scale, selected from 5.25, 10.52, 16.15, 21.13, 21.56, and 21.89;
Compound 1 Citrate Form B is **characterized by** all of the following peaks in an XRPD pattern on a 2 Theta scale, selected from 4.58, 5.95, 8.88, 11.93, 13.79, 14.29, 16.64, 18.90, 21.47, 23.80, 24.25, and 25.85;
Compound 1 Esylate Form A is **characterized by** all of the following peaks in an XRPD pattern on a 2 Theta scale, selected from 3.97, 7.95, 9.89, 10.62, 13.27, 15.08, 16.17, 16.92, 17.01, 20.65, 20.78, 21.44, 22.08, 23.99, 25.43, and 26.52;
Compound 1 HBr Form B is **characterized by** all of the following peaks in an XRPD pattern on a 2 Theta scale, selected from 5.26, 9.91, 10.72, 20.55, 20.69, 21.18, 22.09, 23.24, 24.56, and 25.80;
Compound 1 Maleate Form A is **characterized by** all of the following peaks in an XRPD pattern on a 2 Theta scale, selected from 10.92, 15.13, 15.78, 16.43, 18.09, 18.71, 20.30, 21.99, 22.30, 22.49, 23.32, 23.56, 24.79, 26.41, 26.92, and 29.01;
Compound 1 Mesylate Form A is **characterized by** all of the following peaks in an XRPD pattern on a 2 Theta scale, selected from 5.06, 7.66, 14.79, 15.42, 16.23, 17.20, 17.59, 18.40, 19.33, 19.53, 20.38, 20.73, 21.83, 22.16, 23.14, 24.06, and 24.77;
Compound 1 Mesylate Form B is **characterized by** all of the following peaks in an XRPD pattern on a 2 Theta scale, selected from 14.61, 15.21, 16.17, 17.18, 17.41, 18.46, 19.13, 19.54, 20.15, 20.53, 20.75, 20.98, 21.27, 21.60, 22.03, 23.32, 24.16, 24.42, 24.87, and 25.13;
Compound 1 Mesylate Form C is **characterized by** all of the following peaks in an XRPD pattern on a 2 Theta scale, selected from 7.55, 9.53, 11.49, 12.51, 14.44, 19.04, 20.54, 21.12, 21.24, and 23.15;
Compound 1 Oxalate Form A is **characterized by** all of the following peaks in an XRPD pattern on a 2 Theta scale, selected from 4.90, 5.25, 9.87, 10.54, 12.29, 15.35, 18.67, 19.40, 19.81, 20.26, 22.93, and 24.66;
Compound 1 Oxalate Form B is **characterized by** all of the following peaks in an XRPD pattern on a 2 Theta scale, selected from 4.20, 7.59, 10.53, 15.06, 15.23, 19.52, 21.18, 21.43, 22.59, 24.21, and 25.85;
Compound 1 Oxalate Form C is **characterized by** all of the following peaks in an XRPD pattern on a 2 Theta scale, selected from 7.70, 10.29, 10.59, 13.09, 13.70, 15.33, 17.83, 18.15, 20.11, 21.07, 21.91, and 22.56;
Compound 1 Sulfate Form A is **characterized by** all of the following peaks in an XRPD pattern on a 2 Theta scale, selected from 4.74, 5.85, 9.47, 10.04, 11.74, 11.95, 16.48, 20.22, and 22.74;
Compound 1 Sulfate Form B is **characterized by** all of the following peaks in an XRPD pattern on a 2 Theta scale, selected from 4.27, 8.57, 9.32, 16.94, 19.99, 21.03, 21.44, 22.00, and 26.02;
Compound 1 Sulfate Form C is **characterized by** all of the following peaks in an XRPD pattern on a 2 Theta scale, selected from 3.19, 5.35, 9.62, 12.27, 16.33, 17.11, 18.75, 18.99, 19.36, 19.47, 20.55, 21.01, 21.52, 21.62, 21.89, 22.19, 23.79, 25.07, and 26.06;
Compound 1 Hemitartrate Form B is **characterized by** all of the following peaks in an XRPD pattern on a 2 Theta scale, selected from 5.17, 5.66, 10.10, 10.40, 11.37, 11.55, 14.51, 17.09, 21.23, 21.83, 23.53, 24.26, 25.26, and 26.78;
Compound 1 Tosylate Form A is **characterized by** all of the following peaks in an XRPD pattern on a 2 Theta scale, selected from 8.52, 9.74, 14.28, 16.03, 17.55, 18.94, 19.26, 20.31, 21.02, 21.43, 22.11, 22.44, 22.66, 23.07, 24.55, and 27.70; and
Compound 1 Tosylate Form B is **characterized by** all of the following peaks in an XRPD pattern on a 2 Theta scale, selected from 4.61, 9.25, 12.44, 15.47, 17.76, 19.01, 19.35, 25.91, 26.33, and 27.63.

4. The crystalline salt form of any one of claims 1-3, wherein:
Compound 1 Besylate Form A is **characterized by** all of the following peaks in an XRPD pattern on a 2 Theta scale, selected from 3.78, 7.56, 10.08, 10.42, 11.70, 11.78, 12.00, 12.71, 13.20, 13.92, 14.06, 14.44, 14.93, 15.18, 15.96, 16.21, 16.40, 16.73, 16.86, 17.38, 18.32, 19.07, 20.07, 20.26, 20.51, 20.94, 21.45, 21.60, 22.30, 22.90, 23.05, 23.28, 23.71, 24.28, 24.97, 25.30, 25.69, 26.33, 26.71, 27.29, 27.65, 28.07, 29.22, and 29.50;
Compound 1 Camsylate Form A is **characterized by** all of the following peaks in an XRPD pattern on a 2 Theta scale, selected from 5.55, 7.90, 8.25, 8.38, 9.55, 9.87, 10.26, 11.12, 11.34, 12.50, 12.94, 13.40, 13.65, 14.79, 15.83, 16.16, 16.41, 16.54, 16.82, 17.10, 17.69, 18.02, 18.22, 18.47, 18.91, 19.10, 19.47, 20.04, 20.87, 21.05, 22.16, 22.36, 22.93, 23.71, 24.55, 25.33, 26.02, 26.40, 27.09, 27.33, 27.97, 28.32, 28.68, and 28.98;
Compound 1 Camsylate Form B is **characterized by** all of the following peaks in an XRPD pattern on a 2 Theta scale, selected from 5.25, 8.32, 8.51, 8.76, 9.05, 9.58, 9.88, 10.00, 10.52, 10.80, 11.06, 11.96, 12.24, 12.58, 13.98, 15.62, 15.81, 16.15, 16.70, 17.12, 17.42, 17.71, 18.41, 19.37, 19.55, 20.63, 21.13, 21.56, 21.89, 22.50, 23.04, 24.43, 26.08, 27.07, 27.97, 28.22, and 28.73;
Compound 1 Citrate Form B is **characterized by** all of the following peaks in an XRPD pattern on a 2 Theta scale, selected from 4.58, 5.95, 7.61, 8.88, 9.19, 9.94, 11.93, 13.26, 13.79, 14.29, 14.68, 15.26, 15.95, 16.64, 17.11, 17.87, 18.21, 18.43, 18.90, 19.36, 19.67, 20.14, 20.57, 21.47, 22.30, 22.64, 23.06, 23.80, 24.25, 24.55, 24.81, 25.10, 25.45, 25.54, 25.85, 26.35, 26.51, 26.83, 27.16, 27.57, 27.85, 28.08, and 28.47;
Compound 1 Esylate Form A is **characterized by** all of the following peaks in an XRPD pattern on a 2 Theta scale, selected from 3.97, 7.95, 9.89, 10.62, 10.92, 11.03, 11.34, 11.77, 12.03, 12.53, 12.71, 13.27, 14.00, 14.70, 15.08, 15.94, 16.17, 16.92, 17.01, 17.19, 17.35, 17.61, 18.15, 18.76, 19.03, 19.66, 19.89, 20.26, 20.65, 20.78, 21.44, 22.08, 23.14, 23.27, 23.99, 24.32, 25.43, 25.66, 25.85, 26.52, 26.87, 27.61, 27.96, 28.18, 28.40, and 28.91;
Compound 1 HBr Form B is **characterized by** all of the following peaks in an XRPD pattern on a 2 Theta scale, selected from 5.26, 8.29, 9.75, 9.91, 10.56, 10.72, 12.25, 12.50, 12.75, 13.31, 13.54, 14.11, 14.72, 14.89, 15.87, 16.29, 16.50, 17.36, 17.78, 18.09, 18.44, 18.75, 19.12, 19.24, 19.56, 19.92, 20.40, 20.55, 20.69, 21.18, 22.09, 22.64, 23.24, 24.56, 25.80, 26.52, 27.23, 27.43, 28.13, 28.46, 28.76, 29.08, 29.66, and 30.07;
Compound 1 Maleate Form A is **characterized by** all of the following peaks in an XRPD pattern on a 2 Theta scale, selected from 7.58, 8.34, 10.92, 12.38, 12.87, 14.17, 15.13, 15.78, 16.43, 16.91, 17.51, 18.09, 18.71, 20.30, 21.60, 21.99, 22.30, 22.49, 23.32, 23.56, 23.92, 24.79, 26.04, 26.41, 26.92, 27.62, 28.18, 28.54, and 29.01;
Compound 1 Mesylate Form A is **characterized by** all of the following peaks in an XRPD pattern on a 2 Theta scale, selected from 5.06, 6.65, 7.66, 8.35, 10.16, 10.72, 11.98, 12.62, 13.32, 14.41, 14.79, 15.42, 15.72, 15.89, 16.23, 16.77, 17.20, 17.59, 18.08, 18.40, 18.81, 19.33, 19.53, 19.71, 20.14, 20.38, 20.73, 21.05, 21.56, 21.83, 22.16, 23.14, 24.06, 24.54, 24.77, 25.75, 26.82, 27.71, 27.94, 28.71, 29.27, and 29.85;
Compound 1 Mesylate Form B is **characterized by** all of the following peaks in an XRPD pattern on a 2 Theta scale, selected from 5.14, 6.62, 7.72, 7.89, 10.33, 10.73, 12.74, 13.31, 13.88, 14.23, 14.61, 15.21, 15.52, 15.87, 16.17, 17.18, 17.41, 17.88, 18.15, 18.46, 18.73, 19.13, 19.54, 20.15, 20.53, 20.75, 20.98, 21.27, 21.60, 22.03, 22.28, 23.07, 23.32, 24.16, 24.42, 24.87, 25.13, 25.64, 26.24, 26.95, 27.17, 27.49, 27.77, 28.42, and 29.54;
Compound 1 Mesylate Form C is **characterized by** all of the following peaks in an XRPD pattern on a 2 Theta scale, selected from 7.55, 9.53, 10.25, 11.04, 11.49, 11.65, 12.24, 12.51, 13.88, 14.44, 15.36, 15.66, 16.29, 16.58, 17.16, 17.54, 19.04, 19.20, 19.79, 20.54, 21.12, 21.24, 21.62, 22.22, 23.15, 23.57, 23.99, 24.61, 24.77, 25.22, 25.61, 25.99, 26.60, 26.89, 27.29, 27.91, 29.03, and 29.29;
Compound 1 Oxalate Form A is **characterized by** all of the following peaks in an XRPD pattern on a 2 Theta scale, selected from 4.90, 5.25, 6.20, 8.05, 9.87, 10.54, 12.29, 14.79, 15.35, 15.84, 16.49, 16.89, 17.55, 18.27, 18.67, 19.12, 19.40, 19.81, 20.26, 20.50, 21.06, 21.74, 22.17, 22.93, 23.26, 23.50, 23.81, 24.66, 25.06, 25.95, 26.90, 27.82, and 28.24;
Compound 1 Oxalate Form B is **characterized by** all of the following peaks in an XRPD pattern on a 2 Theta scale, selected from 4.20, 7.59, 8.02, 8.42, 10.53, 12.36, 12.66, 13.09, 13.35, 13.88, 14.15, 14.46, 15.06, 15.23, 17.42, 17.75, 18.28, 19.07, 19.52, 20.33, 21.18, 21.43, 21.74, 22.08, 22.59, 23.53, 24.21, 24.52, 24.71, 25.24, 25.85, 26.24, 26.55, 26.90, 27.43, 28.56, 29.30, and 29.51;
Compound 1 Oxalate Form C is **characterized by** all of the following peaks in an XRPD pattern on a 2 Theta scale, selected from 4.13, 7.70, 10.29, 10.59, 13.09, 13.70, 14.13, 15.33, 15.87, 16.57, 17.19, 17.50, 17.83, 18.15, 18.61, 19.24, 19.42, 20.11, 20.34, 20.64, 21.07, 21.47, 21.59, 21.91, 22.56, 22.92, 23.37, 23.87, 24.35, 25.35, 25.91, 26.47, 26.68, 27.74, 28.47, and 29.14;
Compound 1 Sulfate Form A is **characterized by** all of the following peaks in an XRPD pattern on a 2 Theta scale, selected from 4.74, 5.85, 9.47, 10.04, 11.74, 11.95, 13.77, 14.99, 15.42, 15.71, 16.48, 17.36, 17.66, 17.92, 18.59, 18.95, 19.36, 19.74, 20.22, 20.77, 21.38, 21.86, 22.74, 23.30, 23.70, 24.33, 25.07, 25.32, 25.67, 26.23, and 27.00;
Compound 1 Sulfate Form B is **characterized by** all of the following peaks in an XRPD pattern on a 2 Theta scale, selected from 4.27, 8.57, 9.32, 10.95, 11.29, 12.07, 12.87, 13.25, 13.82, 14.13, 14.67, 16.94, 17.21, 17.64, 19.99, 20.26, 21.03, 21.44, 22.00, 22.71, 23.85, 25.20, and 26.02;
Compound 1 Sulfate Form C is **characterized by** all of the following peaks in an XRPD pattern on a 2 Theta scale, selected from 3.19, 5.35, 9.04, 9.62, 10.73, 11.31, 11.60, 12.27, 12.67, 13.76, 14.31, 14.65, 15.21, 15.63, 16.11, 16.33, 16.63, 17.11, 18.32, 18.75, 18.99, 19.36, 19.47, 20.16, 20.55, 21.01, 21.52, 21.62, 21.89, 22.19, 22.53, 23.19, 23.79, 24.23, 24.81, 25.07, 25.41, 26.06, 26.73, 27.58, 28.16, 28.63, 28.90, 29.22, 29.47, and 29.73;
Compound 1 Hemitartrate Form B is **characterized by** all of the following peaks in an XRPD pattern on a 2 Theta scale, selected from 5.17, 5.66, 7.22, 9.27, 10.10, 10.40, 11.37, 11.55, 12.77, 13.94, 14.51, 15.60, 16.06, 16.48, 17.09, 17.69, 17.90, 18.27, 18.94, 19.15, 19.59, 19.81, 20.06, 20.32, 21.23, 21.83, 22.34, 22.84, 23.53, 24.26, 24.70, 25.26, 25.80, 26.78, 27.29, 27.90, 29.04, and 29.45;
Compound 1 Tosylate Form A is **characterized by** all of the following peaks in an XRPD pattern on a 2 Theta scale, selected from 5.81, 7.05, 8.52, 9.74, 10.40, 10.99, 11.26, 11.66, 12.58, 12.81, 14.28, 14.62, 15.30, 15.80, 16.03, 16.34, 17.12, 17.55, 18.03, 18.94, 19.26, 19.67, 20.31, 20.65, 21.02, 21.43, 22.11, 22.44, 22.66, 22.94, 23.07, 23.54, 23.79, 24.55, 24.88, 25.42, 25.83, 26.10, 26.53, 27.70, 28.11, 28.62, 29.18, 29.40, 29.66, and 29.83; and
Compound 1 Tosylate Form B is **characterized by** all of the following peaks in an XRPD pattern on a 2 Theta scale, selected from 4.61, 8.69, 9.25, 10.68, 12.44, 12.69, 13.76, 14.42, 14.81, 15.47, 16.08, 16.57, 17.45, 17.76, 18.44, 18.83, 19.01, 19.35, 20.42, 20.84, 21.15, 21.69, 22.09, 22.86, 23.12, 24.08, 24.53, 24.86, 25.22, 25.91, 26.33, 26.82, 27.63, 28.61, 29.11, and 29. 77.

5. The crystalline salt form of any one of claims 1-4, wherein:
Compound 1 Besylate Form A is **characterized by** a first endotherm at a temperature of about 144 °C and a second endotherm at a temperature of about 170 °C in a DSC thermogram;
Compound 1 Camsylate Form A is **characterized by** a first endotherm at a temperature of about 110 °C and a second endotherm at a temperature of about 174 °C in a DSC thermogram;
Compound 1 Citrate Form B is **characterized by** an endotherm with an onset temperature at about 175 °C in a DSC thermogram;
Compound 1 Esylate Form A is **characterized by** a first endotherm at a temperature of about 69 °C, a second endotherm at about 156 °C, and a third endotherm at a temperature of about 190 °C, in a DSC thermogram;
Compound 1 Maleate Form A is **characterized by** an endotherm at a temperature of about 117 °C in a DSC thermogram;
Compound 1 Mesylate Form C is **characterized by** a first endotherm with a peak at a temperature of about 69 °C, wherein the first endotherm has a lower limit of about 36 and an upper limit of a temperature of about 96 °C, and a second endotherm with a peak at a temperature of about 208 °C, wherein the second endotherm has a lower limit of a temperature of about 169 and an upper limit of a temperature of about 219 °C;
Compound 1 Hemitartrate Form B is **characterized by** a first endotherm at a temperature of about 111 °C, and a second endotherm at a temperature of about 148 °C in a DSC thermogram;
Compound 1 Tosylate Form A is **characterized by** an endotherm with an onset temperature at about 214 °C in a DSC thermogram; and
Compound 1 Tosylate Form B is **characterized by** a sharp endotherm with an onset temperature at -183 °C in a DSC thermogram.

6. The crystalline salt form of any one of claims 1-5, wherein:
Compound 1 Besylate Form A is **characterized by** a weight loss of about 7.9% over a temperature range of 39-177 °C in a TGA thermogram;
Compound 1 Camsylate Form A is **characterized by** a weight loss of about 5.9% over a temperature range of 38-170 °C in a TGA thermogram;
Compound 1 Citrate Form B is **characterized by** a weight loss beginning at a temperature of about 159 °C and ending at about 220 °C in a TGA thermogram;
Compound 1 Esylate Form A is **characterized by** a weight loss of about 8.5 wt% over a temperature range of 38-188 °C in a TGA thermogram;
Compound 1 Maleate Form A is **characterized by** a weight loss of about 3.1 wt% in the temperature range of 42-146 °C in a TGA thermogram;
Compound 1 Mesylate Form C is **characterized by** a weight loss of from 0.5 to 3.0 wt% in the temperature range of 36-130 °C in a TGA thermogram;
Compound 1 Hemitartrate Form B is **characterized by** a weight loss of about 7.4 wt% in the temperature range of 39-157 °C in a TGA thermogram;
Compound 1 Tosylate Form A is **characterized by** a weight loss of about 0.9 wt% in the temperature range of 72-231 °C in a TGA thermogram; and
Compound 1 Tosylate Form B is **characterized by** a weight loss of about 0.7 wt% in the temperature range of 39-214 °C in a TGA thermogram.

7. The crystalline salt form of any one of claims 1-6, wherein:
Compound 1 Citrate Form B is **characterized by** about a 4.1 wt% increase, as measured by DVS, when taken from an environment of 5% relative humidity to an environment of 95% relative humidity;
Compound 1 Maleate Form A is **characterized by** about a 2.5 wt% increase, as measured by DVS, when taken from an environment of 5% relative humidity to an environment of 95% relative humidity;
Compound 1 Mesylate Form C is **characterized by** about a 5.1 wt% increase, as measured by DVS, when taken from an environment of 5% relative humidity to an environment of 95% relative humidity;
Compound 1 Tosylate Form A is **characterized by** about a 0.9 wt% increase, as measured by DVS, when taken from an environment of 5% relative humidity to an environment of 95% relative humidity; and
Compound 1 Tosylate Form B is **characterized by** about a 0.9 wt% increase, as measured by DVS, when taken from an environment of 5% relative humidity to an environment of 95% relative humidity.

8. A pharmaceutical composition comprising a crystalline salt form of any one of claims 1-7 and a pharmaceutically acceptable excipient.

9. A crystalline salt form of any one of claims 1-7, or a pharmaceutical composition of claim 8 for use in a method of treating a disease, disorder, or syndrome mediated at least in part by modulating *in vivo* activity of a protein kinase, wherein the disease, disorder or syndrome is cancer.

## Patentansprüche

1. Eine kristalline Salzform von Verbindung 1 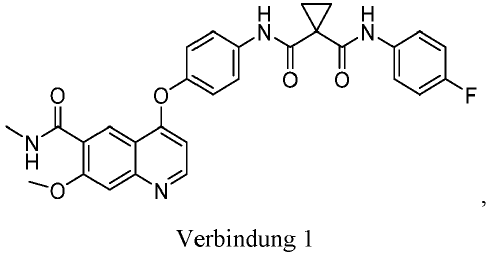 ausgewählt aus der Gruppe, bestehend aus Verbindung-1-Besylatform A, Verbindung-1-Besylatform B, Verbindung-1-Benzoatform A, Verbindung-1-Camsylatform A, Verbindung-1-Camsylatform B, Verbindung-1-Citratform A, Verbindung-1-Citratform B, Verbindung-1-Esylatform A, Verbindung-1-Esylatform B, Verbindung-1-HBr-Form A, Verbindung-1-HBr-Form B, Verbindung-1-Lactatform A, Verbindung-1-Malatform A, Verbindung-1-Maleatform A, Verbindung-1-Maleatform B, Verbindung-1-Mesylatform A, Verbindung-1-Mesylatform B, Verbindung-1-Mesylatform C, Verbindung-1-Oxalatform A, Verbindung-1-Oxalatform B, Verbindung-1-Oxalatform C, Verbindung-1-Propionatform A, Verbindung-1-Succinatform A, Verbindung-1-Sulfatform A, Verbindung-1-Sulfatform B, Verbindung-1-Sulfatform C, Verbindung-1-Tartratform A, Verbindung-1-Hemitartratform B, Verbindung-1-Tartratform C, Verbindung-1-Tosylatform A und Verbindung-1-Tosylatform B, wobei:
Verbindung-1-Besylatform A durch einen oder mehrere Peaks in einem XRPD-Muster auf einer 2-Theta-Skala gekennzeichnet ist, wobei der eine oder die mehreren Peaks aus 3,78, 7,56, 10,08, 10,42, 11,70, 11,78, 12,00, 12,71, 13,20, 13,92, 14,06, 14,44, 14,93, 15,18, 15,96, 16,21, 16,40, 16,73, 16,86, 17,38, 18,32, 19,07, 20,07, 20,26, 20,51, 20,94, 21,45, 21,60, 22,30, 22,90, 23,05, 23,28, 23,71, 24,28, 24,97, 25,30, 25,69, 26,33, 26,71, 27,29, 27,65, 28,07, 29,22 und 29,50 ausgewählt sind,
Verbindung-1-Besylatform B durch das in Figur 4 bereitgestellte XRPD-Muster gekennzeichnet ist;
Verbindung-1-Benzoatform A durch das in Figur 5 bereitgestellte XRPD-Muster gekennzeichnet ist;
Verbindung-1-Camsylatform A durch einen oder mehrere Peaks in einem XRPD-Muster auf einer 2-Theta-Skala gekennzeichnet ist, wobei der eine oder die mehreren Peaks aus 5,55, 7,90, 8,25, 8,38, 9,55, 9,87, 10,26, 11,12, 11,34, 12,50, 12,94, 13,40, 13,65, 14,79, 15,83, 16,16, 16,41, 16,54, 16,82, 17,10, 17,69, 18,02, 18,22, 18,47, 18,91, 19,10, 19,47, 20,04, 20,87, 21,05, 22,16, 22,36, 22,93, 23,71, 24,55, 25,33, 26,02, 26,40, 27,09, 27,33, 27,97, 28,32, 28,68 und 28,98 ausgewählt sind;
Verbindung-1-Camsylatform B durch einen oder mehrere Peaks in einem XRPD-Muster auf einer 2-Theta-Skala gekennzeichnet ist, wobei der eine oder die mehreren Peaks aus 5,25, 8,32, 8,51, 8,76, 9,05, 9,58, 9,88, 10,00, 10,52, 10,80, 11,06, 11,96, 12,24, 12,58, 13,98, 15,62, 15,81, 16,15, 16,70, 17,12, 17,42, 17,71, 18,41, 19,37, 19,55, 20,63, 21,13, 21,56, 21,89, 22,50, 23,04, 24,43, 26,08, 27,07, 27,97, 28,22 und 28,73 ausgewählt sind;
Verbindung-1-Citratform A durch das in Figur 10 bereitgestellte XRPD-Muster gekennzeichnet ist;
Verbindung-1-Citratform B durch einen oder mehrere Peaks in einem XRPD-Muster auf einer 2-Theta-Skala gekennzeichnet ist, wobei der eine oder die mehreren Peaks aus 4,58, 5,95, 7,61, 8,88, 9,19, 9,94, 11,93, 13,26, 13,79, 14,29, 14,68, 15,26, 15,95, 16,64, 17,11, 17,87, 18,21, 18,43, 18,90, 19,36, 19,67, 20,14, 20,57, 21,47, 22,30, 22,64, 23,06, 23,80, 24,25, 24,55, 24,81, 25,10, 25,45, 25,54, 25,85, 26,35, 26,51, 26,83, 27,16, 27,57, 27,85, 28,08 und 28,47 ausgewählt sind;
Verbindung-1-Esylatform A durch einen oder mehrere Peaks in einem XRPD-Muster auf einer 2-Theta-Skala gekennzeichnet ist, wobei der eine oder die mehreren Peaks aus 3,97, 7,95, 9,89, 10,62, 10,92, 11,03, 11,34, 11,77, 12,03, 12,53, 12,71, 13,27, 14,00, 14,70, 15,08, 15,94, 16,17, 16,92, 17,01, 17,19, 17,35, 17,61, 18,15, 18,76, 19,03, 19,66, 19,89, 20,26, 20,65, 20,78, 21,44, 22,08, 23,14, 23,27, 23,99, 24,32, 25,43, 25,66, 25,85, 26,52, 26,87, 27,61, 27,96, 28,18, 28,40 und 28,91 ausgewählt sind;
Verbindung-1-Esylatform B durch das in Figur 18 bereitgestellte XRPD-Muster gekennzeichnet ist;
Verbindung-1-HBr-Form A durch das in Figur 19 bereitgestellte XRPD-Muster gekennzeichnet ist;
Verbindung-1-HBr-Form B durch einen oder mehrere Peaks in einem XRPD-Muster auf einer 2-Theta-Skala gekennzeichnet ist, wobei der eine oder die mehreren Peaks aus 5,26, 8,29, 9,75, 9,91, 10,56, 10,72, 12,25, 12,50, 12,75, 13,31, 13,54, 14,11, 14,72, 14,89, 15,87, 16,29, 16,50, 17,36, 17,78, 18,09, 18,44, 18,75, 19,12, 19,24, 19,56, 19,92, 20,40, 20,55, 20,69, 21,18, 22,09, 22,64, 23,24, 24,56, 25,80, 26,52, 27,23, 27,43, 28,13, 28,46, 28,76, 29,08, 29,66 und 30,07 ausgewählt sind;
Verbindung-1-Lactatform A durch das in Figur 21 bereitgestellte XRPD-Muster gekennzeichnet ist;
Verbindung-1-Malatform A durch das in Figur 22 bereitgestellte XRPD-Muster gekennzeichnet ist;
Verbindung-1-Maleatform A durch einen oder mehrere Peaks in einem XRPD-Muster auf einer 2-Theta-Skala gekennzeichnet ist, wobei der eine oder die mehreren Peaks aus 7,58, 8,34, 10,92, 12,38, 12,87, 14,17, 15,13, 15,78, 16,43, 16,91, 17,51, 18,09, 18,71, 20,30, 21,60, 21,99, 22,30, 22,49, 23,32, 23,56, 23,92, 24,79, 26,04, 26,41, 26,92, 27,62, 28,18, 28,54 und 29,01 ausgewählt sind;
Verbindung-1-Maleatform B durch das in Figur 28 bereitgestellte XRPD-Muster gekennzeichnet ist;
Verbindung-1-Mesylatform A durch einen oder mehrere Peaks in einem XRPD-Muster auf einer 2-Theta-Skala gekennzeichnet ist, wobei der eine oder die mehreren Peaks aus 5,06, 6,65, 7,66, 8,35, 10,16, 10,72, 11,98, 12,62, 13,32, 14,41, 14,79, 15,42, 15,72, 15,89, 16,23, 16,77, 17,20, 17,59, 18,08, 18,40, 18,81, 19,33, 19,53, 19,71, 20,14, 20,38, 20,73, 21,05, 21,56, 21,83, 22,16, 23,14, 24,06, 24,54, 24,77, 25,75, 26,82, 27,71, 27,94, 28,71, 29,27 und 29,85 ausgewählt sind;
Verbindung-1-Mesylatform B durch einen oder mehrere Peaks in einem XRPD-Muster auf einer 2-Theta-Skala gekennzeichnet ist, wobei der eine oder die mehreren Peaks aus 5,14, 6,62, 7,72, 7,89, 10,33, 10,73, 12,74, 13,31, 13,88, 14,23, 14,61, 15,21, 15,52, 15,87, 16,17, 17,18, 17,41, 17,88, 18,15, 18,46, 18,73, 19,13, 19,54, 20,15, 20,53, 20,75, 20,98, 21,27, 21,60, 22,03, 22,28, 23,07, 23,32, 24,16, 24,42, 24,87, 25,13, 25,64, 26,24, 26,95, 27,17, 27,49, 27,77, 28,42 und 29,54 ausgewählt sind;
Verbindung-1-Mesylatform C durch einen oder mehrere Peaks in einem XRPD-Muster auf einer 2-Theta-Skala gekennzeichnet ist, wobei der eine oder die mehreren Peaks aus 7,55, 9,53, 10,25, 11,04, 11,49, 11,65, 12,24, 12,51, 13,88, 14,44, 15,36, 15,66, 16,29, 16,58, 17,16, 17,54, 19,04, 19,20, 19,79, 20,54, 21,12, 21,24, 21,62, 22,22, 23,15, 23,57, 23,99, 24,61, 24,77, 25,22, 25,61, 25,99, 26,60, 26,89, 27,29, 27,91, 29,03 und 29,29 ausgewählt sind;
Verbindung-1-Oxalatform A durch einen oder mehrere Peaks in einem XRPD-Muster auf einer 2-Theta-Skala gekennzeichnet ist, wobei der eine oder die mehreren Peaks aus 4,90, 5,25, 6,20, 8,05, 9,87, 10,54, 12,29, 14,79, 15,35, 15,84, 16,49, 16,89, 17,55, 18,27, 18,67, 19,12, 19,40, 19,81, 20,26, 20,50, 21,06, 21,74, 22,17, 22,93, 23,26, 23,50, 23,81, 24,66, 25,06, 25,95, 26,90, 27,82 und 28,24 ausgewählt sind;
Verbindung-1-Oxalatform B durch einen oder mehrere Peaks in einem XRPD-Muster auf einer 2-Theta-Skala gekennzeichnet ist, wobei der eine oder die mehreren Peaks aus 4,20, 7,59, 8,02, 8,42, 10,53, 12,36, 12,66, 13,09, 13,35, 13,88, 14,15, 14,46, 15,06, 15,23, 17,42, 17,75, 18,28, 19,07, 19,52, 20,33, 21,18, 21,43, 21,74, 22,08, 22,59, 23,53, 24,21, 24,52, 24,71, 25,24, 25,85, 26,24, 26,55, 26,90, 27,43, 28,56, 29,30 und 29,51 ausgewählt sind;
Verbindung-1-Oxalatform C durch einen oder mehrere Peaks in einem XRPD-Muster auf einer 2-Theta-Skala gekennzeichnet ist, wobei der eine oder die mehreren Peaks aus 4,13, 7,70, 10,29, 10,59, 13,09, 13,70, 14,13, 15,33, 15,87, 16,57, 17,19, 17,50, 17,83, 18,15, 18,61, 19,24, 19,42, 20,11, 20,34, 20,64, 21,07, 21,47, 21,59, 21,91, 22,56, 22,92, 23,37, 23,87, 24,35, 25,35, 25,91, 26,47, 26,68, 27,74, 28,47 und 29,14 ausgewählt sind;
Verbindung-1-Propionatform A durch das in Figur 38 bereitgestellte XRPD-Muster gekennzeichnet ist;
Verbindung-1-Succinatform A durch das in Figur 39 bereitgestellte XRPD-Muster gekennzeichnet ist;
Verbindung-1-Sulfatform A durch einen oder mehrere Peaks in einem XRPD-Muster auf einer 2-Theta-Skala gekennzeichnet ist, wobei der eine oder die mehreren Peaks aus 4,74, 5,85, 9,47, 10,04, 11,74, 11,95, 13,77, 14,99, 15,42, 15,71, 16,48, 17,36, 17,66, 17,92, 18,59, 18,95, 19,36, 19,74, 20,22, 20,77, 21,38, 21,86, 22,74, 23,30, 23,70, 24,33, 25,07, 25,32, 25,67, 26,23 und 27,00 ausgewählt sind;
Verbindung-1-Sulfatform B durch einen oder mehrere Peaks in einem XRPD-Muster auf einer 2-Theta-Skala gekennzeichnet ist, wobei der eine oder die mehreren Peaks aus 4,27, 8,57, 9,32, 10,95, 11,29, 12,07, 12,87, 13,25, 13,82, 14,13, 14,67, 16,94, 17,21, 17,64, 19,99, 20,26, 21,03, 21,44, 22,00, 22,71, 23,85, 25,20 und 26,02 ausgewählt sind;
Verbindung-1-Sulfatform C durch einen oder mehrere Peaks in einem XRPD-Muster auf einer 2-Theta-Skala gekennzeichnet ist, wobei der eine oder die mehreren Peaks aus 3,19, 5,35, 9,04, 9,62, 10,73, 11,31, 11,60, 12,27, 12,67, 13,76, 14,31, 14,65, 15,21, 15,63, 16,11, 16,33, 16,63, 17,11, 18,32, 18,75, 18,99, 19,36, 19,47, 20,16, 20,55, 21,01, 21,52, 21,62, 21,89, 22,19, 22,53, 23,19, 23,79, 24,23, 24,81, 25,07, 25,41, 26,06, 26,73, 27,58, 28,16, 28,63, 28,90, 29,22, 29,47 und 29,73 ausgewählt sind;
Verbindung-1-Tartratform A durch das in Figur 43 bereitgestellte XRPD-Muster gekennzeichnet ist;
Verbindung-1-Hemitartratform B durch einen oder mehrere Peaks in einem XRPD-Muster auf einer 2-Theta-Skala gekennzeichnet ist, wobei der eine oder die mehreren Peaks aus 5,17, 5,66, 7,22, 9,27, 10,10, 10,40, 11,37, 11,55, 12,77, 13,94, 14,51, 15,60, 16,06, 16,48, 17,09, 17,69, 17,90, 18,27, 18,94, 19,15, 19,59, 19,81, 20,06, 20,32, 21,23, 21,83, 22,34, 22,84, 23,53, 24,26, 24,70, 25,26, 25,80, 26,78, 27,29, 27,90, 29,04 und 29,45 ausgewählt sind;
Verbindung-1-Tartratform C durch das in Figur 69 bereitgestellte XRPD-Muster gekennzeichnet ist;
Verbindung-1-Tosylatform A durch einen oder mehrere Peaks in einem XRPD-Muster auf einer 2-Theta-Skala gekennzeichnet ist, wobei der eine oder die mehreren Peaks aus 5,81, 7,05, 8,52, 9,74, 10,40, 10,99, 11,26, 11,66, 12,58, 12,81, 14,28, 14,62, 15,30, 15,80, 16,03, 16,34, 17,12, 17,55, 18,03, 18,94, 19,26, 19,67, 20,31, 20,65, 21,02, 21,43, 22,11, 22,44, 22,66, 22,94, 23,07, 23,54, 23,79, 24,55, 24,88, 25,42, 25,83, 26,10, 26,53, 27,70, 28,11, 28,62, 29,18, 29,40, 29,66 und 29,83 ausgewählt sind; und
Verbindung-1-Tosylatform B durch einen oder mehrere Peaks in einem XRPD-Muster auf einer 2-Theta-Skala gekennzeichnet ist, wobei der eine oder die mehreren Peaks aus 4,61, 8,69, 9,25, 10,68, 12,44, 12,69, 13,76, 14,42, 14,81, 15,47, 16,08, 16,57, 17,45, 17,76, 18,44, 18,83, 19,01, 19,35, 20,42, 20,84, 21,15, 21,69, 22,09, 22,86, 23,12, 24,08, 24,53, 24,86, 25,22, 25,91, 26,33, 26,82, 27,63, 28,61, 29,11 und 29,77 ausgewählt sind.

2. Kristalline Salzform nach Anspruch 1, wobei:
Verbindung-1-Besylatform A durch einen oder mehrere Peaks in einem XRPD-Muster auf einer 2-Theta-Skala gekennzeichnet ist, wobei der eine oder die mehreren Peaks aus 3,78, 7,56, 10,42, 20,51, 20,94, 21,60 und 25,30 ausgewählt sind;
Verbindung-1-Camsylatform A durch einen oder mehrere Peaks in einem XRPD-Muster auf einer 2-Theta-Skala gekennzeichnet ist, wobei der eine oder die mehreren Peaks aus 5,55, 9,87, 10,26, 16,16, 16,82, 20,87, 22,36 und 23,71 ausgewählt sind;
Verbindung-1-Camsylatform B durch einen oder mehrere Peaks in einem XRPD-Muster auf einer 2-Theta-Skala gekennzeichnet ist, wobei der eine oder die mehreren Peaks aus 5,25, 10,52, 16,15, 21,13, 21,56 und 21,89 ausgewählt sind;
Verbindung-1-Citratform B durch einen oder mehrere Peaks in einem XRPD-Muster auf einer 2-Theta-Skala gekennzeichnet ist, wobei der eine oder die mehreren Peaks aus 4,58, 5,95, 8,88, 11,93, 13,79, 14,29, 16,64, 18,90, 21,47, 23,80, 24,25 und 25,85 ausgewählt sind;
Verbindung-1-Esylatform A durch einen oder mehrere Peaks in einem XRPD-Muster auf einer 2-Theta-Skala gekennzeichnet ist, wobei der eine oder die mehreren Peaks aus 3,97, 7,95, 9,89, 10,62, 13,27, 15,08, 16,17, 16,92, 17,01, 20,65, 20,78, 21,44, 22,08, 23,99, 25,43 und 26,52 ausgewählt sind;
Verbindung-1-HBr-Form B durch einen oder mehrere Peaks in einem XRPD-Muster auf einer 2-Theta-Skala gekennzeichnet ist, wobei der eine oder die mehreren Peaks aus 5,26, 9,91, 10,72, 20,55, 20,69, 21,18, 22,09, 23,24, 24,56 und 25,80 ausgewählt sind;
Verbindung-1-Maleatform A durch einen oder mehrere Peaks in einem XRPD-Muster auf einer 2-Theta-Skala gekennzeichnet ist, wobei der eine oder die mehreren Peaks aus 10,92, 15,13, 15,78, 16,43, 18,09, 18,71, 20,30, 21,99, 22,30, 22,49, 23,32, 23,56, 24,79, 26,41, 26,92 und 29,01 ausgewählt sind;
Verbindung-1-Mesylatform A durch einen oder mehrere Peaks in einem XRPD-Muster auf einer 2-Theta-Skala gekennzeichnet ist, wobei der eine oder die mehreren Peaks aus 5,06, 7,66, 14,79, 15,42, 16,23, 17,20, 17,59, 18,40, 19,33, 19,53, 20,38, 20,73, 21,83, 22,16, 23,14, 24,06 und 24,77 ausgewählt sind;
Verbindung-1-Mesylatform B durch einen oder mehrere Peaks in einem XRPD-Muster auf einer 2-Theta-Skala gekennzeichnet ist, wobei der eine oder die mehreren Peaks aus 14,61, 15,21, 16,17, 17,18, 17,41, 18,46, 19,13, 19,54, 20,15, 20,53, 20,75, 20,98, 21,27, 21,60, 22,03, 23,32, 24,16, 24,42, 24,87 und 25,13 ausgewählt sind;
Verbindung-1-Mesylatform C durch einen oder mehrere Peaks in einem XRPD-Muster auf einer 2-Theta-Skala gekennzeichnet ist, wobei der eine oder die mehreren Peaks aus 7,55, 9,53, 11,49, 12,51, 14,44, 19,04, 20,54, 21,12, 21,24 und 23,15 ausgewählt sind;
Verbindung-1-Oxalatform A durch einen oder mehrere Peaks in einem XRPD-Muster auf einer 2-Theta-Skala gekennzeichnet ist, wobei der eine oder die mehreren Peaks aus 4,90, 5,25, 9,87, 10,54, 12,29, 15,35, 18,67, 19,40, 19,81, 20,26, 22,93 und 24,66 ausgewählt sind;
Verbindung-1-Oxalatform B durch einen oder mehrere Peaks in einem XRPD-Muster auf einer 2-Theta-Skala gekennzeichnet ist, wobei der eine oder die mehreren Peaks aus 4,20, 7,59, 10,53, 15,06, 15,23, 19,52, 21,18, 21,43, 22,59, 24,21 und 25,85 ausgewählt sind;
Verbindung-1-Oxalatform C durch einen oder mehrere Peaks in einem XRPD-Muster auf einer 2-Theta-Skala gekennzeichnet ist, wobei der eine oder die mehreren Peaks aus 7,70, 10,29, 10,59, 13,09, 13,70, 15,33, 17,83, 18,15, 20,11, 21,07, 21,91 und 22,56 ausgewählt sind;
Verbindung-1-Sulfatform A durch einen oder mehrere Peaks in einem XRPD-Muster auf einer 2-Theta-Skala gekennzeichnet ist, wobei der eine oder die mehreren Peaks aus 4,74, 5,85, 9,47, 10,04, 11,74, 11,95, 16,48, 20,22 und 22,74 ausgewählt sind;
Verbindung-1-Sulfatform B durch einen oder mehrere Peaks in einem XRPD-Muster auf einer 2-Theta-Skala gekennzeichnet ist, wobei der eine oder die mehreren Peaks aus 4,27, 8,57, 9,32, 16,94, 19,99, 21,03, 21,44, 22,00 und 26,02 ausgewählt sind;
Verbindung-1-Sulfatform C durch einen oder mehrere Peaks in einem XRPD-Muster auf einer 2-Theta-Skala gekennzeichnet ist, wobei der eine oder die mehreren Peaks aus 3,19, 5,35, 9,62, 12,27, 16,33, 17,11, 18,75, 18,99, 19,36, 19,47, 20,55, 21,01, 21,52, 21,62, 21,89, 22,19, 23,79, 25,07 und 26,06 ausgewählt sind;
Verbindung-1-Hemitartratform B durch einen oder mehrere Peaks in einem XRPD-Muster auf einer 2-Theta-Skala gekennzeichnet ist, wobei der eine oder die mehreren Peaks aus 5,17, 5,66, 10,10, 10,40, 11,37, 11,55, 14,51, 17,09, 21,23, 21,83, 23,53, 24,26, 25,26 und 26,78 ausgewählt sind;
Verbindung-1-Tosylatform A durch einen oder mehrere Peaks in einem XRPD-Muster auf einer 2-Theta-Skala gekennzeichnet ist, wobei der eine oder die mehreren Peaks aus 8,52, 9,74, 14,28, 16,03, 17,55, 18,94, 19,26, 20,31, 21,02, 21,43, 22,11, 22,44, 22,66, 23,07, 24,55 und 27,70 ausgewählt sind; und
Verbindung-1-Tosylatform B durch einen oder mehrere Peaks in einem XRPD-Muster auf einer 2-Theta-Skala gekennzeichnet ist, wobei der eine oder die mehreren Peaks aus 4,61, 9,25, 12,44, 15,47, 17,76, 19,01, 19,35, 25,91, 26,33 und 27,63 ausgewählt sind.

3. Kristalline Salzform nach Anspruch 1 oder 2, wobei:
Verbindung-1-Besylatform A durch alle der folgenden Peaks in einem XRPD-Muster auf einer 2-Theta-Skala, ausgewählt aus 3,78, 7,56, 10,42, 20,51, 20,94, 21,60 und 25,30, gekennzeichnet ist;
Verbindung-1-Camsylatform A durch alle der folgenden Peaks in einem XRPD-Muster auf einer 2-Theta-Skala, ausgewählt aus 5,55, 9,87, 10,26, 16,16, 16,82, 20,87, 22,36 und 23,71, gekennzeichnet ist;
Verbindung-1-Camsylatform B durch alle der folgenden Peaks in einem XRPD-Muster auf einer 2-Theta-Skala, ausgewählt aus 5,25, 10,52, 16,15, 21,13, 21,56 und 21,89, gekennzeichnet ist;
Verbindung-1-Citratform B durch alle der folgenden Peaks in einem XRPD-Muster auf einer 2-Theta-Skala, ausgewählt aus 4,58, 5,95, 8,88, 11,93, 13,79, 14,29, 16,64, 18,90, 21,47, 23,80, 24,25 und 25,85, gekennzeichnet ist;
Verbindung-1-Esylatform A durch alle der folgenden Peaks in einem XRPD-Muster auf einer 2-Theta-Skala, ausgewählt aus 3,97, 7,95, 9,89, 10,62, 13,27, 15,08, 16,17, 16,92, 17,01, 20,65, 20,78, 21,44, 22,08, 23,99, 25,43 und 26,52, gekennzeichnet ist;
Verbindung-1-HBr-Form B durch alle der folgenden Peaks in einem XRPD-Muster auf einer 2-Theta-Skala, ausgewählt aus 5,26, 9,91, 10,72, 20,55, 20,69, 21,18, 22,09, 23,24, 24,56 und 25,80, gekennzeichnet ist;
Verbindung-1-Maleatform A durch alle der folgenden Peaks in einem XRPD-Muster auf einer 2-Theta-Skala, ausgewählt aus 10,92, 15,13, 15,78, 16,43, 18,09, 18,71, 20,30, 21,99, 22,30, 22,49, 23,32, 23,56, 24,79, 26,41, 26,92 und 29,01, gekennzeichnet ist;
Verbindung-1-Mesylatform A durch alle der folgenden Peaks in einem XRPD-Muster auf einer 2-Theta-Skala, ausgewählt aus 5,06, 7,66, 14,79, 15,42, 16,23, 17,20, 17,59, 18,40, 19,33, 19,53, 20,38, 20,73, 21,83, 22,16, 23,14, 24,06 und 24,77, gekennzeichnet ist;
Verbindung-1-Mesylatform B durch alle der folgenden Peaks in einem XRPD-Muster auf einer 2-Theta-Skala, ausgewählt aus 14,61, 15,21, 16,17, 17,18, 17,41, 18,46, 19,13, 19,54, 20,15, 20,53, 20,75, 20,98, 21,27, 21,60, 22,03, 23,32, 24,16, 24,42, 24,87 und 25,13, gekennzeichnet ist;
Verbindung-1-Mesylatform C durch alle der folgenden Peaks in einem XRPD-Muster auf einer 2-Theta-Skala, ausgewählt aus 7,55, 9,53, 11,49, 12,51, 14,44, 19,04, 20,54, 21,12, 21,24 und 23,15, gekennzeichnet ist;
Verbindung-1-Oxalatform A durch alle der folgenden Peaks in einem XRPD-Muster auf einer 2-Theta-Skala, ausgewählt aus 4,90, 5,25, 9,87, 10,54, 12,29, 15,35, 18,67, 19,40, 19,81, 20,26, 22,93 und 24,66, gekennzeichnet ist;
Verbindung-1-Oxalatform B durch alle der folgenden Peaks in einem XRPD-Muster auf einer 2-Theta-Skala, ausgewählt aus 4,20, 7,59, 10,53, 15,06, 15,23, 19,52, 21,18, 21,43, 22,59, 24,21 und 25,85, gekennzeichnet ist;
Verbindung-1-Oxalatform C durch alle der folgenden Peaks in einem XRPD-Muster auf einer 2-Theta-Skala, ausgewählt aus 7,70, 10,29, 10,59, 13,09, 13,70, 15,33, 17,83, 18,15, 20,11, 21,07, 21,91 und 22,56, gekennzeichnet ist;
Verbindung-1-Sulfatform A durch alle der folgenden Peaks in einem XRPD-Muster auf einer 2-Theta-Skala, ausgewählt aus 4,74, 5,85, 9,47, 10,04, 11,74, 11,95, 16,48, 20,22 und 22,74, gekennzeichnet ist;
Verbindung-1-Sulfatform B durch alle der folgenden Peaks in einem XRPD-Muster auf einer 2-Theta-Skala, ausgewählt aus 4,27, 8,57, 9,32, 16,94, 19,99, 21,03, 21,44, 22,00 und 26,02, gekennzeichnet ist;
Verbindung-1-Sulfatform C durch alle der folgenden Peaks in einem XRPD-Muster auf einer 2-Theta-Skala, ausgewählt aus 3,19, 5,35, 9,62, 12,27, 16,33, 17,11, 18,75, 18,99, 19,36, 19,47, 20,55, 21,01, 21,52, 21,62, 21,89, 22,19, 23,79, 25,07 und 26,06, gekennzeichnet ist;
Verbindung-1-Hemitartratform B durch alle der folgenden Peaks in einem XRPD-Muster auf einer 2-Theta-Skala, ausgewählt aus 5,17, 5,66, 10,10, 10,40, 11,37, 11,55, 14,51, 17,09, 21,23, 21,83, 23,53, 24,26, 25,26 und 26,78, gekennzeichnet ist;
Verbindung-1-Tosylatform A durch alle der folgenden Peaks in einem XRPD-Muster auf einer 2-Theta-Skala, ausgewählt aus 8,52, 9,74, 14,28, 16,03, 17,55, 18,94, 19,26, 20,31, 21,02, 21,43, 22,11, 22,44, 22,66, 23,07, 24,55 und 27,70, gekennzeichnet ist; und
Verbindung-1-Tosylatform B durch alle der folgenden Peaks in einem XRPD-Muster auf einer 2-Theta-Skala, ausgewählt aus 4,61, 9,25, 12,44, 15,47, 17,76, 19,01, 19,35, 25,91, 26,33 und 27,63, gekennzeichnet ist.

4. Kristalline Salzform nach einem der Ansprüche 1-3, wobei:
Verbindung-1-Besylatform A durch alle der folgenden Peaks in einem XRPD-Muster auf einer 2-Theta-Skala, ausgewählt aus 3,78, 7,56, 10,08, 10,42, 11,70, 11,78, 12,00, 12,71, 13,20, 13,92, 14,06, 14,44, 14,93, 15,18, 15,96, 16,21, 16,40, 16,73, 16,86, 17,38, 18,32, 19,07, 20,07, 20,26, 20,51, 20,94, 21,45, 21,60, 22,30, 22,90, 23,05, 23,28, 23,71, 24,28, 24,97, 25,30, 25,69, 26,33, 26,71, 27,29, 27,65, 28,07, 29,22 und 29,50, gekennzeichnet ist;
Verbindung-1-Camsylatform A durch alle der folgenden Peaks in einem XRPD-Muster auf einer 2-Theta-Skala, ausgewählt aus 5,55, 7,90, 8,25, 8,38, 9,55, 9,87, 10,26, 11,12, 11,34, 12,50, 12,94, 13,40, 13,65, 14,79, 15,83, 16,16, 16,41, 16,54, 16,82, 17,10, 17,69, 18,02, 18,22, 18,47, 18,91, 19,10, 19,47, 20,04, 20,87, 21,05, 22,16, 22,36, 22,93, 23,71, 24,55, 25,33, 26,02, 26,40, 27,09, 27,33, 27,97, 28,32, 28,68 und 28,98, gekennzeichnet ist;
Verbindung-1-Camsylatform B durch alle der folgenden Peaks in einem XRPD-Muster auf einer 2-Theta-Skala, ausgewählt aus 5,25, 8,32, 8,51, 8,76, 9,05, 9,58, 9,88, 10,00, 10,52, 10,80, 11,06, 11,96, 12,24, 12,58, 13,98, 15,62, 15,81, 16,15, 16,70, 17,12, 17,42, 17,71, 18,41, 19,37, 19,55, 20,63, 21,13, 21,56, 21,89, 22,50, 23,04, 24,43, 26,08, 27,07, 27,97, 28,22 und 28,73, gekennzeichnet ist;
Verbindung-1-Citratform B durch alle der folgenden Peaks in einem XRPD-Muster auf einer 2-Theta-Skala, ausgewählt aus 4,58, 5,95, 7,61, 8,88, 9,19, 9,94, 11,93, 13,26, 13,79, 14,29, 14,68, 15,26, 15,95, 16,64, 17,11, 17,87, 18,21, 18,43, 18,90, 19,36, 19,67, 20,14, 20,57, 21,47, 22,30, 22,64, 23,06, 23,80, 24,25, 24,55, 24,81, 25,10, 25,45, 25,54, 25,85, 26,35, 26,51, 26,83, 27,16, 27,57, 27,85, 28,08 und 28,47, gekennzeichnet ist;
Verbindung-1-Esylatform A durch alle der folgenden Peaks in einem XRPD-Muster auf einer 2-Theta-Skala, ausgewählt aus 3,97, 7,95, 9,89, 10,62, 10,92, 11,03, 11,34, 11,77, 12,03, 12,53, 12,71, 13,27, 14,00, 14,70, 15,08, 15,94, 16,17, 16,92, 17,01, 17,19, 17,35, 17,61, 18,15, 18,76, 19,03, 19,66, 19,89, 20,26, 20,65, 20,78, 21,44, 22,08, 23,14, 23,27, 23,99, 24,32, 25,43, 25,66, 25,85, 26,52, 26,87, 27,61, 27,96, 28,18, 28,40 und 28,91, gekennzeichnet ist;
Verbindung-1-HBr-Form B durch alle der folgenden Peaks in einem XRPD-Muster auf einer 2-Theta-Skala, ausgewählt aus 5,26, 8,29, 9,75, 9,91, 10,56, 10,72, 12,25, 12,50, 12,75, 13,31, 13,54, 14,11, 14,72, 14,89, 15,87, 16,29, 16,50, 17,36, 17,78, 18,09, 18,44, 18,75, 19,12, 19,24, 19,56, 19,92, 20,40, 20,55, 20,69, 21,18, 22,09, 22,64, 23,24, 24,56, 25,80, 26,52, 27,23, 27,43, 28,13, 28,46, 28,76, 29,08, 29,66 und 30,07, gekennzeichnet ist;
Verbindung-1-Maleatform A durch alle der folgenden Peaks in einem XRPD-Muster auf einer 2-Theta-Skala, ausgewählt aus 7,58, 8,34, 10,92, 12,38, 12,87, 14,17, 15,13, 15,78, 16,43, 16,91, 17,51, 18,09, 18,71, 20,30, 21,60, 21,99, 22,30, 22,49, 23,32, 23,56, 23,92, 24,79, 26,04, 26,41, 26,92, 27,62, 28,18, 28,54 und 29,01, gekennzeichnet ist;
Verbindung-1-Mesylatform A durch alle der folgenden Peaks in einem XRPD-Muster auf einer 2-Theta-Skala, ausgewählt aus 5,06, 6,65, 7,66, 8,35, 10,16, 10,72, 11,98, 12,62, 13,32, 14,41, 14,79, 15,42, 15,72, 15,89, 16,23, 16,77, 17,20, 17,59, 18,08, 18,40, 18,81, 19,33, 19,53, 19,71, 20,14, 20,38, 20,73, 21,05, 21,56, 21,83, 22,16, 23,14, 24,06, 24,54, 24,77, 25,75, 26,82, 27,71, 27,94, 28,71, 29,27 und 29,85, gekennzeichnet ist;
Verbindung-1-Mesylatform B durch alle der folgenden Peaks in einem XRPD-Muster auf einer 2-Theta-Skala, ausgewählt aus 5,14, 6,62, 7,72, 7,89, 10,33, 10,73, 12,74, 13,31, 13,88, 14,23, 14,61, 15,21, 15,52, 15,87, 16,17, 17,18, 17,41, 17,88, 18,15, 18,46, 18,73, 19,13, 19,54, 20,15, 20,53, 20,75, 20,98, 21,27, 21,60, 22,03, 22,28, 23,07, 23,32, 24,16, 24,42, 24,87, 25,13, 25,64, 26,24, 26,95, 27,17, 27,49, 27,77, 28,42 und 29,54, gekennzeichnet ist;
Verbindung-1-Mesylatform C durch alle der folgenden Peaks in einem XRPD-Muster auf einer 2-Theta-Skala, ausgewählt aus 7,55, 9,53, 10,25, 11,04, 11,49, 11,65, 12,24, 12,51, 13,88, 14,44, 15,36, 15,66, 16,29, 16,58, 17,16, 17,54, 19,04, 19,20, 19,79, 20,54, 21,12, 21,24, 21,62, 22,22, 23,15, 23,57, 23,99, 24,61, 24,77, 25,22, 25,61, 25,99, 26,60, 26,89, 27,29, 27,91, 29,03 und 29,29, gekennzeichnet ist;
Verbindung-1-Oxalatform A durch alle der folgenden Peaks in einem XRPD-Muster auf einer 2-Theta-Skala, ausgewählt aus 4,90, 5,25, 6,20, 8,05, 9,87, 10,54, 12,29, 14,79, 15,35, 15,84, 16,49, 16,89, 17,55, 18,27, 18,67, 19,12, 19,40, 19,81, 20,26, 20,50, 21,06, 21,74, 22,17, 22,93, 23,26, 23,50, 23,81, 24,66, 25,06, 25,95, 26,90, 27,82 und 28,24, gekennzeichnet ist;
Verbindung-1-Oxalatform B durch alle der folgenden Peaks in einem XRPD-Muster auf einer 2-Theta-Skala, ausgewählt aus 4,20, 7,59, 8,02, 8,42, 10,53, 12,36, 12,66, 13,09, 13,35, 13,88, 14,15, 14,46, 15,06, 15,23, 17,42, 17,75, 18,28, 19,07, 19,52, 20,33, 21,18, 21,43, 21,74, 22,08, 22,59, 23,53, 24,21, 24,52, 24,71, 25,24, 25,85, 26,24, 26,55, 26,90, 27,43, 28,56, 29,30 und 29,51, gekennzeichnet ist;
Verbindung-1-Oxalatform C durch alle der folgenden Peaks in einem XRPD-Muster auf einer 2-Theta-Skala, ausgewählt aus 4,13, 7,70, 10,29, 10,59, 13,09, 13,70, 14,13, 15,33, 15,87, 16,57, 17,19, 17,50, 17,83, 18,15, 18,61, 19,24, 19,42, 20,11, 20,34, 20,64, 21,07, 21,47, 21,59, 21,91, 22,56, 22,92, 23,37, 23,87, 24,35, 25,35, 25,91, 26,47, 26,68, 27,74, 28,47 und 29,14, gekennzeichnet ist;
Verbindung-1-Sulfatform A durch alle der folgenden Peaks in einem XRPD-Muster auf einer 2-Theta-Skala, ausgewählt aus 4,74, 5,85, 9,47, 10,04, 11,74, 11,95, 13,77, 14,99, 15,42, 15,71, 16,48, 17,36, 17,66, 17,92, 18,59, 18,95, 19,36, 19,74, 20,22, 20,77, 21,38, 21,86, 22,74, 23,30, 23,70, 24,33, 25,07, 25,32, 25,67, 26,23 und 27,00, gekennzeichnet ist;
Verbindung-1-Sulfatform B durch alle der folgenden Peaks in einem XRPD-Muster auf einer 2-Theta-Skala, ausgewählt aus 4,27, 8,57, 9,32, 10,95, 11,29, 12,07, 12,87, 13,25, 13,82, 14,13, 14,67, 16,94, 17,21, 17,64, 19,99, 20,26, 21,03, 21,44, 22,00, 22,71, 23,85, 25,20 und 26,02, gekennzeichnet ist;
Verbindung-1-Sulfatform C durch alle der folgenden Peaks in einem XRPD-Muster auf einer 2-Theta-Skala, ausgewählt aus 3,19, 5,35, 9,04, 9,62, 10,73, 11,31, 11,60, 12,27, 12,67, 13,76, 14,31, 14,65, 15,21, 15,63, 16,11, 16,33, 16,63, 17,11, 18,32, 18,75, 18,99, 19,36, 19,47, 20,16, 20,55, 21,01, 21,52, 21,62, 21,89, 22,19, 22,53, 23,19, 23,79, 24,23, 24,81, 25,07, 25,41, 26,06, 26,73, 27,58, 28,16, 28,63, 28,90, 29,22, 29,47 und 29,73, gekennzeichnet ist;
Verbindung-1-Hemitartratform B durch alle der folgenden Peaks in einem XRPD-Muster auf einer 2-Theta-Skala, ausgewählt aus 5,17, 5,66, 7,22, 9,27, 10,10, 10,40, 11,37, 11,55, 12,77, 13,94, 14,51, 15,60, 16,06, 16,48, 17,09, 17,69, 17,90, 18,27, 18,94, 19,15, 19,59, 19,81, 20,06, 20,32, 21,23, 21,83, 22,34, 22,84, 23,53, 24,26, 24,70, 25,26, 25,80, 26,78, 27,29, 27,90, 29,04 und 29,45, gekennzeichnet ist;
Verbindung-1-Tosylatform A durch alle der folgenden Peaks in einem XRPD-Muster auf einer 2-Theta-Skala, ausgewählt aus 5,81, 7,05, 8,52, 9,74, 10,40, 10,99, 11,26, 11,66, 12,58, 12,81, 14,28, 14,62, 15,30, 15,80, 16,03, 16,34, 17,12, 17,55, 18,03, 18,94, 19,26, 19,67, 20,31, 20,65, 21,02, 21,43, 22,11, 22,44, 22,66, 22,94, 23,07, 23,54, 23,79, 24,55, 24,88, 25,42, 25,83, 26,10, 26,53, 27,70, 28,11, 28,62, 29,18, 29,40, 29,66 und 29,83, gekennzeichnet ist; und
Verbindung-1-Tosylatform B durch alle der folgenden Peaks in einem XRPD-Muster auf einer 2-Theta-Skala, ausgewählt aus 4,61, 8,69, 9,25, 10,68, 12,44, 12,69, 13,76, 14,42, 14,81, 15,47, 16,08, 16,57, 17,45, 17,76, 18,44, 18,83, 19,01, 19,35, 20,42, 20,84, 21,15, 21,69, 22,09, 22,86, 23,12, 24,08, 24,53, 24,86, 25,22, 25,91, 26,33, 26,82, 27,63, 28,61, 29,11 und 29,77, gekennzeichnet ist;

5. Kristalline Salzform nach einem der Ansprüche 1-4, wobei:
Verbindung-1-Besylatform A durch ein erstes endothermes Ereignis bei einer Temperatur von etwa 144 °C und ein zweites endothermes Ereignis bei einer Temperatur von etwa 170 °C in einem DSC-Thermogramm gekennzeichnet ist;
Verbindung-1-Camsylatform A durch ein erstes endothermes Ereignis bei einer Temperatur von etwa 110 °C und ein zweites endothermes Ereignis bei einer Temperatur von etwa 174 °C in einem DSC-Thermogramm gekennzeichnet ist;
Verbindung-1-Citratform B durch ein endothermes Ereignis mit einer Einsetztemperatur von etwa 175 °C in einem DSC-Thermogramm gekennzeichnet ist;
Verbindung-1-Esylatform A durch ein erstes endothermes Ereignis bei einer Temperatur von etwa 69 °C, ein zweites endothermes Ereignis bei etwa 156 °C und ein drittes endothermes Ereignis bei einer Temperatur von etwa 190 °C in einem DSC-Thermogramm gekennzeichnet ist;
Verbindung-1-Maleatform A durch ein endothermes Ereignis bei einer Temperatur von etwa 117 °C in einem DSC-Thermogramm gekennzeichnet ist;
Verbindung-1-Mesylatform C durch ein erstes endothermes Ereignis mit einem Peak bei einer Temperatur von etwa 69 °C, wobei das erste endotherme Ereignis eine Untergrenze von etwa 36 und eine Obergrenze einer Temperatur von etwa 96 °C aufweist, und ein zweites endothermes Ereignis mit einem Peak bei einer Temperatur von etwa 208 °C, wobei das zweite endotherme Ereignis eine Untergrenze einer Temperatur von etwa 169 und eine Obergrenze einer Temperatur von etwa 219 °C aufweist, gekennzeichnet ist;
Verbindung-1-Hemitartratform B durch ein erstes endothermes Ereignis bei einer Temperatur von etwa 111 °C und ein zweites endothermes Ereignis bei einer Temperatur von etwa 148 °C in einem DSC-Thermogramm gekennzeichnet ist;
Verbindung-1-Tosylatform A durch ein endothermes Ereignis mit einer Einsetztemperatur von etwa 214 °C in einem DSC-Thermogramm gekennzeichnet ist; und
Verbindung-1-Tosylatform B durch ein starkes endothermes Ereignis mit einer Einsetztemperatur von ca. 183 °C in einem DSC-Thermogramm gekennzeichnet ist.

6. Kristalline Salzform nach einem der Ansprüche 1-5, wobei:
Verbindung-1-Besylatform A durch einen Gewichtsverlust von etwa 7,9% über einen Temperaturbereich von 39-177 °C in einem TGA-Thermogramm gekennzeichnet ist;
Verbindung-1-Camsylatform A durch einen Gewichtsverlust von etwa 5,9% über einen Temperaturbereich von 38-170 °C in einem TGA-Thermogramm gekennzeichnet ist;
Verbindung-1-Citratform B durch einen Gewichtsverlust, der bei einer Temperatur von etwa 159 °C beginnt und bei etwa 220 °C endet, in einem TGA-Thermogramm gekennzeichnet ist;
Verbindung-1-Esylatform A durch einen Gewichtsverlust von etwa 8,5 Gew.-% über einen Temperaturbereich von 38-188 °C in einem TGA-Thermogramm gekennzeichnet ist;
Verbindung-1-Maleatform A durch einen Gewichtsverlust von etwa 3,1 Gew.-% im Temperaturbereich von 42-146 °C in einem TGA-Thermogramm gekennzeichnet ist;
Verbindung-1-Mesylatform C durch einen Gewichtsverlust von 0,5 bis 3,0 Gew.-% im Temperaturbereich von 36-130 °C in einem TGA-Thermogramm gekennzeichnet ist;
Verbindung-1-Hemitartratform B durch einen Gewichtsverlust von etwa 7,4 Gew.-% im Temperaturbereich von 39-157 °C in einem TGA-Thermogramm gekennzeichnet ist;
Verbindung-1-Tosylatform A durch einen Gewichtsverlust von etwa 0,9 Gew.-% im Temperaturbereich von 72-231 °C in einem TGA-Thermogramm gekennzeichnet ist; und
Verbindung-1-Tosylatform B durch einen Gewichtsverlust von etwa 0.7 Gew.-% im Temperaturbereich von 39-214 °C in einem TGA-Thermogramm gekennzeichnet ist.

7. Kristalline Salzform nach einem der Ansprüche 1-6, wobei:
Verbindung-1-Citratform B durch eine Zunahme von etwa 4,1 Gew.-%, gemessen mittels DVS, bei Verbringen von einer Umgebung mit 5% relativer Feuchte in eine Umgebung mit 95% relativer Feuchte, gekennzeichnet ist;
Verbindung-1-Maleatform A durch eine Zunahme von etwa 2,5 Gew.-%, gemessen mittels DVS, bei Verbringen von einer Umgebung mit 5% relativer Feuchte in eine Umgebung mit 95% relativer Feuchte, gekennzeichnet ist;
Verbindung-1-Mesylatform C durch eine Zunahme von etwa 5,1 Gew.-%, gemessen mittels DVS, bei Verbringen von einer Umgebung mit 5% relativer Feuchte in eine Umgebung mit 95% relativer Feuchte, gekennzeichnet ist;
Verbindung-1-Tosylatform A durch eine Zunahme von etwa 0,9 Gew.-%, gemessen mittels DVS, bei Verbringen von einer Umgebung mit 5% relativer Feuchte in eine Umgebung mit 95% relativer Feuchte, gekennzeichnet ist; und
Verbindung-1-Tosylatform B durch eine Zunahme von etwa 0,9 Gew.-%, gemessen mittels DVS, bei Verbringen von einer Umgebung mit 5% relativer Feuchte in eine Umgebung mit 95% relativer Feuchte, gekennzeichnet ist.

8. Eine pharmazeutische Zusammensetzung, die eine kristalline Salzfor, nach einem der Ansprüche 1-7 und einen pharmazeutisch akzeptablen Hilfsstoff umfasst.

9. Eine kristalline Salzform nach einem der Ansprüche 1-7 oder eine pharmazeutische Zusammensetzung nach Anspruch 8 zur Verwendung in einem Verfahren zur Behandlung einer Krankheit, einer Erkrankung oder eines Syndroms, die oder das zumindest teilweise durch Modulation der In-vivo-Aktivität einer Proteinkinase vermittelt wird, wobei die Krankheit, die Erkrankung oder das Syndrom Krebs ist.

## Revendications

1. Forme de sel cristallin d'un Composé 1 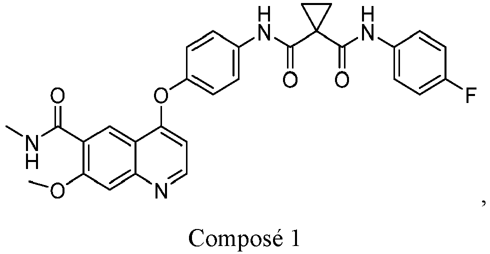 sélectionné dans le groupe constitué du Composé 1 bésylate de forme A, du Composé 1 bésylate de forme B, du Composé 1 benzoate de forme A, du Composé 1 camsylate de forme A, du Composé 1 camsylate de forme B, du Composé 1 citrate de forme A, du Composé 1 citrate de forme B, du Composé 1 ésylate de forme A, du Composé 1 ésylate de forme B, du Composé 1 HBr de forme A, du Composé 1 HBr de forme B, du Composé 1 lactate de forme A, du Composé 1 malate de forme A, du Composé 1 maléate de forme A, du Composé 1 maléate de forme B, du Composé 1 mésylate de forme A, du Composé 1 mésylate de forme B, du Composé 1 mésylate de forme C, du Composé 1 oxalate de forme A, du Composé 1 oxalate de forme B, du Composé 1 oxalate de forme C, du Composé 1 propionate de forme A, du Composé 1 succinate de forme A, du Composé 1 sulfate de forme A, du Composé 1 sulfate de forme B, du Composé 1 sulfate de forme C, du Composé 1 tartrate de forme A, du Composé 1 hémitartrate de forme B, du Composé 1 tartrate de forme C, du Composé 1 tosylate de forme A, et du Composé 1 tosylate de forme B, dans laquelle:
le Composé 1 bésylate de forme A est **caractérisé par** un ou plusieurs pics dans un diagramme de diffraction sur poudre des rayons X (XRPD) en échelle 2 thêta, les un ou plusieurs pics étant sélectionnés parmi 3,78, 7,56, 10,08, 10,42, 11,70, 11,78, 12,00, 12,71, 13,20, 13,92, 14,06, 14,44, 14,93, 15,18, 15,96, 16,21, 16,40, 16,73, 16,86, 17,38, 18,32, 19,07, 20,07, 20,26, 20,51, 20,94, 21,45, 21,60, 22,30, 22,90, 23,05, 23,28, 23,71, 24,28, 24,97, 25,30, 25,69, 26,33, 26,71, 27,29, 27,65, 28,07, 29,22, et 29,50,
le Composé 1 bésylate de forme B est **caractérisé par** le diagramme de XRPD présenté dans la Figure 4;
le Composé 1 benzoate de forme A est **caractérisé par** le diagramme de XRPD présenté dans la Figure 5;
le Composé 1 camsylate de forme A est **caractérisé par** un ou plusieurs pics dans un diagramme de XRPD en échelle 2 thêta, les un ou plusieurs pics étant sélectionnés parmi 5,55, 7,90, 8,25, 8,38, 9,55, 9,87, 10,26, 11,12, 11,34, 12,50, 12,94, 13,40, 13,65, 14,79, 15,83, 16,16, 16,41, 16,54, 16,82, 17,10, 17,69, 18,02, 18,22, 18,47, 18,91, 19,10, 19,47, 20,04, 20,87, 21,05, 22,16, 22,36, 22,93, 23,71, 24,55, 25,33, 26,02, 26,40, 27,09, 27,33, 27,97, 28,32, 28,68, et 28,98;
le Composé 1 camsylate de forme B est **caractérisé par** un ou plusieurs pics dans un diagramme de XRPD en échelle 2 thêta, les un ou plusieurs pics étant sélectionnés parmi 5,25, 8,32, 8,51, 8,76, 9,05, 9,58, 9,88, 10,00, 10,52, 10,80, 11,06, 11,96, 12,24, 12,58, 13,98, 15,62, 15,81, 16,15, 16,70, 17,12, 17,42, 17,71, 18,41, 19,37, 19,55, 20,63, 21,13, 21,56, 21,89, 22,50, 23,04, 24,43, 26,08, 27,07, 27,97, 28,22, et 28,73;
le Composé 1 citrate de forme A est **caractérisé par** le diagramme de XRPD présenté dans la Figure 10;
le Composé 1 citrate de forme B est **caractérisé par** un ou plusieurs pics dans un diagramme de XRPD en échelle 2 thêta, les un ou plusieurs pics étant sélectionnés parmi 4,58, 5,95, 7,61, 8,88, 9,19, 9,94, 11,93, 13,26, 13,79, 14,29, 14,68, 15,26, 15,95, 16,64, 17,11, 17,87, 18,21, 18,43, 18,90, 19,36, 19,67, 20,14, 20,57, 21,47, 22,30, 22,64, 23,06, 23,80, 24,25, 24,55, 24,81, 25,10, 25,45, 25,54, 25,85, 26,35, 26,51, 26,83, 27,16, 27,57, 27,85, 28,08, et 28,47;
le Composé 1 ésylate de forme A est **caractérisé par** un ou plusieurs pics dans un diagramme de XRPD en échelle 2 thêta, les un ou plusieurs pics étant sélectionnés parmi 3,97, 7,95, 9,89, 10,62, 10,92, 11,03, 11,34, 11,77, 12,03, 12,53, 12,71, 13,27, 14,00, 14,70, 15,08, 15,94, 16,17, 16,92, 17,01, 17,19, 17,35, 17,61, 18,15, 18,76, 19,03, 19,66, 19,89, 20,26, 20,65, 20,78, 21,44, 22,08, 23,14, 23,27, 23,99, 24,32, 25,43, 25,66, 25,85, 26,52, 26,87, 27,61, 27,96, 28,18, 28,40, et 28,91;
le Composé 1 ésylate de forme B est **caractérisé par** le diagramme de XRPD présenté dans la Figure 18;
le Composé 1 HBr de forme A est **caractérisé par** le diagramme de XRPD présenté dans la Figure 19;
le Composé 1 HBr de forme B est **caractérisé par** un ou plusieurs pics dans un diagramme de XRPD en échelle 2 thêta, les un ou plusieurs pics étant sélectionnés parmi 5,26, 8,29, 9,75, 9,91, 10,56, 10,72, 12,25, 12,50, 12,75, 13,31, 13,54, 14,11, 14,72, 14,89, 15,87, 16,29, 16,50, 17,36, 17,78, 18,09, 18,44, 18,75, 19,12, 19,24, 19,56, 19,92, 20,40, 20,55, 20,69, 21,18, 22,09, 22,64, 23,24, 24,56, 25,80, 26,52, 27,23, 27,43, 28,13, 28,46, 28,76, 29,08, 29,66, et 30,07;
le Composé 1 lactate de forme A est **caractérisé par** le diagramme de XRPD présenté dans la Figure 21;
le Composé 1 malate de forme A est **caractérisé par** le diagramme de XRPD présenté dans la Figure 22;
le Composé 1 maléate de forme A est **caractérisé par** un ou plusieurs pics dans un diagramme de XRPD en échelle 2 thêta, les un ou plusieurs pics étant sélectionnés parmi 7,58, 8,34, 10,92, 12,38, 12,87, 14,17, 15,13, 15,78, 16,43, 16,91, 17,51, 18,09, 18,71, 20,30, 21,60, 21,99, 22,30, 22,49, 23,32, 23,56, 23,92, 24,79, 26,04, 26,41, 26,92, 27,62, 28,18, 28,54, et 29,01;
le Composé 1 maléate de forme B est **caractérisé par** le diagramme de XRPD présenté dans la Figure 28;
le Composé 1 mésylate de forme A est **caractérisé par** un ou plusieurs pics dans un diagramme de XRPD en échelle 2 thêta, les un ou plusieurs pics étant sélectionnés parmi 5,06, 6,65, 7,66, 8,35, 10,16, 10,72, 11,98, 12,62, 13,32, 14,41, 14,79, 15,42, 15,72, 15,89, 16,23, 16,77, 17,20, 17,59, 18,08, 18,40, 18,81, 19,33, 19,53, 19,71, 20,14, 20,38, 20,73, 21,05, 21,56, 21,83, 22,16, 23,14, 24,06, 24,54, 24,77, 25,75, 26,82, 27,71, 27,94, 28,71, 29,27, et 29,85;
le Composé 1 mésylate de forme B est **caractérisé par** un ou plusieurs pics dans un diagramme de XRPD en échelle 2 thêta, les un ou plusieurs pics étant sélectionnés parmi 5,14, 6,62, 7,72, 7,89, 10,33, 10,73, 12,74, 13,31, 13,88, 14,23, 14,61, 15,21, 15,52, 15,87, 16,17, 17,18, 17,41, 17,88, 18,15, 18,46, 18,73, 19,13, 19,54, 20,15, 20,53, 20,75, 20,98, 21,27, 21,60, 22,03, 22,28, 23,07, 23,32, 24,16, 24,42, 24,87, 25,13, 25,64, 26,24, 26,95, 27,17, 27,49, 27,77, 28,42, et 29,54;
le Composé 1 mésylate de forme C est **caractérisé par** un ou plusieurs pics dans un diagramme de XRPD en échelle 2 thêta, les un ou plusieurs pics étant sélectionnés parmi 7,55, 9,53, 10,25, 11,04, 11,49, 11,65, 12,24, 12,51, 13,88, 14,44, 15,36, 15,66, 16,29, 16,58, 17,16, 17,54, 19,04, 19,20, 19,79, 20,54, 21,12, 21,24, 21,62, 22,22, 23,15, 23,57, 23,99, 24,61, 24,77, 25,22, 25,61, 25,99, 26,60, 26,89, 27,29, 27,91, 29,03, et 29,29;
le Composé 1 oxalate de forme A est **caractérisé par** un ou plusieurs pics dans un diagramme de XRPD en échelle 2 thêta, les un ou plusieurs pics étant sélectionnés parmi 4,90, 5,25, 6,20, 8,05, 9,87, 10,54, 12,29, 14,79, 15,35, 15,84, 16,49, 16,89, 17,55, 18,27, 18,67, 19,12, 19,40, 19,81, 20,26, 20,50, 21,06, 21,74, 22,17, 22,93, 23,26, 23,50, 23,81, 24,66, 25,06, 25,95, 26,90, 27,82, et 28,24;
le Composé 1 oxalate de forme B est **caractérisé par** un ou plusieurs pics dans un diagramme de XRPD en échelle 2 thêta, les un ou plusieurs pics étant sélectionnés parmi 4,20, 7,59, 8,02, 8,42, 10,53, 12,36, 12,66, 13,09, 13,35, 13,88, 14,15, 14,46, 15,06, 15,23, 17,42, 17,75, 18,28, 19,07, 19,52, 20,33, 21,18, 21,43, 21,74, 22,08, 22,59, 23,53, 24,21, 24,52, 24,71, 25,24, 25,85, 26,24, 26,55, 26,90, 27,43, 28,56, 29,30, et 29,51;
le Composé 1 oxalate de forme C est **caractérisé par** un ou plusieurs pics dans un diagramme de XRPD en échelle 2 thêta, les un ou plusieurs pics étant sélectionnés parmi 4,13, 7,70, 10,29, 10,59, 13,09, 13,70, 14,13, 15,33, 15,87, 16,57, 17,19, 17,50, 17,83, 18,15, 18,61, 19,24, 19,42, 20,11, 20,34, 20,64, 21,07, 21,47, 21,59, 21,91, 22,56, 22,92, 23,37, 23,87, 24,35, 25,35, 25,91, 26,47, 26,68, 27,74, 28,47, et 29,14;
le Composé 1 propionate de forme A est **caractérisé par** le diagramme de XRPD présenté dans la Figure 38;
le Composé 1 succinate de forme A est **caractérisé par** le diagramme de XRPD présenté dans la Figure 39;
le Composé 1 sulfate de forme A est **caractérisé par** un ou plusieurs pics dans un diagramme de XRPD en échelle 2 thêta, les un ou plusieurs pics étant sélectionnés parmi 4,74, 5,85, 9,47, 10,04, 11,74, 11,95, 13,77, 14,99, 15,42, 15,71, 16,48, 17,36, 17,66, 17,92, 18,59, 18,95, 19,36, 19,74, 20,22, 20,77, 21,38, 21,86, 22,74, 23,30, 23,70, 24,33, 25,07, 25,32, 25,67, 26,23, et 27,00;
le Composé 1 sulfate de forme B est **caractérisé par** un ou plusieurs pics dans un diagramme de XRPD en échelle 2 thêta, les un ou plusieurs pics étant sélectionnés parmi 4,27, 8,57, 9,32, 10,95, 11,29, 12,07, 12,87, 13,25, 13,82, 14,13, 14,67, 16,94, 17,21, 17,64, 19,99, 20,26, 21,03, 21,44, 22,00, 22,71, 23,85, 25,20, et 26,02;
le Composé 1 sulfate de forme C est **caractérisé par** un ou plusieurs pics dans un diagramme de XRPD en échelle 2 thêta, les un ou plusieurs pics étant sélectionnés parmi 3,19, 5,35, 9,04, 9,62, 10,73, 11,31, 11,60, 12,27, 12,67, 13,76, 14,31, 14,65, 15,21, 15,63, 16,11, 16,33, 16,63, 17,11, 18,32, 18,75, 18,99, 19,36, 19,47, 20,16, 20,55, 21,01, 21,52, 21,62, 21,89, 22,19, 22,53, 23,19, 23,79, 24,23, 24,81, 25,07, 25,41, 26,06, 26,73, 27,58, 28,16, 28,63, 28,90, 29,22, 29,47, et 29,73;
le Composé 1 tartrate de forme A est **caractérisé par** le diagramme de XRPD présenté dans la Figure 43;
le Composé 1 hémitartrate de forme B est **caractérisé par** un ou plusieurs pics dans un diagramme de XRPD en échelle 2 thêta, les un ou plusieurs pics étant sélectionnés parmi 5,17, 5,66, 7,22, 9,27, 10,10, 10,40, 11,37, 11,55, 12,77, 13,94, 14,51, 15,60, 16,06, 16,48, 17,09, 17,69, 17,90, 18,27, 18,94, 19,15, 19,59, 19,81, 20,06, 20,32, 21,23, 21,83, 22,34, 22,84, 23,53, 24,26, 24,70, 25,26, 25,80, 26,78, 27,29, 27,90, 29,04, et 29,45;
le Composé 1 tartrate de forme C est **caractérisé par** le diagramme de XRPD présenté dans la Figure 69;
le Composé 1 tosylate de forme A est **caractérisé par** un ou plusieurs pics dans un diagramme de XRPD en échelle 2 thêta, les un ou plusieurs pics étant sélectionnés parmi 5,81, 7,05, 8,52, 9,74, 10,40, 10,99, 11,26, 11,66, 12,58, 12,81, 14,28, 14,62, 15,30, 15,80, 16,03, 16,34, 17,12, 17,55, 18,03, 18,94, 19,26, 19,67, 20,31, 20,65, 21,02, 21,43, 22,11, 22,44, 22,66, 22,94, 23,07, 23,54, 23,79, 24,55, 24,88, 25,42, 25,83, 26,10, 26,53, 27,70, 28,11, 28,62, 29,18, 29,40, 29,66, et 29,83; et
le Composé 1 tosylate de forme B est **caractérisé par** un ou plusieurs pics dans un diagramme de XRPD en échelle 2 thêta, les un ou plusieurs pics étant sélectionnés parmi 4,61, 8,69, 9,25, 10,68, 12,44, 12,69, 13,76, 14,42, 14,81, 15,47, 16,08, 16,57, 17,45, 17,76, 18,44, 18,83, 19,01, 19,35, 20,42, 20,84, 21,15, 21,69, 22,09, 22,86, 23,12, 24,08, 24,53, 24,86, 25,22, 25,91, 26,33, 26,82, 27,63, 28,61, 29,11, et 29,77.

2. Forme de sel cristallin selon la revendication 1, dans laquelle:
le Composé 1 bésylate de forme A est **caractérisé par** un ou plusieurs pics dans un diagramme de XRPD en échelle 2 thêta, les un ou plusieurs pics étant sélectionnés parmi 3,78, 7,56, 10,42, 20,51, 20,94, 21,60, et 25,30;
le Composé 1 camsylate de forme A est **caractérisé par** un ou plusieurs pics dans un diagramme de XRPD en échelle 2 thêta, les un ou plusieurs pics étant sélectionnés parmi 5,55, 9,87, 10,26, 16,16, 16,82, 20,87, 22,36, et 23,71;
le Composé 1 camsylate de forme B est **caractérisé par** un ou plusieurs pics dans un diagramme de XRPD en échelle 2 thêta, les un ou plusieurs pics étant sélectionnés parmi 5,25, 10,52, 16,15, 21,13, 21,56, et 21,89;
le Composé 1 citrate de forme B est **caractérisé par** un ou plusieurs pics dans un diagramme de XRPD en échelle 2 thêta, les un ou plusieurs pics étant sélectionnés parmi 4,58, 5,95, 8,88, 11,93, 13,79, 14,29, 16,64, 18,90, 21,47, 23,80, 24,25, et 25,85;
le Composé 1 ésylate de forme A est **caractérisé par** un ou plusieurs pics dans un diagramme de XRPD en échelle 2 thêta, les un ou plusieurs pics étant sélectionnés parmi 3,97, 7,95, 9,89, 10,62, 13,27, 15,08, 16,17, 16,92, 17,01, 20,65, 20,78, 21,44, 22,08, 23,99, 25,43, et 26,52;
le Composé 1 HBr de forme B est **caractérisé par** un ou plusieurs pics dans un diagramme de XRPD en échelle 2 thêta, les un ou plusieurs pics étant sélectionnés parmi 5,26, 9,91, 10,72, 20,55, 20,69, 21,18, 22,09, 23,24, 24,56, et 25,80;
le Composé 1 maléate de forme A est **caractérisé par** un ou plusieurs pics dans un diagramme de XRPD en échelle 2 thêta, les un ou plusieurs pics étant sélectionnés parmi 10,92, 15,13, 15,78, 16,43, 18,09, 18,71, 20,30, 21,99, 22,30, 22,49, 23,32, 23,56, 24,79, 26,41, 26,92, et 29,01;
le Composé 1 mésylate de forme A est **caractérisé par** un ou plusieurs pics dans un diagramme de XRPD en échelle 2 thêta, les un ou plusieurs pics étant sélectionnés parmi 5,06, 7,66, 14,79, 15,42, 16,23, 17,20, 17,59, 18,40, 19,33, 19,53, 20,38, 20,73, 21,83, 22,16, 23,14, 24,06, et 24,77;
le Composé 1 mésylate de forme B est **caractérisé par** un ou plusieurs pics dans un diagramme de XRPD en échelle 2 thêta, les un ou plusieurs pics étant sélectionnés parmi 14,61, 15,21, 16,17, 17,18, 17,41, 18,46, 19,13, 19,54, 20,15, 20,53, 20,75, 20,98, 21,27, 21,60, 22,03, 23,32, 24,16, 24,42, 24,87, et 25,13;
le Composé 1 mésylate de forme C est **caractérisé par** un ou plusieurs pics dans un diagramme de XRPD en échelle 2 thêta, les un ou plusieurs pics étant sélectionnés parmi 7,55, 9,53, 11,49, 12,51, 14,44, 19,04, 20,54, 21,12, 21,24, et 23,15;
le Composé 1 oxalate de forme A est **caractérisé par** un ou plusieurs pics dans un diagramme de XRPD en échelle 2 thêta, les un ou plusieurs pics étant sélectionnés parmi 4,90, 5,25, 9,87, 10,54, 12,29, 15,35, 18,67, 19,40, 19,81, 20,26, 22,93, et 24,66;
le Composé 1 oxalate de forme B est **caractérisé par** un ou plusieurs pics dans un diagramme de XRPD en échelle 2 thêta, les un ou plusieurs pics étant sélectionnés parmi 4,20, 7,59, 10,53, 15,06, 15,23, 19,52, 21,18, 21,43, 22,59, 24,21, et 25,85;
le Composé 1 oxalate de forme C est **caractérisé par** un ou plusieurs pics dans un diagramme de XRPD en échelle 2 thêta, les un ou plusieurs pics étant sélectionnés parmi 7,70, 10,29, 10,59, 13,09, 13,70, 15,33, 17,83, 18,15, 20,11, 21,07, 21,91, et 22,56;
le Composé 1 sulfate de forme A est **caractérisé par** un ou plusieurs pics dans un diagramme de XRPD en échelle 2 thêta, les un ou plusieurs pics étant sélectionnés parmi 4,74, 5,85, 9,47, 10,04, 11,74, 11,95, 16,48, 20,22, et 22,74;
le Composé 1 sulfate de forme B est **caractérisé par** un ou plusieurs pics dans un diagramme de XRPD en échelle 2 thêta, les un ou plusieurs pics étant sélectionnés parmi 4,27, 8,57, 9,32, 16,94, 19,99, 21,03, 21,44, 22,00, et 26,02;
le Composé 1 sulfate de forme C est **caractérisé par** un ou plusieurs pics dans un diagramme de XRPD en échelle 2 thêta, les un ou plusieurs pics étant sélectionnés parmi 3,19, 5,35, 9,62, 12,27, 16,33, 17,11, 18,75, 18,99, 19,36, 19,47, 20,55, 21,01, 21,52, 21,62, 21,89, 22,19, 23,79, 25,07, et 26,06;
le Composé 1 hémitartrate de forme B est **caractérisé par** un ou plusieurs pics dans un diagramme de XRPD en échelle 2 thêta, les un ou plusieurs pics étant sélectionnés parmi 5,17, 5,66, 10,10, 10,40, 11,37, 11,55, 14,51, 17,09, 21,23, 21,83, 23,53, 24,26, 25,26, et 26,78;
le Composé 1 tosylate de forme A est **caractérisé par** un ou plusieurs pics dans un diagramme de XRPD en échelle 2 thêta, les un ou plusieurs pics étant sélectionnés parmi 8,52, 9,74, 14,28, 16,03, 17,55, 18,94, 19,26, 20,31, 21,02, 21,43, 22,11, 22,44, 22,66, 23,07, 24,55, et 27,70; et
le Composé 1 tosylate de forme B est **caractérisé par** un ou plusieurs pics dans un diagramme de XRPD en échelle 2 thêta, les un ou plusieurs pics étant sélectionnés parmi 4,61, 9,25, 12,44, 15,47, 17,76, 19,01, 19,35, 25,91, 26,33, et 27,63.

3. Forme de sel cristallin selon la revendication 1 ou 2, dans laquelle:
le Composé 1 bésylate de forme A est **caractérisé par** tous les pics suivants dans un diagramme de XRPD en échelle 2 thêta, sélectionnés parmi 3,78, 7,56, 10,42, 20,51, 20,94, 21,60, et 25,30;
le Composé 1 camsylate de forme A est **caractérisé par** tous les pics suivants dans un diagramme de XRPD en échelle 2 thêta, sélectionnés parmi 5,55, 9,87, 10,26, 16,16, 16,82, 20,87, 22,36, et 23,71;
le Composé 1 camsylate de forme B est **caractérisé par** tous les pics suivants dans un diagramme de XRPD en échelle 2 thêta, sélectionnés parmi 5,25, 10,52, 16,15, 21,13, 21,56, et 21,89;
le Composé 1 citrate de forme B est **caractérisé par** tous les pics suivants dans un diagramme de XRPD en échelle 2 thêta, sélectionnés parmi 4,58, 5,95, 8,88, 11,93, 13,79, 14,29, 16,64, 18,90, 21,47, 23,80, 24,25, et 25,85;
le Composé 1 ésylate de forme A est **caractérisé par** tous les pics suivants dans un diagramme de XRPD en échelle 2 thêta, sélectionnés parmi 3,97, 7,95, 9,89, 10,62, 13,27, 15,08, 16,17, 16,92, 17,01, 20,65, 20,78, 21,44, 22,08, 23,99, 25,43, et 26,52;
le Composé 1 HBr de forme B est **caractérisé par** tous les pics suivants dans un diagramme de XRPD en échelle 2 thêta, sélectionnés parmi 5,26, 9,91, 10,72, 20,55, 20,69, 21,18, 22,09, 23,24, 24,56, et 25,80;
le Composé 1 maléate de forme A est **caractérisé par** tous les pics suivants dans un diagramme de XRPD en échelle 2 thêta, sélectionnés parmi 10,92, 15,13, 15,78, 16,43, 18,09, 18,71, 20,30, 21,99, 22,30, 22,49, 23,32, 23,56, 24,79, 26,41, 26,92, et 29,01;
le Composé 1 mésylate de forme A est **caractérisé par** tous les pics suivants dans un diagramme de XRPD en échelle 2 thêta, sélectionnés parmi 5,06, 7,66, 14,79, 15,42, 16,23, 17,20, 17,59, 18,40, 19,33, 19,53, 20,38, 20,73, 21,83, 22,16, 23,14, 24,06, et 24,77;
le Composé 1 mésylate de forme B est **caractérisé par** tous les pics suivants dans un diagramme de XRPD en échelle 2 thêta, sélectionnés parmi 14,61, 15,21, 16,17, 17,18, 17,41, 18,46, 19,13, 19,54, 20,15, 20,53, 20,75, 20,98, 21,27, 21,60, 22,03, 23,32, 24,16, 24,42, 24,87, et 25,13;
le Composé 1 mésylate de forme C est **caractérisé par** tous les pics suivants dans un diagramme de XRPD en échelle 2 thêta, sélectionnés parmi 7,55, 9,53, 11,49, 12,51, 14,44, 19,04, 20,54, 21,12, 21,24, et 23,15;
le Composé 1 oxalate de forme A est **caractérisé par** tous les pics suivants dans un diagramme de XRPD en échelle 2 thêta, sélectionnés parmi 4,90, 5,25, 9,87, 10,54, 12,29, 15,35, 18,67, 19,40, 19,81, 20,26, 22,93, et 24,66;
le Composé 1 oxalate de forme B est **caractérisé par** tous les pics suivants dans un diagramme de XRPD en échelle 2 thêta, sélectionnés parmi 4,20, 7,59, 10,53, 15,06, 15,23, 19,52, 21,18, 21,43, 22,59, 24,21, et 25,85;
le Composé 1 oxalate de forme C est **caractérisé par** tous les pics suivants dans un diagramme de XRPD en échelle 2 thêta, sélectionnés parmi 7,70, 10,29, 10,59, 13,09, 13,70, 15,33, 17,83, 18,15, 20,11, 21,07, 21,91, et 22,56;
le Composé 1 sulfate de forme A est **caractérisé par** tous les pics suivants dans un diagramme de XRPD en échelle 2 thêta, sélectionnés parmi 4,74, 5,85, 9,47, 10,04, 11,74, 11,95, 16,48, 20,22, et 22,74;
le Composé 1 sulfate de forme B est **caractérisé par** tous les pics suivants dans un diagramme de XRPD en échelle 2 thêta, sélectionnés parmi 4,27, 8,57, 9,32, 16,94, 19,99, 21,03, 21,44, 22,00, et 26,02;
le Composé 1 sulfate de forme C est **caractérisé par** tous les pics suivants dans un diagramme de XRPD en échelle 2 thêta, sélectionnés parmi 3,19, 5,35, 9,62, 12,27, 16,33, 17,11, 18,75, 18,99, 19,36, 19,47, 20,55, 21,01, 21,52, 21,62, 21,89, 22,19, 23,79, 25,07, et 26,06;
le Composé 1 hémitartrate de forme B est **caractérisé par** tous les pics suivants dans un diagramme de XRPD en échelle 2 thêta, sélectionnés parmi 5,17, 5,66, 10,10, 10,40, 11,37, 11,55, 14,51, 17,09, 21,23, 21,83, 23,53, 24,26, 25,26, et 26,78;
le Composé 1 tosylate de forme A est **caractérisé par** tous les pics suivants dans un diagramme de XRPD en échelle 2 thêta, sélectionnés parmi 8,52, 9,74, 14,28, 16,03, 17,55, 18,94, 19,26, 20,31, 21,02, 21,43, 22,11, 22,44, 22,66, 23,07, 24,55, et 27,70; et
le Composé 1 tosylate de forme B est **caractérisé par** tous les pics suivants dans un diagramme de XRPD en échelle 2 thêta, sélectionnés parmi 4,61, 9,25, 12,44, 15,47, 17,76, 19,01, 19,35, 25,91, 26,33, et 27,63.

4. Forme de sel cristallin selon l'une quelconque des revendications 1 à 3, dans laquelle:
le Composé 1 bésylate de forme A est **caractérisé par** tous les pics suivants dans un diagramme de XRPD en échelle 2 thêta, sélectionnés parmi 3,78, 7,56, 10,08, 10,42, 11,70, 11,78, 12,00, 12,71, 13,20, 13,92, 14,06, 14,44, 14,93, 15,18, 15,96, 16,21, 16,40, 16,73, 16,86, 17,38, 18,32, 19,07, 20,07, 20,26, 20,51, 20,94, 21,45, 21,60, 22,30, 22,90, 23,05, 23,28, 23,71, 24,28, 24,97, 25,30, 25,69, 26,33, 26,71, 27,29, 27,65, 28,07, 29,22, et 29,50;
le Composé 1 camsylate de forme A est **caractérisé par** tous les pics suivants dans un diagramme de XRPD en échelle 2 thêta, sélectionnés parmi 5,55, 7,90, 8,25, 8,38, 9,55, 9,87, 10,26, 11,12, 11,34, 12,50, 12,94, 13,40, 13,65, 14,79, 15,83, 16,16, 16,41, 16,54, 16,82, 17,10, 17,69, 18,02, 18,22, 18,47, 18,91, 19,10, 19,47, 20,04, 20,87, 21,05, 22,16, 22,36, 22,93, 23,71, 24,55, 25,33, 26,02, 26,40, 27,09, 27,33, 27,97, 28,32, 28,68, et 28,98;
le Composé 1 camsylate de forme B est **caractérisé par** tous les pics suivants dans un diagramme de XRPD en échelle 2 thêta, sélectionnés parmi 5,25, 8,32, 8,51, 8,76, 9,05, 9,58, 9,88, 10,00, 10,52, 10,80, 11,06, 11,96, 12,24, 12,58, 13,98, 15,62, 15,81, 16,15, 16,70, 17,12, 17,42, 17,71, 18,41, 19,37, 19,55, 20,63, 21,13, 21,56, 21,89, 22,50, 23,04, 24,43, 26,08, 27,07, 27,97, 28,22, et 28,73;
le Composé 1 citrate de forme B est **caractérisé par** tous les pics suivants dans un diagramme de XRPD en échelle 2 thêta, sélectionnés parmi 4,58, 5,95, 7,61, 8,88, 9,19, 9,94, 11,93, 13,26, 13,79, 14,29, 14,68, 15,26, 15,95, 16,64, 17,11, 17,87, 18,21, 18,43, 18,90, 19,36, 19,67, 20,14, 20,57, 21,47, 22,30, 22,64, 23,06, 23,80, 24,25, 24,55, 24,81, 25,10, 25,45, 25,54, 25,85, 26,35, 26,51, 26,83, 27,16, 27,57, 27,85, 28,08, et 28,47;
le Composé 1 ésylate de forme A est **caractérisé par** tous les pics suivants dans un diagramme de XRPD en échelle 2 thêta, sélectionnés parmi 3,97, 7,95, 9,89, 10,62, 10,92, 11,03, 11,34, 11,77, 12,03, 12,53, 12,71, 13,27, 14,00, 14,70, 15,08, 15,94, 16,17, 16,92, 17,01, 17,19, 17,35, 17,61, 18,15, 18,76, 19,03, 19,66, 19,89, 20,26, 20,65, 20,78, 21,44, 22,08, 23,14, 23,27, 23,99, 24,32, 25,43, 25,66, 25,85, 26,52, 26,87, 27,61, 27,96, 28,18, 28,40, et 28,91;
le Composé 1 HBr de forme B est **caractérisé par** tous les pics suivants dans un diagramme de XRPD en échelle 2 thêta, sélectionnés parmi 5,26, 8,29, 9,75, 9,91, 10,56, 10,72, 12,25, 12,50, 12,75, 13,31, 13,54, 14,11, 14,72, 14,89, 15,87, 16,29, 16,50, 17,36, 17,78, 18,09, 18,44, 18,75, 19,12, 19,24, 19,56, 19,92, 20,40, 20,55, 20,69, 21,18, 22,09, 22,64, 23,24, 24,56, 25,80, 26,52, 27,23, 27,43, 28,13, 28,46, 28,76, 29,08, 29,66, et 30,07;
le Composé 1 maléate de forme A est **caractérisé par** tous les pics suivants dans un diagramme de XRPD en échelle 2 thêta, sélectionnés parmi 7,58, 8,34, 10,92, 12,38, 12,87, 14,17, 15,13, 15,78, 16,43, 16,91, 17,51, 18,09, 18,71, 20,30, 21,60, 21,99, 22,30, 22,49, 23,32, 23,56, 23,92, 24,79, 26,04, 26,41, 26,92, 27,62, 28,18, 28,54, et 29,01;
le Composé 1 mésylate de forme A est **caractérisé par** tous les pics suivants dans un diagramme de XRPD en échelle 2 thêta, sélectionnés parmi 5,06, 6,65, 7,66, 8,35, 10,16, 10,72, 11,98, 12,62, 13,32, 14,41, 14,79, 15,42, 15,72, 15,89, 16,23, 16,77, 17,20, 17,59, 18,08, 18,40, 18,81, 19,33, 19,53, 19,71, 20,14, 20,38, 20,73, 21,05, 21,56, 21,83, 22,16, 23,14, 24,06, 24,54, 24,77, 25,75, 26,82, 27,71, 27,94, 28,71, 29,27, et 29,85;
le Composé 1 mésylate de forme B est **caractérisé par** tous les pics suivants dans un diagramme de XRPD en échelle 2 thêta, sélectionnés parmi 5,14, 6,62, 7,72, 7,89, 10,33, 10,73, 12,74, 13,31, 13,88, 14,23, 14,61, 15,21, 15,52, 15,87, 16,17, 17,18, 17,41, 17,88, 18,15, 18,46, 18,73, 19,13, 19,54, 20,15, 20,53, 20,75, 20,98, 21,27, 21,60, 22,03, 22,28, 23,07, 23,32, 24,16, 24,42, 24,87, 25,13, 25,64, 26,24, 26,95, 27,17, 27,49, 27,77, 28,42, et 29,54;
le Composé 1 mésylate de forme C est **caractérisé par** tous les pics suivants dans un diagramme de XRPD en échelle 2 thêta, sélectionnés parmi 7,55, 9,53, 10,25, 11,04, 11,49, 11,65, 12,24, 12,51, 13,88, 14,44, 15,36, 15,66, 16,29, 16,58, 17,16, 17,54, 19,04, 19,20, 19,79, 20,54, 21,12, 21,24, 21,62, 22,22, 23,15, 23,57, 23,99, 24,61, 24,77, 25,22, 25,61, 25,99, 26,60, 26,89, 27,29, 27,91, 29,03, et 29,29;
le Composé 1 oxalate de forme A est **caractérisé par** tous les pics suivants dans un diagramme de XRPD en échelle 2 thêta, sélectionnés parmi 4,90, 5,25, 6,20, 8,05, 9,87, 10,54, 12,29, 14,79, 15,35, 15,84, 16,49, 16,89, 17,55, 18,27, 18,67, 19,12, 19,40, 19,81, 20,26, 20,50, 21,06, 21,74, 22,17, 22,93, 23,26, 23,50, 23,81, 24,66, 25,06, 25,95, 26,90, 27,82, et 28,24;
le Composé 1 oxalate de forme B est **caractérisé par** tous les pics suivants dans un diagramme de XRPD en échelle 2 thêta, sélectionnés parmi 4,20, 7,59, 8,02, 8,42, 10,53, 12,36, 12,66, 13,09, 13,35, 13,88, 14,15, 14,46, 15,06, 15,23, 17,42, 17,75, 18,28, 19,07, 19,52, 20,33, 21,18, 21,43, 21,74, 22,08, 22,59, 23,53, 24,21, 24,52, 24,71, 25,24, 25,85, 26,24, 26,55, 26,90, 27,43, 28,56, 29,30, et 29,51;
le Composé 1 oxalate de forme C est **caractérisé par** tous les pics suivants dans un diagramme de XRPD en échelle 2 thêta, sélectionnés parmi 4,13, 7,70, 10,29, 10,59, 13,09, 13,70, 14,13, 15,33, 15,87, 16,57, 17,19, 17,50, 17,83, 18,15, 18,61, 19,24, 19,42, 20,11, 20,34, 20,64, 21,07, 21,47, 21,59, 21,91, 22,56, 22,92, 23,37, 23,87, 24,35, 25,35, 25,91, 26,47, 26,68, 27,74, 28,47, et 29,14;
le Composé 1 sulfate de forme A est **caractérisé par** tous les pics suivants dans un diagramme de XRPD en échelle 2 thêta, sélectionnés parmi 4,74, 5,85, 9,47, 10,04, 11,74, 11,95, 13,77, 14,99, 15,42, 15,71, 16,48, 17,36, 17,66, 17,92, 18,59, 18,95, 19,36, 19,74, 20,22, 20,77, 21,38, 21,86, 22,74, 23,30, 23,70, 24,33, 25,07, 25,32, 25,67, 26,23, et 27,00;
le Composé 1 sulfate de forme B est **caractérisé par** tous les pics suivants dans un diagramme de XRPD en échelle 2 thêta, sélectionnés parmi 4,27, 8,57, 9,32, 10,95, 11,29, 12,07, 12,87, 13,25, 13,82, 14,13, 14,67, 16,94, 17,21, 17,64, 19,99, 20,26, 21,03, 21,44, 22,00, 22,71, 23,85, 25,20, et 26,02;
le Composé 1 sulfate de forme C est **caractérisé par** tous les pics suivants dans un diagramme de XRPD en échelle 2 thêta, sélectionnés parmi 3,19, 5,35, 9,04, 9,62, 10,73, 11,31, 11,60, 12,27, 12,67, 13,76, 14,31, 14,65, 15,21, 15,63, 16,11, 16,33, 16,63, 17,11, 18,32, 18,75, 18,99, 19,36, 19,47, 20,16, 20,55, 21,01, 21,52, 21,62, 21,89, 22,19, 22,53, 23,19, 23,79, 24,23, 24,81, 25,07, 25,41, 26,06, 26,73, 27,58, 28,16, 28,63, 28,90, 29,22, 29,47, et 29,73;
le Composé 1 hémitartrate de forme B est **caractérisé par** tous les pics suivants dans un diagramme de XRPD en échelle 2 thêta, sélectionnés parmi 5,17, 5,66, 7,22, 9,27, 10,10, 10,40, 11,37, 11,55, 12,77, 13,94, 14,51, 15,60, 16,06, 16,48, 17,09, 17,69, 17,90, 18,27, 18,94, 19,15, 19,59, 19,81, 20,06, 20,32, 21,23, 21,83, 22,34, 22,84, 23,53, 24,26, 24,70, 25,26, 25,80, 26,78, 27,29, 27,90, 29,04, et 29,45;
le Composé 1 tosylate de forme A est **caractérisé par** tous les pics suivants dans un diagramme de XRPD en échelle 2 thêta, sélectionnés parmi 5,81, 7,05, 8,52, 9,74, 10,40, 10,99, 11,26, 11,66, 12,58, 12,81, 14,28, 14,62, 15,30, 15,80, 16,03, 16,34, 17,12, 17,55, 18,03, 18,94, 19,26, 19,67, 20,31, 20,65, 21,02, 21,43, 22,11, 22,44, 22,66, 22,94, 23,07, 23,54, 23,79, 24,55, 24,88, 25,42, 25,83, 26,10, 26,53, 27,70, 28,11, 28,62, 29,18, 29,40, 29,66, et 29,83; et
le Composé 1 tosylate de forme B est **caractérisé par** tous les pics suivants dans un diagramme de XRPD en échelle 2 thêta, sélectionnés parmi 4,61, 8,69, 9,25, 10,68, 12,44, 12,69, 13,76, 14,42, 14,81, 15,47, 16,08, 16,57, 17,45, 17,76, 18,44, 18,83, 19,01, 19,35, 20,42, 20,84, 21,15, 21,69, 22,09, 22,86, 23,12, 24,08, 24,53, 24,86, 25,22, 25,91, 26,33, 26,82, 27,63, 28,61, 29,11, et 29,77.

5. Forme de sel cristallin selon l'une quelconque des revendications 1 à 4, dans laquelle:
le Composé 1 bésylate de forme A est **caractérisé par** un premier endotherme à une température d'environ 144 °C et un deuxième endotherme à une température d'environ 170 °C dans un thermogramme de DSC;
le Composé 1 camsylate de forme A est **caractérisé par** un premier endotherme à une température d'environ 110 °C et un deuxième endotherme à une température d'environ 174 °C dans un thermogramme de DSC;
le Composé 1 citrate de forme B est **caractérisé par** un endotherme commençant à une température d'environ 175 °C dans un thermogramme de DSC;
le Composé 1 ésylate de forme A est **caractérisé par** un premier endotherme à une température d'environ 69 °C, un deuxième endotherme à une température d'environ 156 °C, et un troisième endotherme à une température d'environ 190 °C, dans un thermogramme de DSC;
le Composé 1 maléate de forme A est **caractérisé par** un endotherme à une température d'environ 117 °C dans un thermogramme de DSC;
le Composé 1 mésylate de forme C est **caractérisé par** un premier endotherme présentant un pic à une température d'environ 69 °C, le premier endotherme ayant une limite inférieure à une température d'environ 36 °C et une limite supérieure à une température d'environ 96 °C, et un deuxième endotherme présentant un pic à une température d'environ 208 °C, le deuxième endotherme ayant une limite inférieure à une température d'environ 169 °C et une limite supérieure à une température d'environ 219 °C;
le Composé 1 hémitartrate de forme B est **caractérisé par** un premier endotherme à une température d'environ 111 °C et un deuxième endotherme à une température d'environ 148 °C dans un thermogramme de DSC;
le Composé 1 tosylate de forme A est **caractérisé par** un endotherme commençant à une température d'environ 214 °C dans un thermogramme de DSC; et
le Composé 1 tosylate de forme B est **caractérisé par** un endotherme prononcé commençant à une température d'environ 183 °C dans un thermogramme de DSC.

6. Forme de sel cristallin selon l'une quelconque des revendications 1 à 5, dans laquelle:
le Composé 1 bésylate de forme A se **caractérise par** une perte de poids d'environ 7,9% dans une plage de températures de 39 °C à 177 °C dans un thermogramme de TGA;
le Composé 1 camsylate de forme A se **caractérise par** une perte de poids d'environ 5,9% dans une plage de températures de 38 °C à 170 °C dans un thermogramme de TGA;
le Composé 1 citrate de forme B se **caractérise par** une perte de poids commençant à une température d'environ 159 °C et se terminant à environ 220 °C dans un thermogramme de TGA;
le Composé 1 ésylate de forme A se **caractérise par** une perte de poids d'environ 8,5% en poids dans une plage de températures de 38 °C à 188 °C dans un thermogramme de TGA;
le Composé 1 maléate de forme A se **caractérise par** une perte de poids d'environ 3,1% en poids dans la plage de températures de 42 °C à 146 °C dans un thermogramme de TGA;
le Composé 1 mésylate de forme C se **caractérise par** une perte de poids de 0,5% à 3,0% en poids dans la plage de températures de 36 °C à 130 °C dans un thermogramme de TGA;
le Composé 1 hémitartrate de forme B se **caractérise par** une perte de poids d'environ 7,4% en poids dans la plage de températures de 39 °C à 157 °C dans un thermogramme de TGA;
le Composé 1 tosylate de forme A se **caractérise par** une perte de poids d'environ 0,9% en poids dans la plage de températures de 72 °C à 231 °C dans un thermogramme de TGA; et
le Composé 1 tosylate de forme B se **caractérise par** une perte de poids d'environ 0,7% en poids dans la plage de températures de 39 °C à 214 °C dans un thermogramme de TGA.

7. Forme de sel cristallin selon l'une quelconque des revendications 1 à 6, dans laquelle:
le Composé 1 citrate de forme B se **caractérise par** une augmentation d'environ 4,1% en poids, mesurée par DVS, lorsqu'il est transféré d'un environnement à 5% d'humidité relative à un environnement à 95% d'humidité relative;
le Composé 1 maléate de forme A se **caractérise par** une augmentation d'environ 2,5% en poids, mesurée par DVS, lorsqu'il est transféré d'un environnement à 5% d'humidité relative à un environnement à 95% d'humidité relative;
le Composé 1 mésylate de forme C se **caractérise par** une augmentation d'environ 5,1% en poids, mesurée par DVS, lorsqu'il est transféré d'un environnement à 5% d'humidité relative à un environnement à 95% d'humidité relative;
le Composé 1 tosylate de forme A se **caractérise par** une augmentation d'environ 0,9% en poids, mesurée par DVS, lorsqu'il est transféré d'un environnement à 5% d'humidité relative à un environnement à 95% d'humidité relative; et
le Composé 1 tosylate de forme B se **caractérise par** une augmentation d'environ 0,9% en poids, mesurée par DVS, lorsqu'il est transféré d'un environnement à 5% d'humidité relative à un environnement à 95% d'humidité relative.

8. Composition pharmaceutique comprenant une forme de sel cristallin selon l'une quelconque des revendications 1 à 7 et un excipient pharmaceutiquement acceptable.

9. Forme de sel cristallin selon l'une quelconque des revendications 1 à 7, ou composition pharmaceutique selon la revendication 8, destinée à une utilisation dans une méthode de traitement d'une maladie, d'une affection ou d'un syndrome médié(e) au moins en partie par la modulation de l'activité *in vivo* d'une protéine kinase, la maladie, l'affection ou le syndrome étant un cancer.
